# EUROPEAN PATENT APPLICATION

(11) **EP 3 884 940 A1**
(43) Date of publication of application: **29.09.2021**
(21) Application number: 19888010.6
(22) Date of filing: 20.11.2019
(51) Int. Cl.: A61K 31/422, A61K 31/454, A61P 31/04, C07D 413/06, C07D 413/14

(54) **NOVEL IMIDAZOLE DERIVATIVE**

(30) Priority: 21.11.2018 JP 2018218448
(71) Applicant: Taisho Pharmaceutical Co., Ltd., Tokyo 170-8633 (JP)
(72) Inventor: TAKASHIMA, Hajime, Tokyo 170-8633 (JP); MATSUDA, Yohei, Tokyo 170-8633 (JP); OGATA, Yuya, Tokyo 170-8633 (JP); SASAMOTO, Naoki, Tokyo 170-8633 (JP); TSURUTA, Risa, Tokyo 170-8633 (JP); USHIYAMA, Fumihito, Tokyo 170-8633 (JP); UEKI, Kaori, Tokyo 170-8633 (JP); TANAKA, Nozomi, Tokyo 170-8633 (JP)
(74) Representative: Hoffmann Eitle
(86) International application number: PCT/JP2019/045362
(87) International publication number: WO 2020/105660

(57) **Abstract**

The present invention provides a new imidazole derivative represented by the following formula or a pharmaceutically acceptable salt thereof which exhibits potent antimicrobial activity based on a LpxC-inhibiting action against gram-negative bacteria such as *Pseudomonas aeruginosa, Escherichia coli,* and *Klebsiella pneumoniae,* and their drug-resistant strains.

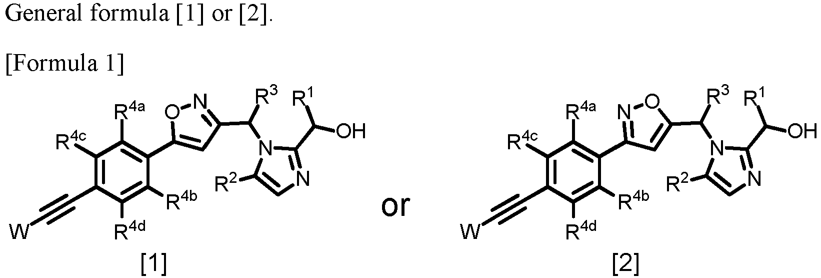

## Description

### TECHNICAL FIELD

The present invention relates to novel imidazole derivatives or salts thereof, which exhibit an activity for inhibiting uridyldiphospho(UDP)-3-O-acyl-N-acetylglucosamine deacetylase (LpxC) and antimicrobial pharmaceuticals comprising the same as active ingredients.

### BACKGROUND ART

Gram-negative bacteria have an outer membrane composed of a lipid bilayer inexistent in gram-positive bacteria, and thus tend to be more resistant to drugs, as compared with gram-positive bacteria, due to the problem of drug permeability. Gram-negative bacteria are also known to have a plurality of drug efflux proteins, which are known to be involved in drug resistance (Non Patent Literature 1). Furthermore, lipopolysaccharide (LPS), one of the main constituents of the outer membrane, greatly takes part in toxicity as an endotoxin.

As effective drugs for gram-negative bacterial infectious diseases, carbapenem drugs, quinolone drugs, aminoglycoside drugs, and the like are known, but in recent years, a large number of *Pseudomonas aeruginosa* and Enterobacteriaceae (such as *Klebsiella pneumoniae* and *Escherichia coli*) which exhibit resistance to these drugs are reported in medical settings (Non Patent Literatures 2 and 3). Carbapenem-resistant enterobacteriaceae (CRE) is one of them and exhibits a high degree of resistance to a lot of drugs including carbapenem drugs. CRE has been rapidly spreading in the world, but the drug effective against CRE infectious disease is rare and the treatment thereof is difficult. Also, drug resistance of *Pseudomonas aeruginosa* has become a major problem. In particular, multidrug resistant *Pseudomonas aeruginosa* for which none of the carbapenem drugs, quinolone drugs, and aminoglycoside drugs are effective has often isolated in a clinical setting (Non Patent Literature 4) and the drug effective against that is rare, which makes the treatment also difficult. Thus, infectious diseases caused by resistance gram-negative bacteria have become major problems as intractable infectious diseases spreading in the world and there is a keen demand for the development of novel infectious disease therapeutic drugs having a mechanism of action.

UDP-3-O-acyl-N-acetylglucosamine deacetylase (LpxC) is an enzyme in charge of the synthesis of lipid A (hydrophobic anchor of LPS which is the constituent of the outer membrane). Lipid A biosynthesis consists of reactions in 10 steps, and LpxC catalyzes the second step of the biosynthesis reactions to remove the acetyl group of UDP-3-O-acyl-N-acetylglucosamine (Non Patent Literature 5). Lipid A is a component essential for the formation of the outer membrane, and is consequently indispensable for the survival of gram-negative bacteria (Non Patent Literature 6). LpxC is one of the rate-determining important enzymes during the process of lipid A biosynthesis, and is an indispensable enzyme for lipid A biosynthesis. Thus, a drug inhibiting the activity of LpxC is highly expected to be capable of becoming an antimicrobial agent effective against gram-negative bacteria including *Pseudomonas aeruginosa* and *Klebsiella pneumoniae*, particularly against gram-negative bacterial infectious disease exhibiting drug resistance, because such a drug has a mechanism of action different from those of conventional drugs.

Although there is a number of reports on LpxC inhibitors (Non Patent Literatures 7 to 10), but almost all of them are inhibitors having a hydroxamic acid structure. This is because LpxC is an enzyme having a zinc atom in the active center and the above inhibitor strongly bonds thereto. However, toxicological problems are concerned because hydroxamic acid is a non-selective metal chelator.

On the other hand, reports on the LpxC inhibitor having no hydroxamic acid are very few. In recent years, non-hydroxamic acid LpxC inhibitors are reported from the group of Cohen et al. and Forge (Patent Literatures 1 to 3). They have also reported non-hydroxamic acid LpxC inhibitors in a meeting (Non Patent Literature 11), but the structure thereof is not disclosed.

Thus, regardless of the high need for new infectious disease therapeutic drugs based on LpxC inhibition which exhibits potent antimicrobial activity against gram-negative bacteria such as *Pseudomonas aeruginosa* and *Klebsiella pneumoniae* and their drug-resistant strains, there are only a few reports on the LpxC inhibitor having a non-hydroxamic acid structure, and thus, an LpxC inhibitor having a non-hydroxamic acid structure containing a new unconventional skeleton is needed.

### CITATION LIST

### NON PATENT LITERATURE

NPL 1: Antimicrobial Resistance (2002) Mar 1, 34, p. 634-640.
NPL 2: J. Antimicrob. Chemother. (2003) Jan 14, 51, p.347-352.
NPL 3: Virulence. (2017), 8(4), p. 460-469.
NPL 4: Jpn. J. Antibiotics (2006), 59(5), p.355-363.
NPL 5: J.Biol.Chem. (1995) Dec 22, 270, p.30384-30391.
NPL 6: J.Bacteriol. (1987), 169, p.5408-5415
NPL 7: Curr. Top. Med. Chem. (2016), 6(7), p.2379-2430.
NPL 8: Cold Spring Harb Perspect Med. (2016), 6(7), a025304.
NPL 9: Expert. Opin. Ther. Pat. (2017), 27(11), p.1227-1250.
NPL 10: Mini Rev. Med. Chem. (2018), 18(4), p.310-323.
NPL 11: ASM Microbe 2018 (June 7-11), poster #643.

### PATENT LITERATURE

PTL 1: International Publication No. WO 2015/085238
PTL 2: International Publication No. WO 2017/083431
PTL 3: International Publication No. WO 2017/083434

### SUMMARY OF INVENTION

### TECHNICAL PROBLEM

An object of the present invention is to find out an LpxC inhibitor having a non-hydroxamic acid structure and to provide a new compound which exhibits potent antimicrobial activity against gram-negative bacteria such as *Pseudomonas aeruginosa* and *Klebsiella pneumoniae*, and their drug-resistant strains, and which is useful as a pharmaceutical product.

### SOLUTION TO PROBLEM

The present inventors have conducted intensive studies in an attempt to find out a new non-hydroxamic acid compound having LpxC-inhibiting action, and as a result, found that a compound represented by the following general formula [1] or a pharmaceutically acceptable salt thereof attains the above object, thereby completed the present invention.

The present invention will be described below.

The present invention is:
(1) A compound represented by general formula [1] or [2] or a pharmaceutically acceptable salt thereof: 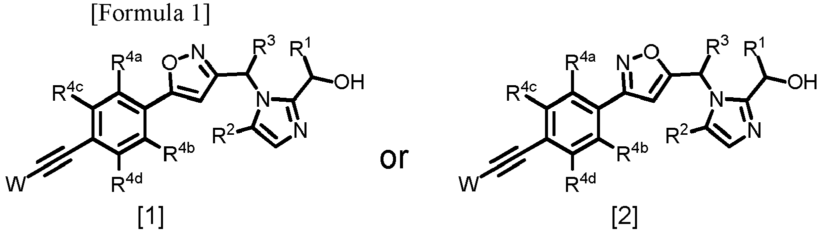 wherein
   R¹ represents a C₁₋₄ alkyl group,
   R² represents a hydrogen atom or a C₁₋₄ alkyl group,
   R³ represents a hydrogen atom, an amino C₁₋₄ alkyl group (the amino group in the amino C₁₋₄ alkyl group may be substituted with 1 to 2 C₁₋₄ alkyl groups which are the same or different), or a hydroxy C₁₋₄ alkyl group,
   R^{4a}, R^{4b}, R^{4c}, and R^{4d} are the same or different and each represent a hydrogen atom, a halogen atom, or a C₁₋₄ alkyl group,
   W represents any one of the structures in the following formula group [3], and is any one of groups represented by 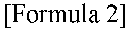 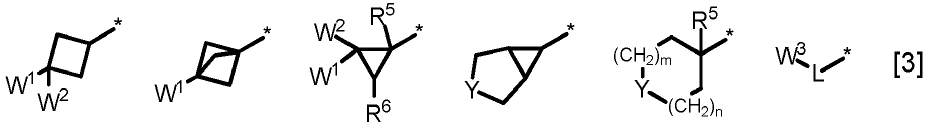 wherein
      W¹ represents A^{1a}- or A^{1b}-B¹-,
      A^{1a} represents a hydrogen atom, a hydroxy group, an amino group, a carboxy group, a hydroxy C₁₋₄ alkyl group, an amino C₁₋₄ alkyl group, or a C₂₋₆ alkoxyalkyl group (the hydroxy C₁₋₄ alkyl group, the amino C₁₋₄ alkyl group, and the C₂₋₆ alkoxyalkyl group may be substituted with 1 to 3 hydroxy groups),
      A^{1b} represents a hydrogen atom, a C₁₋₄ alkyl group, a C₁₋₄ hydroxyalkyl group, a C₃₋₆ cycloalkyl group, or a saturated heterocyclyl group containing an oxygen atom in the ring,
      B¹ represents -NR¹¹- or -CONR¹¹-,
      W² represents a hydrogen atom, a hydroxy group, a cyano group, a carbamoyl group, or a hydroxy C₁₋₄ alkyl group,
      W³ represents a hydrogen atom, a hydroxy group, -NR¹¹R¹³, or a C₁₋₄ alkoxy group,
      L represents a C₁₋₆ alkylene group,
      Y represents W⁴-N, an oxygen atom, or SO₂,
      W⁴ represents A^{2a}-, A^{2b}-X¹-, A^{2a}-B²-, A^{2b}-X²-B²-, or Q-X²-,
      A^{2a} represents a hydrogen atom or a C₁₋₄ alkyl group,
      A^{2b} represents a hydrogen atom, a hydroxy group, an amino group, a carboxy group, a C₁₋₄ alkylamino group, or a C₁₋₄ alkoxy group,
      B² represents -CO-, -OCO-, -NR¹¹CO-, -NR¹¹COCO-, -SO₂-, or -NR¹¹SO₂-,
      X¹ represents a C₁₋₆ alkylene group (the C₁₋₆ alkylene group may be substituted with 1 to 3 hydroxy groups),
      X² represents a C₁₋₆ alkylene group (the C₁₋₆ alkylene group may be substituted with 1 to 3 hydroxy groups) or a bond,
      Q represents a saturated heterocyclyl group containing an oxygen atom in the ring,
      R⁵ represents a hydrogen atom, a hydroxy group, a C₁₋₄ alkyl group, -NR¹¹COR¹², or -NR¹¹R¹²,
      R⁶ represents a hydrogen atom or a hydroxy C₁₋₄ alkyl group,
      R¹¹ and R¹² are the same or different and each represent a hydrogen atom or a C₁₋₄ alkyl group,
      R¹³ represents a hydrogen atom or a C₃₋₆ cycloalkyl group, and
      m and n are the same or different and each represent 0 or 1;
(2) A compound represented by general formula [1] or [2] or a pharmaceutically acceptable salt thereof: 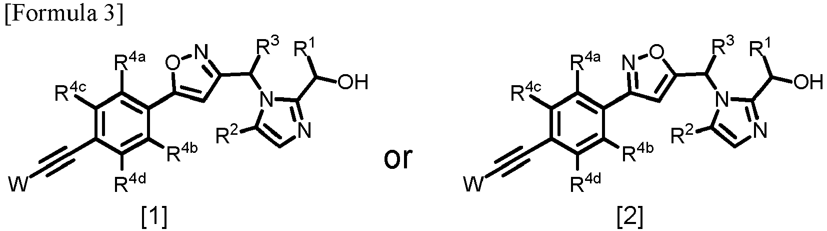 wherein
   R¹ represents a C₁₋₄ alkyl group,
   R² represents a hydrogen atom or a C₁₋₄ alkyl group,
   R³ represents a hydrogen atom, an amino C₁₋₄ alkyl group (the amino group in the amino C₁₋₄ alkyl group may be substituted with 1 to 2 C₁₋₄ alkyl groups which are the same or different), or a hydroxy C₁₋₄ alkyl group,
   R^{4a}, R^{4b}, R^{4c}, and R^{4d} are the same or different and each represent a hydrogen atom, a halogen atom, or a C₁₋₄ alkyl group,
   W represents any one of the structures in the following formula group [3], and is any one of groups represented by 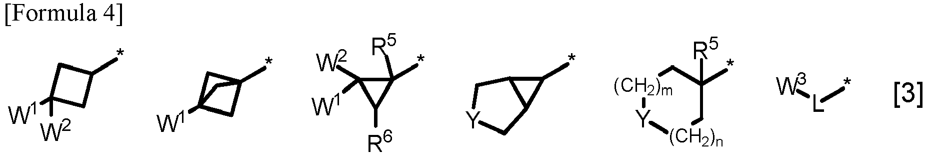 wherein
      W¹ represents A^{1a}- or A^{1b}-B¹-,
      A^{1a} represents a hydrogen atom, a hydroxy group, an amino group, a carboxy group, a hydroxy C₁₋₄ alkyl group, an amino C₁₋₄ alkyl group, or a C₂₋₆ alkoxyalkyl group (the hydroxy C₁₋₄ alkyl group, the amino C₁₋₄ alkyl group, and the C₂₋₆ alkoxyalkyl group may be substituted with 1 to 3 hydroxy groups),
      A^{1b} represents a hydrogen atom, a C₁₋₄ alkyl group, a C₁₋₄ hydroxyalkyl group, a C₃₋₆ cycloalkyl group, or a saturated heterocyclyl group containing an oxygen atom in the ring,
      B¹ represents -NR¹¹- or -CONR¹¹-,
      W² represents a hydrogen atom, a hydroxy group, a cyano group, a carbamoyl group, or a hydroxy C₁₋₄ alkyl group,
      W³ represents a hydrogen atom, a hydroxy group, -NR¹¹R¹³, or a C₁₋₄ alkoxy group,
      L represents a C₁₋₆ alkylene group,
      Y represents W⁴-N, an oxygen atom, or SO₂,
      W⁴ represents A^{2a}-, A^{2b}-X¹-, A^{2a}-B²-, A^{2b}-X²-B²-, or Q-X²-,
      A^{2a} represents a hydrogen atom or a C₁₋₄ alkyl group,
      A^{2b} represents a hydrogen atom, a hydroxy group, an amino group, a carboxy group, a C₁₋₄ alkylamino group, or a C₁₋₄ alkoxy group,
      B² represents -CO-, -OCO-, -NR¹¹CO- , -NR¹¹COCO-, -SO₂-, or -NR¹¹SO₂-,
      X¹ represents a C₁₋₆ alkylene group (the C₁₋₆ alkylene group may be substituted with 1 to 3 hydroxy groups),
      X² represents a C₁₋₆ alkylene group (the C₁₋₆ alkylene group may be substituted with 1 to 3 hydroxy groups) or a bond,
      Q represents a saturated heterocyclyl group containing an oxygen atom in the ring,
      R⁵ represents a hydrogen atom, a hydroxy group, a C₁₋₄ alkyl group, or -NR¹¹R¹²,
      R⁶ represents a hydrogen atom or a hydroxy C₁₋₄ alkyl group,
      R¹¹ and R¹² are the same or different and each represent a hydrogen atom or a C₁₋₄ alkyl group,
      R¹³ represents a hydrogen atom or a C₃₋₆ cycloalkyl group, and m and n are the same or different and each represent 0 or 1;
(3) The compound or the pharmaceutically acceptable salt thereof according to (1) or (2), wherein R¹ is a methyl group;
(4) The compound or the pharmaceutically acceptable salt thereof according to any one of (1) to (3), wherein R² is a hydrogen atom;
(5) The compound or the pharmaceutically acceptable salt thereof according to any one of (1) to (4), wherein all of R^{4a}, R^{4b}, R^{4c}, and R^{4d} are hydrogen atoms;
(6) The compound or the pharmaceutically acceptable salt thereof according to any one of (1) to (5), wherein R³ is a hydrogen atom, an aminomethyl group, or a hydroxymethyl group;
(7) The compound or the pharmaceutically acceptable salt thereof according to any one of (1) to (6), wherein W is formula [4] 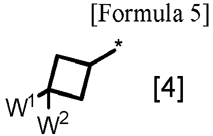 wherein
   W¹ represents a hydroxy group or A^{1b}-B¹-,
   A^{1b} represents a hydrogen atom or a C₁₋₄ alkyl group,
   B¹ represents -CONR¹¹-,
   R¹¹ represents a hydrogen atom or a C₁₋₄ alkyl group, and
   W² represents a hydrogen atom;
(8) The compound or the pharmaceutically acceptable salt thereof according to any one of (1) to (6), wherein W is formula [5] 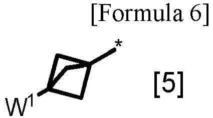 wherein
   W¹ represents an amino group;
(9) The compound or the pharmaceutically acceptable salt thereof according to any one of (1) to (6), wherein W is formula [6] 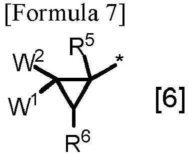 wherein
   W¹ represents a hydrogen atom, a hydroxy C₁₋₄ alkyl group, or a C₂₋₆ alkoxyalkyl group,
   W² represents a hydrogen atom,
   R⁵ represents a hydrogen atom or an amino group, and
   R⁶ represents a hydrogen atom;
(10) The compound or the pharmaceutically acceptable salt thereof according to any one of (1) to (6), wherein W is formula [7] 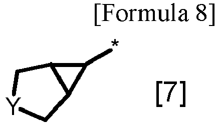 wherein
   Y represents W⁴-N,
   W⁴ represents A^{2b}-X¹- or A^{2a}-B²-,
   A^{2a} represents a hydrogen atom or a C₁₋₄ alkyl group,
   A^{2b} represents a hydrogen atom, a hydroxy group, or a carboxy group,
   B² represents -CO-, and
   X¹ represents a C₁₋₆ alkylene group (the C₁₋₆ alkylene group may be substituted with 1 to 3 hydroxy groups);
(11) The compound or the pharmaceutically acceptable salt thereof according to any one of (1) to (6), wherein W is formula [8] 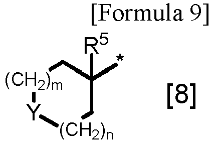 wherein
   Y represents an oxygen atom,
   m represents 0,
   n represents 0 or 1, and
   R⁵ represents a hydrogen atom or a C₁₋₄ alkyl group;
(12) A pharmaceutical composition comprising the compound or the pharmaceutically acceptable salt thereof according to any one of (1) to (11) as an active ingredient;
(13) An LpxC inhibitor comprising the compound or the pharmaceutically acceptable salt thereof according to any one of the items (1) to (11) as an active ingredient; and
(14) An antimicrobial agent comprising the compound or the pharmaceutically acceptable salt thereof according to any one of the items (1) to (11) as an active ingredient.

### ADVANTAGEOUS EFFECTS OF INVENTION

The compound or the pharmaceutically acceptable salt thereof according to the present invention has a strong LpxC-inhibiting action and exhibits potent antimicrobial activity against gram-negative bacteria. Thus, the compound or the pharmaceutically acceptable salt thereof is useful as a pharmaceutical composition and as an antimicrobial agent against these bacteria as causative bacteria.

### DESCRIPTION OF EMBODIMENTS

The present invention will be described in further detail below.

The terms and phrases used herein will be explained first.

In the present invention, "n-" means normal, "i-" iso, "s-" secondary, "tert-" or "t-" tertiary, "o-" ortho, "m-" meta, and "p-" para.

The "halogen atom" means a fluorine atom, a chlorine atom, a bromine atom, and an iodine atom.

The "C₁₋₄ alkyl group" refers to a straight-chain or branched-chain alkyl group having 1 to 4 carbon atoms, and examples thereof include a methyl group, an ethyl group, an n-propyl group, an n-butyl group, an isopropyl group, an isobutyl group, a t-butyl group, and a s-butyl group.

The "C₁₋₆ alkyl group" refers to a straight-chain or branched-chain alkyl group having 1 to 6 carbon atoms, and examples thereof include, in addition to the specific examples of the "C₁₋₄ alkyl group", an n-pentyl group, an n-hexyl group, an isopentyl group, a neopentyl group, a t-pentyl group, and a 1,2-dimethylpropyl group.

The "C₃₋₆ cycloalkyl group" refers to a cycloalkyl group having 3 to 6 carbon atoms, and examples thereof include a cyclopropyl group, a cyclobutyl group, a cyclopentyl group, and a cyclohexyl group.

The "hydroxy C₁₋₄ alkyl group" refers to an alkyl group in which 1 to 3 hydrogen atoms of the above-mentioned "C₁₋₄ alkyl group" are substituted with a hydroxy group or hydroxy groups, and examples thereof include a hydroxymethyl group, a 1-hydroxyethyl group, a 2-hydroxyethyl group, a 1-hydroxypropyl group, a 2-hydroxypropyl group, a 3-hydroxypropyl group, a 1-hydroxybutyl group, a 2-hydroxybutyl group, a 3-hydroxybutyl group, a 4-hydroxybutyl group, a 1,2-dihydroxyethyl group, a 1,2-dihydroxypropyl group, a 2,3-dihydroxypropyl group, a 1,3-dihydroxyisopropyl group, and a 1,2,3-trihydroxypropyl group.

The "amino C₁₋₄ alkyl group" refers to an alkyl group in which 1 to 3 hydrogen atoms in the above-mentioned "C₁₋₄ alkyl group" are substituted with an amino group or amino groups, and examples thereof include an aminomethyl group, a 1-aminoethyl group, a 2-aminoethyl group, a 1-aminopropyl group, a 2-aminopropyl group, a 3-aminopropyl group, a 1-aminobutyl group, a 2-aminobutyl group, a 3-aminobutyl group, a 4-aminobutyl group, a 1,2-diaminoethyl group, a 1,2-diaminopropyl group, a 2,3-diaminopropyl group, a 1,3-diaminoisopropyl group, and a 1,2,3-triaminopropyl group.

The "C₁₋₄ alkoxy group" refers to a straight-chain or branched-chain alkoxy group having 1 to 4 carbon atoms, and examples thereof include a methoxy group, an ethoxy group, a 1-propoxy group, an isopropoxy group, a 1-butoxy group, a 1-methyl-1-propoxy group, and a t-butoxy group.

The "C₂₋₆ alkoxyalkyl group" refers to an alkoxyalkyl group having a total of 2 to 6 carbon atoms, and examples thereof include a methoxymethyl group, a methoxyethyl group, an ethoxyethyl group, a tert-butoxyethyl group, a methoxypropyl group, an ethoxypropyl group, and a methoxybutyl group.

The "C₁₋₄ alkylamino group" refers to a straight-chain or branched-chain alkylamino group having 1 to 4 carbon atoms, and examples thereof include a methylamino group, an ethylamino group, an n-propylamino group, an isopropylamino group, an n-butylamino group, a s-butylamino group, and a t-butylamino group.

The "C₂₋₆ alkanoyl group" refers to a straight-chain or branched-chain alkanoyl group having 2 to 6 carbon atoms, and examples thereof include an acetyl group, a propionyl group, a butyryl group, and a pivaloyl group.

The "C₁₋₆ alkoxycarbonyl group" refers to a carbonyl group having a straight-chain or branched-chain alkoxy group having 1 to 6 carbon atoms, and examples thereof include a methoxycarbonyl group, an ethoxycarbonyl group, an isopropoxycarbonyl group, and a tert-butoxycarbonyl group.

The "aryl group" refers to a monocyclic to tetracyclic aromatic carbocyclic group composed of 6 to 18 carbon atoms, and examples thereof include a phenyl group, a naphthyl group, an anthryl group, a phenanthrenyl group, a tetracenyl group, and a pyrenyl group.

The "C₁₋₄ alkylene group" refers to a straight-chain or branched-chain alkylene group having 1 to 4 carbon atoms, and examples thereof include -CH₂-, -(CH₂)₂-, -(CH₂)₃-, - CH₂-CH(CH₃)-, -C(CH₃)₂-, -(CH₂)₄-, -(CH₂)₂-CH(CH₃)-, -CH₂-C(CH₃)₂-, -CH₂-CH(CH₃)-CH₂-, and -CH(CH₃)-(CH₂)₂-.

The "C₁₋₆ alkylene group" refers to a straight-chain or branched-chain alkylene group having 1 to 6 carbon atoms, and examples thereof include, in addition to specific examples of the "C₁₋₄ alkylene group", -(CH₂)₅-, -(CH₂)₃-CH(CH₃)-, -(CH₂)₂-CH(C₂H₅)-,-(CH₂)₆-, -(CH₂)₃-CH(CH₃)-CH₂-, and -CH₂-CH(CH₃)-(CH₂)₃-.

The "saturated heterocyclyl group containing an oxygen atom in the ring" refers to a monocyclic group which has a ring constituted by 4 to 7 atoms, contains 1 or 2 oxygen atoms in the ring, and is constituted only by saturated bonds. Examples thereof include an oxetan-2-yl group, an oxetan-3-yl group, a tetrahydrofuran-2-yl group, a tetrahydrofuran-3-yl group, a tetrahydropyran-2-yl group, a tetrahydropyran-3-yl group, a tetrahydropyran-4-yl group, and a 1,4-dioxane-2-yl group.

The "protective group for a hydroxy group" includes all groups usable usually as protective groups for a hydroxy group, and includes, for example, the groups described in P.G.M. Wuts et al., Protective Groups in Organic Synthesis 4th Ed., 2006, John Wiley & Sons, INC. Examples thereof include a C₁₋₆ alkyl group optionally substituted with a C₁₋₄ alkoxy group (a methyl group, a methoxymethyl group, a tert-butoxymethyl group, etc.), a benzyloxymethyl group, a tetrahydropyranyl group, a tetrahydrofuranyl group, a benzyl group optionally substituted with a substituent selected from "a halogen atom, a C₁₋₄ alkoxy group, and a nitro group" (a benzyl group, a p-methoxybenzyl group, a p-nitrobenzyl group, a p-chlorobenzyl group, etc.), a C₁₋₄ alkoxycarbonyl group optionally substituted with 1 to 3 substituents selected from "a halogen atom and an aryl group" (a methoxycarbonyl group, a tert-butoxycarbonyl group, a 2,2,2-trichloroethoxycarbonyl group, a benzyloxycarbonyl group, a 9-fluorenylmethoxycarbonyl group, etc.), a benzoyl group, a C₂₋₆ alkanoyl group optionally substituted with 1 to 3 halogen atoms (an acetyl group, a chloroacetyl group, a trichloroacetyl group, a trifluoroacetyl group, a pivaloyl group, etc.), and a silyl group having 3 substituents which are the same or different and are selected from "a C₁₋₆ alkyl group and an aryl group" (a trimethylsilyl group, a triethylsilyl group, a tert-butyldimethylsilyl group, a tert-butyldiphenylsilyl group, etc.).

The "optionally protected hydroxy group" means an unprotected or protected hydroxy group.

The "protected hydroxy group" means a hydroxy group protected with a "protective group for a hydroxy group".

The "protected hydroxy C₁₋₄ alkyl group" means a protected hydroxy C₁₋₄ alkyl group in which a hydroxy group is protected with a "protective group for a hydroxy group".

The "optionally protected hydroxy C₁₋₄ alkyl group" means a hydroxy C₁₋₄ alkyl group or a protected hydroxy C₁₋₄ alkyl group.

The "protective group for an amino group" includes all groups usable as usually amino protective groups, and includes, for example, the groups described in P.G.M. Wuts et al., Protective Groups in Organic Synthesis 4th Ed., 2006, John Wiley & Sons, INC. Examples thereof include a benzyl group optionally substituted with a substituent selected from "a halogen atom, a C₁₋₄ alkoxy group, and a nitro group" (a benzyl group, a p-methoxybenzyl group, a p-nitrobenzyl group, a p-chlorobenzyl group, etc.), a C₁₋₆ alkoxycarbonyl group optionally substituted with 1 to 3 substituents selected from "a halogen atom and an aryl group" (a methoxycarbonyl group, a tert-butoxycarbonyl group, a 2,2,2-trichloroethoxycarbonyl group, a benzyloxycarbonyl group, a 9-fluorenylmethoxycarbonyl group, etc.), an allyl group, a dialkylaminoalkylidene group (an N,N-dimethylaminomethylene group, an N,N-diethylaminomethylene group, etc.), a formyl group, a C₂₋₆ alkanoyl group optionally substituted with 1 to 3 halogen atoms (an acetyl group, a chloroacetyl group, a trichloroacetyl group, a trifluoroacetyl group, a pivaloyl group, etc.) and a benzoyl group.

The "optionally protected amino group" means an unprotected or protected amino group.

The "protected amino group" means an amino group protected with a "protective group for an amino group".

The "protected amino C₁₋₄ alkyl group" means a protected amino C₁₋₄ alkyl group in which an amino group is protected with a "protective group for an amino group".

The "optionally protected amino C₁₋₄ alkyl group" means an amino C₁₋₄ alkyl group or a protected amino C₁₋₄ alkyl group.

The "protective group for a carboxy group" includes all groups usable as usual carboxy protective groups, and includes, for example, the groups described in P.G.M. Wuts et al., Protective Groups in Organic Synthesis 4th Ed., 2006, John Wiley & Sons, INC. Examples thereof include a C₁₋₆ alkyl group optionally substituted with a C₁₋₄ alkoxy group (a methyl group, an ethyl group, a tert-butyl group, a methoxymethyl group, a tert-butoxymethyl group, etc.), and a benzyl group optionally substituted with a substituent selected from "a halogen atom, a C₁₋₄ alkoxy group, and a nitro group" (a benzyl group, a p-methoxybenzyl group, a p-nitrobenzyl group, a p-chlorobenzyl group, etc.).

The "optionally protected carboxy group" means an unprotected or protected carboxy group.

The "protected carboxy group" means a carboxy group protected with a "protective group for a carboxy group".

The "protected formyl group" includes a formyl group protected with any of groups usable as usual formyl protective groups, and includes, for example, the groups described in P.G.M. Wuts et al., Protective Groups in Organic Synthesis 4th Ed., 2006, John Wiley & Sons, INC. Examples thereof include a 1,1-dimethoxymethyl group, a 1,1-diethoxymethyl group, a 1,3-dioxanyl group, 1,3-dioxolanyl group, 1,3-dithianyl group, and a 1,3-dithiolanyl group.

The "optionally protected formyl group" means an unprotected or protected formyl group.

The "protective group for an acetylene group" includes all groups usable usually as protective groups for an acetylene group, and includes, for example, the groups described in P.G.M. Wuts et al., Protective Groups in Organic Synthesis 4th Ed., 2006, John Wiley & Sons, INC. Examples thereof include a silyl group having 3 substituents which are the same or different and are selected from "a C₁₋₆ alkyl group and an aryl group" (a trimethylsilyl group, a triethylsilyl group, a triisopropylsilyl group, a tert-butyldimethylsilyl group, a tert-butyldiphenylsilyl group, etc.).

Examples of the "leaving group" include a halogen atom, a methanesulfonyloxy group, a trifluoromethanesulfonyloxy group, and a p-toluenesulfonyloxy group.

The "antimicrobial agent" means a substance which has the ability to act on bacteria, such as gram-positive bacteria or gram-negative bacteria, thereby suppressing their growth or destroying them. The antimicrobial agent may be one which keeps down propagation of bacteria, or kills some of bacteria to decrease their count. Examples of gram-positive bacteria include the genus Staphylococcus (*Staphylococcus aureus*, *Staphylococcus epidermidis*, etc.), the genus Streptococcus (*Streptococcus pyogenes*, *Streptococcus agalactiae*, *Streptococcus pneumoniae*, etc.), and the genus Enterococcus (*Enterococcus faecalis*, *Enterococcus faecium*, etc.). Examples of gram-negative bacteria include the genus Pseudomonas (*Pseudomonas aeruginosa*, etc.), the genus Escherichia (*Escherichia coli*, etc.), the genus Klebsiella (*Klebsiella pneumoniae*, *Klebsiella oxytoca*, etc.), the genus Haemophilus *(Haemophilus influenzae, Haemophilus parainfluenzae*, etc.), the genus Bordetella (*Bordetella pertussis*, *Bordetella bronchiseptica*, etc.), the genus Serratia (*Serratia marcescens*, etc.), the genus Proteus (*Proteus mirabilis*, etc.), the genus Enterobacter (*Enterobacter cloacae*, etc.), the genus Campylobacter (*Campylobacter jejuni*, etc.), the genus Citrobacter, the genus Vibrio (*Vibrio parahaemolyticus*, *Vibrio cholerae*, etc.), the genus Morganella (*Morganella morganii*, etc.), the genus Salmonella (*Salmonella typhi*, *Salmonella paratyphi*, etc.), the genus Shigella (*Shigella dysenteriae*, etc.), the genus Acinetobacter (*Acinetobacter baumannii*, *Acinetobacter calcoaceticus*, etc.), the genus Legionella (*Legionella pneumophila*, etc.), the genus Bacteroides (*Bacteroides fragilis*, etc.), the genus Neisseria (*Neisseria gonorrhoeae*, *Neisseria meningitides*, etc.), the genus Moraxella (*Moraxella catarrhalis*, etc.), the genus Chlamydia (*Chlamydia trachomatis*, *Chlamydia psittaci*, etc.), and the genus Helicobacter (*Helicobacter pylori*, etc.).

The preferred embodiments of the compound of the present invention are as follows.

Preferred R¹ is a methyl group.

Preferred R² is a hydrogen atom or a methyl group, and more preferred R² is a hydrogen atom.

Preferred R³ is a hydrogen atom, an aminomethyl group, or a hydroxymethyl group, and more preferred R³ is a hydrogen atom or an aminomethyl group.

Preferred R^{4a} is a hydrogen atom or a fluorine atom, and more preferred R^{4a} is a hydrogen atom.

Preferred R^{4b} is a hydrogen atom or a fluorine atom, and more preferred R^{4b} is a hydrogen atom.

Preferred R^{4c} is a hydrogen atom or a fluorine atom, and more preferred R^{4c} is a hydrogen atom.

Preferred R^{4d} is a hydrogen atom or a fluorine atom, and more preferred R^{4d} is a hydrogen atom.

Preferred embodiment of the compound of the present invention is a compound represented by formula [9a] or [9b]: 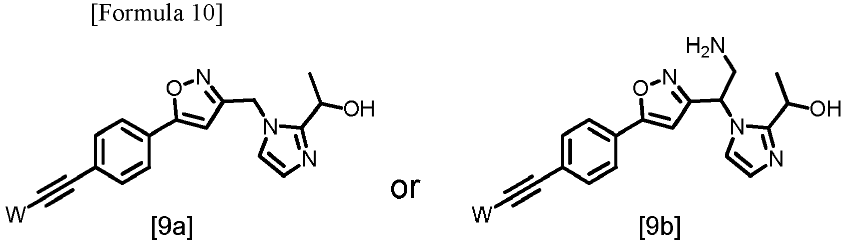 and more preferred embodiment is an optical isomer having an absolute configuration represented by formula [10a] or [10b]:

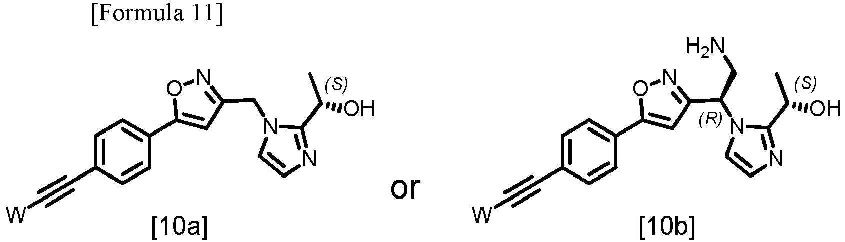

When W is formula [4], 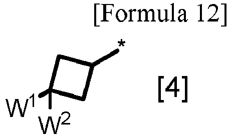 preferred embodiment is one in which W¹ is a hydroxy group or A^{1b}-CONR¹¹-, A^{1b} is a hydrogen atom or a C₁₋₄ alkyl group, R¹¹ is a hydrogen atom or a methyl group, and W² is a hydrogen atom. Further preferred embodiment of W¹ is a hydroxy group.

When W is formula [5], 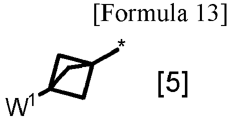 preferred embodiment is one in which W¹ is an amino group.

When W is formula [6], 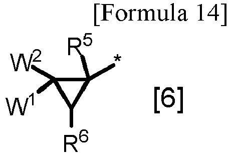 a preferred embodiment is one in which W¹ is a hydroxy C₁₋₄ alkyl group or a C₂₋₆ alkoxyalkyl group, W² is a hydrogen atom, R⁵ is a hydrogen atom, and R⁶ is a hydrogen atom. Further preferred embodiment of W¹ is a hydroxymethyl group. Another preferred embodiment is one in which W¹ is a hydrogen atom, W² is a hydrogen atom, R⁵ is a hydrogen atom, and R⁶ is an amino group.

When W is formula [7], 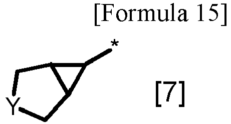 a preferred embodiment is one in which Y is W⁴-N, W⁴ is an A^{2b}-X¹-, A^{2b} is a hydroxy group or a carboxy group, and X¹ is a C₁₋₆ alkylene group. Another preferred embodiment is one in which Y is W⁴-N, W⁴ is A^{2a}-CO-, and A^{2a} is a hydrogen atom or a methyl group.

When W is formula [8], 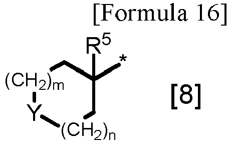 a preferred embodiment is one in which Y is an oxygen atom, m is 0, n is 0 or 1, and R⁵ is a hydrogen atom or a methyl group.

The compound of the present invention can exist as tautomers, stereoisomers such as geometrical isomers, and optical isomers, and the present invention includes them. The present invention also includes various hydrates, solvates and polymorphic substances of the compounds of the invention and the salts thereof.

In the present invention, the pharmaceutically acceptable salts refer to salts which are used in chemotherapy and prevention of bacterial infections. Examples thereof include salts with acids such as acetic acid, propionic acid, butyric acid, formic acid, trifluoroacetic acid, maleic acid, tartaric acid, citric acid, stearic acid, succinic acid, ethylsuccinic acid, malonic acid, lactobionic acid, gluconic acid, glucoheptonic acid, benzoic acid, methanesulfonic acid, ethanesulfonic acid, 2-hydroxyethanesulfonic acid, benzenesulfonic acid, para toluenesulfonic acid (tosic acid), laurylsulfuric acid, malic acid, aspartic acid, glutamic acid, adipic acid, cysteine, N-acetylcysteine, hydrochloric acid, hydrobromic acid, phosphoric acid, sulfuric acid, hydriodic acid, nicotinic acid, oxalic acid, picric acid, thiocyanic acid, undecanoic acid, acrylic polymer, and carboxyvinyl polymer; salts with inorganic bases such as lithium salt, sodium salt, potassium salt, magnesium salt, and calcium salt; salts with organic amines such as morpholine and piperidine; and salts with amino acids.

In order to use the compound of the present invention as a medicament, the compound of the present invention is used in an effective amount and may be in any form of a solid composition, a liquid composition, and other compositions, and the most suitable form is selected as needed. The above composition can be made into a medicinal preparation upon combination with one or more pharmaceutically acceptable carriers, excipients or diluents. Examples of the above carriers, excipients, and diluents include water, lactose, dextrose, fructose, sucrose, sorbitol, mannitol, polyethylene glycol, propylene glycol, starch, gum, gelatin, alginate, calcium silicate, calcium phosphate, cellulose, aqueous syrup, methyl cellulose, polyvinyl pyrrolidone, alkylparahydroxybenzosorbate, talc, magnesium stearate, stearic acid, glycerin, sesame oil, olive oil, soy oil, and various other seed oils. Moreover, the above carriers, excipients, or diluents can be mixed, as needed, with commonly used additives such as thickeners, binders, disintegrants, pH regulators, and solvents, and can be prepared as an oral or parenteral drug, such as tablets, pills, capsules, granules, powders, liquids, emulsions, suspensions, ointments, injections, or skin patchs, by a customary pharmaceutical technology.

The compound of the present invention can be administered parenterally (intravenously, intravenous drip, intramuscularly, subcutaneously, subcutaneously, etc.), oral or topically (inhalation, nasally, etc.) to an adult patient in a dose of 10 to 10,000 mg as a single daily dose or as divided portions per day. This dose can be increased or decreased, as appropriate, according to the type of the disease to be treated, or the age, body weight, sex, symptoms, etc. of the patient. The compound of the present invention can also be used in combination with other drugs.

The compound of the present invention can be synthesized, for example, by methods to be shown below, but the present invention is in no way limited to these methods of producing the compound.

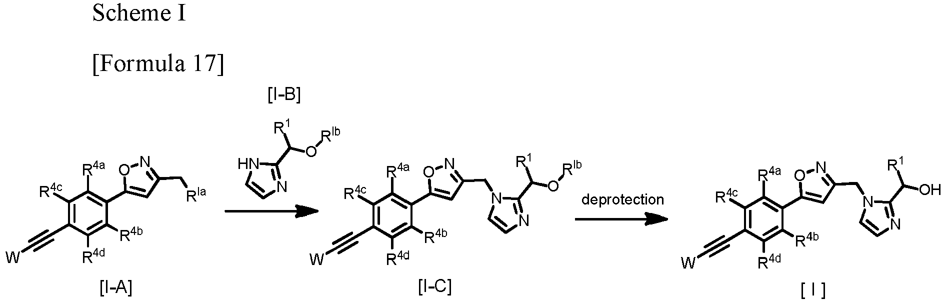

Compound [1] of the present invention represented by general formula [I] can be synthesized by the process shown in scheme I. A compound represented by general formula [I-A] (where R^{Ia} is a hydroxy group or a leaving group, and the other symbols are as defined above) and a compound represented by general formula [I-B] (where R^{Ib} is a protective group for a hydroxy group, and the other symbols are as defined above) are reacted under conditions of Mitsunobu reaction when R^{Ia} is a hydroxy group, or in the presence of a base when R^{Ia} is a leaving group, whereby a compound represented by general formula [I-C] (where the symbols are as defined above) can be obtained. Furthermore, an appropriate deprotection reaction is performed in accordance with the type of the protective group R^{Ib}, whereby a compound represented by general formula [I] (where the symbols are as defined above) can be obtained.

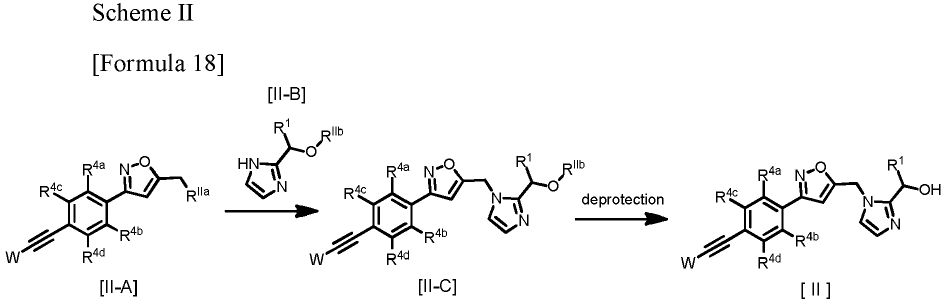

Compound [2] of the present invention represented by general formula [II] can be synthesized by the process shown in scheme II. A compound represented by general formula [II-A] (where R^{IIa} is a hydroxy group or a leaving group, and the other symbols are as defined above) and a compound represented by general formula [II-B] (where R^{IIb} is a protective group for a hydroxy group, and the other symbols are as defined above) are reacted under conditions of Mitsunobu reaction when R^{IIa} is a hydroxy group, or in the presence of a base when R^{IIa} is a leaving group, whereby a compound represented by general formula [II-C] (where the symbols are as defined above) can be obtained. Furthermore, an appropriate deprotection reaction is performed in accordance with the type of the protective group R^{IIb}, whereby a compound represented by general formula [II] (where the symbols are as defined above) can be obtained. 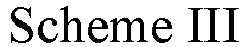 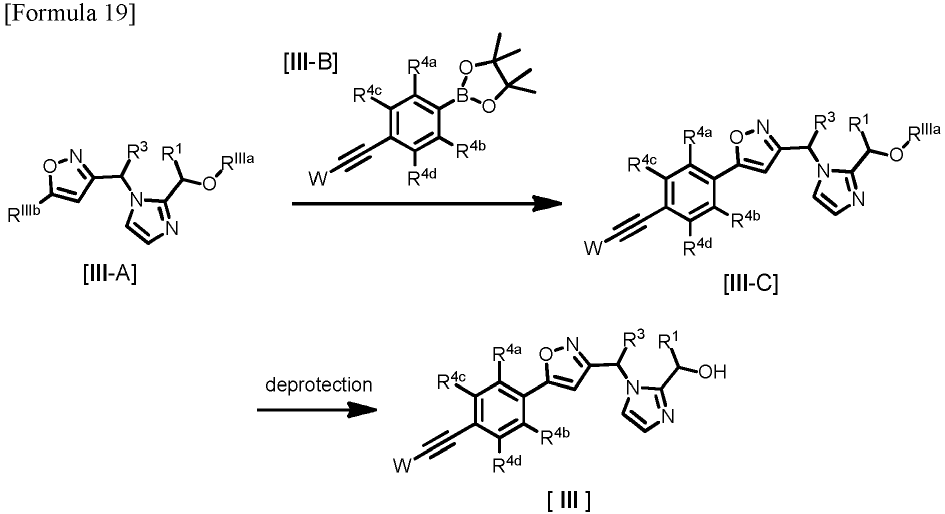 Compound [1] of the present invention represented by general formula [III] can be synthesized by the process shown in scheme III. Boronic ester compounds represented by general formula [III-A] (where R^{IIIa} is a protective group for a hydroxy group, R^{IIIb} is a leaving group, and the other symbols are as defined above) and general formula [III-B] (where the symbols are as defined above) or a boronic compound equivalent thereto are subjected to a coupling reaction in the presence of a catalyst such as tetrakis(triphenylphosphine)palladium and a base, and in the presence or absence of a ligand, whereby a compound represented by general formula [III-C] (where the symbols are as defined above) can be obtained. Furthermore, an appropriate deprotection reaction is performed in accordance with the type of a protective group, whereby the compound represented by general formula [III] (where the symbols are as defined above) can be obtained. 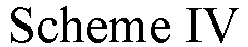 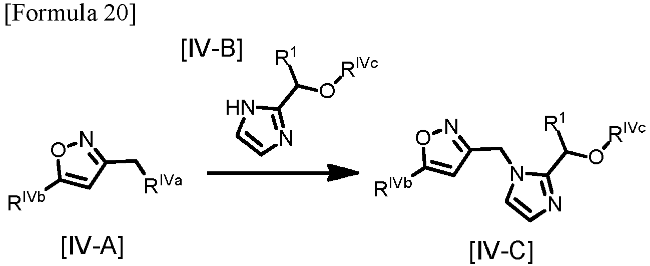 A compound represented by general formula [IV-A] (where R^{IVa} is a hydroxy group or a leaving group, R^{IVb} is a leaving group, and the other symbols are as defined above) and a compound represented by general formula [IV-B] (where R^{IVc} is a protective group for a hydroxy group, and the other symbols are as defined above) are reacted under conditions of Mitsunobu reaction when R^{IVa} is a hydroxy group, or in the presence of a base when R^{IVa} is a leaving group, whereby a compound represented by general formula [IV-C] (where the symbols are as defined above) can be obtained.

From the compound represented by general formula [IV-C], the compound of the present invention represented by general formula [1] can be obtained in accordance with the method of Scheme III.

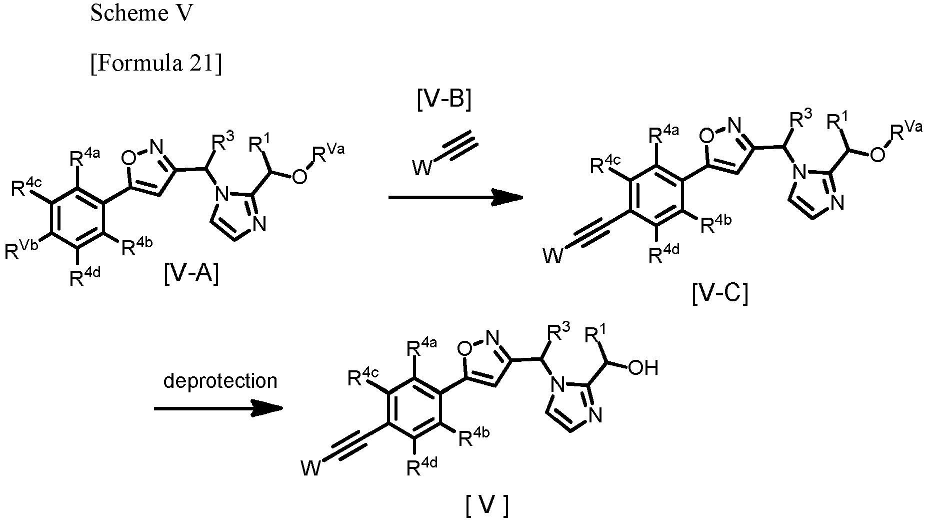

Compound [1] of the present invention represented by general formula [V] can be synthesized by the process shown in scheme V. Compounds represented by general formula [V-A] (where R^{Va} is a protective group for a hydroxy group, R^{Vb} is a leaving group, and the other symbols are as defined above) and general formula [V-B] (where the symbols are as defined above) are subjected to a Sonogashira coupling reaction in the presence of a catalyst such as tetrakis(triphenylphosphine)palladium and copper iodide and a base, and in the presence or absence of a ligand, whereby a compound represented by general formula [V-C] (where the symbols are as defined above) can be obtained. Furthermore, an appropriate deprotection reaction is performed in accordance with the type of a protective group, whereby the compound represented by general formula [V] (where the symbols are as defined above) can be obtained.

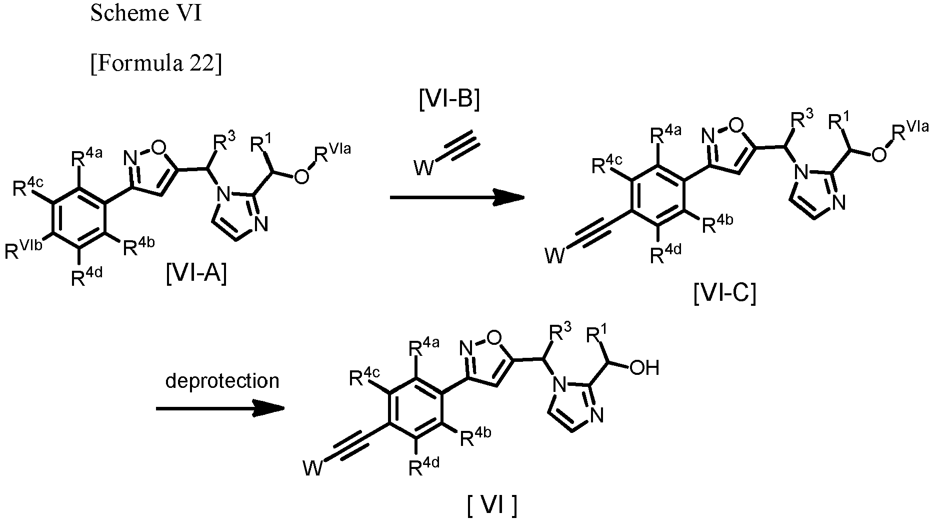

Compound [2] of the present invention represented by general formula [VI] can be synthesized by the process shown in scheme VI. Compounds represented by general formula [VI-A] (where R^{VIa} is a protective group for a hydroxy group, R^{VIb} is a leaving group, and the other symbols are as defined above) and general formula [VI-B] (where the symbols are as defined above) are subjected to a Sonogashira coupling reaction in the presence of a catalyst such as tetrakis(triphenylphosphine)palladium and copper iodide and a base, and in the presence or absence of a ligand, whereby a compound represented by general formula [VI-C] (where the symbols are as defined above) can be obtained. Furthermore, an appropriate deprotection reaction is performed in accordance with the type of a protective group, whereby the compound represented by general formula [VI] (where the symbols are as defined above) can be obtained.

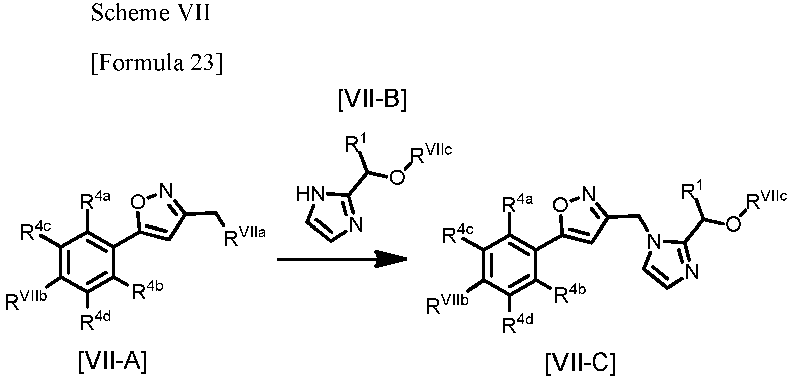

A compound represented by general formula [VII-A] (where R^{VIIa} is a hydroxy group or a leaving group, R^{VIIb} is a leaving group, and the other symbols are as defined above) and a compound represented by general formula [VII-B] (where R^{VIIc} is a protective group for a hydroxy group, and the other symbols are as defined above) are reacted under conditions of Mitsunobu reaction when R^{VIIa} is a hydroxy group, or in the presence of a base when R^{VIIa} is a leaving group, whereby a compound represented by general formula [VII-C] (where the symbols are as defined above) can be obtained.

From the compound represented by general formula [VII-C], the compound of the present invention represented by general formula [1] can be obtained in accordance with the method of Scheme V. 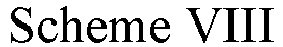 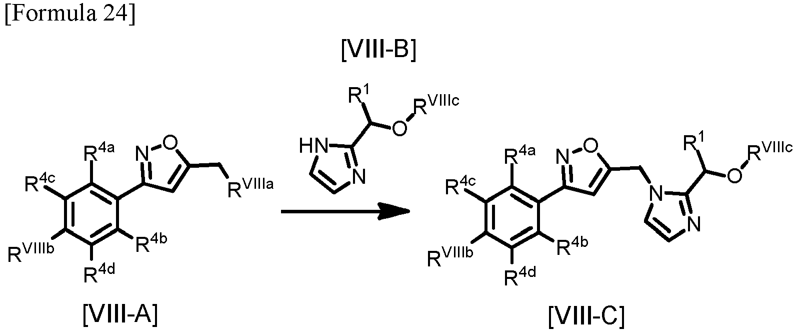 A compound represented by general formula [VIII-A] (where R^{VIIIa} is a hydroxy group or a leaving group, R^{VIIIb} is a leaving group, and the other symbols are as defined above) and a compound represented by general formula [VIII-B] (where R^{VIIIc} is a protective group for a hydroxy group, and the other symbols are as defined above) are reacted under conditions of Mitsunobu reaction when R^{VIIIa} is a hydroxy group, or in the presence of a base when R^{VIIIa} is a leaving group, whereby a compound represented by general formula [VIII-C] (where the symbols are as defined above) can be obtained.

From the compound represented by general formula [VIII-C], the compound of the present invention represented by general formula [2] can be obtained in accordance with the method of Scheme VI.

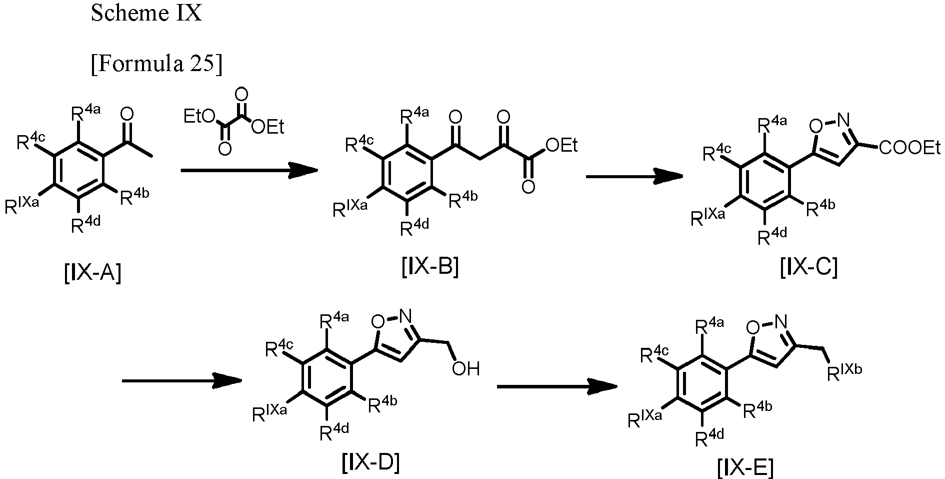

A compound represented by general formula [IX-A] (where R^{IXa} is a leaving group, and the other symbols are as defined above) and an oxalic diester compound such as oxalic acid diethyl ester are allowed to act in the presence of a base to obtain a compound represented by general formula [IX-B] (where the symbols are as defined above). This is reacted with hydroxylamine, whereby a compound represented by general formula [IX-C] (where the symbols are as defined above) in which an isoxazole ring is formed can be obtained. The compound represented by general formula [IX-C] is reduced by a reducing agent such as LiBH₄, whereby a compound represented by general formula [IX-D] (where the symbols are as defined above) can be obtained. Furthermore, the compound represented by general formula [IX-D] is allowed to act with methanesulfonic acid chloride, trifluoromethanesulfonic acid chloride, p-toluenesulfonyl chloride, or the like in the presence of a base, whereby a compound represented by general formula [IX-E] (where R^{IXb} is a leaving group, and the other symbols are as defined above) can be obtained.

From the compound represented by general formula [IX-D] or [IX-E], the compound of the present invention represented by general formula [1] can be obtained in accordance with the method of scheme VII.

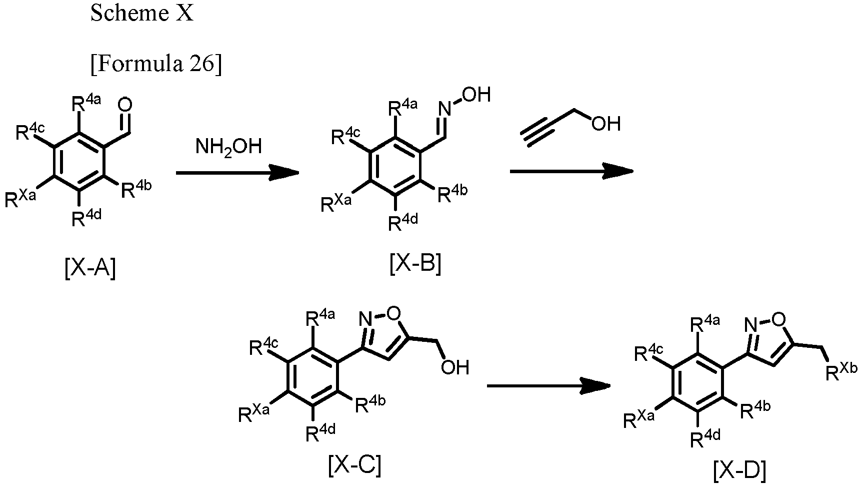

A compound represented by general formula [X-A] (where R^{Xa} is a leaving group, and the other symbols are as defined above) is allowed to act with hydroxylamine to obtain a compound represented by general formula [X-B] (where the symbols are as defined above). The compound represented by general formula [X-B] is allowed to act with propargyl alcohol in the presence of N-chlorosuccinimide and a base, or in the presence of bis(trifluoroacetoxy)iodo]benzene, whereby a compound represented by general formula [X-C] (where the symbols are as defined above) can be obtained. Furthermore, the compound represented by general formula [X-C] is allowed to act with methanesulfonic acid chloride, trifluoromethanesulfonic acid chloride, p-toluenesulfonyl chloride, or the like in the presence of a base, whereby a compound represented by general formula [X-D] (where R^{Xb} is a leaving group, and the other symbols are as defined above) can be obtained.

From the compound represented by general formula [X-C] or [X-D], the compound of the present invention represented by general formula [2] can be obtained in accordance with the method of scheme VIII.

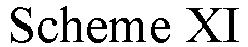

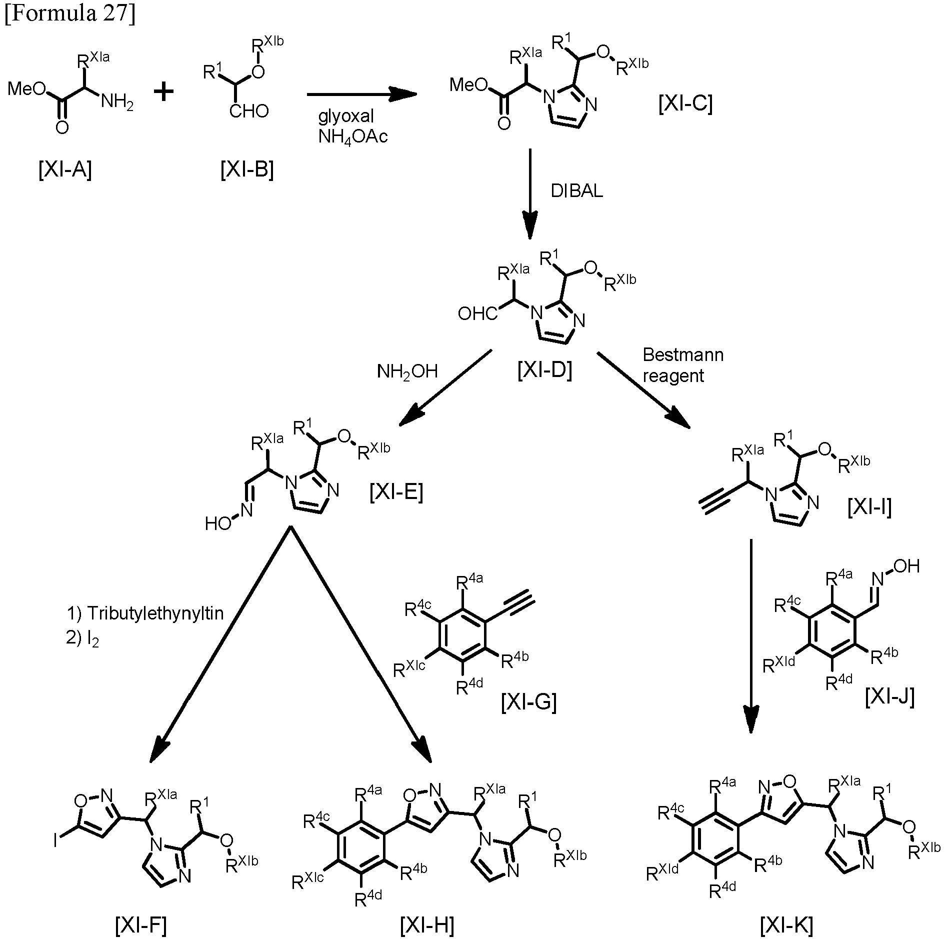

An amino acid ester compound represented by general formula [XI-A] (where R^{XIa} is a protected C₁₋₄ aminoalkyl group or an optionally protected C₁₋₄ hydroxyalkyl group), a compound represented by general formula [XI-B] (where R^{XIb} is a protective group for a hydroxy group, and the other symbols are as defined above), and an ammonia source such as glyoxal and ammonium acetate are reacted to obtain a compound represented by general formula [XI-C] (where the symbols are as defined above). The compound represented by general formula [XI-C] is reduced with DIBAL, whereby an aldehyde compound represented by general formula [XI-D] (where the symbols are as defined above) can be obtained. The compound represented by general formula [XI-D] can also be obtained by reducing the compound represented by general formula [XI-C] with a reducing agent such as LiBH₄, and then allowing to act an oxidizing agent.

The compound represented by general formula [XI-D] is reacted with hydroxylamine, whereby a compound represented by general formula [XI-E] (where the symbols are as defined above) can be obtained. The compound represented by general formula [XI-E] is reacted with tributylethynyltin to form an isoxazole ring, and it is further reacted with iodine, whereby a compound represented by general formula [XI-F] (where the symbols are as defined above) can be obtained.

The compound represented by general formula [XI-E] is reacted with N-chlorosuccinimide and a compound represented by general formula [XI-G] (where R^{XIc} is a leaving group, and the other symbols are as defined above) in the presence of a base, whereby a compound represented by general formula [XI-H] (where the symbols are as defined above) can be obtained.

The compound represented by general formula [XI-D] is reacted with the Bestmann reagent (dimethyl (1-diazo-2-oxopropyl)phosphonate) in the presence of a base, a compound represented by general formula [XI-I] (where the symbols are as defined above) can be obtained. The compound represented by general formula [XI-I] is reacted with N-chlorosuccinimide and a compound represented by general formula [XI-J] (where R^{XId} is a leaving group, and the other symbols are as defined above) in the presence of a base, whereby a compound represented by general formula [XI-K] (where the symbols are as defined above) can be obtained.

From the compound represented by general formula [XI-F], the compound of the present invention represented by general formula [1] can be obtained in accordance with the method of scheme III.

From the compound represented by general formula [XI-H], the compound of the present invention represented by general formula [1] can be obtained in accordance with the method of scheme V.

From the compound represented by general formula [XI-K], the compound of the present invention represented by general formula [2] can be obtained in accordance with the method of scheme VI.

In the methods of synthesis shown above, the sequence of the reaction steps can be changed as needed. If an amino group, a hydroxy group, a formyl group, and a carboxy group are present in the compounds obtained in the respective reaction steps and their intermediates, the reactions can be performed by appropriately changing combinations of protection or deprotection of the functional groups thereof with the protective group for them.

Unless otherwise specified, examples of the base used in any of the above reactions include sodium carbonate, potassium carbonate, cesium carbonate, sodium bicarbonate, potassium bicarbonate, sodium acetate, potassium acetate, potassium hydrate, sodium hydroxide, lithium hydroxide, sodium amide, sodium methoxide, sodium ethoxide, sodium tert-butoxide, potassium tert-butoxide, sodium hydride, lithium hydride, triethylamine, diisopropylethylamine, dimethylaniline, diethylaniline, pyridine, pyrrolidine, and N-methylmorpholine.

Examples of the acid include inorganic acids such as hydrochloric acid, hydrobromic acid, sulfuric acid, nitric acid, phosphoric acid, and polyphosphoric acid, and organic acids such as p-toluenesulfonic acid, methanesulfonic acid, trifluoroacetic acid, formic acid, and acetic acid.

Examples of the condensing agent include O-(7-azabenzotriazol-1-yl)-N,N,N',N'-tetramethyluronium hexafluorophosphate, 1-(3-dimethylaminopropyl)-3-ethylcarbodiimide hydrochloride, dicyclohexylcarbodiimide, carbonyldiimidazole, 2-chloro-1-methylpyridinium iodide, 4-(4,6-dimethoxy-1,3,5-triazin-2-yl)-4-methylmorpholine chloride, O-(7-azabenzotriazol-1-yl)-N,N,N',N'-tetramethyluronium hexafluorophosphate, and benzotriazole-1-yl-oxy-tris-pyrrolidino-phosphonium hexafluorophosphate.

Examples of the activator used in employing the method conducted via an acid chloride or an acid anhydride include thionyl chloride, oxalyl chloride, phosphoryl chloride, acetic anhydride, and chloroformic esters.

Examples of the catalyst include palladium acetate, palladium chloride, bis(triphenylphosphine)palladium(II) dichloride, tetrakis(triphenylphosphine)palladium, bis(acetonitrile)dichloropalladium, bis(benzonitrile)dichloropalladium, tris(dibenzylideneacetone)dipalladium, bis(dibenzylideneacetone)palladium, bis(tricyclohexylphosphine)dichloropalladium, bis(tri-o-tolylphosphine)dichloropalladium, bis(tri-t-butylphosphine)dichloropalladium, [1,3-bis(2,6-diisopropylphenyl)imidazol-2-ylidene](3-chloropyridyl)palladium(II) dichloride, [1,3-bis(2,6-di-3-pentylphenyl)imidazol-2-ylidene](3-chloropyridyl)palladium(II) dichloride, 1,1'-bis(diphenylphosphino)ferrocenepalladium(II) dichloride, tris{tris[3,5-bis(trifluoromethyl)phenyl]phosphine}palladium(0), palladium on carbon, palladium hydroxide, and copper iodide.

Examples of the ligand include tri-tert-butylphosphine, tricyclohexylphosphine, triphenylphosphine, tritolylphosphine, tributyl phosphite, tricyclohexyl phosphite, triphenyl phosphite, 1,1'-bis(diphenylphosphino)ferrocene, 2,2'-bis(diphenylphosphino)-1,1'-binaphthyl, 2-dicyclohexylphosphino-2',6'-dimethoxybiphenyl, 2-dicyclohexylphosphino-2',4',6'-triisopropylbiphenyl, 2-(di-tert-butylphosphino)-2',4',6'-triisopropylbiphenyl, and 2-(di-tert-butylphosphino)biphenyl.

Examples of the oxidizing agent include m-chloroperbenzoic acid, hydrogen peroxide, potassium peroxymonosulfate, Oxone (R), 2-iodoxybenzoic acid, Dess-Martin periodinane (Dess-Martin reagent, 1,1,1-triacetoxy-1,1-dihydro-1,2-benziodoxol-3(1H)-one), potassium permanganate, chromium oxide, potassium dichromate, urea hydrogen peroxide adduct/phthalic anhydride, tert-butylhydroperoxide, cumene hydroperoxide, selenium dioxide, lead acetate (IV), t-butyl hypochlorite, and sodium hypochlorite.

Examples of the reducing agent include hydrogenated complex compounds such as lithium aluminum hydride, sodium triacetoxyborohydride, sodium cyanoborohydride, sodium borohydride, lithium borohydride, and diisobutylaluminum hydride; boranes such as picoline borane, borane-pyridine complex, and borane-THF complex; and sodium, and sodium amalgam.

Examples of the metal salt include zinc chloride, zirconium chloride, indium chloride, and magnesium chloride.

The solvent is not limited, if it is stable under the reaction conditions concerned, is inert, and does not impede the reaction, and examples of the solvent include polar solvents (e.g., water and alcoholic solvents such as methanol, ethanol, and isopropanol), inert solvents (e.g., halogenated hydrocarbon-based solvents such as chloroform, methylene chloride, dichloroethane, and carbon tetrachloride, ether-based solvents such as diethyl ether, diisopropyl ether, tetrahydrofuran, 2-methyltetrahydrofuran, 1,4-dioxane, and dimethoxyethane, aprotic solvents such as dimethylformamide, dimethyl sulfoxide, ethyl acetate, tert-butyl acetate, acetonitrile, and propionitrile, aromatics such as benzene, toluene, and anisole, or hydrocarbons such as n-hexane and cyclohexane), or mixtures of these solvents.

The reaction can be performed at an appropriate temperature selected from a range of from -78°C to the boiling point of the solvent used in the reaction, at ordinary pressure or under pressurized conditions, under microwave irradiation, or the like.

### EXAMPLES

Hereinbelow, the present invention will be described in further detail by Reference Examples, Examples, and Test Examples. The compound of the present invention is in no way limited to the compounds described in Examples presented below.

Unless otherwise described, the support used in OH type silica gel column chromatography was a pre-packed column such as SNAP Ultra produced by Biotage Ltd. or REVELERIS produced by BUCHI, and the support used in NH type silica gel chromatography was a pre-packed column such as SNAP Cartridge produced by Biotage Ltd. or REVELERIS Amino produced by BUCHI. OH type silica gel thin-layer chromatography used for preparative TLC was PLC plate silica gel 60 F₂₅₄ produced by Merck Millipore Corporation and NH type silica gel thin-layer chromatography used for preparative TLC was NH₂ Silica Gel 60 F₂₅₄ Plate-Wako produced by Wako Pure Chemical Industries, Ltd.

Purification by preparative HPLC instrument was conducted using Agilent 1260 Infinity, Agilent 6130 (ESI: electron spray ionisation), or Agient 385-ELSD.
Column:
   YMC-Actus Triart C18, 5.0 µm, Φ30 x 50 mm.
   Xbridge Prep C18, 5.0 µm OBD, Φ30 x 50 mm.
   Waters XSlect CSH, 5.0 µm, Φ30 x 50 mm.
Eluent: A (water + 0.1% formic acid), B (acetonitrile + 0.1% formic acid).

Purification was performed under any of the following conditions 1 to 4.
1. Flow velocity of 50 mL/min; 0 to 0.5 min (A/B = 90/10), 0.5 to 7.5 min (gradient elution with A/B = 90/10 to 20/80), 7.5 to 7.95 min (A/B = 20/80), 7.95 to 8.0 min ((gradient elution with A/B = 20/80 to 5/95), 8.0 to 9.0 min (A/B = 5/95).
2. Flow velocity of 50 mL/min; 0 to 0.5 min (A/B = 95/5), 0.5 to 7.5 min (gradient elution with A/B = 95/5 to 50/50), 7.5 to 7.95 min (A/B = 50/50), 7.95 to 8.0 min ((gradient elution with A/B = 50/50 to 5/95), 8.0 to 9.0 min (A/B = 5/95).
3. Flow velocity of 50 mL/min; 0 to 0.5 min (A/B = 80/20), 0.5 to 7.0 min (gradient elution with A/B = 80/20 to 5/95), 7.0 to 7.45 min (A/B = 5/95), 7.45 to 7.5 min ((gradient elution with A/B = 5/95 to 1/99), 7.5 to 9.0 min (A/B = 1/99).
4. Flow velocity of 40 mL/min; 0 to 2.0 min (A/B = 90/10), 2.0 to 11.0 min (gradient elution with A/B = 90/10 to 20/80), 11.0 to 12.0 min ((gradient elution with A/B = 20/80 to 5/95), 12.0 to 13.5 min (A/B = 5/95).

MS spectrum measurements and retention time (min) measurements of compounds were performed using any of the following LCMS instrument and measurement conditions (A) to (D).
(A)
   Instrument: Agilent 1290 Infinity (LC), Agilent 6130 or 6150 (Quadrupole LC/MS), Agilent 385-ELSD (ELSD).
   Column: ACQUITY CSH C18 (Waters), 1.7 µm, Φ2.1 x 50 mm.
   Ionization method: ESI.
   Detection method: 254 nm, 210 nm, or ELSD.
   Eluent: A (water + 0.1% formic acid), B (acetonitrile + 0.1% formic acid).
   Flow velocity: 0.8 mL/min; 0.0 to 0.8 min (gradient elution with A/B = 95/5 to 60/40), 0.8 to 1.08 min (gradient elution with A/B = 60/40 to 1/99), 1.08 to 1.38 min (A/B = 1/99).
(B) The instrument, column, ionization method, detection method, and eluent were the same as (A).
   Flow velocity: 0.8 mL/min; 0.0 to 1.2 min (gradient elution with A/B = 80/20 to 1/99), 1.2 to 1.4 min (A/B = 1/99).
(C) The instrument, column, ionization method, detection method, and eluent were the same as (A).
   Flow velocity: 0.8 mL/min; 0.0 to 0.8 min (gradient elution with A/B = 70/30 to 1/99), 0.8 to 1.4 min (A/B = 1/99).
(D)
   Instrument: LCMS-2010EV (Shimadzu).
   Column: XR-ODS (Shimadzu), 2.2 µm, Φ2.0 x 30 mm.
   Ionization method: ESI/APCI (Atmospheric pressure chemical ionization) dual.
   Detection method: 254 nm, 210 nm.
   Eluent: A (water + 0.1% formic acid), B (acetonitrile + 0.1% formic acid).
   Flow velocity: 0.6 mL/min; 0.0 to 0.5 min (A/B = 90/10), 0.5 to 1.5 min (gradient elution with A/B = 90/10 to 60/40), 1.5 to 2.5 min (gradient elution with A/B = 60/40 to 1/99), 2.5 to 5.0 min (A/B = 1/99).
      MS spectrum measurements of compounds were performed using the following MS instrument and measurement conditions (E).
(E)
   Instrument: LCMS-IT-TOF (Shimazdu).
   Ionization method: ESI/APCI (Atmospheric pressure chemical ionization) dual.
   Eluent: 90% methanol.
   Flow velocity: 0.2 mL/min

As a microwave reactor, Initiator+ produced by Biotage was used.

NMR SPECTRUM measurements were performed using JNM-ECA600 (600 MHz, JEOL), JNM-ECA500 (500 MHz, JEOL), or AVANCE III HD 400 (400 MHz, BRUKER). Chemical shifts indicate proton NMR (¹H-NMR), tetramethylsilane was used as an internal standard, and δ values were represented in ppm. Abbreviations in the NMR data are as follows:
s: Singlet
d: Doublet
dd: Double doublet
dt: Double triplet
t: Triplet
td: Triple doublet
tt: Triple triplet
q: Quartet
quin: Quintet
m: Multiplet
br: Broad signal

Chemical names of compounds and synthetic intermediates were generated using the ACD/Name 2015 (ACD Labs 2015 LSM, Advanced Chemistry Development Inc.) software.

Abbreviations in the following description are shown below.
(+)-CSA: (+)-10-camphorsulfonic acid
APCI: Atmospheric pressure chemical ionization
aq.: Aqueous solution
Bestmann reagent: Dimethyl (1-diazo-2-oxopropyl)phosphonate
Boc: tert-Butoxycarbonyl group
BocNH: tert-Butoxycarbonylamino group
Bn: Benzyl group
Bu: Butyl group
DBU: Diazabicycloundecene
DEAD: Diethyl azodicarboxylate
DIBAL: Diisobutylaluminum hydride
DIPEA: N,N-Diisopropylethylamine
DMAP: N,N-Dimethyl-4-aminopyridine
DMF: N,N-Dimethylformamide
DMP: Dess-Martin periodinane (Dess-Martin reagent)
DMSO-d₆: Hexadeuterodimethyl sulfoxide
DOX: 1,4-Dioxane
DPPA: Diphenylphosphoryl azide
ee: Enantiomeric excess
ESI: Electrospray ionization
Et: Ethyl group
EtOAc: Ethyl acetate
HATU: O-(7-Azabenzotriazol-1-yl)-N,N,N',N'-tetramethyluroniumhexafluorophosphate
HOBt-H₂O: 1-Hydroxybenzotriazole monohydrate
IPA: Isopropyl alcohol
IPE: Diisopropylether
LAH: Lithium aluminum hydride
LC: Liquid chromatography
LDA: Lithium diisopropylamide
Me: Methyl group
MeCN: Acetonitrile
MeOH: Methanol
Ms: Methanesulfonyl group
MsCl: Methanesulfonyl chloride
NCS: N-chlorosuccinimide
NMM: N-methylmorpholine
NMP: 1-Methyl-2-pyrrolidone
NOE: Nuclear Overhauser effect
OTBS: tert-Butyldimethylsilyloxy group
OTHP: Tetrahydropyranyloxy group
PEPPSI-IPr: [1,3-Bis(2,6-diisopropylphenyl)imidazol-2-ylidene] (3 - chloropyridyl)palladium(II) dichloride
PEPPSI-IPent: [1,3-Bis(2,6-di-3-pentylphenyl)imidazol-2-ylidene](3-chloropyridyl)palladium(II) dichloride
PdCl₂(dppf): 1,1'-Bis(diphenylphosphino)ferrocenepalladium(II) dichloride PdCl₂(PPh₃)₂: Bis(triphenylphosphine)palladium(II) dichloride PPTS: Pyridine p-toluenesulfonate
(p-Tol)₃P: Tri(4-methylphenyl)phosphine
pTsOH-H₂O: p-Toluenesulfonic acid monohydrate
SFC: Supercritical flow chromatography
Superstable Pd(0): Tris{tris[3,5-bis(trifluoromethyl)phenyl]phosphine}palladium(0)
T3P: Propylphosphonic anhydride
TBAF: Tetra-n-butylammonium fluoride
TBDPS: tert-Butyldiphenylsilyl group
TBS: tert-Butyldimethylsilyl group
TBSCl: tert-Butyldimethylsilyl chloride
TBSO: tert-Butyldimethylsilyloxy group
TEA: Triethylamine
TFA: Trifluoroacetic acid
THF: Tetrahydrofuran
THP: Tetrahydropyranyl group
THPO: Tetrahydropyranyloxy group
TMAD: N,N,N',N'-tetramethylazodicarboxamide
TMS: Trimethylsilyl group
TIPS: Triisopropylsilyl group
TsCl; p-Toluenesulfonic acid chloride
WSC·HCl: 1-Ethyl-3-(3-dimethylaminopropyl)carbodiimide hydrochloride
XPhos: 2-Dicyclohexylphosphino-2',4',6'-triisopropylbiphenyl

### Reference Example 1

### 2-{(1S)-1-[(Oxan-2-yl)oxy]ethyl}-1H-imidazole (intermediate A)

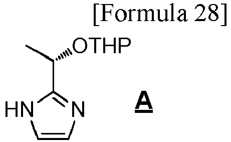

### A-1) Synthesis of 2-{(1S)-1-[(oxan-2-yl)oxy]ethyl}-1H-imidazole (intermediate A)

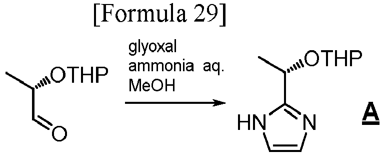

To a MeOH (0.77 L) solution of (2S)-2-tetrahydropyran-2-yloxypropanal (36 g), a 28% ammonia water (92 mL) and an 8.8 mol/L-aqueous glyoxal solution (78 mL) were sequentially added, and the reaction mixture was added at room temperature for 16 hours. The reaction mixture was concentrated under reduced pressure, and the residue was adsorbed on ISOLUTE(registered trademark) HM-N and purified by OH type silica gel column chromatography (gradient elution with chloroform/methanol = 100/0 to 92/8) to obtain 50 g of a brown oily crude product. Then, 45 g of the crude was dissolved in chloroform (0.50 L), and activated carbon (10 g) and NH type silica gel (0.20 L) were added, and the mixture was stirred at room temperature for 2 hours. The activated carbon and NH type silica gel were filtered off, the filtrate was concentrated under reduced pressure, and the residue was adsorbed on ISOLUTE(registered trademark) HM-N and purified by NH type silica gel column chromatography (gradient elution with n-hexane/chloroform = 50/50 to 0/100) to obtain intermediate A (pale pink solid, 28 g, yield 63%).

The ¹H-NMR and LCMS data are as shown in Table 1.

### Reference Example 2

### 2-[(1S)-1-{[tert-Butyl(dimethyl)silyl]oxy}ethyl]-1H-imidazole (intermediate B)

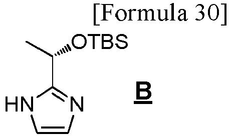

### B-1) Synthesis of 2-[(1S)-1-{[tert-butyl(dimethyl)silyl]oxy}ethyl]-1H-imidazole (intermediate B)

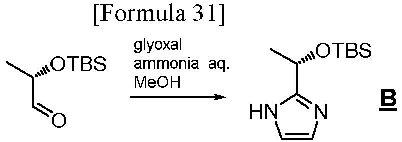

To MeOH (5.5 mL) of (2S)-2-{[tert-butyl(dimethyl)silyl]oxy}propanal (0.51 g), 28% ammonia water (0.77 mL) and 8.8 mol/L-glyoxal (0.62 mL) were added at room temperature, and the mixture was stirred for 22 hours. After concentration of the reaction mixture, the residue was purified by OH type silica gel column chromatography (gradient elution with chloroform/MeOH = 100/0 to 85/15) to obtain intermediate B (pale yellow solid, 0.34 g, yield 55%).

The 1H-NMR and LCMS data are as shown in Table 1.

### Reference Example 3

### 5-Iodo-3-[(2-{(1S)-1-[(oxan-2-yl)oxy]ethyl}-1H-imidazol-1-yl)methyl]-1,2-oxazole (intermediate C)

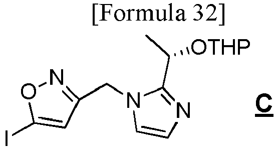

### C-1) Synthesis of 3-(((tert-butyldimethylsilyl)oxy)methyl)-5-iodoisoxazole

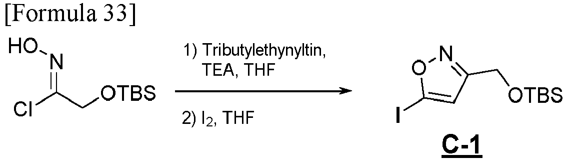

To a mixture of (1Z)-2-[tert-butyl(dimethyl)silyl]oxy-N-hydroxy-acetimidoyl chloride (8.3 g) and tributyl(ethynyl)tin (12 g), a THF (37 mL) solution of TEA (10 mL) was added dropwise under ice cooling. The reaction mixture was stirred at room temperature for 1 hour and then quenched by addition of water, the organic layer was washed with brine and dried over magnesium sulfate, and the solvent was distilled off under reduced pressure. The residue was dissolved in THF (60 mL), and a THF (33 mL) solution of iodine (11 g) was added dropwise under ice cooling. The reaction mixture was stirred at room temperature for 40 minutes and then quenched by addition of an aqueous sodium thiosulfate solution and an aqueous saturated sodium bicarbonate solution, followed by extracting with ethyl acetate. The organic layer was washed with brine and dried over magnesium sulfate, and the solvent was distilled off under reduced pressure. The residue was purified by OH type silica gel column chromatography (gradient elution with n-hexane/ethyl acetate = 99/1 to 85/15) to obtain title compound C-1 (pale yellow oil, 5.0 g, yield 39%).
MS (ESI) m/z = 340 [M+1]⁺
LCMS retention time: 0.968 min (condition C)

### C-2) Synthesis of (5-iodo-1,2-oxazol-3-yl)methanol

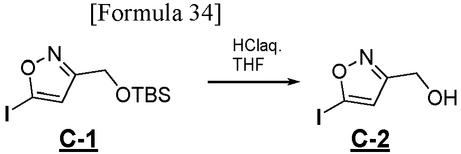

To a THF (22 mL) solution of compound C-1 (2.2 g), 1 mol/L-hydrochloric acid (2.2 mL) was added, and the mixture was stirred at room temperature for 7 hours. To the reaction mixture, a 1 mol/L-aqueous sodium hydroxide solution was added for neutralization, and the mixture was extracted with chloroform. The organic layer was separated using a phase separator and the solvent was concentrated under reduced pressure. The residue was purified by OH type silica gel column chromatography (gradient elution with n-hexane/ethyl acetate = 80/20 to 70/30) to obtain title compound C-2 (colorless solid, 0.49 g, yield 34%).
MS (ESI) m/z = 226 [M+1]⁺
LCMS retention time: 0.496 min (condition B)

### C-3) Synthesis of 5-iodo-3-[(2-{(1S)-1-[(oxan-2-yl)oxy]ethyl}-1H-imidazol-1-yl)methyl]-1,2-oxazole (intermediate C)

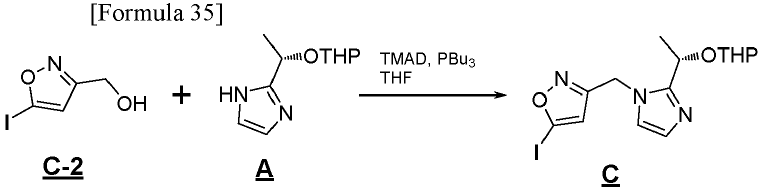

Tributylphosphine (1.2 mL) was added to a THF (15 mL) mixture of compound C-2 (0.95 g), intermediate A (0.95 g), and TMAD (0.84 g), and the mixture was stirred at room temperature for 4 hours. The reaction mixture was diluted with ethyl acetate and water was added. The organic layer was extracted, washed with brine, dried over magnesium sulfate, and concentrated under reduced pressure. The residue was purified by OH type silica gel column chromatography (gradient elution with n-hexane/ethyl acetate = 85/15 to 0/100) to obtain intermediate C (light brown oil, 0.70 g, yield 46%).

The ¹H-NMR and LCMS data are as shown in Table 1.

### Reference Example 4

### (2S)-2-(5-Iodoisoxazol-3-yl)-2-(2-((1S)-1-((tetrahydro-2H-pyran-2-yl)oxy)ethyl)-1H-imidazol-1-yl)ethanol (intermediate D)

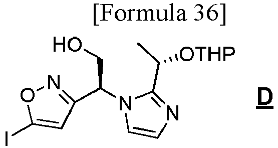

### D-1) Synthesis of methyl (2R)-3-hydroxy-2-(2-{(1S)-1-[(oxan-2-yl)oxy]ethyl}-1H-imidazol-1-yl)propanoate

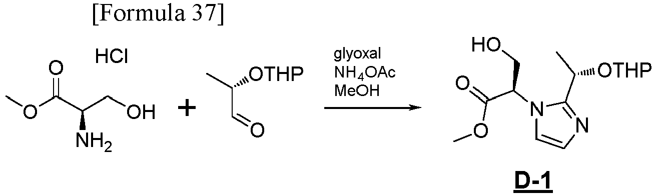

After (2S)-2-[(oxan-2-yl)oxy]propanal (0.76 g) was dissolved in MeOH (11 mL) and an 8.8 mol/L-aqueous glyoxal solution (0.37 mL) was added under ice cooling, methyl D-serinate hydrochloride (0.50 g) was added at room temperature, and the mixture was stirred for 10 min. Furthermore, ammonium acetate (0.25 g) was added, followed by stirring at room temperature for 3 days. After the reaction mixture was concentrated under reduced pressure, the resulting residue was purified by OH type silica gel column chromatography (gradient elution with n-hexane/ethyl acetate = 9/1 to 0/1) to obtain title compound D-1 (pale yellow oil, 0.20 g, yield 21%).
MS (ESI) m/z = 299 [M+1]⁺
LCMS retention time: 0.588 min (condition A)

### D-2) Synthesis of methyl (2R)-3-{[tert-butyl(dimethyl)silyl]oxy}-2-(2-{(1S)-1-[(oxan-2-yl)oxy]ethyl}-1H-imidazol-1-yl)propanoate

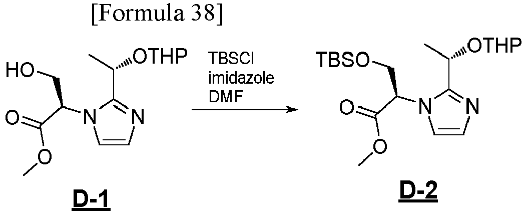

D-1 (0.20 g) was dissolved in DMF (2.2 mL), and imidazole (68 mg) and TBSCl (0.12 g) were added under ice cooling, and then, the mixture was stirred at room temperature for 30 min. Furthermore, imidazole (68 mg) and TBSCl (0.12 g) were added and the mixture was stirred for 30 min. Water was added to the reaction mixture, and the mixture was stirred and then extracted with ethyl acetate. The organic layer was concentrated under reduced pressure and the resulting residue was purified by OH type silica gel column chromatography (gradient elution with n-hexane/ethyl acetate = 9/1 to 4/6) to obtain title compound D-2 (colorless oil, 0.19 g, yield 69%).
MS (ESI) m/z = 413 [M+1]⁺
LCMS retention time: 0.858 min (condition B)

### D-3) Synthesis of N-[(1E,2S)-3-{[tert-butyl(dimethyl)silyl]oxy}-2-(2-{(1S)-1-[(oxan-2-yl)oxy]ethyl}-1H-imidazol-1-yl)propylidene]hydroxylamine

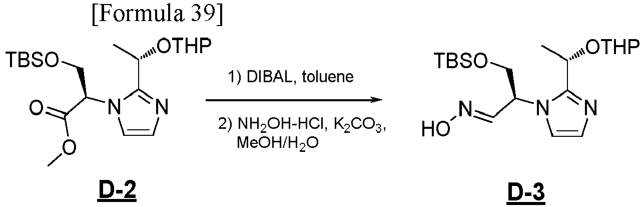

D-2 (0.19 g) was dissolved in toluene (2.3 mL), and a 1.0 mol/L-DIBAL-toluene solution (0.51 mL) was added at -78°C, followed by stirring for 1 hour. Furthermore, after a 1.0 moL/L-DIBAL-toluene solution (0.51 mL) was added and the mixture was stirred for 30 min, MeOH was added to terminate the reaction, and then, the mixture was returned to room temperature, followed by adding water, an aqueous Rochelle's salt solution, and ethyl acetate, and stirring for 1 hour. The filtrate obtained by Celite filtration was separated. The organic layer was dried over magnesium sulfate, filtered, and washed with ethyl acetate, and the resulting filtrate was concentrated under reduced pressure and dried. The resulting residue was dissolved in MeOH (0.92 mL) and water (0.92 mL), hydroxylamine hydrochloride (32 mg) and potassium carbonate (32 mg) were added at room temperature, and the mixture was stirred for 16 hours. The reaction mixture was concentrated under reduced pressure, followed by extraction with chloroform. The aqueous layer was separated by using a phase separator, the organic layer was concentrated under reduced pressure and dried, and the resulting residue was purified by OH type silica gel column chromatography (gradient elution with n-hexane/ethyl acetate = 9/1 to 0/1) to obtain title compound D-3 (colorless oil, 0.16 g, yield 85%).
MS (ESI) m/z = 398 [M+1]⁺
LCMS retention time: 0.776 min (condition B)

### D-4) Synthesis of 3-[(1S)-2-{[tert-butyl(dimethyl)silyl]oxy}-1-(2-{(1S)-1-[(oxan-2-yl)oxy]ethyl}-1H-imidazol-1-yl)ethyl]-5-iodo-1,2-oxazole

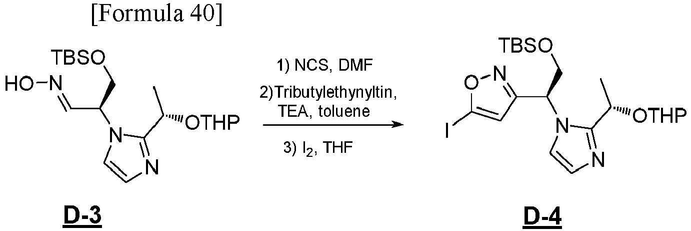

To a DMF (2.0 mL) solution of D-3 (0.16 g), N-chlorosuccinimide (52 mg) was added and stirred in a water bath for 1 hour. Furthermore, N-chlorosuccinimide (52 mg) was added, and the mixture was stirred for 30 min. The reaction mixture was diluted with toluene and washed with water for 3 times. The organic layer was dried over magnesium sulfate, filtered, and then, washed with toluene (50 mL). To the resulting solution, tributyl(ethynyl)tin (0.18 g) and TEA (0.20 mL) were sequentially added, and the mixture was stirred at room temperature for 1.5 hours. Water was added to the reaction mixture to terminate the reaction, and the aqueous layer was extracted with ethyl acetate. The organic layer was dried over magnesium sulfate, and then, filtered, washed with ethyl acetate, and concentrated under reduced pressure. To a THF (1.1 mL) solution of the resulting residue, a THF solution of iodine (0.14 g) was added, and the mixture was stirred at room temperature for 30 min. Furthermore, a THF solution of iodine (0.14 g) was added, and the mixture was stirred for 15 min. To the reaction mixture, an aqueous saturated sodium thiosulfate solution and an aqueous saturated sodium bicarbonate solution were added to terminate the reaction, and the aqueous layer was extracted with ethyl acetate. The organic layer was dried over magnesium sulfate, and then, concentrated under reduced pressure. The resulting residue was purified by OH type silica gel column chromatography (gradient elution with n-hexane/ethyl acetate = 1/0 to 1/1) to obtain title compound D-4 (pale yellow oil, 53 mg, yield 17%).
MS (ESI) m/z = 548 [M+1]⁺
LCMS retention time: 0.957 min (condition B)

### D-5) Synthesis of (2S)-2-(5-iodoisoxazol-3-yl)-2-(2-((1S)-1-((tetrahydro-2H-pyran-2-yl)oxy)ethyl)-1H-imidazol-1-yl)ethanol (intermediate D)

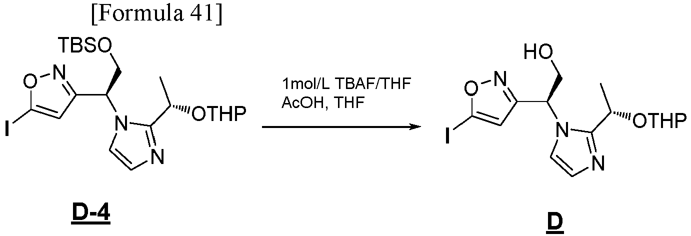

To a THF (1.8 mL) solution of compound D-4 (0.10 g), acetic acid (21 µL) and TBAF (0.37 mL, 1 mol/L-THF solution) were added under ice cooling, and then, stirred at room temperature for 3 hours. The reaction mixture was added to an aqueous saturated ammonium chloride solution and extracted with ethyl acetate for 3 times. The organic layer was washed with brine and dried over magnesium sulfate, and then, the solvent was distilled off under reduced pressure. The residue was purified by OH type silica gel column chromatography (gradient elution with ethyl acetate/methanol = 100/0 to 95/5) to obtain intermediate D (colorless oil, 79 mg, yield 99%).

The ¹H-NMR and LCMS data are as shown in Table 1.

### Reference Example 5

### tert-Butyl [(2R)-2-(5-iodo-1,2-oxazol-3-yl)-2-(2-{(1S)-1-[(oxan-2-yl)oxy]ethyl}-1H-imidazol-1-yl)ethyl]carbamate (intermediate E)

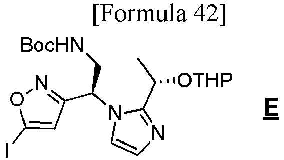

### E-1) Synthesis of methyl (2R)-3-[(tert-butoxycarbonyl)amino]-2-(2-{(1S)-1-[(oxan-2-yl)oxy]ethyl}-1H-imidazol-1-yl)propanoate

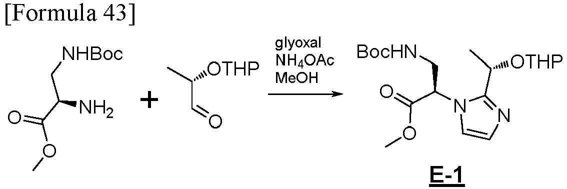

To a methanol (80 mL) solution of (2S)-2-tetrahydropyran-2-yloxypropanal (7.5 g), an 8.8 mol/L-aqueous glyoxal solution (4.2 mL) and a methanol (40 mL) solution of methyl (2R)-2-amino-3-hydroxy-propionate (8.0 g) were added. After the mixture was stirred at room temperature for 10 min, ammonium acetate (2.8 g) was added, and the mixture was stirred at room temperature for 3 days. The reaction mixture was concentrated under reduced pressure, and the resulting residue was purified by OH type silica gel column chromatography (gradient elution with n-hexane/ethyl acetate = 90/10 to 0/100) to obtain title compound E-1 (yellow amorphous, 4.4 g, yield 30%).
MS (ESI) m/z = 398 [M+1]⁺
LCMS retention time: 0.559 min (condition B)

### E-2) Synthesis of tert-butyl [(2R,3E)-3-(hydroxyimino)-2-(2-{(1S)-1-[(oxan-2-yl)oxy]ethyl}-1H-imidazol-1-yl)propyl]carbamate

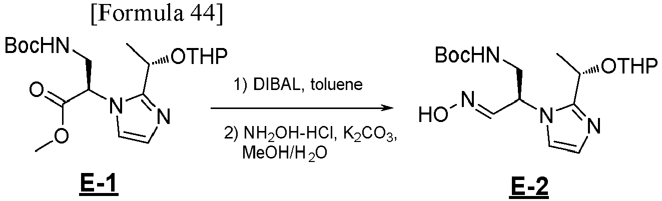

To a toluene (55 mL) solution of compound E-1 (4.4 g), a 1.0 mol/L-DIBAL-toluene solution (44 mL) was added dropwise while maintaining the reaction mixture at -70°C or less. After the reaction mixture was stirred for 15 min, methanol (50 mL) was added while maintaining -67°C or less. Furthermore, a 50% aqueous Rochelle's salt solution (100 mL) and ethyl acetate (50 mL) were added, and the mixture was warmed to room temperature and stirred for 3 hours. The organic layer was extracted with ethyl acetate, dried over sodium sulfate, and concentrated under reduced pressure to obtain a crude product (4.3 g). The resulting crude product was dissolved in methanol (55 mL) and water (55 mL), and hydroxylamine hydrochloride (0.84 g) and potassium carbonate (0.76 g) were added, followed by stirring at room temperature for 16 hours. The reaction mixture was concentrated under reduced pressure, and then extracted with chloroform, separated using a phase separator, and concentrated under reduced pressure. The residue was purified by OH type silica gel column chromatography (gradient elution with n-hexane/ethyl acetate = 90/10 to 0/100) to obtain title compound E-2 (colorless amorphous, 3.2 g, yield 76%).
MS (ESI) m/z = 383 [M+1]⁺
LCMS retention time: 0.478 min (condition B)

### E-3) Synthesis of tert-butyl [(2R)-2-(5-iodo-1,2-oxazol-3-yl)-2-(2-{(1S)-1-[(oxan-2-yl)oxy]ethyl}-1H-imidazol-1-yl)ethyl]carbamate (intermediate E)

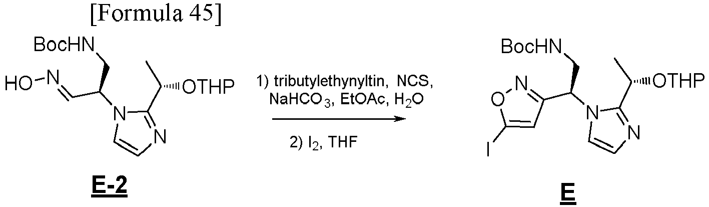

To an ethyl acetate (83 mL) solution of compound E-2 (3.2 g), tributyl(ethynyl)tin (4.8 mL), sodium bicarbonate (2.1 g), and water (8.3 mL) were added at room temperature. After stirring for 10 min, N-chlorosuccinimide (2.2 g) was added in small portions, and the mixture was further stirred for 1 hour. The reaction mixture was diluted with ethyl acetate, separated with an aqueous saturated sodium bicarbonate solution, followed by washing with brine. The organic layer was dried over magnesium sulfate and filtered off, and the filtrate was concentrated under reduced pressure to obtain a crude product. The resulting crude product was dissolved in THF (20 mL), and a THF (22 mL) solution of iodine (5.3 g) was added under ice cooling. After stirring for 1 hour, an aqueous saturated sodium thiosulfate solution and an aqueous saturated sodium bicarbonate solution were added to the reaction mixture, and the mixture was extracted twice with ethyl acetate. The organic layer was dried over sodium sulfate and filtered off, the filtrate was concentrated under reduced pressure, and the residue was purified by OH type silica gel column chromatography (gradient elution with n-hexane/ethyl acetate = 90/10 to 50/50) to obtain intermediate E (pale yellow amorphous, 1.7 g, yield 38%).

The ¹H-NMR and LCMS data are as shown in Table 1.

### Reference Example 6

### 5-(4-Iodophenyl)-3-[(2-{(1S)-1-[(oxan-2-yl)oxy]ethyl}-1H-imidazol-1-yl)methyl]-1,2-oxazole (intermediate F)

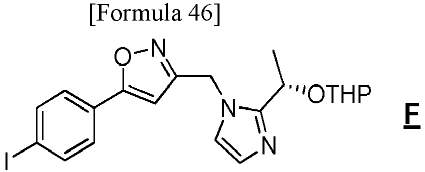

### F-1) Synthesis of [5-(4-iodophenyl)-1,2-oxazol-3-yl]methyl 4-methylbenzene-1-sulfonate

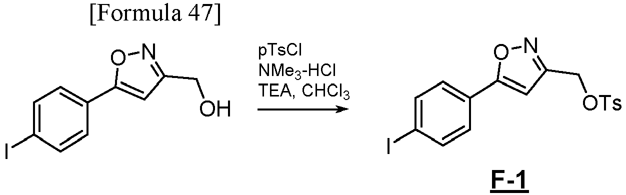

[5-(4-Iodophenyl)isoxazol-3-yl]methanol (5.2 g) was suspended in chloroform (60 mL) and ice-cooled, and TEA (3.6 mL), trimethylamine hydrochloride (0.41 g), and p-toluenesulfonyl chloride (3.6 g) were sequentially added. After the mixture was stirred for 1.5 hours under ice cooling, water was added to the reaction mixture, and the mixture was extracted with chloroform, dried over magnesium sulfate, separated, and concentrated under reduced pressure to obtain title compound F-1 (colorless solid, 7.3 g, yield 93%).
MS (ESI) m/z = 478 [M+23]⁺
LCMS retention time: 1.309 min (condition B)

### F-2) Synthesis of 5-(4-iodophenyl)-3-[(2-{(1S)-1-[(oxan-2-yl)oxy]ethyl}-1H-imidazol-1-yl)methyl]-1,2-oxazole (intermediate F)

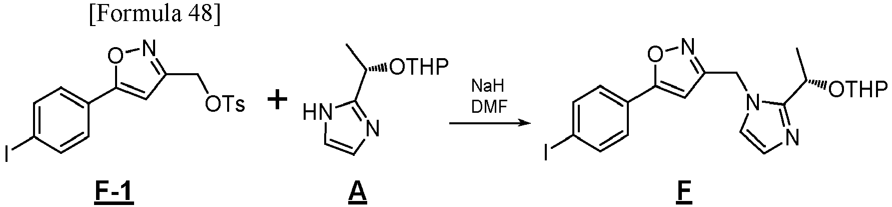

A DMF (50 mL) solution of intermediate A was ice-cooled, 60% sodium hydride (0.77 g) was added under a nitrogen flow, and the mixture was stirred for 20 min. Subsequently, a DMF (70 mL) solution of F-1 (7.3 g) was added dropwise. The mixture was gradually warmed to room temperature and stirred for 1.5 hours. To the reaction mixture, an aqueous saturated sodium bicarbonate solution was added, the mixture was extracted with ethyl acetate, which was further subjected to separation operation twice with an aqueous saturated sodium bicarbonate solution. The organic layer was dried over magnesium sulfate and separated, ISOLUTE(registered trademark) HM-N was added, and the solvent was concentrated under reduced pressure. The residue was purified twice by OH type silica gel column chromatography (gradient elution with chloroform/methanol = 99/1 to 93/7) to obtain a crude product. This was dissolved in ethyl acetate, which was subjected to separation operation three times with an aqueous saturated sodium bicarbonate solution, the organic layer was dried over magnesium sulfate and separated, and the solvent was concentrated under reduced pressure to obtain intermediate F (light brown syrup, 7.3 g, yield 95%).

The ¹H-NMR and LCMS data are as shown in Table 1.

### Reference Example 7

### tert-Butyl [(2R)-2-[5-(4-iodophenyl)-1,2-oxazol-3-yl]-2-(2-{(1S)-1-[(oxan-2-yl)oxy]ethyl}-1H-imidazol-1-yl)ethyl]carbamate (intermediate G)

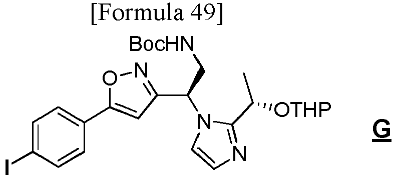

### G-1) Synthesis of tert-butyl [(2R)-2-[5-(4-iodophenyl)-1,2-oxazol-3-yl]-2-(2-{(1S)-1-[(oxan-2-yl)oxy]ethyl}-1H-imidazol-1-yl)ethyl]carbamate (intermediate G)

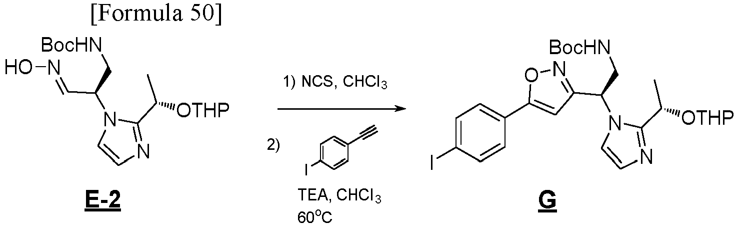

To a chloroform (30 mL) solution of compound E-2 (2.0 g), N-chlorosuccinimide (0.83 g) was added and stirred at room temperature for 1.5 hours (solution A).

On the other hand, TEA (2.2 mL) was added to a chloroform (30 mL) solution of 1-ethynyl-4-iodobenzene (5.9 g), and solution A was added dropwise in small portions over 1.5 hours while heating and stirring the mixture in an oil bath at 62°C. After completing the dropwise addition, the mixture was stirred for 5 min as it was. The reaction mixture was left to cool, and then diluted with chloroform, ISOLUTE(registered trademark) HM-N was added, and the solvent was concentrated under reduced pressure. The residue was purified by OH type silica gel column chromatography (gradient elution with chloroform/methanol = 100/0 to 93/7) to obtain intermediate G (pale yellow amorphous, 1.9 g, yield 60%).

The ¹H-NMR and LCMS data are as shown in Table 1.

### Reference Example 8

### tert-Butyl ((R)-2-(2-((S)-1-((tert-butyldimethylsilyl)oxy)ethyl)-1H-imidazol-1-yl)-2-(5-(4-iodophenyl)isoxazol-3-yl)ethyl)carbamate (intermediate H)

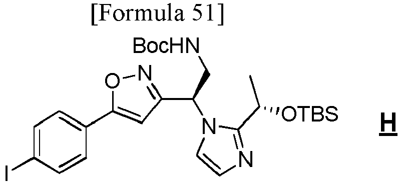

### H-1) Synthesis of tert-butyl {(2R)-2-{2-[(1S)-1-hydroxyethyl]-1H-imidazol-1-yl}-2-[5-(4-iodophenyl)-1,2-oxazol-3-yl]ethyl}carbamate

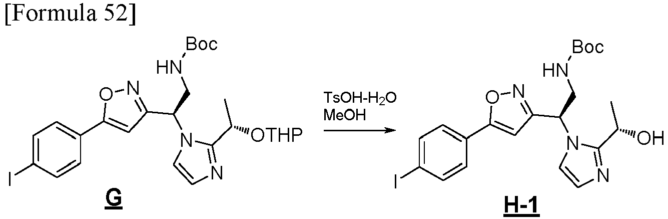

To a MeOH (5.0 mL) solution of intermediate G (0.22 g), pTsOH-H₂O (0.21 g) was added and stirred at room temperature for 3 hours. To the reaction mixture, a 20% aqueous potassium carbonate solution was added, the mixture was extracted with chloroform and separated using a phase separator, and the solvent was concentrated under reduced pressure to obtain title compound H-1 (light brown amorphous, 0.18 g, yield 97%).
MS (ESI) m/z = 525 [M+1]⁺
LCMS retention time: 0.738 min (condition B)

### H-2) Synthesis of tert-butyl ((R)-2-(2-((S)-1-((tert-butyldimethylsilyl)oxy)ethyl)-1H-imidazol-1-yl)-2-(5-(4-iodophenyl)isoxazol-3-yl)ethyl)carbamate (intermediate H)

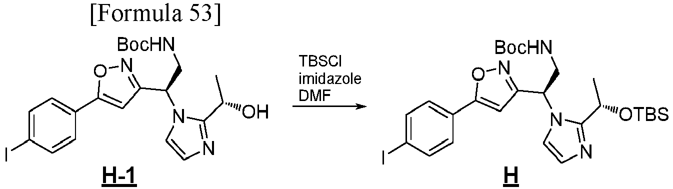

Compound H-1 (0.18 g) was dissolved in DMF (3.0 mL), and then, imidazole (74 mg) and TBSCl (82 mg) were sequentially added under ice cooling, followed by stirring at room temperature for 45 min. To the reaction mixture, an aqueous saturated sodium bicarbonate solution was added, and the mixture was extracted with EtOAc, which was washed twice with an aqueous saturated sodium bicarbonate solution. To the organic layer, ISOLUTE(registered trademark) HM-N was added, and the solvent was concentrated under reduced pressure. The residue was purified by OH type silica gel column chromatography (gradient elution with n-hexane/ethyl acetate = 70/30 to 0/100) to obtain intermediate H (pale yellow oil, 0.12 g, yield 50%).

The ¹H-NMR and LCMS data are as shown in Table 1.

### Reference Example 9

### (4-Iodophenyl)-5-[(2-{(1S)-1-[(oxan-2-yl)oxy]ethyl}-1H-imidazol-1-yl)methyl]-1,2-oxazole (intermediate I)

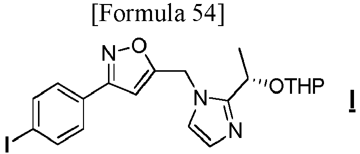

### I-1) Synthesis of (4-iodophenyl)-5-[(2-{(1S)-1-[(oxan-2-yl)oxy]ethyl}-1H-imidazol-1-yl)methyl]-1,2-oxazole (intermediate I)

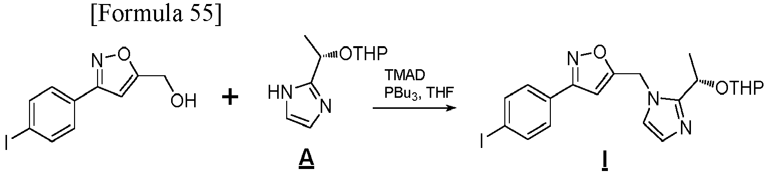

To a THF (4.7 mL) solution of [3-(4-iodophenyl)-1,2-oxazol-5-yl]methanol (0.35 g), compound A (0.30 g), and TMAD (0.26 g), tributylphosphine (0.37 mL) was added at room temperature. After stirring at room temperature for 22 hours, the reaction mixture was diluted with ethyl acetate, and sequentially washed with water and brine. After the organic layer was dried over magnesium sulfate, the solvent was distilled off under reduced pressure. The residue was sequentially purified by NH type silica gel column chromatography (gradient elution with n-hexane/ethyl acetate = 80/20 to 0/100) and OH type silica gel column chromatography (gradient elution with n-hexane/ethyl acetate = 30/70 to 0/100) to obtain intermediate I (pale yellow oil, 0.36 g, yield 65%).

The ¹H-NMR and LCMS data are as shown in Table 1.

### Reference Example 10

### tert-Butyl (1R,5S,6s)-6-ethynyl-3-azabicyclo[3.1.0]hexan-3-carboxylate (intermediate J)

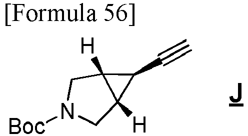

### J-1) Synthesis of tert-butyl (1R,5S,6s)-6-ethynyl-3-azabicyclo[3.1.0]hexan-3-carboxylate (intermediate J)

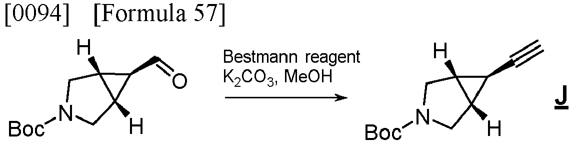

To a methanol (7.1 mL) mixture of tert-butyl (1R,5S,6r)-6-formyl-3-azabicyclo[3.1.0]hexan-3-carboxylate (0.30 g) and potassium carbonate (0.39 g), the Bestmann reagent (0.26 mL) was added at room temperature. After stirring at room temperature for 3 hours, the reaction mixture was poured into water, which was extracted three times with chloroform. The organic layer was washed with brine and dried over magnesium sulfate, and then, the solvent was distilled off under reduced pressure. The residue was purified by OH type silica gel column chromatography (gradient elution with n-hexane/ethyl acetate = 95/5 to 40/60) to obtain intermediate J (colorless solid, 0.26 g, yield 87%).

The ¹H-NMR and LCMS data are as shown in Table 1.

### Reference Example 11

### (1R,5S,6s)-6-Ethynyl-3-azabicyclo[3.1.0]hexane hydrochloride (intermediate K)

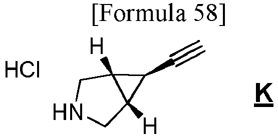

### K-1) Synthesis of (1R,5S,6s)-6-ethynyl-3-azabicyclo[3.1.0]hexane hydrochloride (intermediate K)

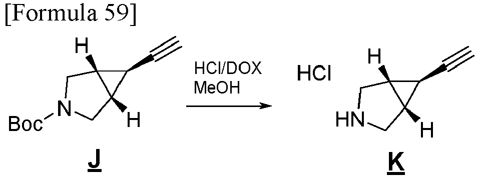

To a MeOH(2.0 mL)/1,4-dioxane (10 mL) solution of compound J (2.0 g), a 4 mol/L hydrochloric acid-dioxane solution (20 mL) was added under ice cooling, and the mixture was stirred at room temperature for 1 hour. The reaction mixture was concentrated under reduced pressure and dried, and the resulting residue was suspended in ethyl acetate, which was stirred and collected by filtration to obtain intermediate K (brown solid, 1.3 g, yield 95%).

The ¹H-NMR and LCMS data are as shown in Table 1.

Table 1 shows the structural formulas, ¹H-NMR data, MS (m/z) data, LCMS retention times (min), and methods of intermediates A to K.

**[Table 1-1]**

| Intermediate | Structural formula | ¹H-NMR | MS (m/z) | Retention time Method |
|---|---|---|---|---|
| A | 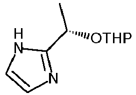 | ¹H NMR (600 MHz, CHLOROFORM-d) δ ppm 1.47 - 1.93 (m, 9 H), 3.50 - 3.57 (m, 1 H), 3.92 - 4.00 (m, 1 H), [4.55 - 4. 59]4. 68 - 4.72 (m, 1 H), [4.84 - 4. 89]5. 02 - 5.08 (m, 1 H), 6.94 - 7.09 (m, 2 H), [9.20 - 9. 45] 9. 83 - 10.05 (m, 1 H) | ESI 197[M+1]⁺ | 0.228 (B) |
| B | 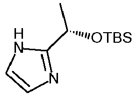 | ¹H NMR (400 MHz, CHLOROFORM-d) δ ppm 0.07 (s, 3 H), 0.13 (s, 3 H), 0.93 (s, 9 H), 1.52 (d, J=6.4 Hz, 3 H), 5.05 (q, J=6.2 Hz, 1 H), 6.98 (br s, 2 H), 9.11 (br s, 1 H) | ESI 227[M+1]⁺ | 0.575 (B) |
| C | 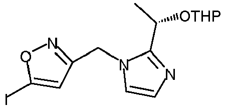 | ¹H NMR (400 MHz, CHLOROFORM-d) δ ppm 1. 34 - 1.69 (m, 8 H), 1.74 - 1.86 (m, 1 H), 3.41 - 3.56 (m, 1 H), 3.79 - 3.99 (m, 1 H), 4.12 (q, J=7.2 Hz, 1 H), 5.01 - 5.15 (m, 1 H), 5. 28 - 5.48 (m, 2 H), 6. 21 - 6.40 (m, 1 H), 6.83 - 6.90 (m, 1 H). 6. 97 - 7. 06 (m. 1 H) | ESI 404[M+1]⁺ | 0.661 (A) |
| D | 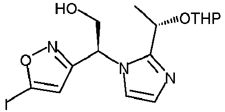 | ¹H NMR (400 MHz, CHLOROFORM-d) δ ppm 1.39 - 1.91 (m, 9 H), 3.42 - 3.58 (m, 1 H), 3.81 - 3.96 (m, 1 H), 4. 16 - 4.35 (m, 2 H), 4.50 - 4.77 (m, 1 H), 5.07 - 5.19 (m, 1 H), 5.88 - 6.12 (m, 1 H), 6.14 - 6.39 (m, 1 H), 6.93 - 7.08 (m, 2 H) | ESI 434[M+1]⁺ | 0.817 (A) |
| E | 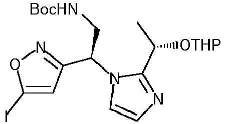 | ¹H NMR (400 MHz, CHLOROFORM-d) δ ppm 1. 35 - 1.41 (m, 9 H), 1.44 - 1.87 (m, 9 H), 3.42 - 3.56 (m, 1 H), 3.79 - 4.02 (m, 3 H), 4.46 - 4.52 (m, 0.3 H), 4.70 (br d, J=5. 1 Hz, 0.7 H), 5. 04 - 5.27 (m, 2 H), 5.95 (dd, J=8.8, 6. 1 Hz, 0.3 H), 6.05 - 6.12 (m, 0.7 H), 6.19 (s, 0.3 H), 6.37 (s, 0.7 H), 6.91 (s, 0.7 H), 6.96 (s, 0.3 H), 7.01 (s, 0.7 H), 7.05 (s, 0.3 H) | ESI 533[M+1]⁺ | 0.712 (B) |
| F | 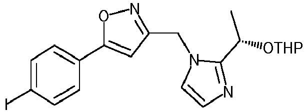 | ¹H NMR (400 MHz, CHLOROFORM-d) δ ppm 1.41 - 1.56 (m, 4 H), 1.62 - 1.70 (m, 3 H), 1.71 - 1.88 (m, 2 H), 3.42 - 3.59 (m, 1 H), 3.80 - 3.96 (m, 1 H), 4.47 - 4.76 (m, 1 H), 5.06 - 5.19 (m, 1 H), 5.32 - 5.51 (m, 2 H), 6.22 - 6.43 (m, 1 H), 6.92 (s, 1 H), 6.99 - 7.08 (m, 1 H), 7.45 (d, J=8.1 Hz, 2 H), 7.81 (d, J=8.1 Hz, 2 H) | ESI 480[M+1]⁺ | 0.765 (B) |

**[Table 1-2]**

| Intermediate | Structural formula | ¹H-NMR | MS (m/z) | Retention time Method |
|---|---|---|---|---|
| G | 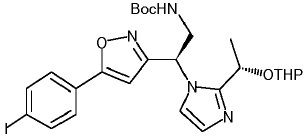 | ¹H NMR (600 MHz, CHLOROFORM-d) δ ppm 1. 37 - 1.43 (m, 9 H), 1.44 - 1. 56 (m, 4 H), 1.65 - 1.74 (m, 3 H), 1.75 - 1.87 (m, 2 H), 3.46 - 3.59 (m, 1 H), 3.85 - 3.96 (m, 2 H), 3.97 - 4.08 (m, 1 H), 4.50 - 4.77 (m, 1 H), 5.05 - 5.35 (m, 2 H), 5.90 - 6.14 (m, 1 H), 6.20 - 6.44 (m, 1 H), 6.87 - 7.10 (m, 2 H), 7.40 - 7.47 (m, 2 H), 7.77 - 7.85 (m. 2 H) | ESI 609[M+1]⁺ | 0.978 (B) |
| H | 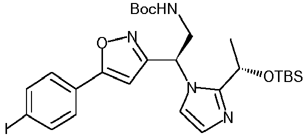 | ¹H NMR (400 MHz, CHLOROFORM-d) δ ppm 0.01 (s, 3 H), 0.11 (s, 3 H), 0.81 (s, 9 H), 1.41 (s, 9 H), 1.66 (d, J=6.5 Hz, 3 H), 3.70 - 3.89 (m, 1 H), 3.99 - 4. 19 (m, 1 H), 4.98 (br s, 1 H), 5.29 (br d, J=6.8 Hz, 1 H), 6.12 - 6.30 (m, 2 H), 6.89 (s, 1 H), 6.98 (s, 1 H), 7.41 (d, J=7.8 Hz, 2 H), 7.80 (d, J=7.8 Hz. 2 H) | ESI 639[M+1]⁺ | 1.080 (B) |
| I | 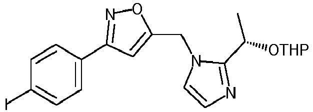 | ¹H NMR (400 MHz, CHLOROFORM-d) δ ppm 1.42 - 1.86 (m, 9 H), 3.43 - 3. 57 (m, 1 H), 3.82 - 3.95 (m, 1 H), 4.47 (br s, 0.75 H), 4.69 (br d, J=5. 6 Hz, 0.25 H), 5.08 - 5. 19 (m, 1 H), 5.41 - 5.67 (m, 2 H), 6.26 - 6.34 (m, 1 H), 6.94 - 7.09 (m, 2 H), 7.48 (m, J=8.3 Hz, 2 H), 7.80 (m, J=8. 3 Hz, 2 H) | ESI 480[M+11' | 0.761 (B) |
| J | 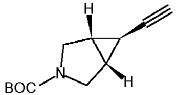 | ¹H NMR (400 MHz, CHLOROFORM-d) δ ppm 1.11 (br s, 1 H), 1.43 (s, 9 H), 1.82 (br s, 2 H), 1.88 (s, 1 H), 3.35 (br s, 2 H), 3.47 - 3.61 (m, 1 H), 3.61 - 3.72 (m, 1 H) | ESI/APCI 230 [M+23]⁺ | 0.200 (E) |
| K | 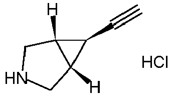 | ¹H NMR (400 MHz, METHANOL-d₄) δ ppm 1.37 - 1.48 (m, 1 H), 2.13 (s, 2 H), 2.29 - 2.36 (m, 1 H), 3.41 - 3.51 (m, 4 H) | ESI/APCI 108[M+1]⁺ | 0.385 (E) |

The methods of producing the compounds of the present invention will be described in detail by way of the following Examples.

### Example 1

### 5-{4-[3-({2-[(1S)-1-Hydroxyethyl]-1H-imidazol-1-yl}methyl)-1,2-oxazol-5-yl]phenyl}pent-4-yn-1-ol (compound 1)

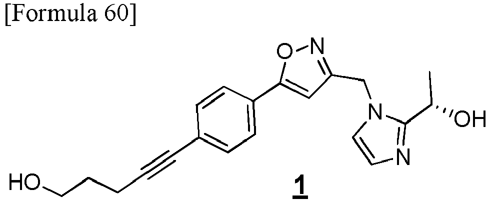

### 1-1) Synthesis of tert-butyl((5-(4-iodophenyl)pent-4-yn-1-yl)oxy)dimethylsilane

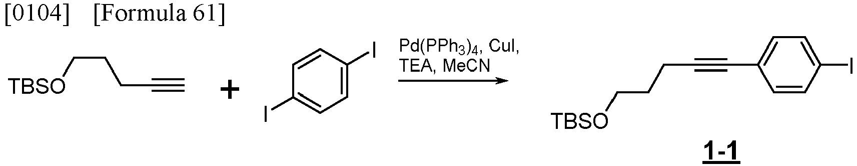

1,4-Diiodobenzene (5.0 g), copper iodide (I) (96 mg), and tetrakis(triphenylphosphine)palladium(0) (0.29 g) were weighed into a flask, the system was replaced with nitrogen, and then, TEA (50 mL) was added. To the resulting suspension, an acetonitrile (5.0 mL) solution of tert-butyl(dimethyl)[(pent-4-yn-1-yl)oxy]silane (1.0 g) was added, and thereafter, the mixture was stirred at 50°C for 2 hours. After the reaction mixture was filtered through Celite to remove the insolubles, the filtrate was concentrated under reduced pressure. The residue was purified by OH type silica gel column chromatography (gradient elution with n-hexane/chloroform = 85/15 to 70/30) to obtain title compound 1-1 (colorless oil, 1.4 g, yield 71%).
MS (ESI/APCI) m/z = 401 [M+1]⁺
LCMS retention time: 0.200 min (condition E)

### 1-2) Synthesis of tert-butyldimethyl((5-(4-(4,4,5,5-tetramethyl-1,3,2-dioxaborolan-2-yl)phenyl)pent-4-yn-1-yl)oxy)silane

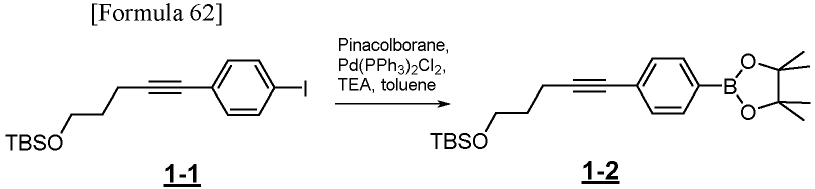

Compound 1-1 (0.30 g) and PdCl₂(PPh₃)₂ (53 mg) were weighed into a three-neck flask, the system was replaced with nitrogen, and toluene (7.5 mL) was added. To the resulting mixture, TEA (2.1 mL) and 4,4,5,5-tetramethyl-1,3,2-dioxaborolane (0.27 mL) were sequentially added, and the mixture was stirred at 100°C for 1 hour. To the reaction mixture, an aqueous saturated ammonium chloride solution was added to terminate the reaction, followed by extraction twice with ethyl acetate. After the organic layer was dried over magnesium sulfate, the solvent was concentrated under reduced pressure. The residue was purified by OH type silica gel column chromatography (gradient elution with n-hexane/ethyl acetate = 100/0 to 90/10) to obtain title compound 1-2 (0.17 g, 56%). ¹H NMR (400 MHz, CHLOROFORM-d) δ ppm 0.06 - 0.09 (m, 6 H), 0.90 - 0.93 (m, 12 H), 1.34 (s, 9 H), 1.75 - 1.84 (m, 2 H), 2.46 - 2.53 (m, 2 H), 3.72 - 3.79 (m, 2 H), 7.14 - 7.21 (m, 2 H), 7.35 - 7.40 (m, 2 H)

### 1-3) Synthesis of 5-{4-[3-({2-[(1S)-1-hydroxyethyl]-1H-imidazol-1-yl}methyl)-1,2-oxazol-5-yl]phenyl}pent-4-yn-1-ol (compound 1)

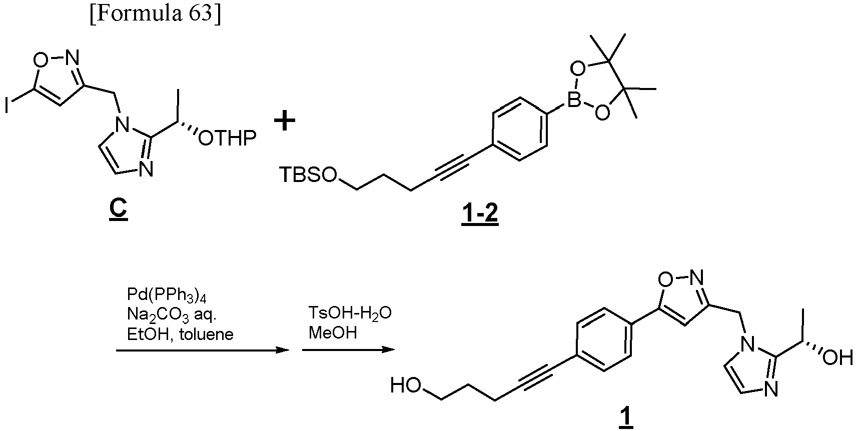

Compound C (90 mg), compound 1-2 (0.11 g), and tetrakis(triphenylphosphine)palladium(0) (26 mg) were weighed into a three-neck flask, the system was replaced with nitrogen, and toluene (0.45 mL), ethanol (0.22 mL), and a 2 mol/L-aqueous sodium carbonate solution (0.22 mL) were added. After the mixture was stirred at 80°C for 2 hours, tetrakis(triphenylphosphine)palladium(0) (26 mg) was added, and the mixture was further stirred at 80°C for 2 hours. The reaction mixture was allowed to cool, and then poured into water, followed by extraction twice with ethyl acetate. After the organic layer was washed with brine and dried over magnesium sulfate, the solvent was distilled off under reduced pressure. The residue was purified by OH type silica gel column chromatography (gradient elution with chloroform/methanol = 100/0 to 95/5), and to a methanol (1.1 mL) solution of the resulting crude product, pTsOH-H₂O (0.11 g) was added, and then, the mixture was stirred for 16 hours. The reaction mixture was poured into an aqueous saturated sodium bicarbonate solution, which was extracted twice with a chloroform/methanol mixed solvent (95:5). After the organic layer was dried over magnesium sulfate, the solvent was distilled off under reduced pressure. The residue was purified by NH type silica gel column chromatography (gradient elution with ethyl acetate/methanol = 100/0 to 90/10) to obtain title compound 1 (pale yellow oil, 15 mg, yield 19%).

The ¹H-NMR and LCMS data are as shown in Table 2.

### Example 2

### (1S)-1-{1-[(5-{4-[(1-Aminocyclopropyl)ethynyl]phenyl}-1,2-oxazol-3-yl)methyl]-1H-imidazol-2-yl}ethan-1-ol (compound 4)

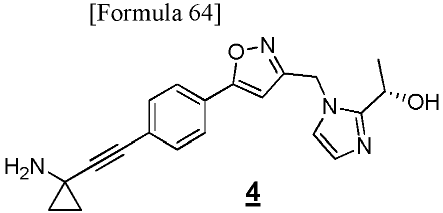

### 4-1) Synthesis of 2,2,2-trifluoro-N-{1-[(4-{3-[(2-{(1S)-1-[(oxan-2-yl)oxy]ethyl}-1H-imidazol-1-yl)methyl]-1,2-oxazol-5-yl}phenyl)ethynyl]cyclopropyl}acetamide

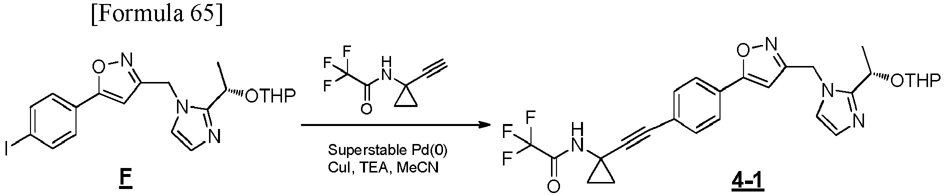

To an acetonitrile (15 mL) solution of intermediate F (0.70 g), Superstable Pd(0) (0.15 g), copper iodide (28 mg), and TEA (1.0 mL), an acetonitrile (2.0 mL) solution of N-(1-ethynylcyclopropyl)-2,2,2-trifluoroacetamide (0.41 g) was added dropwise at room temperature under a nitrogen atmosphere. After stirring at room temperature for 1.5 hours, the reaction mixture was diluted with ethyl acetate and filtered through Celite and NH type silica gel, and then, the solvent was distilled off under reduced pressure. The residue was purified by NH type silica gel column chromatography (gradient elution with n-hexane/ethyl acetate = 75/25 to 0/100) to obtain title compound 4-1 (pale yellow amorphous, 0.68 g, yield 88%).
MS (ESI) m/z = 529 [M+1]⁺
LCMS retention time: 0.720 min (condition B)

### 4-2)1-[(4-{3-[(2-{(1S)-1-[(Oxan-2-yl)oxy]ethyl}-1H-imidazol-1-yl)methyl]-1,2-oxazol-5-yl}phenyl)ethynyl]cyclopropan-1-amine

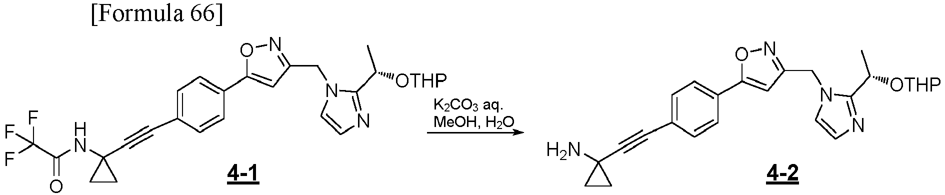

To a methanol (25 mL) solution of compound 4-1 (0.68 g), a 20% aqueous potassium carbonate solution (5.4 g) was added, and the mixture was stirred at room temperature for 19 hours. Furthermore, a 20% aqueous potassium carbonate solution (5.4 g) was added, and the mixture was stirred at room temperature for 4 hours. Water was added to the reaction mixture, and the mixture was extracted with chloroform. After the organic layer was separated using a phase separator, the solvent was distilled off under reduced pressure. The residue was purified by NH type silica gel column chromatography (gradient elution with chloroform/methanol = 100/0 to 94/6) to obtain title compound 4-2 (pale yellow syrup, 0.53 g, yield 95%).
MS (ESI) m/z = 433 [M+1]⁺
LCMS retention time: 0.686 min (condition A)

### 4-3) Synthesis of (1S)-1-{1-[(5-{4-[(1-aminocyclopropyl)ethynyl]phenyl}-1,2-oxazol-3-yl)methyl]-1H-imidazol-2-yl}ethan-1-ol (compound 4)

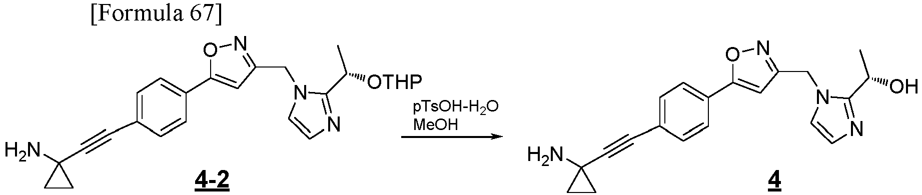

To a methanol (8.5 mL) solution of compound 4-2 (0.37 g), pTsOH-H₂O (0.65 g) was added, and the mixture was stirred at room temperature for 2 hours. To the reaction mixture, a 20% aqueous potassium carbonate solution was added, and the mixture was extracted with chloroform. The organic layer was separated using a phase separator, ISOLUTE(registered trademark) HM-N was added, and the solvent was concentrated under reduced pressure. The residue was purified by NH type silica gel column chromatography (gradient elution with chloroform/methanol = 100/0 to 90/10) to obtain title compound 4 (colorless amorphous, 0.27 g, yield 90%).

The ¹H-NMR and LCMS data are as shown in Table 2.

### Example 3

### (1S)-1-(1-{[5-(4-{[(1R,5S,6s)-3-Azabicyclo[3.1.0]hexan-6-yl]ethynyl}phenyl)-1,2-oxazol-3-yl]methyl}-1H-imidazol-2-yl)ethan-1-ol (compound 10)

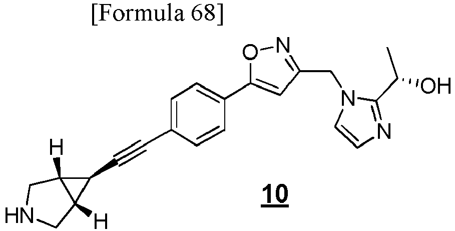

### 10-1) Synthesis of tert-butyl (1R,5S,6s)-6-[(4-{3-[(2-{(1S)-1-[(oxan-2-yl)oxy]ethyl}-1H-imidazol-1-yl)methyl]-1,2-oxazol-5-yl}phenyl)ethynyl]-3-azabicyclo[3.1.0]hexan-3-carboxylate

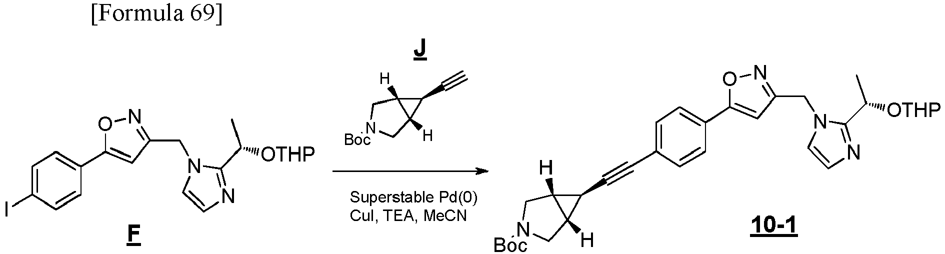

To an acetonitrile (2.5 mL) solution of intermediate F (0.12 g), Superstable Pd(0) (27 mg), copper iodide (5 mg), and TEA (0.17 mL), an acetonitrile (1.0 mL) solution of intermediate J was added dropwise at room temperature under a nitrogen atmosphere. After stirring at room temperature for 1.5 hours, the reaction mixture was diluted with ethyl acetate and filtered through Celite and NH type silica gel, and then, the solvent was distilled off under reduced pressure. The residue was purified by NH type silica gel column chromatography (gradient elution with n-hexane/ethyl acetate = 50/50 to 0/100 to gradient elution with chloroform/methanol = 99/1 to 95/5) to obtain title compound 10-1 (colorless amorphous, 0.12 g, yield 88%).
MS (ESI) m/z = 559 [M+1]⁺
LCMS retention time: 0.923 min (condition B)

### 10-2) Synthesis of (1S)-1-(1-{[5-(4-{[(1R,5S,6s)-3-azabicyclo[3.1.0]hexan-6-yl]ethynyl}phenyl)-1,2-oxazol-3 -yl]methyl}-1H-imidazol-2-yl)ethan-1-ol (compound 10)

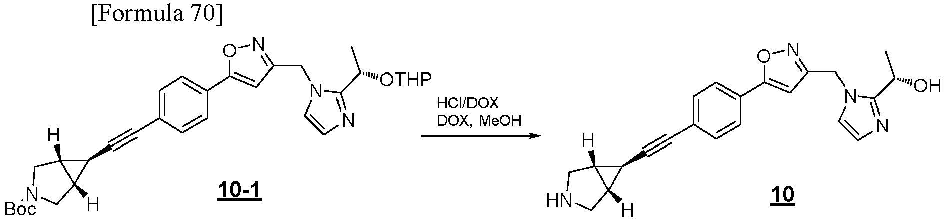

To compound 10-1 (0.12 g), DOX (2.0 mL), methanol (0.40 mL), and a 4 mol/L-hydrochloric acid-DOX solution (1.0 mL) were added, and the mixture was stirred at room temperature for 100 min. After the reaction mixture was concentrated under reduced pressure, a 20% aqueous potassium carbonate solution was added, and the mixture was extracted with chloroform. The organic layer was separated using a phase separator, ISOLUTE(registered trademark) HM-N was added, and the solvent was concentrated under reduced pressure. The residue was purified by NH type silica gel column chromatography (gradient elution with chloroform/methanol = 99/1 to 90/10) to obtain title compound 10 (colorless solid, 49 mg, yield 61%).

The ¹H-NMR and LCMS data are as shown in Table 2.

### Example 4

### (2S)-3-[(1R,5S,6R)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]propan-1,2-diol (compound 11)

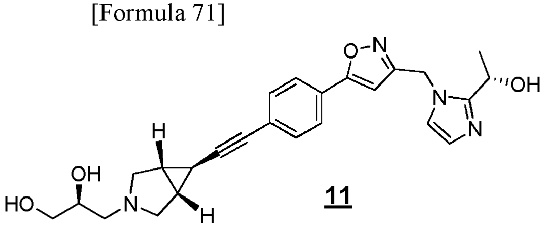

### 11-1) Synthesis of (2S)-3-[(1R,5S, 6R)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1,2-diol (compound 11)

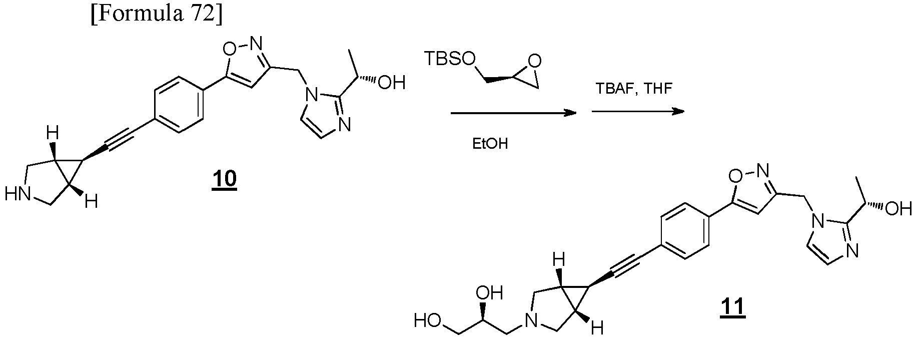

To an ethanol (1.0 mL) suspension of compound 10 (29 mg), (S)-glycidoxy-tert-butyldimethylsilane (73 mg) was added, and the mixture was stirred at 60°C for 4.5 hours. After allowing to cool, the reaction mixture was concentrated under reduced pressure, and the residue was purified by NH type silica gel column chromatography (gradient elution with chloroform/methanol = 99/1 to 95/5) to obtain a crude product (39 mg). The resulting crude product (39 mg) was dissolved in THF (1.0 mL), a THF solution (93 µL) of 1 mol/L TBAF was added, and the mixture was stirred at room temperature for 40 minutes. To the reaction mixture, a 20% aqueous potassium carbonate solution was added, and the mixture was extracted with a chloroform/methanol mixed solvent. The organic layer was separated using a phase separator, ISOLUTE(registered trademark) HM-N was added, and the solvent was concentrated under reduced pressure. The residue was purified by NH type silica gel column chromatography (gradient elution with chloroform/methanol = 99/1 to 90/10) to obtain title compound 11 (colorless amorphous, 29 mg, yield 83%).

The ¹H-NMR and LCMS data are as shown in Table 2.

### Example 5

### (1S)-1-{1-[(5-{4-[(1-Methylazetidin-3-yl)ethynyl]phenyl}-1,2-oxazol-3-yl)methyl]-1H-imidazol-2-yl}ethan-1-ol (compound 12)

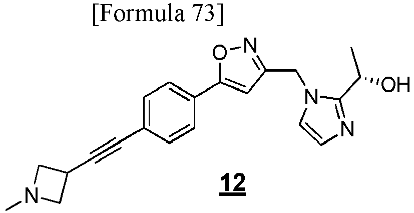

### 12-1) Synthesis of (1S)-1-{1-[(5-{4-[(azetidin-3-yl)ethynyl]phenyl}-1,2-oxazol-3-yl)methyl]-1H-imidazol-2-yl}ethan-1-ol

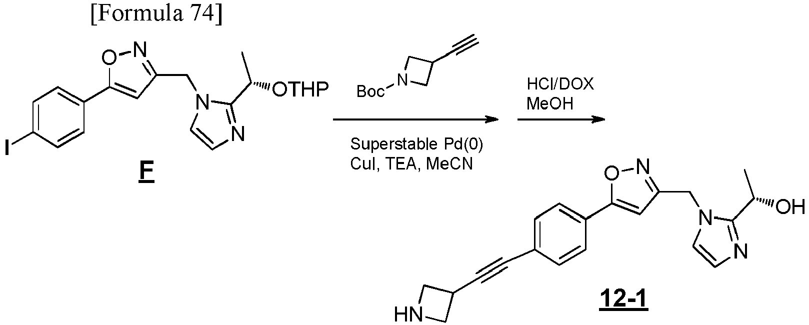

Compound 12-1 was produced by the same methods as in 10-1 and 10-2 with the use of the corresponding starting materials.
MS (ESI) m/z = 175 [M+2]²⁺
LCMS retention time: 0.362 min (condition A)

### 12-2) Synthesis of (1S)-1-{1-[(5-{4-[(1-Methylazetidin-3-yl)ethynyl]phenyl}-1,2-oxazol-3-yl)methyl]-1H-imidazol-2-yl}ethan-1-ol (compound 12)

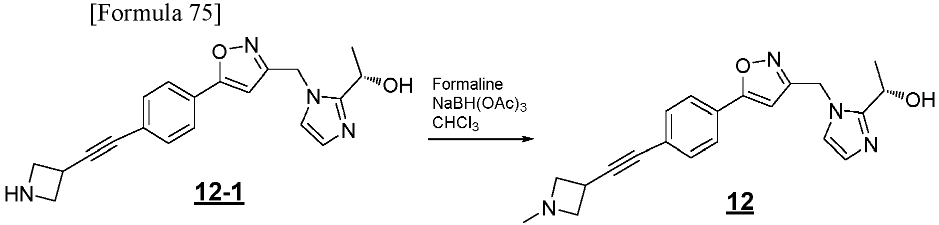

To a chloroform (0.80 mL) solution of compound 12-1 (14 mg), a 37% aqueous formaldehyde solution (12 µL) and sodium triacetoxyborohydride (17 mg) were added, and the mixture was stirred at room temperature overnight. Furthermore, a 37% aqueous formaldehyde solution (12 µL) and sodium triacetoxyborohydride (17 mg) were added, and the mixture was stirred at room temperature for 1 hour. The reaction mixture was poured into an aqueous saturated sodium bicarbonate solution, and the mixture was extracted with chloroform. After the organic layer was separated using a phase separator, the solvent was distilled off under reduced pressure. The residue was purified by OH type silica gel column chromatography (gradient elution with chloroform/methanol = 100/0 to 88/12) to obtain title compound 12 (colorless solid, 12 mg, yield 82%).

The ¹H-NMR and LCMS data are as shown in Table 2.

### Example 6

### (2S)-3-[(1R,5S,6R)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]propan-1,2-diol (compound 13)

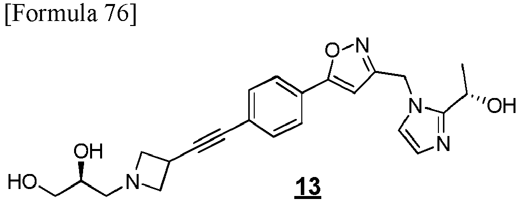

### 13-1) Synthesis of (1S)-1-{1-[(5-{4-[(azetidin-3-yl)ethynyl]phenyl}-1,2-oxazol-3-yl)methyl]-1H-imidazol-2-yl}ethan-1-ol

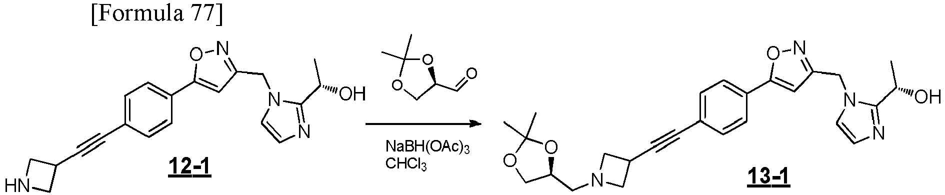

Compound 13-1 was produced by the same methods as in 12-2 with the use of the corresponding starting materials.
MS (ESI) m/z = 463 [M+1]⁺
LCMS retention time: 0.623 min (condition A)

### 13-2) Synthesis of (2S)-3-[(1R,5S,6R)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1,2-diol (compound 13)

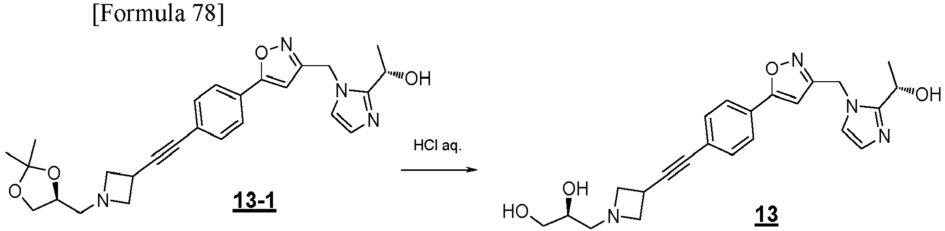

After compound 13-1 (10 mg) was dissolved in 1 mol/L-hydrochloric acid (1.0 mL), 1 mol/L-hydrochloric acid (1.0 mL) was further added, and the mixture was stirred at room temperature for 3 hours. To the reaction mixture, a 1 mol/L-aqueous sodium hydroxide solution and an aqueous saturated potassium carbonate solution were added, followed by extraction with chloroform. The organic layer was separated using a phase separator, and then, the solvent was distilled off under reduced pressure. The residue was purified by preparative TLC (OH type silica gel, developing solvent; chloroform/methanol = 88/12) to obtain title compound 13 (pale yellow oil, 1.7 mg, yield 19%).

The ¹H-NMR and LCMS data are as shown in Table 2.

### Example 7

### 5-{3,5-Difluoro-4-[3-({2-[(1S)-1-hydroxyethyl]-1H-imidazol-1-yl}methyl)-1,2-oxazol-5-yl]phenyl}pent-4-yn-1-ol (compound 14)

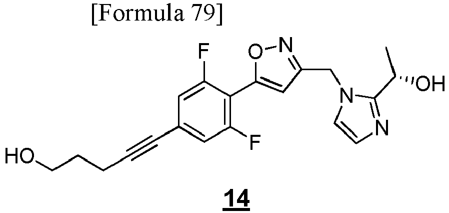

### 14-1) Synthesis of ethyl 5-(4-bromo-2,6-difluorophenyl)-1,2-oxazol-3-carboxylate

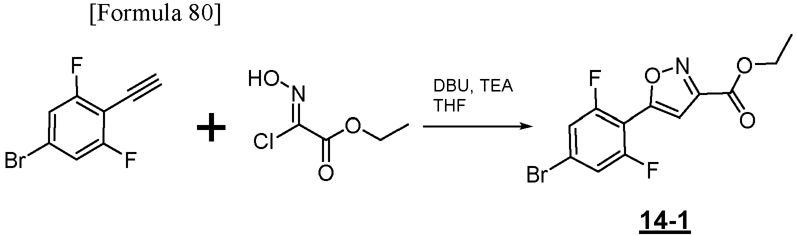

To a THF (18 mL) solution of 5-bromo-2-ethynyl-1,3-difluorobenzene (0.40 g), DBU (0.84 g) and ethyl (2Z)-chloro(hydroxyimino)acetate (0.55 g) were added, and the mixture was irradiated in a microwave reactor at 130°C for 1 hour. TEA (0.77 mL) and ethyl (2Z)-chloro(hydroxyimino)acetate (0.55 g) were further added, and after the mixture was irradiated in a microwave reactor at 130°C for 1 hour, water was added to the reaction mixture. The aqueous layer was extracted with EtOAc, and the organic layer was dried over magnesium sulfate and concentrated under reduced pressure. The residue was purified by OH type silica gel column chromatography (gradient elution with n-hexane/ethyl acetate = 98/2 to 85/15) to obtain title compound 14-1 (colorless solid, 0.36 g, yield 59%).
¹H NMR (400 MHz, CHLOROFORM-d) δ ppm 1.45 (t, J=7.1 Hz, 3 H), 4.49 (q, J=7.1 Hz, 2 H), 7.10 (s, 1 H), 7.28 (br s, 1 H), 7.30 (s, 1 H)

### 14-2) Synthesis of [5-(4-bromo-2,6-difluorophenyl)-1,2-oxazol-3-yl]methanol

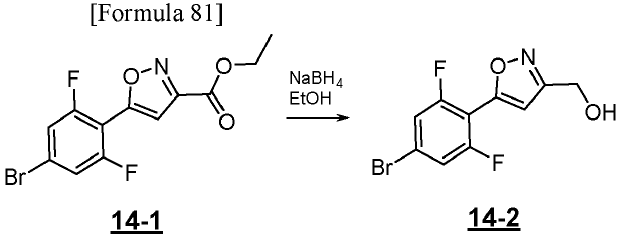

To an ethanol (2.9 mL) solution of compound 14-1 (0.19 g), sodium borohydride (0.11 g) was added under ice cooling, and the mixture was stirred at room temperature for 2 hours. Water was added to the reaction mixture, and the aqueous layer was extracted with chloroform. The organic layer was collected using a phase separator and concentrated under reduced pressure. The residue was purified by OH type silica gel column chromatography (gradient elution with n-hexane/ethyl acetate = 95/5 to 70/30) to obtain title compound 14-2 (colorless solid, 0.13 g, yield 75%).
MS (ESI) m/z = 290 [M+1]⁺
LCMS retention time: 0.922 min (condition B)

### 14-3) Synthesis of 5-(4-bromo-2,6-difluorophenyl)-3-[(2-{(1S)-1-[(oxan-2-yl)oxy]ethyl}-1H-imidazol-1-yl)methyl]-1,2-oxazole

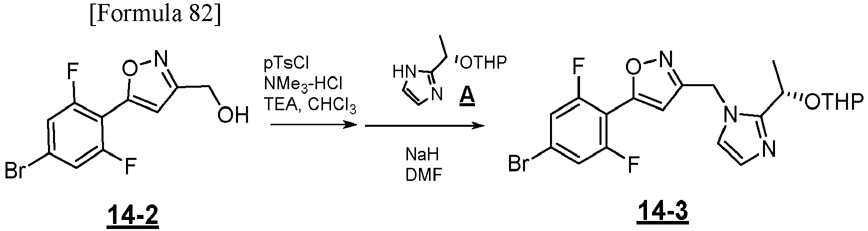

Compound 14-3 was produced by the same methods as in F-1 and F-2 of Reference Example 6 with the use of the corresponding starting materials.
MS (ESI) m/z = 468 [M+1]⁺
LCMS retention time: 0.753 min (condition B)

### 14-4) Synthesis of 5-[4-(5-{[tert-butyl(dimethyl)silyl]oxy}pent-1-yn-1-yl)-2,6-difluorophenyl -3-[(2-{(1S)-1-[(oxan-2-yl)oxy]ethyl}-1H-imidazol-1-yl)methyl]-1,2-oxazole

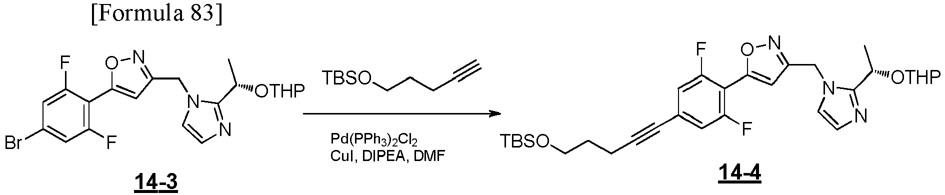

To a DMF (1.6 mL) solution of compound 14-3 (75 mg), DIPEA (0.36 mL), copper iodide (I) (6.1 mg), and Pd(PPh₃)₂Cl₂ (11 mg) were added, and the mixture was stirred at 60°C for 5 minutes. Then, a DMF (1.0 mL) solution of tert-butyl(dimethyl)[(pent-4-yn-1-yl)oxy]silane (95 mg) was added, and the mixture was stirred at 60°C for 1 hour. After allowing to cool to room temperature, the reaction mixture was diluted with EtOAc, and washed with water and brine. The organic layer was dried over magnesium sulfate, and then, concentrated under reduced pressure. The residue was purified by OH type silica gel column chromatography (gradient elution with n-hexane/ethyl acetate = 80/20 to 0/100) to obtain title compound 14-4 (yellow oil, 66 mg, yield 70%).
MS (ESI) m/z = 586 [M+1]⁺
LCMS retention time: 1.188 min (condition B)

### 14-5) Synthesis of 5-{3,5-difluoro-4-[3-({2-[(1S)-1-hydroxyethyl]-1H-imidazol-1-yl}methyl)-1,2-oxazol-5-yl]phenyl}pent-4-yn-1-ol (compound 14)

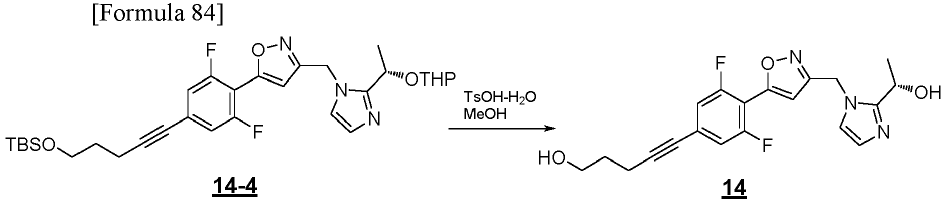

To a MeOH (1.1 mL) solution of compound 14-4 (66 mg), pTsOH-H₂O (64 mg) was added, and the mixture was stirred at room temperature for 1 hour. To the reaction mixture, a 20% aqueous potassium carbonate solution was added, and the mixture was extracted with a chloroform/MeOH (10:1) mixed solvent. After the organic layer was separated using a phase separator, the solvent was distilled off under reduced pressure. The residue was purified by NH type silica gel column chromatography (gradient elution with chloroform/MeOH = 100/0 to 95/5) to obtain title compound 14 (colorless solid, 22 mg, yield 50%).

The ¹H-NMR and LCMS data are as shown in Table 2.

### Example 8

### (R)-2-Amino-1-((1S,2S)-2-((4-(3-((2-((S)-1-hydroxyethyl)-1H-imidazol-1-yl)methyl)isoxazol-5-yl)phenyl)ethynyl)cyclopropyl)ethanol (compound 15)

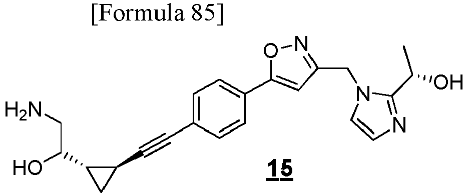

### 15-1) Synthesis of tert-butyl ((R)-2-((1S,2S)-2-((benzyloxy)methyl)cyclopropyl)-2-hydroxyethyl)carbamate

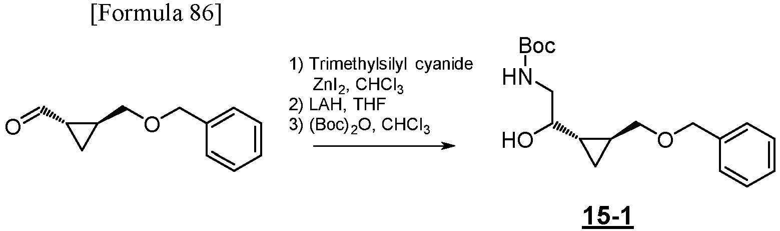

To a chloroform (6.3 mL) solution of (1S,2S)-2-[(benzyloxy)methyl]cyclopropan-1-carbaldehyde (0.60 g), zinc iodide (10 mg) was added and ice-cooled. After trimethylsilyl cyanide (0.79 mL) was added, the mixture was stirred at room temperature overnight. The reaction mixture was ice-cooled, and THF (5.0 mL) and LAH (0.30 g) were added, and the mixture was stirred for 1 hour. To the reaction mixture, water (0.25 mL), a 1 mol/L-aqueous sodium hydroxide solution (0.25 mL), and water (0.75 mL) were sequentially added, followed by stirring for 1 hour. After the resulting mixture was filtered through Celite, the filtrate was concentrated under reduced pressure. The residue was dissolved in chloroform (8.7 mL), di-tert-butyl dicarbonate (0.57 g) was added, and the mixture was stirred at room temperature for 2 hours. The reaction mixture was concentrated under reduced pressure, and the resulting residue was purified by OH type silica gel column chromatography (gradient elution with n-hexane/ethyl acetate = 90/10 to 20/80) to obtain title compound 15-1 (colorless oil, 0.73 g, yield 88%).
MS (ESI) m/z = 344 [M+23]⁺
LCMS retention time: 1.013 min (condition B)

### 15-2) Synthesis of tert-butyl ((R)-2-((1S,2S)-2-((benzyloxy)methyl)cyclopropyl)-2-((tert-butyldimethylsilyl)oxy)ethyl)carbamate

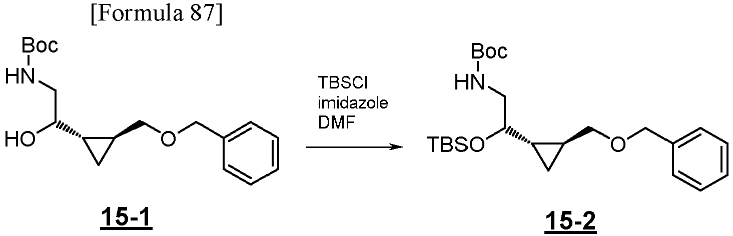

To a DMF (11 mL) solution of compound 15-1 (0.73 g), imidazole (0.31 g) and TBSCl (0.51 g) were added, and the mixture was stirred at room temperature for 1 hour. The reaction mixture was poured into water, and the mixture was extracted with a n-hexane/ethyl acetate (20:80) mixed solvent. The organic layer was washed with water, and then dried over magnesium sulfate, filtered, and concentrated under reduced pressure. The residue was purified by OH type silica gel column chromatography (gradient elution with n-hexane/ethyl acetate = 95/5 to 70/30) to obtain title compound 15-2 (pale yellow oil, 0.94 g, yield 95%).
¹H NMR (600 MHz, CHLOROFORM-d) δ ppm 0.04 - 0.07 (m, 6 H), 0.39 - 0.48 (m, 1 H), 0.52 - 0.59 (m, 1 H), 0.70 - 0.77 (m, 1 H), 0.87 - 0.92 (m, 9 H), 1.02 - 1.17 (m, 1 H), 1.43 (s, 9 H), 3.15 - 3.32 (m, 3 H), 3.33 - 3.46 (m, 2 H), 4.50 - 4.53 (m, 2 H), 7.32 - 7.35 (m, 5 H)

### 15-3) Synthesis of tert-butyl ((R)-2-((tert-butyldimethylsilyl)oxy)-2-((1S,2S)-2-(hydroxymethyl)cyclopropyl)ethyl)carbamate

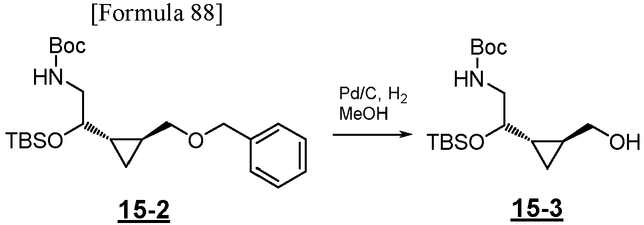

To a methanol (10 mL) solution of compound 15-2 (0.90 g), 10% palladium-carbon (0.18 g) was added at room temperature, and then, the mixture was vigorously stirred overnight under a hydrogen atmosphere. After the reaction mixture was diluted with methanol and ammonia water, the insolubles were filtered off. The filtrate was concentrated under reduced pressure, and the resulting residue was purified by OH type silica gel column chromatography (gradient elution with n-hexane/ethyl acetate = 97/3 to 50/50) to obtain title compound 15-3 (colorless oil, 0.26 g, yield 37%).
MS (ESI) m/z = 368 [M+23]⁺
LCMS retention time: 1.274 min (condition B)

### 15-4) Synthesis of tert-butyl ((R)-2-((tert-butyldimethylsilyl)oxy)-2-((1S,2S)-2-formyl cyclopropyl)ethyl)carbamate

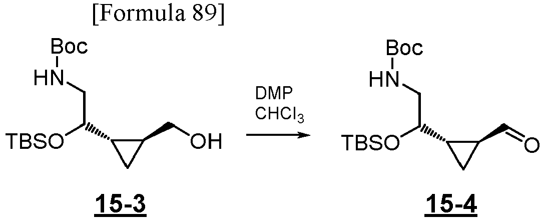

To a chloroform (6.9 mL) solution of compound 15-3 (0.24 g), DMP (0.32 g) was added at room temperature. After the mixture was stirred at room temperature for 1 hour, DMP (0.10 g) was added, and the mixture was stirred for 1 hour. To the reaction mixture, an aqueous saturated sodium bicarbonate solution was added, followed by extraction with chloroform. The organic layer was separated using a phase separator, and the solvent was distilled off under reduced pressure. The residue was purified by OH type silica gel column chromatography (gradient elution with n-hexane/ethyl acetate = 95/5 to 60/40) to obtain title compound 15-4 (colorless oil, 0.19 g, yield 79%).
¹H NMR (400 MHz, CHLOROFORM-d) δ ppm 0.04 - 0.10 (m, 6 H), 0.85 - 0.94 (m, 9 H), 1.05 - 1.20 (m, 1 H), 1.44 (s, 9 H), 1.60 (br s, 2 H), 1.80 - 1.94 (m, 1 H), 3.07 - 3.21 (m, 1 H), 3.24 (br s, 1 H), 3.54 - 3.64 (m, 1 H), 4.73 (br s, 1 H), 9.05 - 9.18 (m, 1 H)

### 15-5) Synthesis of tert-butyl ((R)-2-((tert-butyldimethylsilyl)oxy)-2-((1S,2S)-2-ethynylcyclopropyl)ethyl)carbamate

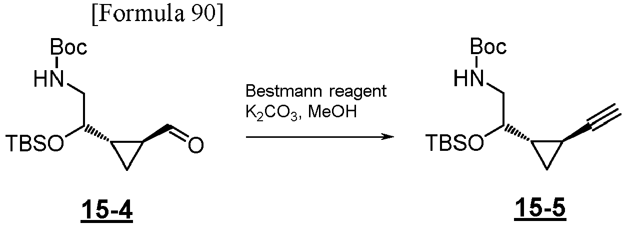

Compound 15-5 was produced by the same methods as in J-1 of Reference Example 10 with the use of the corresponding starting materials.
¹H NMR (400 MHz, CHLOROFORM-d) δ ppm 0.02 - 0.12 (m, 6 H), 0.69 - 0.85 (m, 1 H), 0.85 - 0.92 (m, 9 H), 1.14 - 1.30 (m, 2 H), 1.44 (s, 9 H), 1.78 (s, 1 H), 3.01 - 3.20 (m, 1 H), 3.21 - 3.58 (m, 2 H), 4.72 (br s, 1 H)

### 15-6) Synthesis of (R)-2-amino-1-((1S,2S)-2-((4-(3-((2-((S)-1-hydroxyethyl)-1H-imidazol-1-yl)methyl)isoxazol-5-yl)phenyl)ethynyl)cyclopropyl)ethanol (compound 15)

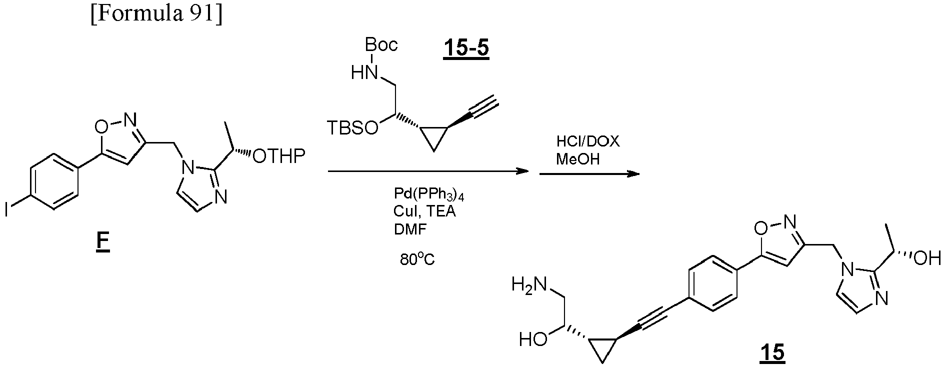

Compound 15 was produced by the same methods as in 10-1 and 10-2 of Example 3 with the use of the corresponding starting materials.

The ¹H-NMR and LCMS data are as shown in Table 2.

### Example 9

### 5-(4-{3-[(1S)-2-Hydroxy-1-{2-[(1S)-1-hydroxyethyl]-1H-imidazol-1-yl}ethyl]-1,2-oxazol-5-yl}phenyl)pent-4-yn-1-ol (compound 16)

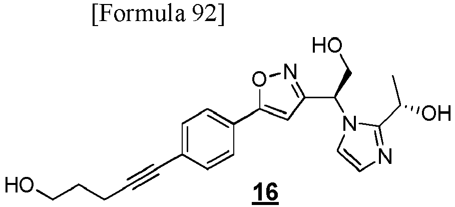

### 16-1) Synthesis of 5-(4-{3-[(1S)-2-hydroxy-1-{2-[(1S)-1-hydroxyethyl]-1H-imidazol-1-yl}ethyl]-1,2-oxazol-5-yl}phenyl)pent-4-yn-1-ol (compound 16)

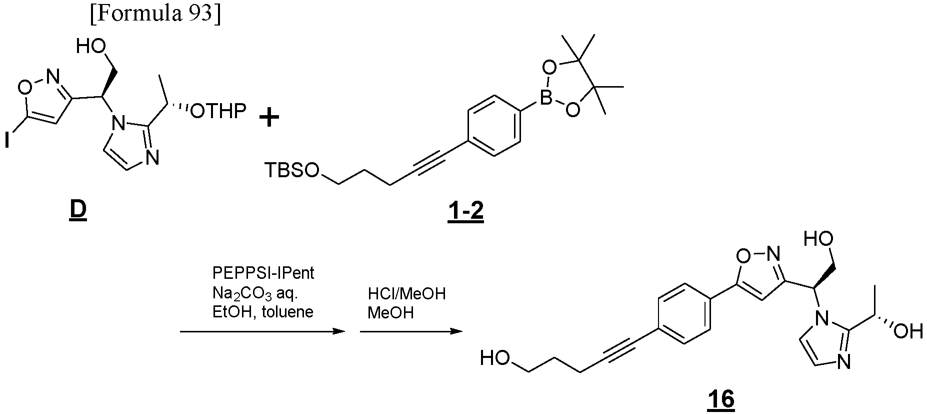

Intermediate D (50 mg), compound 1-2 (69 mg), and PEPPSI-IPent (9.2 mg) were weighed into a microwave reactor vial, the system was replaced with nitrogen, and then, toluene (0.46 mL), ethanol (0.23 mL), and a 2 mol/L-aqueous sodium carbonate solution (0.23 mL) were sequentially added. The resulting mixture was irradiated in a microwave reactor at 110°C for 2.5 hours. The reaction mixture was poured into water, and the mixture was extracted three times with chloroform. After the organic layer was dried over magnesium sulfate, the solvent was distilled off under reduced pressure to obtain a crude product. After the resulting crude product was dissolved in methanol (0.58 mL), a 2 mol/L-hydrochloric acid-methanol solution (0.58 mL) was added. After stirring at room temperature for 2 hours, the reaction mixture was concentrated under reduced pressure. To the residue, a 2 mol/L-aqueous sodium carbonate solution was added, followed by extraction three times with a chloroform/methanol mixed solvent (90:10). After the organic layer was dried over magnesium sulfate, the solvent was distilled off under reduced pressure. The residue was purified by NH type silica gel column chromatography (gradient elution with ethyl acetate/methanol = 100/0 to 90/10) to obtain title compound 16 (colorless oil, 15 mg, yield 34%).

The ¹H-NMR and LCMS data are as shown in Table 2.

### Example 10

### 5-(4-{3-[(1R)-2-Amino-1-{2-[(1S)-1-hydroxyethyl]-1H-imidazol-1-yl}ethyl]-1,2-oxazol-5-yl}phenyl)pent-4-yn-1-ol (compound 18)

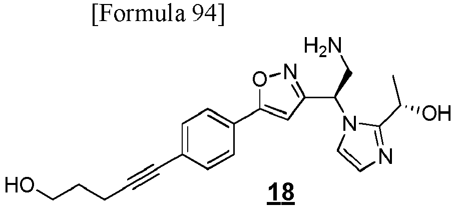

### 18-1) Synthesis of (2R)-2-{5-[4-(5-{[tert-butyl(dimethyl)silyl]oxy}pent-1-yn-1-yl)phenyl]-1,2-oxazol-3-yl}-2-(2-{(1S)-1-[(oxan-2-yl)oxy]ethyl}-1H-imidazol-1-yl)ethan-1-amine

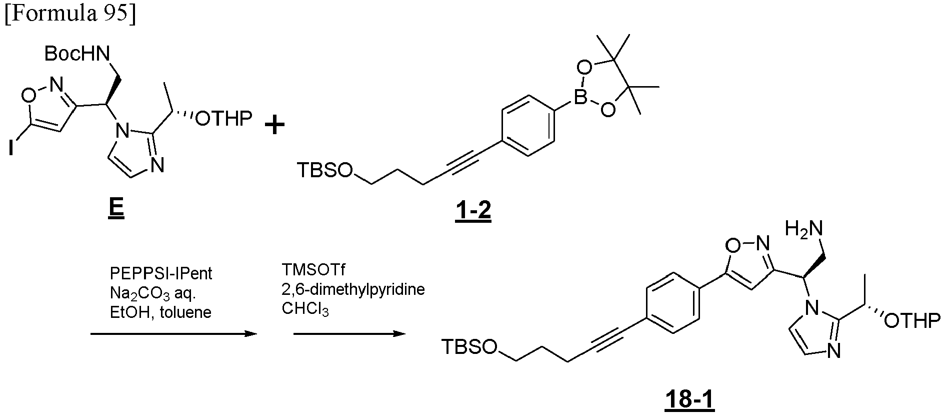

Intermediate E (70 mg), compound 16-1 (79 mg), and PEPPSI-IPent (10 mg) were weighed, the system was replaced with nitrogen, and then, toluene (0.53 mL), ethanol (0.53 mL), and a 2 mol/L-aqueous sodium carbonate solution (0.26 mL) were sequentially added, followed by stirring at 100°C for 2 hours. The reaction mixture was poured into water and extracted three times with ethyl acetate. The organic layer was washed with an aqueous saturated sodium chloride solution, and dried over magnesium sulfate, and then, the solvent was distilled off under reduced pressure. The residue was purified by OH type silica gel column chromatography (gradient elution with n-hexane/ethyl acetate = 50/50 to 0/100). After the resulting crude product was dissolved in chloroform (0.66 mL), 2,6-dimethylpyridine (0.12 mL) and trimethylsilyl trifluoromethanesulfonate (0.19 mL) were sequentially added under ice cooling. After stirring for 15 min, the reaction mixture was warmed to room temperature and stirred for 2 hours. The reaction mixture was poured into an aqueous saturated sodium bicarbonate solution, followed by extraction three times with chloroform. The organic layer was dried over magnesium sulfate, and then, filtered and concentrated under reduced pressure. The residue was purified by OH type silica gel column chromatography (gradient elution with chloroform/methanol = 100/0 to 90/10) to obtain title compound 18-1 (pale yellow oil, 12 mg, yield 16%).
MS (ESI) m/z = 579 [M+1]⁺
LCMS retention time: 0.945 min (condition B)

### 18-2) Synthesis of 5-(4-{3-[(1R)-2-amino-1-{2-[(1S)-1-hydroxyethyl]-1H-imidazol-1-yl}ethyl]-1,2-oxazol-5-yl}phenyl)pent-4-yn-1-ol (compound 18)

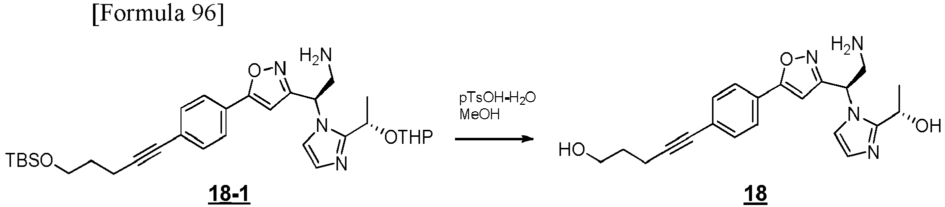

Compound 18 was produced by the same methods as in 14-5 with the use of the corresponding starting materials.

The ¹H-NMR and LCMS data are as shown in Table 2.

### Example 11

### (1 S)-1-(1-{ [5-(4-{[(1R,5S,6s)-3-(2-Hydroxy ethyl)-3-azabicyclo[3.1.0]hexan-6-yl]ethynyl}phenyl)-1,2-oxazol-3-yl]methyl}-1H-imidazol-2-yl)ethan-1-ol (compound 19)

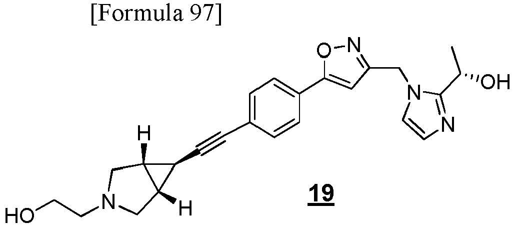

### 19-1) Synthesis of (1S)-1-(1-{[5-(4-{[(1R,5S,6s)-3-(2-{[tert-butyl(dimethyl)silyl]oxy}ethyl)-3-azabicyclo[3.1.0]hexan-6-yl]ethynyl}phenyl)-1,2-oxazol-3-yl]methyl}-1H-imidazol-2-yl)ethan-1-ol

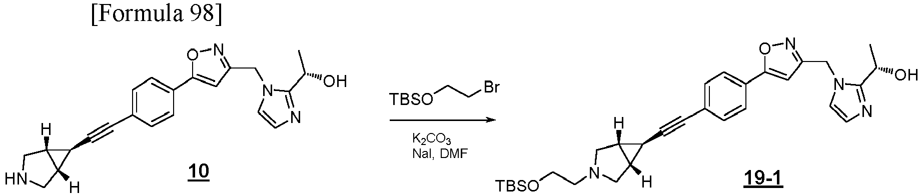

Compound 10 (50 mg) and (2-bromoethoxy)(tert-butyl)dimethylsilane (35 mg) were dissolved in DMF (1.3 mL), potassium carbonate (55 mg) was added, and the mixture was stirred at 80°C for 1 hour. Sodium iodide (20 mg) was added, and the mixture was stirred at 80°C for 5 hours and at room temperature for 14 hours. To the reaction mixture, an aqueous saturated sodium bicarbonate solution was added, and the mixture was stirred, followed by extraction with ethyl acetate. The organic layer was concentrated under reduced pressure, and the resulting residue was purified by NH type silica gel column chromatography (gradient elution with n-hexane/ethyl acetate = 9/1 to 0/1) to obtain title compound 19-1 (colorless amorphous, 59 mg, yield 83%).
MS (ESI) m/z = 533 [M+1]⁺
LCMS retention time: 0.589 min (condition B)

### 19-2) Synthesis of (1S)-1-(1-{[5-(4-{[(1R,5S,6s)-3-(2-hydroxyethyl)-3-azabicyclo[3.1.0]hexan-6-yl]ethynyl}phenyl)-1,2-oxazol-3-yl]methyl}-1H-imidazol-2-yl)ethan-1-ol (compound 19)

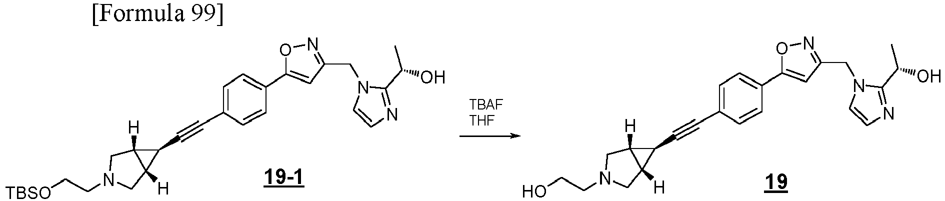

To a THF (0.55 mL) solution of compound 19-1 (59 mg), TBAF (1 mol/L-THF solution)(0.12 mL) was added under ice cooling, and the mixture was stirred for 3 hours. The reaction mixture was concentrated, a 2 mol/L-aqueous sodium carbonate solution was added, and the mixture was extracted with a 10% methanol/chloroform solution. The organic layer was dried over magnesium sulfate and concentrated under reduced pressure. The residue was purified by NH type silica gel column chromatography (gradient elution with chloroform/methanol = 1/0 to 9/1) to obtain title compound 19 (colorless solid, 45 mg, yield 97%).

The ¹H-NMR and LCMS data are as shown in Table 2.

### Example 12

### (1S)-1-(1-{(1R)-2-Amino-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol (compound 22)

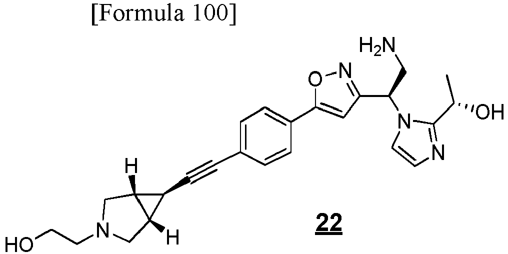

### 22-1) Synthesis of (1R,5S,6s)-3-(2-{[tert-butyl(dimethyl)silyl]oxy}ethyl)-6-ethynyl-3-azabicyclo[3.1.0]hexane

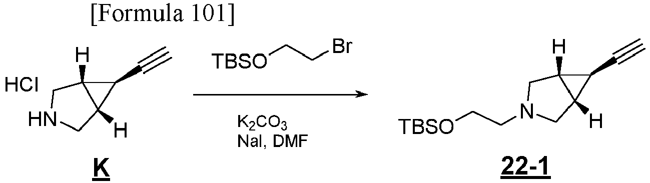

Intermediate K (0.39 g) and (2-bromoethoxy)(tert-butyl)dimethylsilane (0.71 g) were dissolved in DMF (14 mL), potassium carbonate (1.1 g) and sodium iodide (0.41 g) were added, and the mixture was stirred at 80°C for 2 hours. Water was added to the reaction mixture, and the mixture was stirred, followed by extraction with ethyl acetate. The residue obtained by concentrating the organic layer under reduced pressure was purified by OH type silica gel column chromatography (gradient elution with n-hexane/ethyl acetate = 9/1 to 4/6) to obtain title compound 22-1 (pale yellow oil, 0.54 g, yield 75%).
¹H NMR (400 MHz, CHLOROFORM-d) δ ppm 0.00 (s, 6 H), 0.84 (s, 9 H), 1.59 - 1.68 (m, 3 H), 1.76 (s, 1 H), 2.33 (br d, J=9.3 Hz, 2 H), 2.50 (t, J=6.2 Hz, 2 H), 3.03 (d, J=9.3 Hz, 2 H), 3.59 (t, J=6.2 Hz, 2 H)

### 22-2) Synthesis of tert-butyl [(2R)-2-[5-(4-{[(1R,5S,6s)-3-(2-{[tert-butyl(dimethyl)silyl]oxy}ethyl)-3-azabicyclo[3.1.0]hexan-6-yl]ethynyl}phenyl)-1,2-oxazol-3-yl]-2-{2-[(1S)-1-{[tert-butyl(dimethyl)silyl]oxy}ethyl]-1H-imidazol-1-yl}ethyl]carbamate

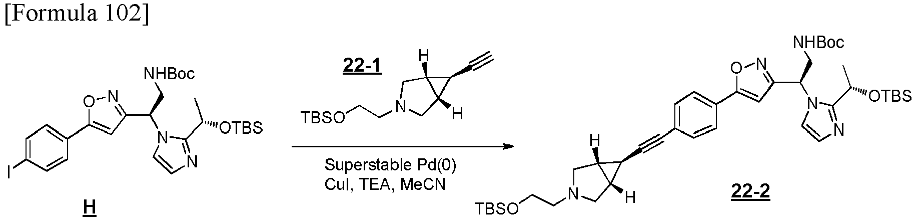

Compound 22-2 was produced by the same methods as in 10-1 of Example 18 with the use of the corresponding starting materials.
MS (ESI) m/z = 776 [M+1]⁺
LCMS retention time: 0.901 min (condition B)

### 22-3) Synthesis of (1S)-1-(1-{(1R)-2-amino-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol (compound 22)

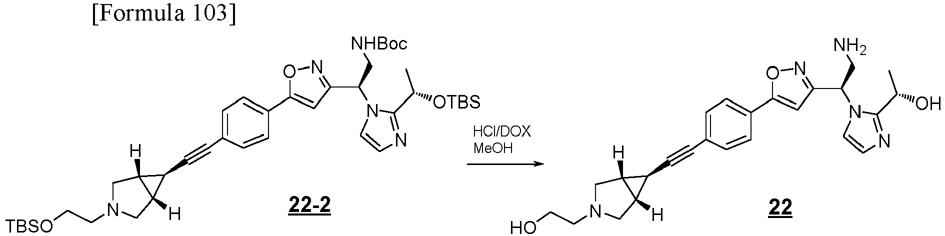

To compound 22-2 (63 mg), methanol (0.10 mL) and a 4 mol/L-hydrochloric acid-DOX solution (2.0 mL) were added, and the mixture was stirred at room temperature for 5 hours. After the reaction mixture was concentrated under reduced pressure, a 20% aqueous potassium carbonate solution was added, and the mixture was extracted with a chloroform/methanol mixed solvent. The organic layer was separated using a phase separator, and the solvent was concentrated under reduced pressure. The residue was purified by NH type silica gel column chromatography (gradient elution with chloroform/methanol = 100/0 to 90/10) to obtain title compound 22 (colorless amorphous, 19 mg, yield 53%).

The ¹H-NMR and LCMS data are as shown in Table 2.

### Example 13

### 1-Amino-3-({4-[3-({2-[(1S)-1-hydroxyethyl]-1H-imidazol-1-yl}methyl)-1,2-oxazol-5-yl]phenyl}ethynyl)cyclobutane-1-carbonitrile (compound 24)

### 1-Amino-3-({4-[3-({2-[(1S)-1-hydroxyethyl]-1H-imidazol-1-yl}methyl)-1,2-oxazol-5-yl]phenyl}ethynyl)cyclobutane-1-carboxamide (compound 25)

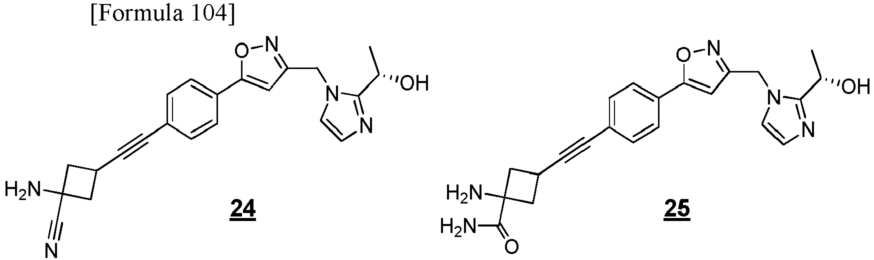

### 24-1) Synthesis of 1-amino-3-({[tert-butyl(dimethyl)silyl]oxy}methyl)cyclobutane-1-carbonitrile

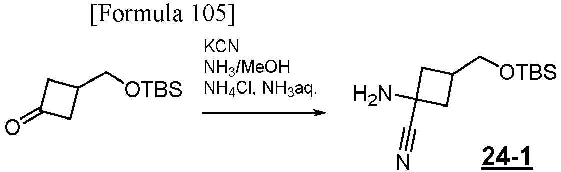

To 3-[[tert-butyl(dimethyl)silyl]oxymethyl]cyclobutanone (0.64 g), a 7 mol/L-ammonia-methanol solution (2.8 mL) was added at 0°C, and the mixture was stirred at the same temperature for 1 hour. A solution obtained by dissolving ammonium chloride (0.40 g) and potassium cyanide (0.25 g) in a 28% aqueous ammonia solution (5.6 mL) was slowly added dropwise at 0°C, and the mixture was stirred at room temperature overnight. Brine was added to the reaction mixture, and the mixture was extracted three times with chloroform. The organic layer was washed with brine and dried over magnesium sulfate, and the solvent was distilled off under reduced pressure. The residue was purified by OH type silica gel column chromatography (gradient elution with n-hexane/ethyl acetate = 100/0 to 20/80) to obtain title compound 24-1 (colorless solid, 0.15 g, yield 21%).
MS (ESI) m/z = 241 [M+1]⁺
LCMS retention time: 0.774 min (condition B)

### 24-2) Synthesis of tert-butyl [1-cyano-3-(hydroxymethyl)cyclobutyl]carbamate

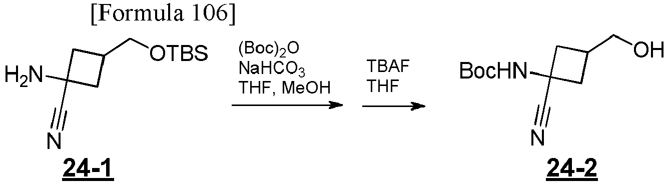

To a THF (4.7 mL) solution of compound 24-1 (0.11 g), di-tert-butyl dicarbonate (0.16 g) was added, and the mixture was stirred at room temperature overnight. Furthermore, an aqueous saturated sodium bicarbonate solution (1.0 mL) and methanol (3.0 mL) were added, and the mixture was stirred at room temperature for 5 days. Water was added to the reaction mixture, and the mixture was extracted three times with ethyl acetate. The organic layer was dried over magnesium sulfate, and then, filtered and concentrated under reduced pressure to obtain a crude product (0.20 g). To a THF (4.0 mL) solution of the resulting crude product (0.20 g), TBAF (1.2 mL, 1 mol/L-THF solution) was added, and the mixture was stirred for 1 hour. To the reaction mixture, water and brine were added, followed by extraction with ethyl acetate. The organic layer was dried over magnesium sulfate, and then, filtered and concentrated under reduced pressure. The residue was purified by OH type silica gel column chromatography (gradient elution with ethyl acetate/methanol = 85/15 to 25/75) to obtain title compound 24-2 (colorless oil, 59 mg, yield 45%).
¹H NMR (400 MHz, CHLOROFORM-d) δ ppm 1.48 (s, 9 H), 2.17 - 2.26 (m, 2 H), 2.68 - 2.83 (m, 3 H), 3.63 - 3.68 (m, 2 H), 4.89 (br s, 1 H)

### 24-3) Synthesis of tert-butyl (1-cyano-3-formylcyclobutyl)carbamate

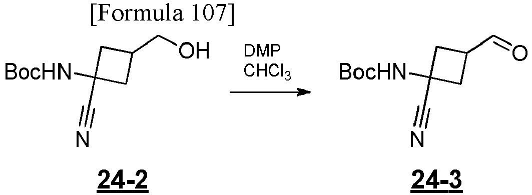

To a chloroform (3.1 mL) solution of compound 24-2 (0.14 g), DMP (0.31 g) was added under ice cooling. After the reaction mixture was warmed to room temperature, the mixture was stirred for 1 hour. The precipitated solid was separated, and an aqueous saturated sodium bicarbonate solution and brine were added, followed by extraction with chloroform. After the organic layer was separated using a phase separator, the solvent was distilled off under reduced pressure. The residue was purified by OH type silica gel column chromatography (gradient elution with n-hexane/ethyl acetate = 85/15 to 25/75) to obtain title compound 24-3 (colorless oil, 59 mg, yield 43%).
¹H NMR (400 MHz, CHLOROFORM-d) δ ppm 1.55 (s, 9 H), 2.53 - 2.66 (m, 2 H), 2.87 - 3.02 (m, 2 H), 3.33 - 3.46 (m, 1 H), 4.91 (br s, 1 H), 9.77 (s, 1 H)

### 24-4) Synthesis of tert-butyl (1-cyano-3-ethynylcyclobutyl)carbamate

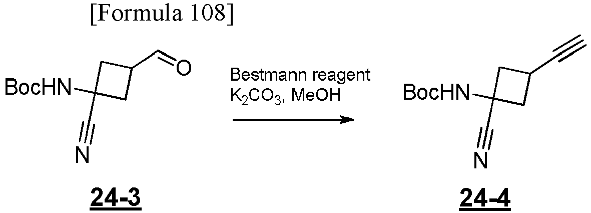

To a methanol (1.3 mL) mixture of compound 24-3 (59 mg) and potassium carbonate (73 mg), the Bestmann reagent (48 µL) was added at room temperature. After stirring at room temperature for 3 hours, the reaction mixture was poured into water, which was extracted three times with chloroform. The organic layer was washed with brine and dried over magnesium sulfate, and then, the solvent was distilled off under reduced pressure. The residue was purified by OH type silica gel column chromatography (gradient elution with n-hexane/ethyl acetate = 90/10 to 60/40) to obtain title compound 24-4 (yellow oil, 37 mg, yield 64%).
¹H NMR (400 MHz, CHLOROFORM-d) δ ppm 1.48 (s, 9 H), 2.25 (s, 1 H), 2.37 - 2.64 (m, 2 H), 3.00 - 3.13 (m, 2 H), 3.15 - 3.28 (m, 1 H), 4.71 - 5.12 (m, 1 H)

### 24-5) Synthesis of tert-butyl {1-cyano-3-[(4-{3-[(2-{(1S)-1-[(oxan-2-yl)oxy]ethyl}-1H-imidazol-1-yl)methyl]-1,2-oxazol-5-yl}phenyl)ethynyl]cyclobutyl}carbamate

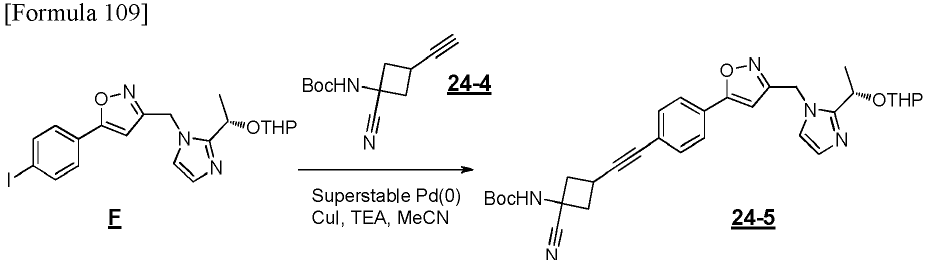

Compound 24-5 was produced by the same methods as in 10-1 with the use of the corresponding starting materials.
MS (ESI) m/z = 572 [M+1]⁺
LCMS retention time: 0.821 min (condition B)

### 24-6) Synthesis of 1-amino-3-({4-[3-({2-[(1S)-1-hydroxyethyl]-1H-imidazol-1-yl}methyl)-1,2-oxazol-5-yl]phenyl}ethynyl)cyclobutane-1-carbonitrile (compound 24) and 1-amino-3-({4-[3-({2-[(1S)-1-hydroxyethyl]-1H-imidazol-1-yl}methyl)-1,2-oxazol-5-yl]phenyl}ethynyl)cyclobutane-1-carboxamide (compound 25)

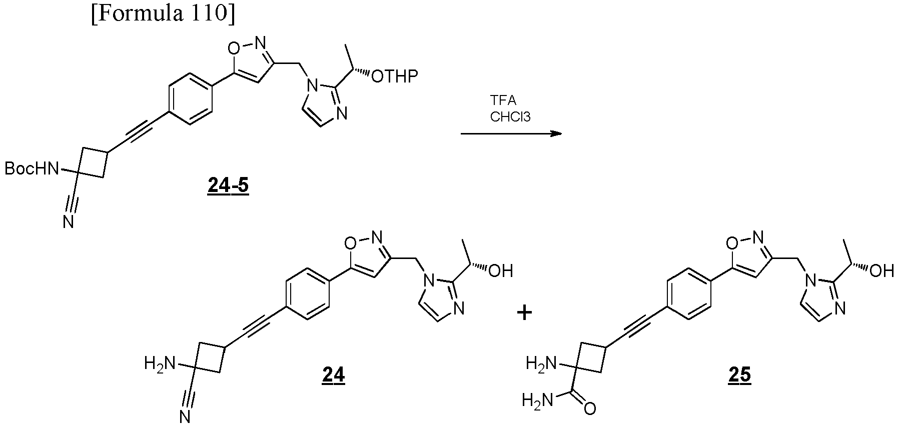

To a chloroform (0.80 mL) solution of compound 24-5 (40 mg), TFA (0.48 mL) was added, and the mixture was stirred for 2 hours. The reaction mixture was concentrated, a 20% aqueous potassium carbonate solution was added to the residue under ice cooling, and the mixture was extracted with chloroform. The organic layer was separated using a phase separator, ISOLUTE(registered trademark) HM-N was added, and the solvent was concentrated under reduced pressure. The residue was purified by NH type silica gel column chromatography (gradient elution with chloroform/methanol = 100/0 to 88/12) to obtain title compound 24 (pale yellow oil, 5.5 mg, yield 20%) and title compound 25 (colorless solid, 5.4 mg, yield 19%).

The ¹H-NMR and LCMS data are as shown in Table 2.

### Example 14

### (1S)-1-[1-({5-[4-(Cyclopropylethynyl)phenyl]-1,2-oxazol-3-yl}methyl)-1H-imidazol-2-yl]ethan-1-ol (compound 26)

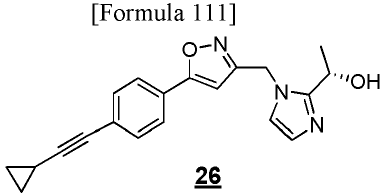

### 26-1) Synthesis of 5-[4-(cyclopropylethynyl)phenyl]-3-[(2-{(1S)-1-[(oxan-2-yl)oxy]ethyl}-1H-imidazol-1-yl)methyl]-1,2-oxazole

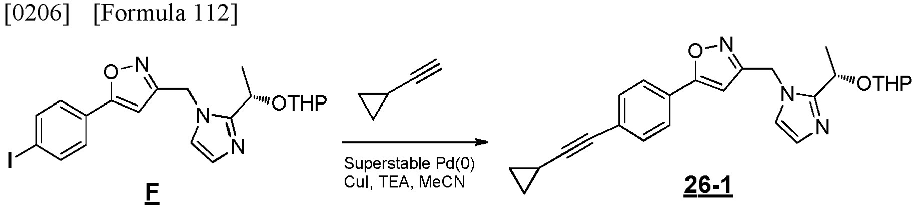

Compound 26-1 was produced by the same methods as in 4-1 of Example 2 with the use of the corresponding starting materials.
MS (ESI) m/z = 418 [M+1]⁺
LCMS retention time: 0.807 min (condition B)

### 26-2) Synthesis of (1S)-1-[1-({5-[4-(cyclopropylethynyl)phenyl]-1,2-oxazol-3-yl}methyl)-1H-imidazol-2-yl]ethan-1-ol (compound 26)

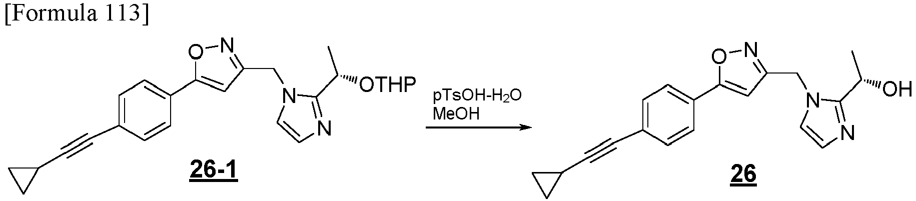

Compound 26 was produced by the same methods as in 4-3 of Example 2 with the use of the corresponding starting materials.

The ¹H-NMR and LCMS data are as shown in Table 2.

### Example 15

### 1-[(1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]ethan-1-one (compound 37)

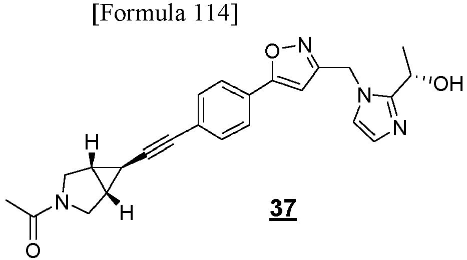

### 37-1) Synthesis of 1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]ethan-1-one (compound 37)

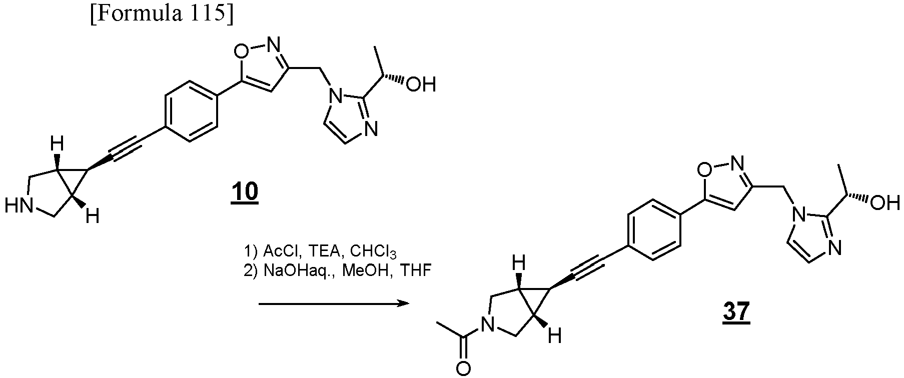

To a chloroform (0.80 mL) suspension of compound 10 (15 mg), TEA (17 µL) and acetyl chloride (6.3 µL) were added under ice cooling, and the mixture was stirred at room temperature for 2 hours. Water was added to the reaction mixture, and the mixture was extracted with chloroform. The aqueous layer was separated using a phase separator, and the organic layer was concentrated under reduced pressure and dried to obtain a crude product (22 mg). To the resulting crude product (22 mg), THF (0.20 mL), methanol (0.10 mL), and a 1 mol/L-aqueous sodium hydroxide solution (0.20 mL) were added, and the mixture was stirred at room temperature for 0.5 hours. Water was added to the reaction mixture, and the mixture was extracted with chloroform. The organic layer was separated using a phase separator, ISOLUTE(registered trademark) HM-N was added, and the mixture was concentrated under reduced pressure. The residue was purified by NH type silica gel column chromatography (gradient elution with chloroform/methanol = 99/1 to 89/11) to obtain title compound 37 (colorless solid, 16 mg, yield 98%).

The ¹H-NMR and LCMS data are as shown in Table 2.

### Example 16

### (1S)-1-{1-[(1R)-1-[5-(4-{[(1R,5S,6s)-3-(2-Hydroxyethyl)-3-azabicyclo[3.1.0]hexan-6-yl]ethynyl}phenyl)-1,2-oxazol-3-yl]-2-(methylamino)ethyl]-1H-imidazol-2-yl}ethan-1-ol (compound 38)

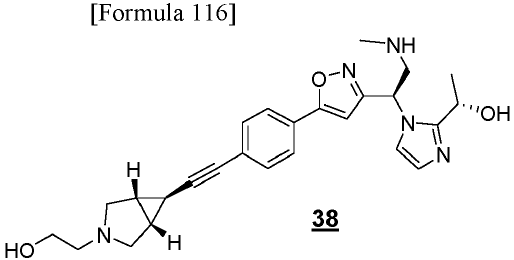

### 38-1) Synthesis of tert-butyl [(2R)-2-(5-iodo-1,2-oxazol-3-yl)-2-(2-{(1S)-1-[(oxan-2-yl)oxy]ethyl}-1H-imidazol-1-yl)ethyl]methylcarbamate

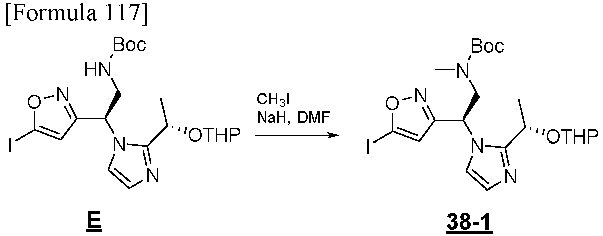

To a DMF (1.8 mL) solution of intermediate E (0.10 g), sodium hydride (15 mg, purity 60%) and iodomethane (27 µL) were added under ice cooling. After the reaction mixture was warmed to room temperature, the mixture was stirred for 15 minutes. Iodomethane (12 µL) was added at the same temperature, and the mixture was further stirred for 30 minutes. To the reaction mixture, an aqueous saturated ammonium chloride solution was added to terminate the reaction. After the resulting mixture was extracted with ethyl acetate, the organic layer was concentrated. The residue was purified by OH type silica gel column chromatography (gradient elution with n-hexane/ethyl acetate = 80/20 to 0/100) to obtain title compound 38-1 (colorless solid, 57 mg, yield 56%).
MS (ESI) m/z = 547 [M+1]⁺
LCMS retention time: 0.768 min (condition B)

### 38-2) Synthesis of tert-butyl (1R,5S,6s)-6-[(4-bromophenyl)ethynyl]-3-azabicyclo[3.1.0]hexan-3-carboxylate

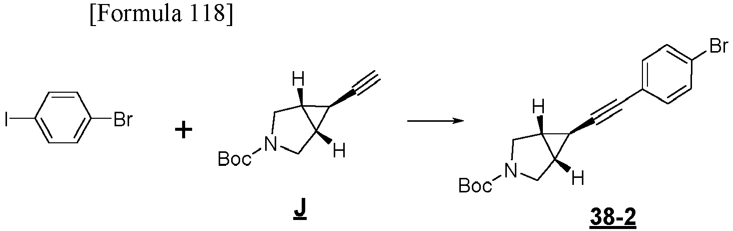

To an acetonitrile (15 mL) solution of 1-bromo-4-iodobenzene (0.82 g), Superstable Pd(0) (0.26 g), copper iodide (46 mg), and TEA (1.7 mL), an acetonitrile (65 mL) solution of compound J (0.50 g) was added dropwise at room temperature under a nitrogen atmosphere. After stirring at room temperature for 5 minutes, the reaction mixture was diluted with ethyl acetate and filtered through Celite and NH type silica gel, and then, the solvent was distilled off under reduced pressure. The residue was purified by NH type silica gel column chromatography (gradient elution with n-hexane/ethyl acetate = 95/5 to 80/20) to obtain title compound 38-2 (colorless solid, 0.50 g, yield 57%).
¹H NMR (400 MHz, CHLOROFORM-d) δ ppm 1.27 - 1.35 (m, 1 H), 1.44 (s, 9 H), 1.83 - 1.97 (m, 2 H), 3.30 - 3.49 (m, 2 H), 3.57 - 3.79 (m, 2 H), 7.22 (d, J=8.4 Hz, 2 H), 7.40 (d, J=8.4 Hz, 2 H)

### 38-3) Synthesis of (1R,5S,6s)-6-[(4-bromophenyl)ethynyl]-3-azabicyclo[3.1.0]hexane

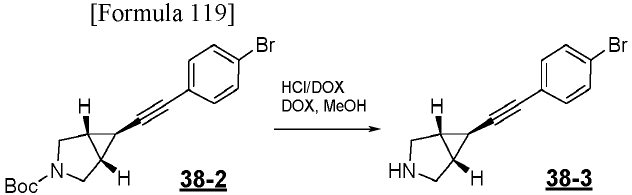

Compound 38-3 was produced by the same methods as in 10-2 of Example 3 with the use of the corresponding starting materials.
MS (ESI) m/z = 262 [M+1]⁺
LCMS retention time: 0.566 min (condition B)

### 38-4) Synthesis of (1R,5S,6s)-6-[(4-bromophenyl)ethynyl]-3-{2-[(oxan-2-yl)oxy]ethyl}-3-azabicyclo[3.1.0]hexane

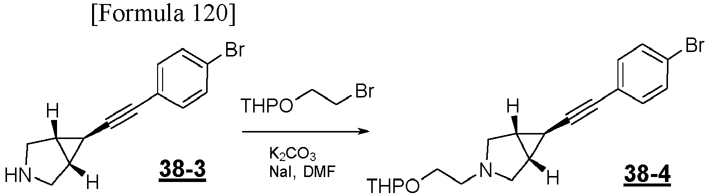

To a DMF (14 mL) solution of compound 38-3 (0.36 g) and sodium iodide (0.21 g), potassium carbonate (0.57 g) and 2-(2-bromoethoxy)oxane (0.32 g) were added, and the mixture was stirred at 80°C for 5 hours. After an aqueous saturated sodium bicarbonate solution was added to the reaction mixture, the mixture was extracted with ethyl acetate. After the organic layer was concentrated under reduced pressure, the resulting residue was purified by OH type silica gel column chromatography (gradient elution with n-hexane/ethyl acetate = 90/10 to 50/50) to obtain title compound 38-4 (yellow oil, 0.41 g, yield 76%).
MS (ESI) m/z = 390 [M+1]⁺
LCMS retention time: 0.709 min (condition B)

### 38-5) Synthesis of (1R,5S,6s)-3-{2-[(oxan-2-yl)oxy]ethyl}-6-{[4-(4,4,5,5-tetramethyl-1,3,2-dioxaborolan-2-yl)phenyl]ethynyl}-3-azabicyclo[3.1.0]hexane

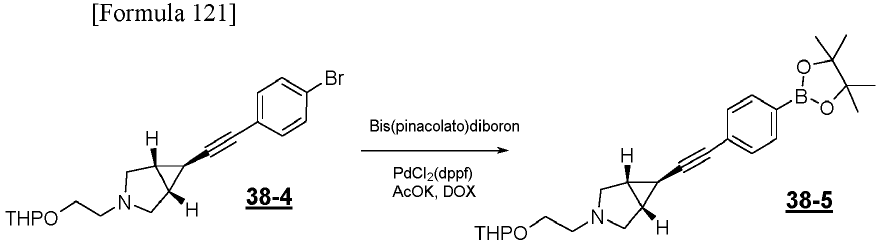

To a DOX (2.0 mL) solution of compound 38-4 (0.23 g), potassium acetate (0.16 g), 4,4,4',4',5,5,5',5'-octamethyl-2,2'-bi-l,3,2-dioxaborolane (0.19 g), and PdCl₂ (dppf) (22 mg) were added, followed by nitrogen replacement. The mixture was stirred at 100°C for 1.5 hours. Then, 4,4,4',4',5,5,5',5'-octamethyl-2,2'-bi-1,3,2-dioxaborolane (90 mg) was added, and the mixture was stirred at the same temperature for 1 hour. After allowing to cool to room temperature, the reaction mixture was diluted with ethyl acetate, and washed with water and brine. The organic layer was dried over magnesium sulfate and concentrated under reduced pressure. The residue was purified by OH type silica gel column chromatography (gradient elution with n-hexane/ethyl acetate = 90/10 to 0/100) to obtain compound 38-5 (orange oil, 88 mg, yield 34%).
MS (ESI) m/z = 438 [M+1]⁺
LCMS retention time: 0.802 min (condition B)

### 38-6) Synthesis of (1S)-1-{1-[(1R)-1-[5-(4-{[(1R,5S,6s)-3-(2-hydroxyethyl)-3-azabicyclo[3.1.0]hexan-6-yl]ethynyl}phenyl)-1,2-oxazol-3-yl]-2-(methylamino)ethyl]-1H-imidazol-2-yl}ethan-1-ol (compound 38)

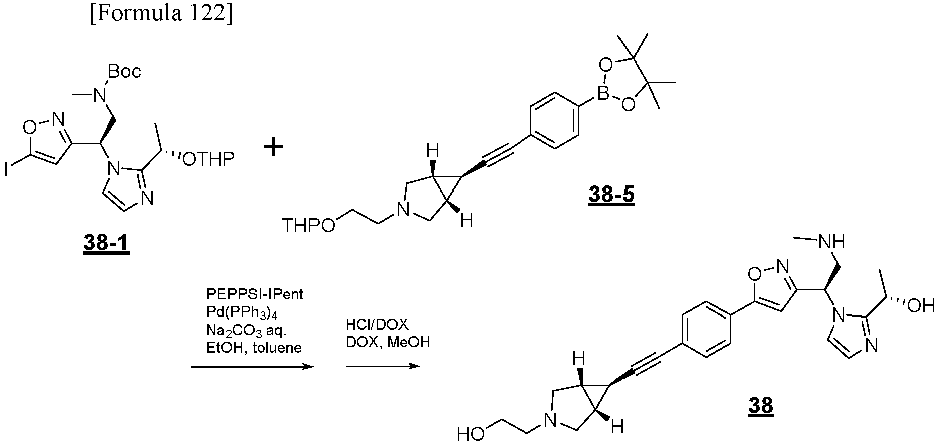

Compound 38 was produced by the same methods as in 16-1 of Example 9 with the use of the corresponding starting materials.

The ¹H-NMR and LCMS data are as shown in Table 2.

### Example 17

### (1S)-1-(1-{(1R)-2-(Dimethylamino)-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol (compound 39)

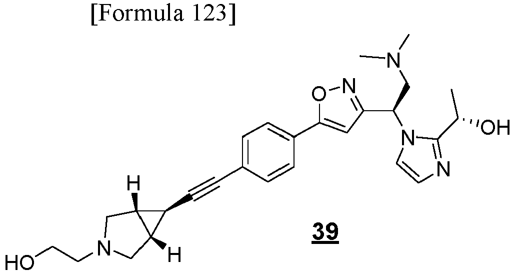

### 39-1) Synthesis of (1S)-1-(1-{(1R)-2-(dimethylamino)-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol (compound 39)

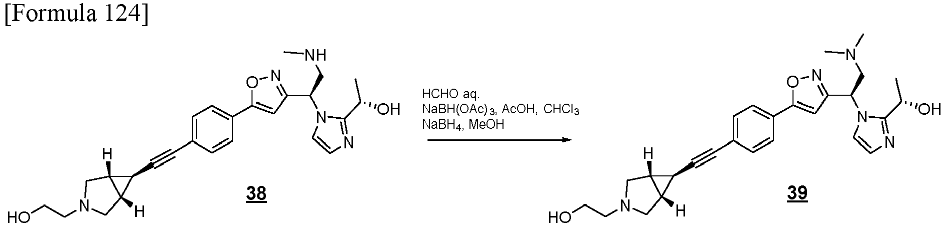

To a chloroform (0.48 mL) solution of compound 38 (11 mg), acetic acid (4.1 µL), a 35% aqueous formaldehyde solution (11 µL), and sodium triacetoxyborohydride (20 mg) were added, and the mixture was stirred at room temperature for 4 hours. The reaction mixture was concentrated, and the residue was dissolved in methanol (0.24 mL). A 35% aqueous formaldehyde solution (5.5 µL) and sodium borohydride (7.2 mg) were added under ice cooling, and the mixture was stirred at room temperature for 1 hour. The reaction mixture was added in small portions to the ice-cooled aqueous saturated sodium bicarbonate solution, and the mixture was extracted with chloroform. The resulting organic layer was dried over magnesium sulfate and concentrated under reduced pressure. The residue was purified by NH type silica gel column chromatography (gradient elution with chloroform/methanol = 99/1 to 89/11), and subsequently by preparative TLC (NH type silica gel, developing solvent; chloroform/methanol = 90/10) to obtain title compound 39 (yellow solid, 5.4 mg, yield 48%).

The ¹H-NMR and LCMS data are as shown in Table 2.

### Example 18

### (1S)-1-[1-({5-[4-({(1R,5S,6s)-3-[(Oxetan-3-yl)methyl]-3-azabicyclo[3.1.0]hexan-6-yl}ethynyl)phenyl]-1,2-oxazol-3-yl}methyl)-1H-imidazol-2-yl]ethan-1-ol (compound 42)

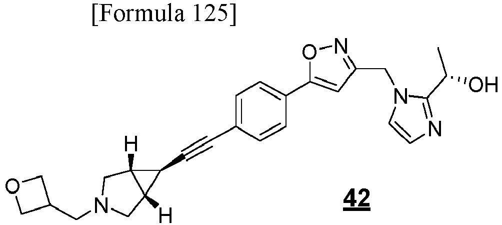

### 42-1) Synthesis of (1S)-1-[1-({5-[4-({(1R,5S,6s)-3-[(oxetan-3-yl)methyl]-3-azabicyclo[3.1.0]hexan-6-yl}ethynyl)phenyl]-1,2-oxazol-3-yl}methyl)-1H-imidazol-2-yl]ethan-1-ol (compound 42)

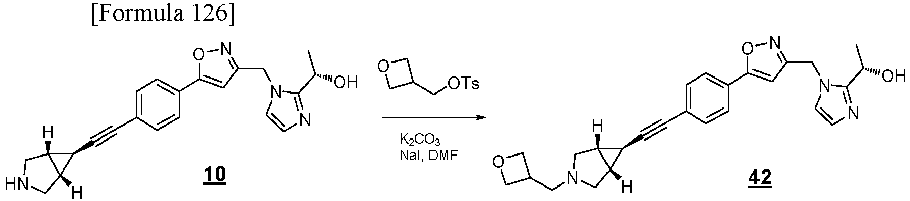

To a DMF (0.40 mL) solution of compound 10 (15 mg) and sodium iodide (6.0 mg), potassium carbonate (17 mg) and (oxetan-3-yl)methyl 4-methylbenzene-1-sulfonate (7.3 µL) were added, and the mixture was stirred at 85°C for 5 hours, and subsequently stirred at room temperature overnight. To the reaction mixture, an aqueous saturated sodium bicarbonate solution was added, followed by extraction with ethyl acetate. After the organic layer was concentrated under reduced pressure, the resulting residue was purified by NH type silica gel column chromatography (gradient elution with n-hexane/ethyl acetate = 90/10 to 0/100) to obtain title compound 42 (colorless amorphous, 13 mg, yield 73%).

The ¹H-NMR and LCMS data are as shown in Table 2.

### Example 19

### (1S)-1-(1-{[5-(4-{[1-(Methylamino)cyclopropyl]ethynyl}phenyl)-1,2-oxazol-3-yl]methyl}-1H-imidazol-2-yl)ethan-1-ol (compound 45)

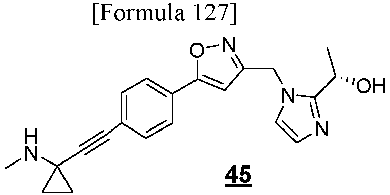

### 45-1) Synthesis of (1S)-1-(1-{[5-(4-{[1-(methylamino)cyclopropyl]ethynyl}phenyl)-1,2-oxazol-3-yl]methyl}-1H-imidazol-2-yl)ethan-1-ol (compound 45)

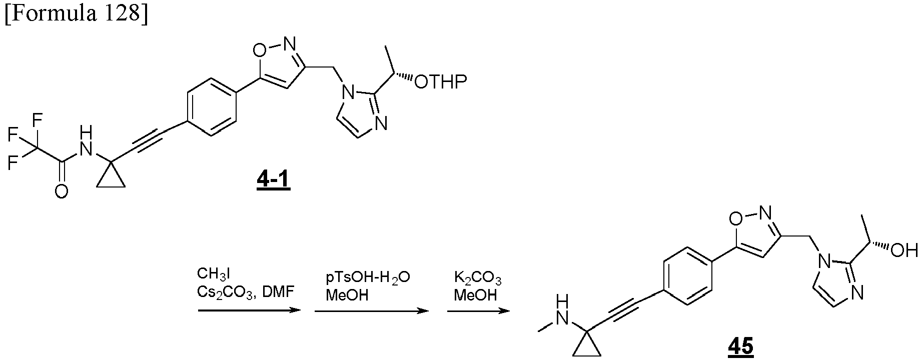

After cesium carbonate (62 mg) and iodomethane (7.1 µL) were added to a DMF (0.47 mL) solution of compound 4-1 (50 mg), the mixture was stirred at room temperature for 1 hour. Iodomethane (4.0 µL) was added, and the mixture was further stirred for 1.5 hours. The reaction mixture was poured into an aqueous saturated sodium bicarbonate solution, and the mixture was extracted three times with ethyl acetate. The organic layer was dried over magnesium sulfate, and then filtered and concentrated under reduced pressure. The residue was purified by OH type silica gel column chromatography (gradient elution with n-hexane/ethyl acetate = 50/50 to 0/100) to obtain a crude product (52 mg). Using this, compound 45 was produced by the same methods as in 4-3 and 4-2.

The ¹H-NMR and LCMS data are as shown in Table 2.

### Example 20

### trans-3-({4-[3-({2-[(1S)-1-Hydroxyethyl]-1H-imidazol-1-yl}methyl)-1,2-oxazol-5-yl]phenyl}ethynyl)cyclobutan-1-ol (compound 54)

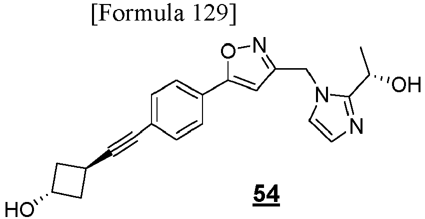

### 54-1) Synthesis of trans-3-{[tert-butyl(dimethyl)silyl]oxy}cyclobutane-1-carbaldehyde

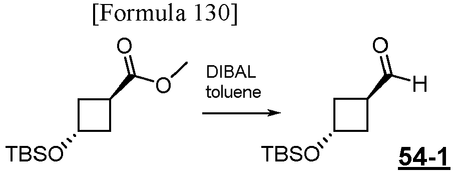

Methyl trans-3-{[tert-butyl(dimethyl)silyl]oxy}cyclobutane-1-carboxylate (1.7 g) was dissolved in toluene (34 mL), and a 1.0 mol/L-DIBAL-toluene solution (7.1 mL) was added dropwise at -78°C, and thereafter, the mixture was stirred for 15 minutes. After methanol (20 mL) was added at the same temperature to terminate the reaction, a 50% aqueous Rochelle's salt solution (50 mL) and ethyl acetate (50 mL) were added, and the mixture was stirred at room temperature for 3 hours. The organic layer was separated and dried over sodium sulfate, and the filtrate was concentrated under reduced pressure. The residue was purified by OH type silica gel column chromatography (gradient elution with n-hexane/ethyl acetate = 95/5 to 80/20) to obtain title compound 54-1 (colorless oil, 1.2 g, yield 84%).
¹H NMR (400 MHz, CHLOROFORM-d) δ ppm 0.00 (s, 6 H), 0.84 (s, 9 H), 2.07 - 2.23 (m, 2 H), 2.46 - 2.60 (m, 2 H), 2.97 (br t, J=10.3 Hz, 1 H), 4.27 (quin, J=7.3 Hz, 1 H), 9.79 (s, 1 H)

### 54-2) Synthesis of tert-butyl[(trans-3-ethynylcyclobutyl)oxy]dimethylsilane

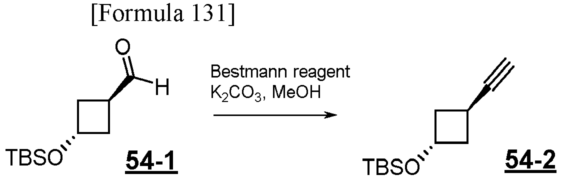

After potassium carbonate (1.1 g) was added to a methanol (19 mL) solution of compound 54-1 (0.82 g) under ice cooling, the Bestmann reagent (0.63 mL) was added dropwise, and the mixture was stirred at 0°C for 5 hours. To the reaction mixture, saturated ammonium chloride water was added, the mixture was extracted with chloroform, the aqueous layer was separated, and the organic layer was concentrated under reduced pressure. The resulting residue was purified by OH type silica gel column chromatography (gradient elution with n-hexane/ethyl acetate = 98/2 to 9/1) to obtain title compound 54-2 (colorless oil, 0.36 g, yield 45%).
¹H NMR (400 MHz, CHLOROFORM-d) δ ppm 0.00 (s, 6 H), 0.84 (s, 9 H), 2.07 (s, 1 H), 2.12 - 2.24 (m, 2 H), 2.26 - 2.38 (m, 2 H), 2.79 - 2.89 (m, 1 H), 4.53 (quin, J=7.1 Hz, 1 H)

### 54-3) Synthesis of trans-3-({4-[3-({2-[(1S)-1-hydroxyethyl]-1H-imidazol-1-yl}methyl)-1,2-oxazol-5-yl]phenyl}ethynyl)cyclobutan-1-ol (compound 54)

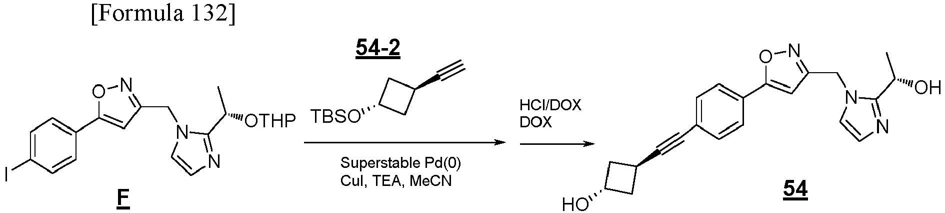

Compound 54 was produced by the same methods as in 10-1 and 10-2 of Example 3 with the use of the corresponding starting materials.

The ¹H-NMR and LCMS data are as shown in Table 2.

### Example 21

### (1S)-1-(1-{[5-(4-{[(1R,5S,6s)-3-(Oxetan-3-yl)-3-azabicyclo[3.1.0]hexan-6-yl]ethynyl}phenyl)-1,2-oxazol-3-yl]methyl}-1H-imidazol-2-yl)ethan-1-ol (compound 59)

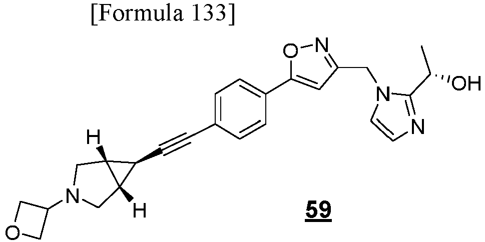

### 59-1) Synthesis of (1R,5S,6s)-6-ethynyl-3-(oxetan-3-yl)-3-azabicyclo[3.1.0]hexane

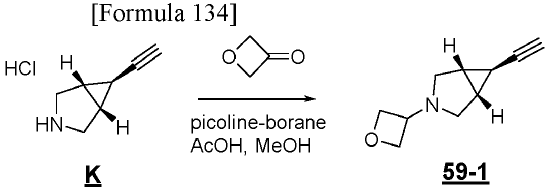

Acetic acid (24 µL) and oxetan-3-one (20 mg) were added to a methanol (1.4 mL) solution of intermediate K (20 mg), and the mixture was stirred at room temperature for 30 min. To this solution, 2-picoline borane (45 mg) was added, and the mixture was stirred at room temperature for 16 hours. An aqueous saturated sodium bicarbonate solution was added to the reaction mixture to terminate the reaction, followed by extraction with diethyl ether. The organic layer was concentrated, and the residue was purified by OH type silica gel column chromatography (gradient elution with n-hexane/ethyl acetate = 70/30 to 0/100) to obtain title compound 59-1 (yellow oil, 19 mg, yield 83%).
¹H NMR (400 MHz, CHLOROFORM-d) δ ppm 1.74 (s, 3 H), 1.82 (s, 1 H), 2.28 - 2.42 (m, 2 H), 3.01 (m, J=8.9 Hz, 2 H), 3.61 - 3.82 (m, 1 H), 4.54 (t, J=6.6 Hz, 2 H), 4.63 (t, J=6.6 Hz, 2 H)

### 59-2) Synthesis of (1S)-1-(1-{[5-(4-{[(1R,5S,6s)-3-(oxetan-3-yl)-3-azabicyclo[3.1.0]hexan-6-yl]ethynyl}phenyl)-1,2-oxazol-3-yl]methyl}-1H-imidazol-2-yl)ethan-1-ol (compound 59)

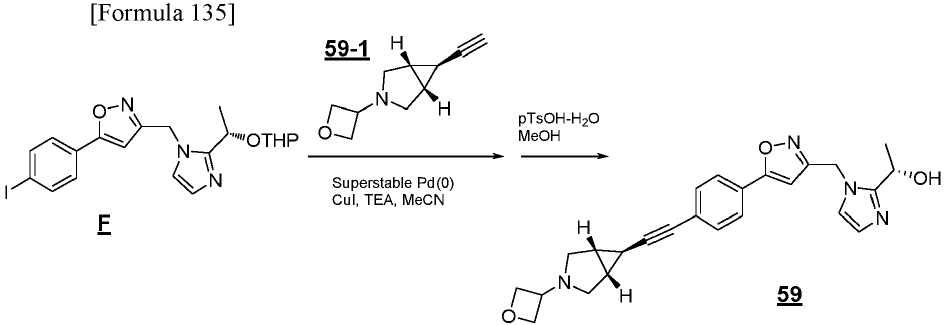

Compound 59 was produced by the same methods as in 4-1 and 4-3 of Example 2 with the use of the corresponding starting materials.

The ¹H-NMR and LCMS data are as shown in Table 2.

### Example 22

### 3-Amino-1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1-one (compound 61)

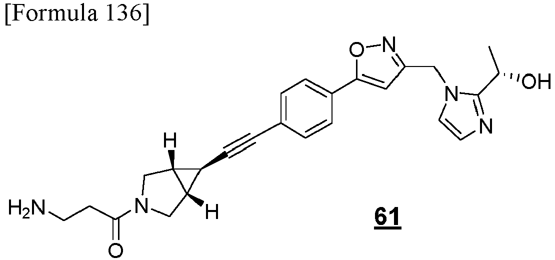

### 61-1) Synthesis of (S)-1-(1-((5-(4-(((1R,5S,6s)-3-(3-((tert-butoxycarbonyl)amino)propanoyl)-3-azabicyclo[3.1.0]hexan-6-yl)ethynyl)phenyl)isoxazol-3-yl)methyl)-1H-imidazol-2-yl)ethyl 3-((tert-butoxycarbonyl)amino)propanoate

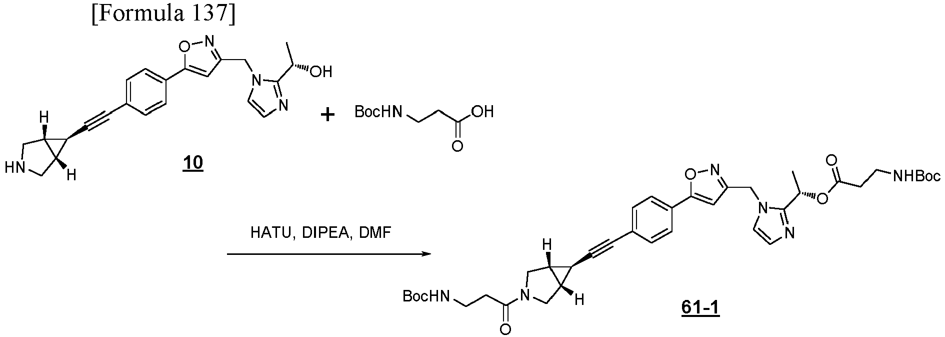

To a DMF (1.0 mL) solution of compound 10 (30 mg), HATU (40 mg), DIPEA (35 µL), and 3-(tert-butoxycarbonylamino)propionic acid (20 mg) were added, and the mixture was stirred at room temperature for 5 days. To the reaction mixture, an aqueous saturated sodium bicarbonate solution was added, and the mixture was extracted with ethyl acetate. The organic layer was washed twice with an aqueous saturated sodium bicarbonate solution, ISOLUTE(registered trademark) HM-N was added, and the solvent was concentrated under reduced pressure. The residue was purified by NH type silica gel column chromatography (gradient elution with n-hexane/ethyl acetate = 20/80 to 0/100 to gradient elution with chloroform/methanol = 98/2 to 90/10) to obtain title compound 61-1 (colorless amorphous, 22 mg, yield 38%).
MS (ESI) m/z = 717 [M+1]⁺
LCMS retention time: 0.833 min (condition B)

### 61-2) Synthesis of tert-butyl {3-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-3-oxopropyl}carbamate

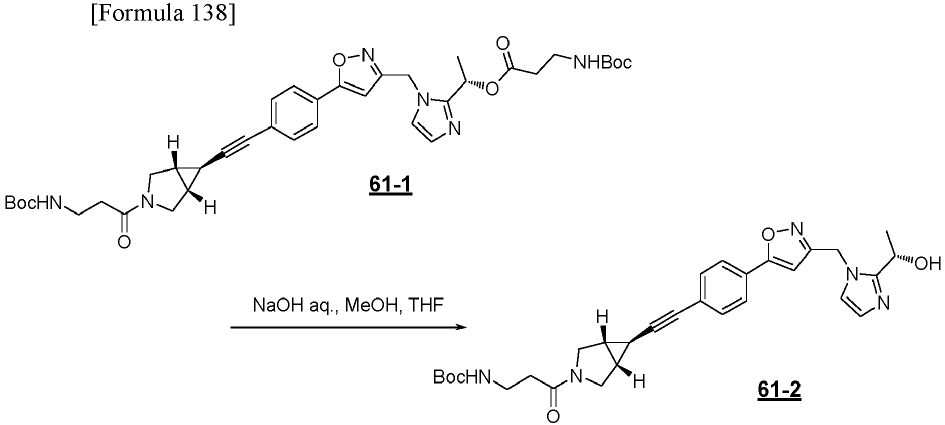

To a THF (0.20 mL) solution of compound 61-1 (21 mg), methanol (0.20 mL) and a 2 mol/L-aqueous sodium hydroxide solution (0.20 mL) were added, and the mixture was stirred at room temperature for 2 hours. To the reaction mixture, a 20% aqueous potassium carbonate solution was added, and the mixture was extracted with chloroform. The organic layer was separated using a phase separator, the solvent was concentrated under reduced pressure to obtain title compound 61-2 (colorless amorphous, 16 mg, yield 100%).
MS (ESI) m/z = 546 [M+1]⁺
LCMS retention time: 0.618 min (condition B)

### 61-3) Synthesis of 3-amino-1-[(1R,5S,6s)-6-({4-[3-({2-[(1S)-1-hydroxyethyl]-1H-imidazol-1-yl}methyl)-1,2-oxazol-5-yl]phenyllethynyl)-3-azabicyclo[3.1.0]hexan-3-yl]propan-1-one (compound 61)

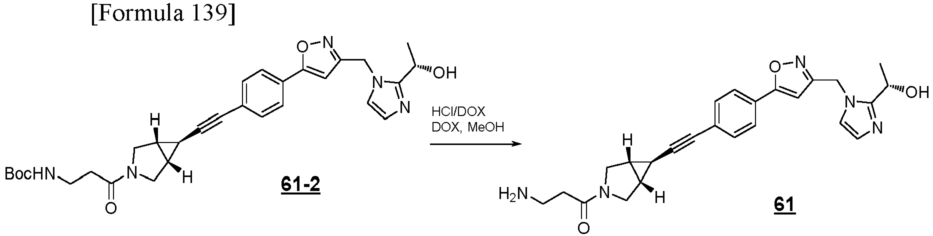

Compound 61 was produced by the same methods as in 10-2 of Example 3 with the use of the corresponding starting materials.

The ¹H-NMR and LCMS data are as shown in Table 2.

### Example 23

### (1S)-1-{1-[(5-{4-[(1-Aminocyclopropyl)ethynyl]phenyl}-1,2-oxazol-3-yl)methyl]-5-methyl-1H-imidazol-2-yl}ethan-1-ol (compound 64)

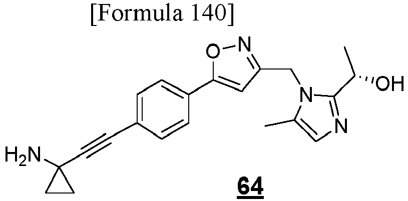

### 64-1) Synthesis of 5-methyl-2-{(1S)-1-[(oxan-2-yl)oxy]ethyl}-1H-imidazole

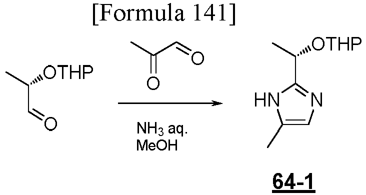

To a methanol (20 mL) solution of (2S)-2-tetrahydropyran-2-yloxypropanal (1.0 g), a 28% ammonia water (1.8 mL) and a 40% aqueous solution (2.3 g) of 2-oxopropanal were added, and the mixture was stirred at room temperature for 5 days. To the reaction mixture, a 20% aqueous potassium carbonate solution was added, the mixture was extracted with chloroform and separated using a phase separator, and the organic layer was concentrated under reduced pressure. The residue was purified by NH type silica gel column chromatography (gradient elution with chloroform/methanol = 100/0 to 95/5) and further purified by OH type silica gel column chromatography (gradient elution with n-hexane/ethyl acetate = 20/80 to 0/100 to gradient elution with chloroform/methanol = 98/2 to 92/8) to obtain title compound 64-1 (yellow syrup, 0.83 g, yield 62%).
MS (ESI) m/z = 211 [M+1]⁺
LCMS retention time: 0.504 min (condition A)

### 64-2) Synthesis of 5-(4-iodophenyl)-3-[(5-methyl-2-{(1S)-1-[(oxan-2-yl)oxy]ethyl}-1H-imidazol-1-yl)methyl]-1,2-oxazole (64-1a) and 5-(4-iodophenyl)-3-[(4-methyl-2-{(1S)-1-[(oxan-2-yl)oxy]ethyl}-1H-imidazol-1-yl)methyl]-1,2-oxazole (64-2b)

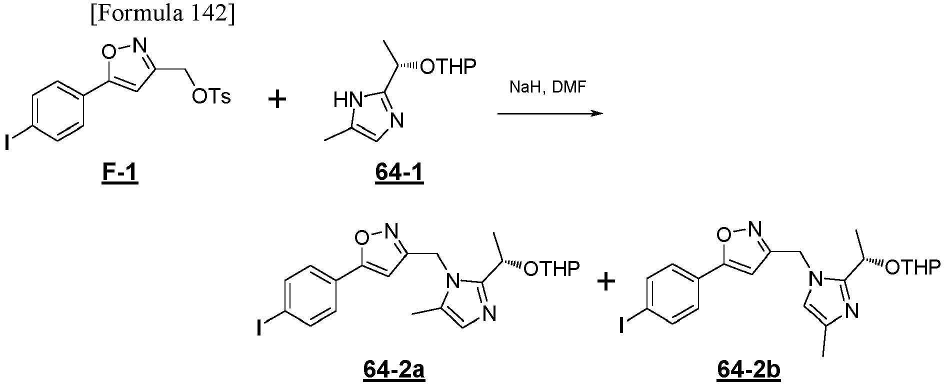

A DMF (5.0 mL) solution of compound 64-1 (0.44 g) was ice-cooled, 60% sodium hydride (92 mg) was added thereto, and the mixture was stirred. After heating to 60°C, a DMF (5.0 mL) solution of compound F-1 (0.87 g) was added, and the mixture was stirred for 1 hour. After the reaction mixture was allowed to cool, an aqueous saturated sodium bicarbonate solution was poured, and the mixture was extracted with ethyl acetate. The organic layer was washed three times with brine, dried over magnesium sulfate, and concentrated under reduced pressure.

The residue was purified by OH type silica gel column chromatography (gradient elution with chloroform/methanol = 98/2 to 94/6) and further purified by NH type silica gel column chromatography (gradient elution with n-hexane/ethyl acetate = 75/25 to 0/100) to obtain title compound 64-2a (colorless amorphous, 0.12 g, yield 12%) and title compound 64-2b (pale yellow syrup, 0.40 g, yield 42%).

The position of the methyl group in the imidazole ring of compound 64-2a and compound 64-2b was determined by the NOE analysis of ¹H-NMR (CDCl₃) shown below. The numbers represent shift values δ of hydrogen atoms (median value, ppm), and the arrows indicate that correlation was found in NOE.

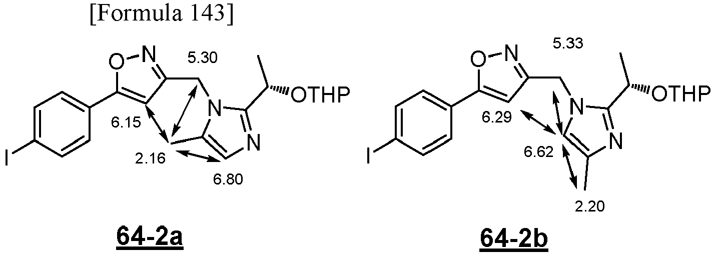

### Compound 64-2a

MS (ESI) m/z = 494 [M+1]⁺
LCMS retention time: 0.763 min (condition B)

### Compound 64-2b

MS (ESI) m/z = 494 [M+1]⁺
LCMS retention time: 0.761 min (condition B)

### 64-3) Synthesis of (1S)-1-{1-[(5-{4-[(1-aminocyclopropyl)ethynyl]phenyl}-1,2-oxazol-3-yl)methyl]-5-methyl-1H-imidazol-2-yl}ethan-1-ol (compound 64)

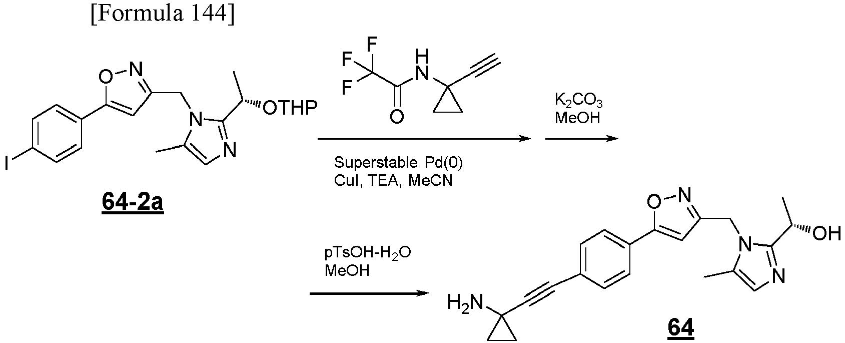

Compound 64 was produced by the same methods as in 4-1 to 4-3 of Example 2 with the use of the corresponding starting materials.

The ¹H-NMR and LCMS data are as shown in Table 2.

### Example 24

### (1S)-1-(1-{[5-(4-{[trans-2-(Methoxymethyl)cyclopropyl]ethynyl}phenyl)-1,2-oxazol-3-yl]methyl}-1H-imidazol-2-yl)ethan-1-ol (compound 66)

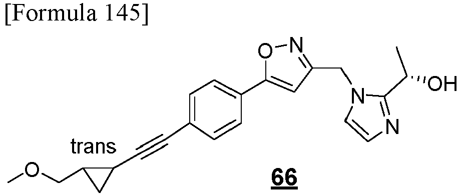

### 66-1) Synthesis of (1S)-1-(1-{[5-(4-{[trans-2-(methoxymethyl)cyclopropyl]ethynyl}phenyl)-1,2-oxazol-3-yl]methyl}-1H-imidazol-2-yl)ethan-1-ol (compound 66)

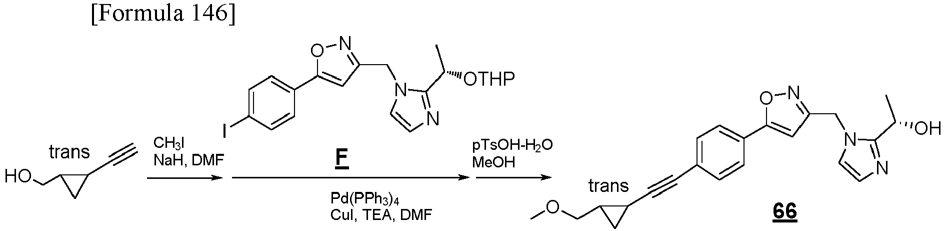

To a DMF (0.74 mL) solution of trans-1-ethynyl-2-(hydroxymethyl)cyclopropane (0.25 g, 34 wt% THF mixture), iodomethane (61 µL) and 60% sodium hydride (35 mg) were added under ice cooling, and the reaction mixture was warmed to room temperature. Since the raw materials were left, iodomethane (61 µL) and 60% sodium hydride (35 mg) were further added under ice cooling. After the mixture was stirred at room temperature for 20 min, the reaction mixture was ice-cooled, and the reaction was quenched by addition of water. The reaction mixture was extracted with diethyl ether, and the organic layer was dried over magnesium sulfate and concentrated to obtain a crude product. Compound 66 was produced by the same methods as in 10-1 and 4-3 with the use of the obtained crude product and intermediate F.

The ¹H-NMR and LCMS data are as shown in Table 2.

### Example 25

### 4-[(1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]butanoic acid (compound 67)

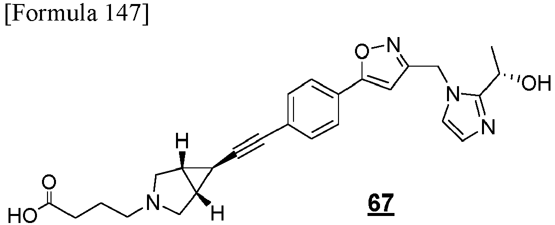

### 67-1) Synthesis of ethyl 4-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]butanoate

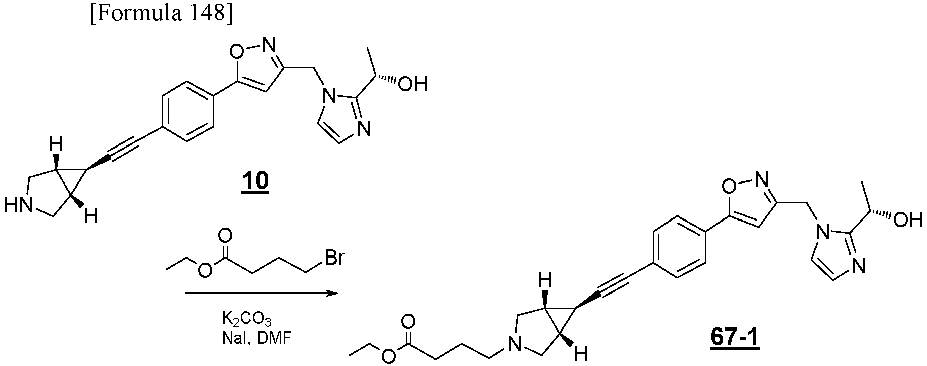

Compound 10 (0.30 g) and ethyl 4-bromobutyrate (0.17 g) were dissolved in DMF (7.5 mL), potassium carbonate (0.33 g) and sodium iodide (0.12 g) were added, and the mixture was stirred at 80°C for 80 min. After the reaction mixture was allowed to cool, a 20% aqueous potassium carbonate solution was added, and the mixture was extracted with ethyl acetate. After the organic layer was washed twice with a 20% aqueous potassium carbonate solution, ISOLUTE(registered trademark) HM-N was added, and the solvent was concentrated under reduced pressure. The residue was purified by NH type silica gel column chromatography (gradient elution with n-hexane/ethyl acetate = 25/75 to 0/100 to gradient elution with chloroform/methanol = 99/1 to 95/5) to obtain title compound 67-1 (pale yellow amorphous, 0.29 g, yield 75%).
MS (ESI) m/z = 245 [M+2]²⁺
LCMS retention time: 0.834 min (condition A)

### 67-2) Synthesis of 4-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]butanoic acid (compound 67)

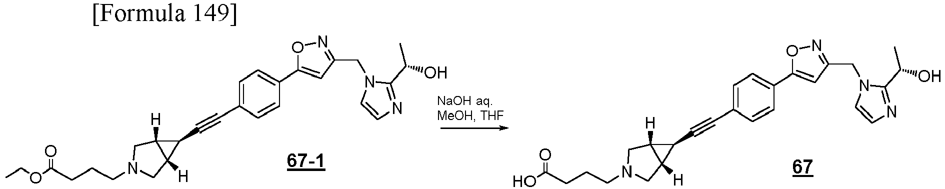

To a methanol (3.0 mL)/THF (3.0 mL) solution of compound 67-1 (0.29 g), a 2 mol/L-aqueous sodium hydroxide solution (3.0 mL) was added, and the mixture was stirred at room temperature for 2 hours. The reaction mixture was placed in an ice bath, and 1 mol/L-hydrochloric acid was added so that the mixture became neutral, and then, the solvent was distilled off under reduced pressure. This was dissolved in water, which was charged into a column packed with Diaion(registered trademark) HP-20, and water was passed through the column for desalting, followed by elution with methanol. The solvent was distilled of under reduced pressure to obtain title compound 67 (pale yellow amorphous, 0.21 g, yield 77%).

The ¹H-NMR and LCMS data are as shown in Table 2.

### Example 26

### trans-3-[(4-{3-[(1R)-2-Amino-1-{2-[(1S)-1-hydroxyethyl]-1H-imidazol-1-yl}ethyl]-1,2-oxazol-5-yl}phenyl)ethynyl]cyclobutan-1-ol (compound 70)

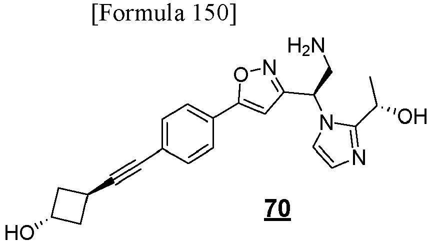

### 70-1) Synthesis of tert-butyl [(2R)-2-(5-{4-[(trans-3-{[tert-butyl(dimethyl)silyl]oxy}cyclobutyl)ethynyl]phenyl}-1,2-oxazol-3-yl)-2-{2-[(1S)-1-{[tert-butyl(dimethyl)silyl]oxy}ethyl]-1H-imidazol-1-yl}ethyl]carbamate

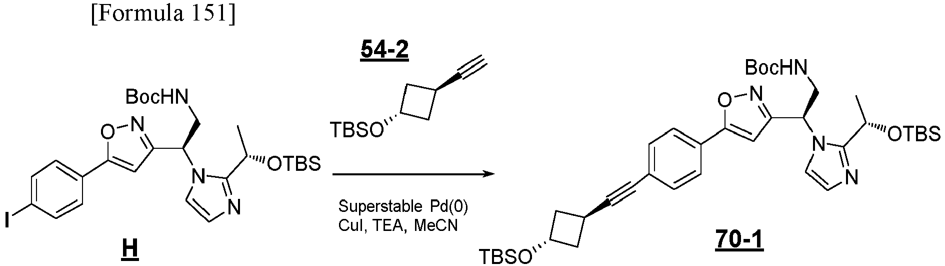

Compound 70-1 was produced by the same methods as in 10-1 of Example 3 with the use of the corresponding starting materials.
¹H NMR (400 MHz, CHLOROFORM-d) δ ppm 0.00 (s, 6 H), 0.05 (s, 6 H), 0.81 (s, 9 H), 0.89 (s, 9 H), 1.41 (s, 9 H), 1.66 (d, J=6.4 Hz, 3 H), 2.25 - 2.38 (m, 2 H), 2.39 - 2.50 (m, 2 H), 3.08 - 3.20 (m, 1 H), 3.76 - 3.86 (m, 1 H), 4.08 (dt, J=14.1, 7.2 Hz, 1 H), 4.55 - 4.66 (m, 1 H), 4.99 (br s, 1 H), 5.24 - 5.33 (m, 1 H), 6.14 - 6.27 (m, 2 H), 6.90 (s, 1 H), 6.97 (s, 1 H), 7.46 (d, J=7.9 Hz, 2 H), 7.60 (d, J=7.9 Hz, 2 H)

### 70-2) Synthesis of trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutan-1-ol (compound 70)

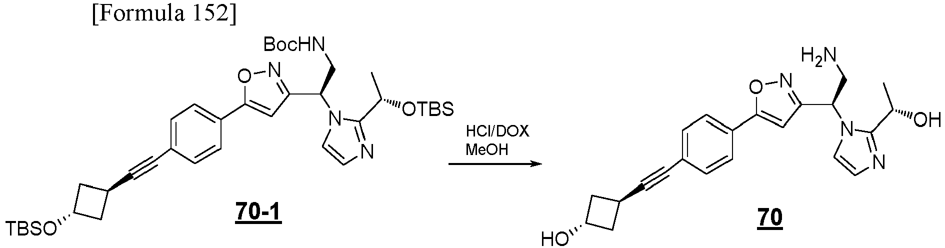

To compound 70-1 (0.54 g), methanol (1.0 mL) and a 4 mol/L-hydrochloric acid-DOX solution (10 mL) were added, and the mixture was stirred at room temperature for 5 hours. After the reaction mixture was concentrated under reduced pressure, a 20% aqueous potassium carbonate solution was added, and the mixture was extracted with a chloroform/methanol mixed solvent. The organic layer was separated using a phase separator, and the solvent was concentrated under reduced pressure. The residue was purified by NH type silica gel column chromatography (gradient elution with chloroform/methanol = 100/0 to 95/5) to obtain title compound 70 (colorless amorphous, 0.27 g, yield 94%).

The ¹H-NMR and LCMS data are as shown in Table 2.

### Example 27

### (1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexane-3-carboxamide (compound 71)

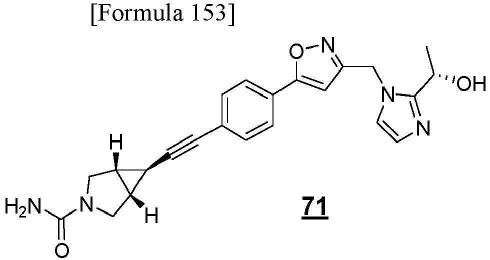

### 71-1) Synthesis of (1R,5S,6s)-6-({4-[3-({2-[(1S)-1-{[tert-butyl(dimethyl)silyl]oxy}ethyl]-1H-imidazol-1-yl}methyl)-1,2-oxazol-5-yl]phenyl}ethynyl)-3-azabicyclo[3.1.0]hexane)

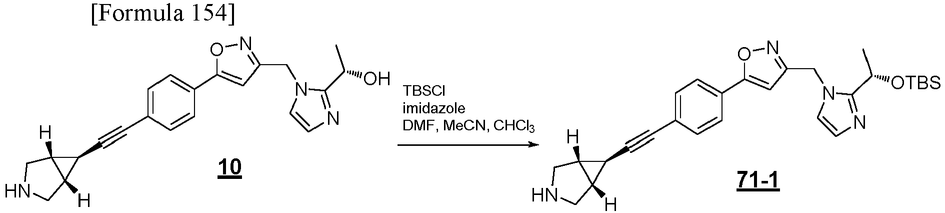

To a chloroform (6.8 mL) and acetonitrile (6.0 mL) suspension of compound 10 (0.23 g), imidazole (62 mg) and TBSC1 (0.12 g) were added, and the mixture was stirred at room temperature for 1.5 hours. Since the reaction did not proceed, the reaction mixture was concentrated, DMF (10 mL) was added to the residue for dissolution, and imidazole (62 mg) and TBSC1 (0.12 g) were added, followed by stirring at room temperature for 1 hour. To the reaction mixture, a 20% aqueous potassium carbonate solution was added, and the mixture was extracted with ethyl acetate. The organic layer was washed with brine and dried over magnesium sulfate, ISOLUTE(registered trademark) HM-N was added, and the solvent was distilled off under reduced pressure. The residue was purified by NH type silica gel column chromatography (gradient elution with chloroform/methanol = 99/1 to 90/10) to obtain title compound 71-1 (pale yellow amorphous, 0.14 g, yield 49%).
MS (ESI) m/z = 489 [M+1]⁺
LCMS retention time: 0.569 min (condition B)

### 71-2) Synthesis of (1R,5S,6s)-6-({4-[3-({2-[(1S)-I-{[tert-butyl(dimethyl)silyl]oxy}ethyl]-1H-imidazol-1-yl}methyl)-1,2-oxazol-5-yl]phenyl}ethynyl)-3-azabicyclo[3.1.0]hexane-3-carboxamide

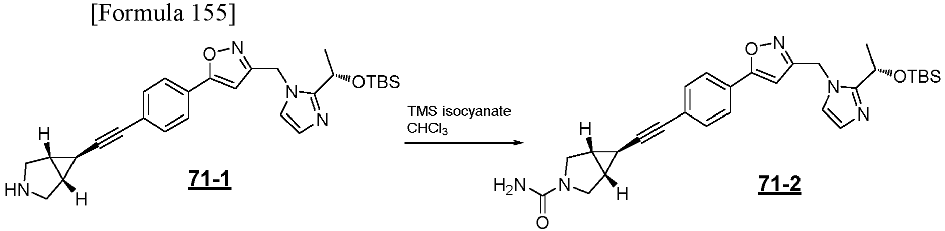

To a chloroform (1.5 mL) solution of compound 71-1 (38 mg), isocyanato(trimethyl)silane (33 µL) was added, and the mixture was stirred at room temperature for 2 hours. The reaction mixture was concentrated under reduced pressure, and the resulting residue was purified by NH type silica gel column chromatography (gradient elution with chloroform/methanol = 99/1 to 90/10) to obtain title compound 71-2 (colorless oil, 22 mg, yield 54%).
MS (ESI) m/z = 532 [M+1]⁺
LCMS retention time: 1.202 min (condition A)

### 71-3) Synthesis of (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carboxamide (compound 71)

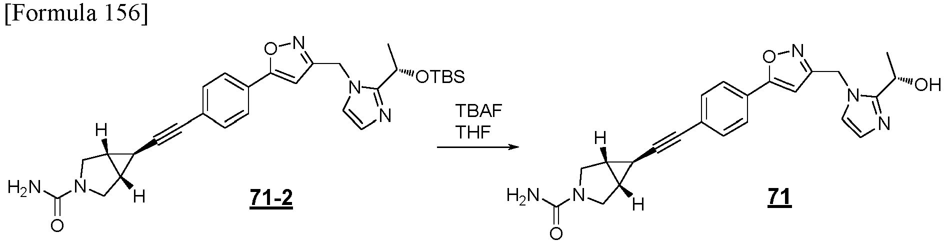

Compound 71 was produced by the same methods as in 19-2 of Example 11 with the use of the corresponding starting materials.

The ¹H-NMR and LCMS data are as shown in Table 2.

### Example 28

### (1S)-1-(1-{[5-(4-{[(1R,5S,6s)-3-(Methanesulfonyl)-3-azabicyclo[3.1.0]hexan-6-yl]ethynyl}phenyl)-1,2-oxazol-3-yl]methyl}-1H-imidazol-2-yl)ethan-1-ol (compound 72)

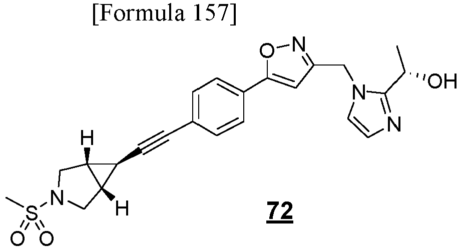

### 72-1) Synthesis of (1R,5S,6s)-6-({4-[3-({2-[(1S)-1-{[tert-butyl(dimethyl)silyl]oxy}ethyl]-1H-imidazol-1-yl}methyl)-1,2-oxazol-5-yl]phenyl}ethynyl)-3-(methanesulfonyl)-3-azabicyclo[3.1.0]hexane

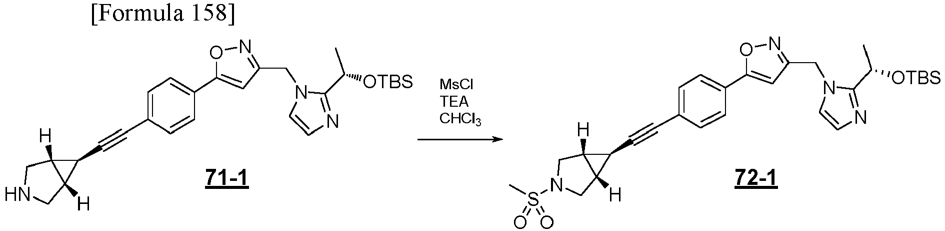

To a chloroform (1.1 mL) solution of compound 71-1 (27 mg), TEA (23 µL) and MsCl (9 µL) were added under ice cooling, and the mixture was stirred at room temperature for 1 hour. To the reaction mixture, an aqueous saturated sodium bicarbonate solution was added, the mixture was extracted with chloroform, and the organic layer was concentrated under reduced pressure. The residue was purified by OH type silica gel column chromatography (gradient elution with chloroform/methanol = 100/0 to 90/10) to obtain title compound 72-1 (yellow solid, 22 mg, yield 69%).
MS (ESI) m/z = 567 [M+1]⁺
LCMS retention time: 0.867 min (condition B)

### 72-2) Synthesis of (1S)-1-(1-{[5-(4-{[(1R,5S,6s)-3-(methanesulfonyl)-3-azabicyclo[3.1.0]hexan-6-yl]ethynyl}phenyl)-1,2-oxazol-3-yl]methyl}-1H-imidazol-2-yl)ethan-1-ol (compound 72)

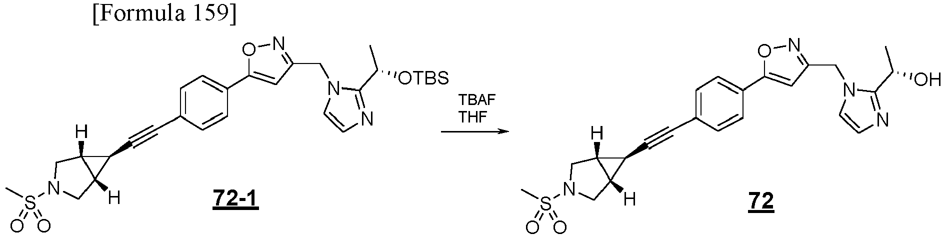

Compound 72 was produced by the same methods as in 19-2 of Example 11 with the use of the corresponding starting materials.

The ¹H-NMR and LCMS data are as shown in Table 2.

### Example 29

### (1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexane-3-carbaldehyde (compound 73)

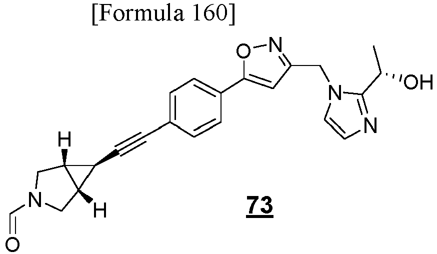

### 73-1) Synthesis of (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carbaldehyde (compound 73)

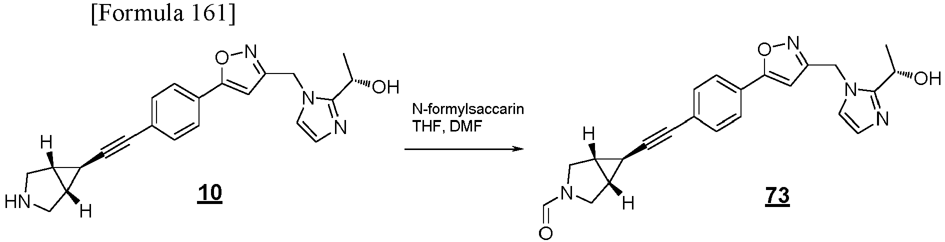

Compound 10 (20 mg) was dissolved in THF (1.0 mL) and DMF (0.50 mL), N-formyl saccharin (12 mg) was added, and the mixture was stirred at room temperature for 1 hour. The reaction mixture was diluted with chloroform, an aqueous saturated sodium bicarbonate solution was added, and the mixture was extracted with a chloroform/methanol mixed solvent and then concentrated under reduced pressure. The residue was purified by NH type silica gel column chromatography (gradient elution with ethyl acetate/methanol = 100/0 to 90/10) to obtain title compound 73 (colorless solid, 15 mg, yield 72%).

The ¹H-NMR and LCMS data are as shown in Table 2.

### Example 30

### 1-[(1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]-2-methoxyethan-1-one (compound 76)

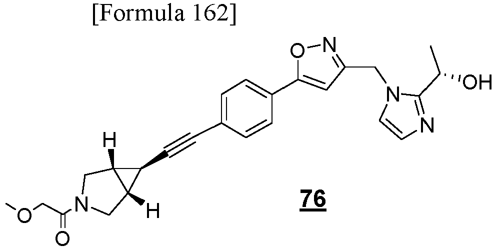

### 76-1) Synthesis of 1-[(1R,5S,6s)-6-({4-[3-({2-[(lS)-1-hydroxyethyl]-1H-imidazol-1-yl}methyl)-1,2-oxazol-5-yl]phenyl}ethynyl)-3-azabicyclo[3.1.0]hexan-3-yl]-2-methoxyethan-1-one (compound 76)

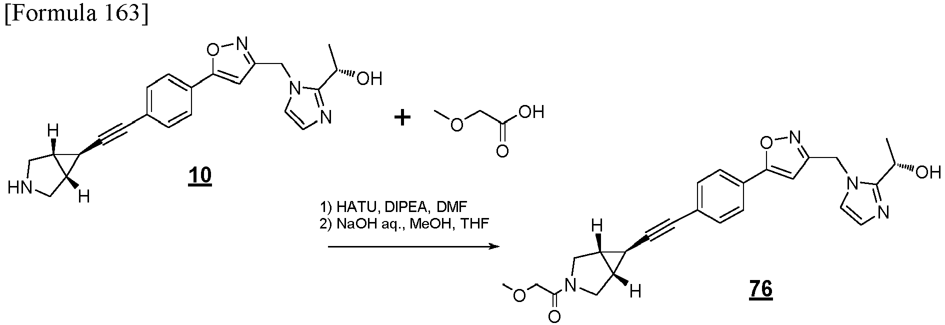

To a DMF (1.0 mL) solution of compound 10 (30 mg), 2-methoxyacetic acid (16 mg), HATU (67 mg), and DIPEA (42 µL) were added, and the mixture was stirred at room temperature for 3 hours. To the reaction mixture, an aqueous saturated sodium bicarbonate solution was added, the mixture was extracted with ethyl acetate, and the organic layer was concentrated under reduced pressure. To the resulting residue, THF (0.50 mL), methanol (0.50 mL), and a 2 mol/L-aqueous sodium hydroxide solution (0.50 mL) were added, and the mixture was stirred at room temperature for 1 hour. To the reaction mixture, a 20% aqueous potassium carbonate solution was added, and the mixture was extracted with chloroform. The organic layer was separated using a phase separator, ISOLUTE(registered trademark) HM-N was added, and the mixture was concentrated under reduced pressure. The residue was purified by NH type silica gel column chromatography (gradient elution with chloroform/methanol = 99/1 to 94/6) to obtain title compound 76 (colorless amorphous, 32 mg, yield 88%).

The ¹H-NMR and LCMS data are as shown in Table 2.

### Example 31

### Methyl (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carboxylate (compound 78)

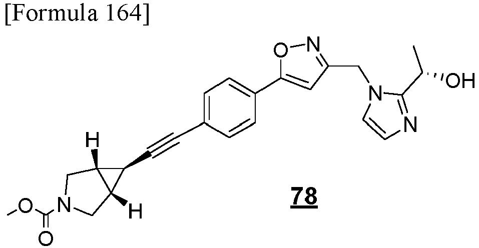

### 78-1) Synthesis of methyl (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carboxylate (compound 78)

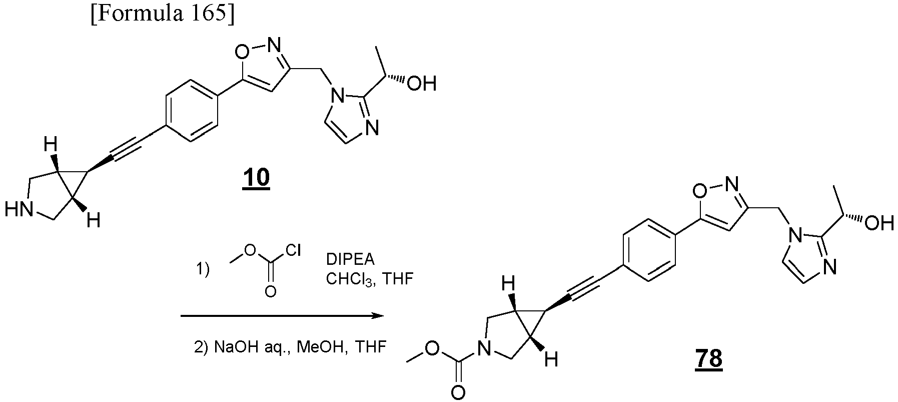

A chloroform (1.0 mL) and THF (1.0 mL) suspension of compound 10 (30 mg) was ice-cooled, DIPEA (42 µL) and methyl chloroformate (17 mg) were added, and the mixture was stirred at room temperature for 1 hour. To the reaction mixture, a 20% aqueous potassium carbonate solution was added, the mixture was extracted with chloroform, and the organic layer was separated using a phase separator and concentrated under reduced pressure. To the resulting residue, THF (0.50 mL), methanol (0.50 mL), and a 2 mol/L-aqueous sodium hydroxide solution (0.50 mL) were added, and the mixture was stirred at room temperature for 30 min. To the reaction mixture, a 20% aqueous potassium carbonate solution was added, and the mixture was extracted with chloroform. The organic layer was separated using a phase separator, ISOLUTE(registered trademark) HM-N was added, and the mixture was concentrated under reduced pressure. The residue was purified by NH type silica gel column chromatography (gradient elution with chloroform/methanol = 99/1 to 94/6) to obtain title compound 78 (colorless amorphous, 34 mg, yield 99%).

The ¹H-NMR and LCMS data are as shown in Table 2.

### Example 32

### (1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexane-3-sulfonamide hydrochloride (compound 81)

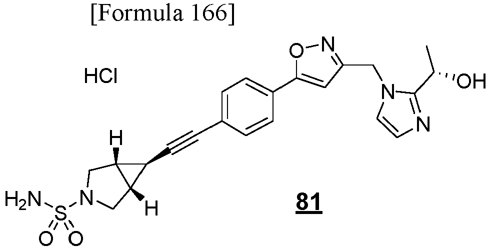

### 81-1) Synthesis of tert-butyl [(1R,5S,6s)-6-({4-[3-({2-[(1S)-1-{[tert-butyl(dimethyl)silyl]oxy}ethyl]-1H-imidazol-1-yl}methyl)-1,2-oxazol-5-yl]phenyl}ethynyl)-3-azabicyclo[3.1.0]hexane-3-sulfonyl]carbamate

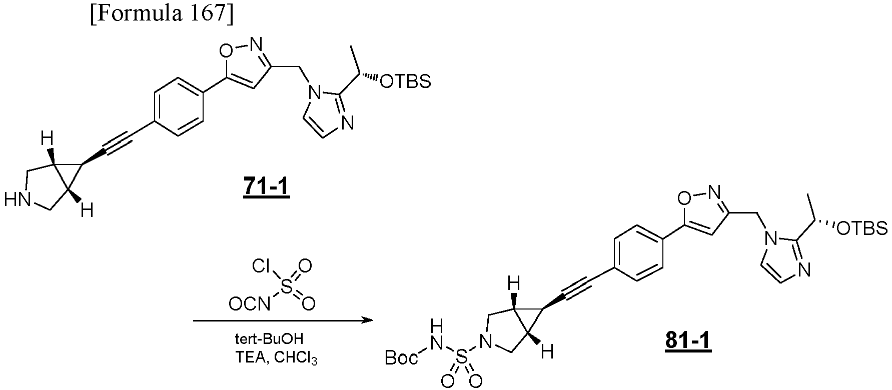

To a chloroform (1.2 mL) solution of sulfurisocyanatidoyl chloride (5.3 µL), 2-methylpropan-2-ol (8.1 µL) was added under ice cooling, and the mixture was stirred at the same temperature for 10 minutes. Then, TEA (10 µL) and compound 71-1 (30 mg) were added, and the mixture was stirred at the same temperature for 30 minutes. Furthermore, sulfurisocyanatidoyl chloride (5.3 µL) and 2-methylpropan-2-ol (8.1 µL) were added, and the mixture was stirred for 30 minutes. After water was added to the reaction mixture and stirred, the mixture was extracted with chloroform, followed by washing with brine. The aqueous layer was separated using a phase separator, the organic layer was concentrated under reduced pressure, and then, the resulting residue was purified by OH type silica gel column chromatography (gradient elution with chloroform/methanol = 99/1 to 83/17) to obtain title compound 81-1 (colorless oil, 24 mg, yield 60%).
MS (ESI) m/z = 668 [M+1]⁺
LCMS retention time: 0.992 min (condition B)

### 81-2) Synthesis of (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-sulfonamide hydrochloride (compound 81)

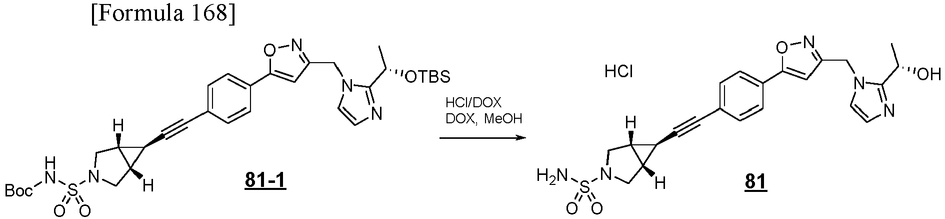

To compound 81-1 (24 mg), DOX (0.20 mL), methanol (0.30 mL), and a 4 mol/L-hydrochloric acid-DOX solution (1.0 mL) were added, and the mixture was stirred at room temperature for 60 minutes. The reaction mixture was concentrated under reduced pressure. To the resulting residue, methanol (0.30 mL) and a 4 mol/L-hydrochloric acid-DOX solution (1.0 mL) were added, and the mixture was further stirred at room temperature for 30 minutes. The reaction mixture was concentrated under reduced pressure and dried. To the resulting residue, ethyl acetate was added, and the solid was collected by filtration to obtain title compound 81 (colorless solid, 12 mg, yield 69%).

The ¹H-NMR and LCMS data are as shown in Table 2.

### Example 33

### (1S)-1-{1-[(1R)-2-Amino-1-{3-[4-(cyclopropylethynyl)phenyl]-1,2-oxazol-5-yl}ethyl]-1H-imidazol-2-yl}ethan-1-ol (compound 82)

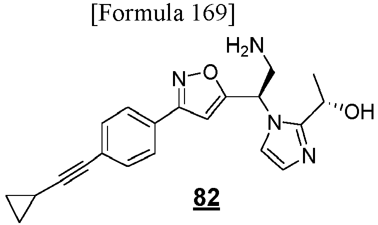

### 82-1) Synthesis of tert-butyl [(2S)-2-(2-{(1S)-1-[(oxan-2-yl)oxy]ethyl}-1H-imidazol-1-yl)but-3-yn-1-yl]carbamate

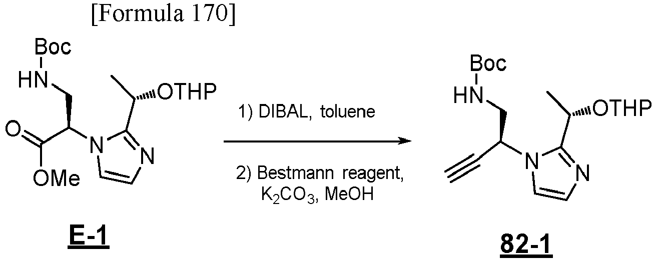

Compound 82-1 was produced by the same methods as in 54-1 and 54-2 of Example 20 with the use of the corresponding starting materials.
MS (ESI) m/z = 364 [M+1]⁺
LCMS retention time: 0.558 min (condition B)

### 82-2) Synthesis of tert-butyl [(2R)-2-[3-(4-iodophenyl)-1,2-oxazol-5-yl]-2-(2-{(1S)-1-[(oxan-2-yl)oxy]ethyl}-1H-imidazol-1-yl)ethyl]carbamate

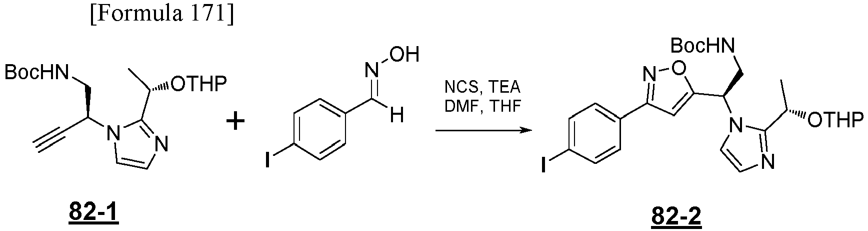

To a DMF (1.6 mL) solution of N-[(E)-(4-iodophenyl)methylidene]hydroxylamine (0.15 g), N-chlorosuccinimide (85 mg) was added, and the mixture was stirred at 55°C for 30 min. The mixture was returned to room temperature, which was subjected to separation operation with ethyl acetate and water. The organic layer was washed with water and brine, dried over magnesium sulfate, and concentrated under reduced pressure. The resulting residue was dissolved in THF (4.9 mL), compound 82-1 (0.18 g) and TEA (0.14 mL) were sequentially added, and the mixture was stirred at 55°C for 3 hours and then stirred at room temperature for 3 days. The reaction mixture was concentrated under reduced pressure, and chloroform and water were added to the resulting residue and stirred, followed by separation. The organic layer was concentrated under reduced pressure, and the resulting residue was purified by OH type silica gel column chromatography (gradient elution with n-hexane/ethyl acetate = 9/1 to 4/6) to obtain compound 82-2 (yellow oil, 0.12 g, yield 41%).
MS (ESI) m/z = 609 [M+1]⁺
LCMS retention time: 0.927 min (condition B)

### 82-3) Synthesis of (1S)-1-{1-[(1R)-2-amino-1-{3-[4-(cyclopropylethynyl)phenyl]-1,2-oxazol-5-yl}ethyl]-1H-imidazol-2-yl}ethan-1-ol (compound 82)

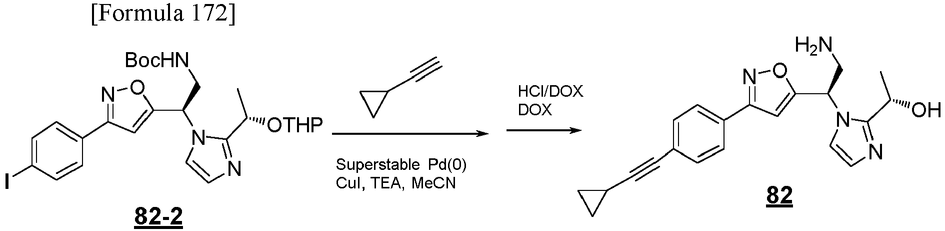

Compound 82 was produced by the same methods as in 10-1 and 10-2 of Example 3 with the use of the corresponding starting materials.

The ¹H-NMR and LCMS data are as shown in Table 2.

### Example 34

### cis-3-({4-[3-({2-[(1S)-1-Hydroxyethyl]-1H-imidazol-1-yl}methyl)-1,2-oxazol-5-yl]phenyl}ethynyl)cyclobutan-1-ol (compound 88)

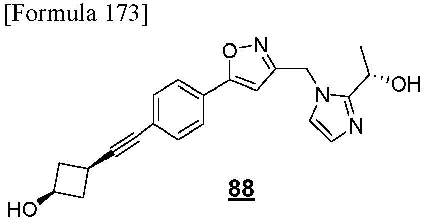

### 88-1) Synthesis of 5-{4-[(cis-3-{[tert-butyl(dimethyl)silyl]oxy}cyclobutyl)ethynyl]phenyl}-3-[(2-{(1S)-1-[(oxan-2-yl)oxy]ethyl}-1H-imidazol-1-yl)methyl]-1,2-oxazole

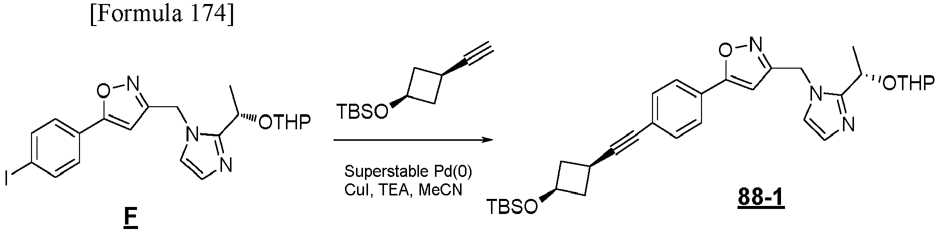

Compound 88-1 was produced by the same methods as in 10-1 of Example 3 with the use of the corresponding starting materials.
MS (ESI) m/z = 562 [M+1]⁺
LCMS retention time: 1.167 min (condition B)

### 88-2) Synthesis of cis-3-({4-[3-({2-[(1S)-1-hydroxyethyl]-1H-imidazol-1-yl}methyl)-1,2-oxazol-5-yl]phenyl}ethynyl)cyclobutan-1-ol (compound 88)

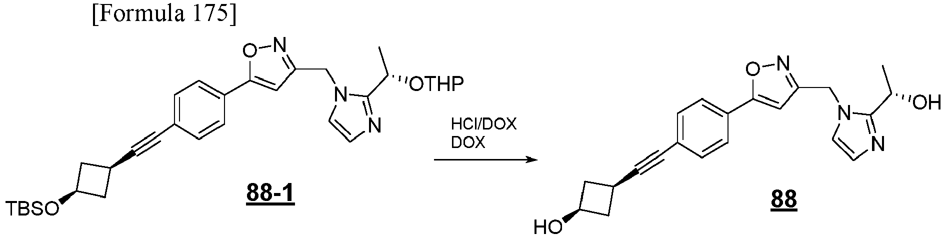

Compound 88 was produced by the same methods as in 10-2 of Example 3 with the use of the corresponding starting materials.

The ¹H-NMR and LCMS data are as shown in Table 2.

### Example 35

### trans-3-[(4-{3-[(1R)-2-Amino-1-{2-[(1S)-1-hydroxyethyl]-1H-imidazol-1-yl}ethyl]-1,2-oxazol-5-yl}phenyl)ethynyl]-1-(hydroxymethyl)cyclobutan-1-ol (compound 89)

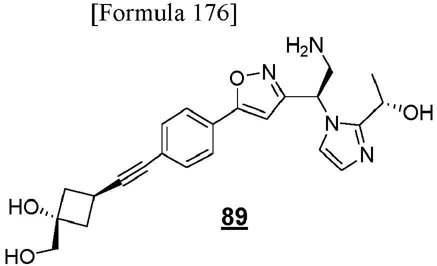

### 89-1) Synthesis of methyl (2r,4r)-6,6-dimethyl-5,7-dioxaspiro[3.4]octane-2-carboxylate (89-1a) and methyl (2s,4s)-6,6-dimethyl-5,7-dioxaspiro[3.4]octane-2-carboxylate (89-1b)

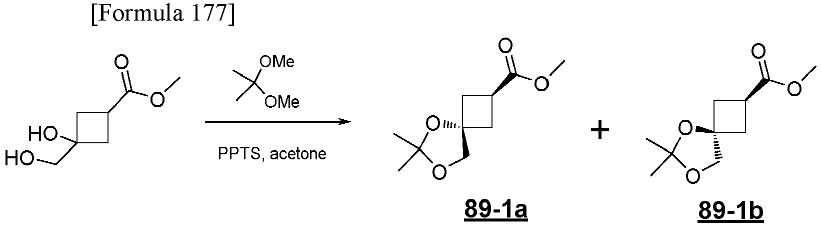

Methyl 3-hydroxy-3-(hydroxymethyl)cyclobutane-1-carboxylate (0.41 g) was dissolved in acetone (25 mL), 2,2-dimethoxypropane (6.2 mL) and PPTS (64 mg) were added, and the mixture was stirred at room temperature for 3 days. The reaction mixture was concentrated, and the resulting residue was purified by OH type silica gel column chromatography (gradient elution with n-hexane/ethyl acetate = 95/5 to 80/20) to obtain compound 89-1a (pale yellow oil, 0.23 g, yield 45%) and compound 89-1b (pale yellow oil, 0.18 g, yield 36%).

### Compound 89-1a

¹H NMR (400 MHz, CHLOROFORM-d) δ ppm 1.36 (s, 6 H), 2.41 - 2.51 (m, 2 H), 2.58 - 2.68 (m, 2 H), 3.06 (tt, J=9.9, 4.7 Hz, 1 H), 3.69 (s, 3 H), 4.02 (s, 2 H)

### Compound 89-1b

¹H NMR (400 MHz, CHLOROFORM-d) δ ppm 1.36 (s, 6 H), 2.34 - 2.47 (m, 2 H), 2.57 - 2.71 (m, 3 H), 3.69 (s, 3 H), 3.95 (s, 2 H)

### 89-2) Synthesis of (2r,4r)-2-ethynyl-6,6-dimethyl-5,7-dioxaspiro[3.4]octane

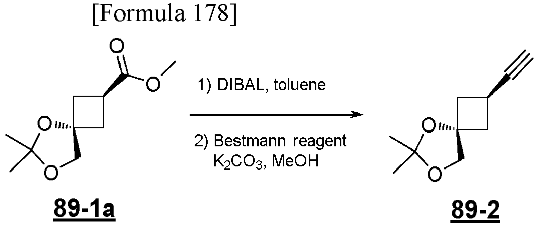

Compound 89-2 was produced by the same methods as in 54-1 and 54-2 of Example 20 with the use of the corresponding starting materials.
¹H NMR (400 MHz, CHLOROFORM-d) δ ppm 1.36 (s, 6 H), 2.13 (s, 1 H), 2.22 - 2.32 (m, 2 H), 2.59 - 2.72 (m, 2 H), 2.89 - 3.00 (m, 1 H), 4.09 (s, 2 H)

### 89-3) Synthesis of trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]-1-(hydroxymethyl)cyclobutan-1-ol (compound 89)

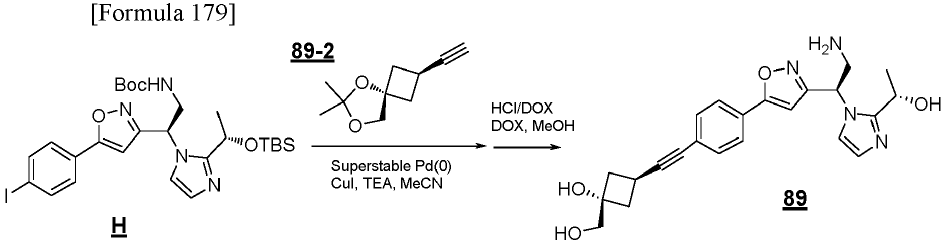

Compound 89 was produced by the same methods as in 10-1 and 22-3 with the use of the corresponding starting materials.

The ¹H-NMR and LCMS data are as shown in Table 2.

### Example 36

### 1-{(1R,5S,6s)-6-[(4-{3-[(1R)-2-Amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}ethan-1-one (compound 91)

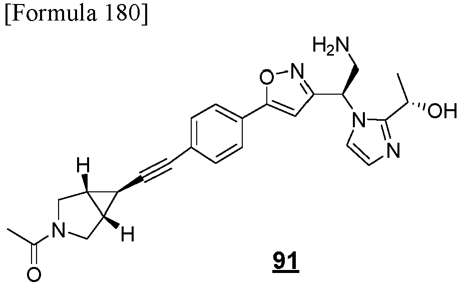

### 91-1) Synthesis of 1-[(1R,5S,6s)-6-ethynyl-3-azabicyclo[3.1.0]hexan-3-yl]ethan-1-one

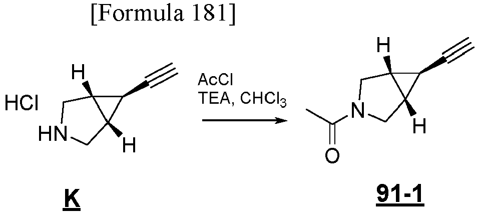

To a chloroform (1.0 mL) suspension of intermediate K (78 mg), TEA (0.38 mL) and acetyl chloride (86 µL) were added under ice cooling, and the mixture was stirred at room temperature for 2 hours. Thereafter, the reaction mixture was ice-cooled, TEA (0.76 mL) and acetyl chloride (86 µL) were added, and the mixture was stirred at room temperature for 30 min. Water was added to the reaction mixture, the mixture was extracted with chloroform, and the organic layer was concentrated under reduced pressure. The residue was purified by NH type silica gel column chromatography (gradient elution with chloroform/methanol = 100/0 to 90/10) and OH type silica gel column chromatography (gradient elution with n-hexane/ethyl acetate = 75/25 to 0/100) to obtain title compound 91-1 (pale yellow solid, 61 mg, yield 75%).
MS (ESI) m/z = 150 [M+1]⁺
LCMS retention time: 0.478 min (condition B)

### 91-2) Synthesis of 1-{(1R,5S,6s)-6-[(4-{3-[(1R)-2-amino-1-{2-[(1S)-1-hydroxy ethyl]-1H-imidazol-1-yl}ethyl]-1,2-oxazol-5-yl}phenyl)ethynyl]-3-azabicyclo[3.1.0]hexan-3-yl}ethan-1-one (compound 91)

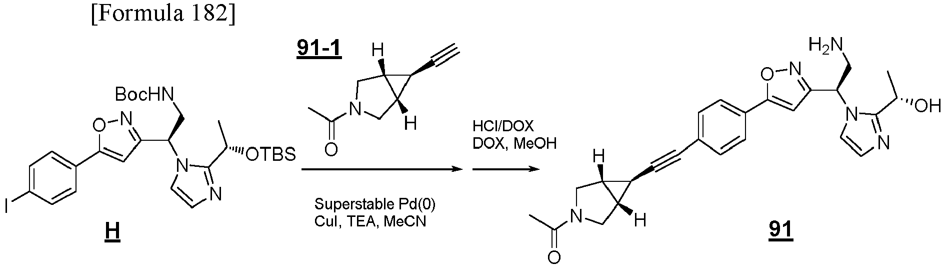

Compound 91 was produced by the same methods as in 10-1 and 22-3 with the use of the corresponding starting materials.

The ¹H-NMR and LCMS data are as shown in Table 2.

### Example 37

### (1R,5S,6s)-6-({4-[3-({2-[(1S)-1-Hydroxyethyl]-1H-imidazol-1-yl}methyl)-1,2-oxazol-5-yl]phenyl}ethynyl)-N-methyl-3-azabicyclo[3.1.0]hexan-3-carboxamide (compound 92)

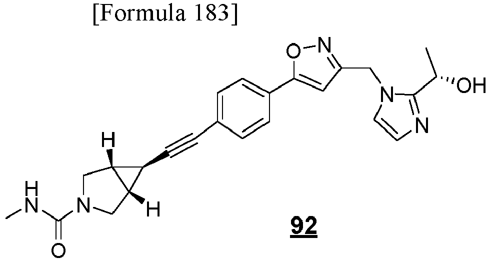

### 92-1) Synthesis of (1R,5S,6s)-6-({4-[3-({2-[(1S)-1-{[tert-butyl(dimethyl)silyl]oxy}ethyl]-1H-imidazol-1-yl}methyl)-1,2-oxazol-5-yl]phenyl}ethynyl)-N-methyl-3-azabicyclo[3.1,0]hexane-3-carboxamide

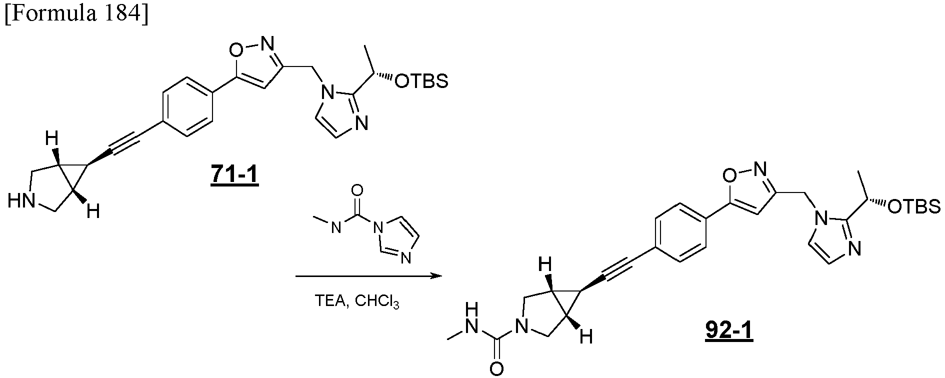

To a chloroform (0.80 mL) solution of 71-1 (40 mg), TEA (34 µL) and N-methylimidazol-1-carboxamide (25 mg) were added under ice cooling, followed by stirring at room temperature overnight, and the reaction mixture was concentrated under reduced pressure. The residue was purified by NH type silica gel column chromatography (gradient elution with ethyl acetate/methanol = 100/0 to 90/10) and OH type silica gel column chromatography (gradient elution with chloroform/methanol = 100/0 to 90/10) to obtain title compound 92-1 (colorless oil, 28 mg, yield 63%).
MS (ESI) m/z = 546 [M+1]⁺
LCMS retention time: 0.814 min (condition B)

### 92-2) Synthesis of (1R,5S,6s)-6-({4-[3-({2-[(1S)-1-hydroxyethyl]-1H-imidazol-1-yl}methyl)-1,2-oxazol-5-yl]phenyl}ethynyl)-N-methyl-3-azabicyclo[3.1.0]hexane-3-carboxamide (compound 92)

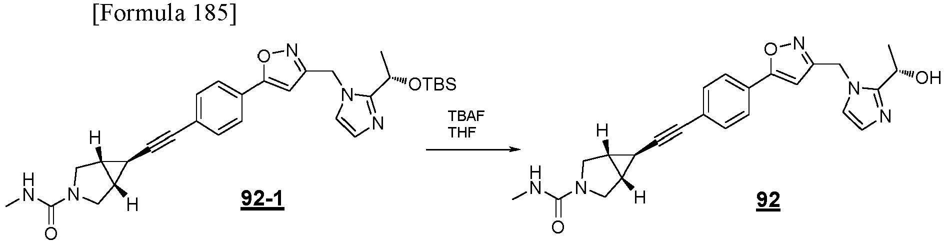

Compound 92 was produced by the same methods as in 19-2 of Example 11 with the use of the corresponding starting materials.

The ¹H-NMR and LCMS data are as shown in Table 2.

### Example 38

### (1S)-1-(1-{(1R)-2-Amino-1-[5-(4-{[(1R,5S,6s)-3-(2-methoxyethyl)-3-azabicyclo[3.1.0]hexan-6-yl]ethynyl}phenyl)-1,2-oxazol-3-yl]ethyl}-1H-imidazol-2-yl)ethan-1-ol (compound 101)

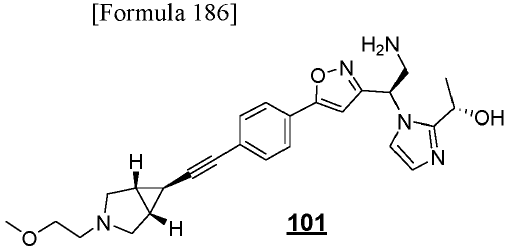

### 101-1) Synthesis of (1R,5S,6s)-6-ethynyl-3-(2-methoxyethyl)-3-azabicyclo[3.1.0]hexane

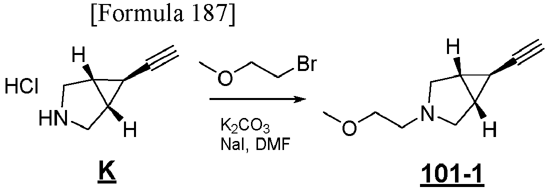

To a DMF (2.5 mL) suspension of intermediate K (72 mg), potassium carbonate (0.35 g), sodium iodide (75 mg), and 1-bromo-2-methoxyethane (72 µL) were added under ice cooling, and the mixture was heated and stirred at 85°C for 1 hour. After the reaction mixture was allowed to cool, an aqueous saturated sodium bicarbonate solution was added, the mixture was extracted with an ethyl acetate/toluene mixed solvent, and the organic layer was dried over magnesium sulfate and concentrated under reduced pressure. The residue was purified by OH type silica gel column chromatography (gradient elution with n-hexane/ethyl acetate = 50/50 to 0/100) to obtain title compound 101-1 (orange oil, 61 mg, yield 74%).
MS (ESI) m/z = 166 [M+1]⁺
LCMS retention time: 0.242 min (condition A)

### 101-2) Synthesis of (1S)-1-(1-{(1R)-2-amino-1-[5-(4-{[(1R,5S,6s)-3-(2-methoxyethyl)-3-azabicyclo[3.1.0]hexan-6-yl]ethynyl}phenyl)-1,2-oxazol-3-yl]ethyl}-1H-imidazol-2-yl)ethan-1-ol (compound 101)

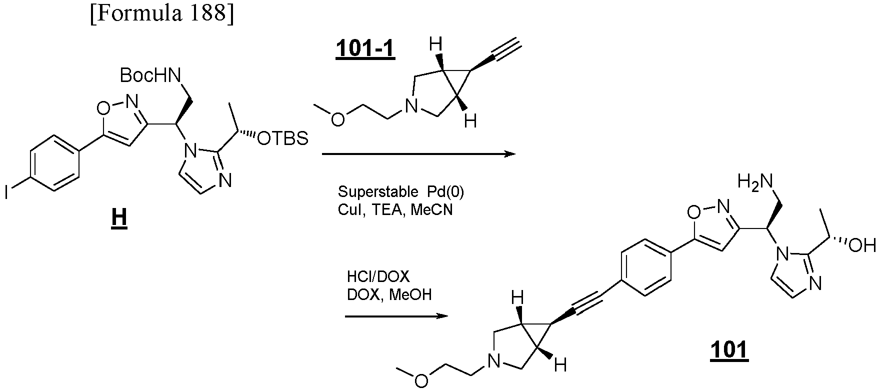

Compound 101 was produced by the same methods as in 10-1 of Example 3 and 22-3 of Example 12 with the use of the corresponding starting materials.

The ¹H-NMR and LCMS data are as shown in Table 2.

### Example 39

### N-[trans-3-({4-[3-({2-[(1S)-1-Hydroxyethyl]-1H-imidazol-1-yl}methyl)-1,2-oxazol-5-yl]phenyl}ethynyl)cyclobutyl]formamide (compound 103)

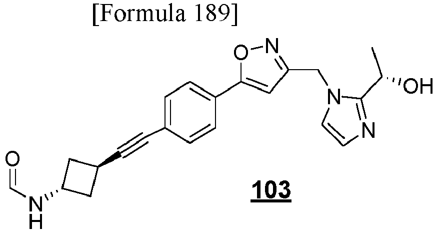

### 103-1) Synthesis of tert-butyl (trans-3-ethynylcyclobutyl)carbamate

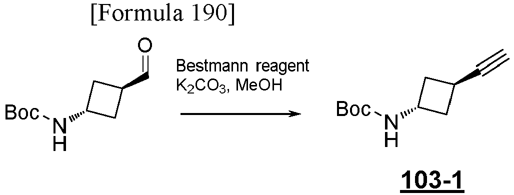

Compound 103-1 was produced by the same methods as in 54-2 of Example 20 with the use of the corresponding starting materials.
¹H NMR (400 MHz, CHLOROFORM-d) δ ppm 1.44 (s, 9 H), 2.09 - 2.24 (m, 3 H), 2.39 - 2.51 (m, 2 H), 2.88 - 2.97 (m, 1 H), 4.25 - 4.52 (m, 1 H), 4.55 - 4.76 (m, 1 H)

### 103-2) Synthesis of trans-3-ethynylcyclobutan-1-amine hydrochloride

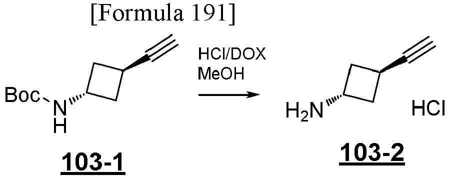

To a methanol (0.20 mL) solution of compound 103-1 (89 mg), a 4 mol/L-hydrochloric acid-DOX solution (1.0 mL) was added, and the mixture was stirred at room temperature for 1 hour. The reaction mixture was concentrated under reduced pressure to obtain compound 103-2 (brown solid, 59 mg, yield 99%).
¹H NMR (400 MHz, METHANOL-d4) δ ppm 2.35 - 2.61 (m, 5 H), 3.04 - 3.22 (m, 1 H), 3.85 - 4.00 (m, 1 H)

### 103-3) Synthesis of N-(trans-3-ethynylcyclobutyl)formamide

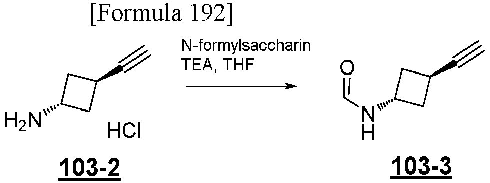

To a THF (2.0 mL) solution of compound 103-2 (59 mg), TEA (63 µL) and N-formyl saccharin (0.11 g) were sequentially added under ice cooling. After the mixture was stirred at room temperature for 1 hour, TEA (63 µL) was added, and the mixture was further stirred at room temperature for 1 hour. The reaction mixture was diluted with chloroform, an aqueous saturated sodium bicarbonate solution was added, and the mixture was extracted three times with a chloroform/methanol mixed solvent. The organic layer was separated using a phase separator and concentrated under reduced pressure. The residue was purified by OH type silica gel column chromatography (gradient elution with n-hexane/ethyl acetate = 75/25 to 0/100 to gradient elution with ethyl acetate/methanol = 99/1 to 90/10) to obtain title compound 103-3 (colorless solid, 35 mg, yield 63%).
MS (ESI) m/z = 124 [M+1]⁺
LCMS retention time: 0.441 min (condition B)

### 103-4) Synthesis of N-[trans-3-({4-[3-({2-[(1S)-1-hydroxyethyl]-1H-imidazol-1-yl}methyl)-1,2-oxazol-5-yl]phenyl}ethynyl)cyclobutyl]formamide (compound 103)

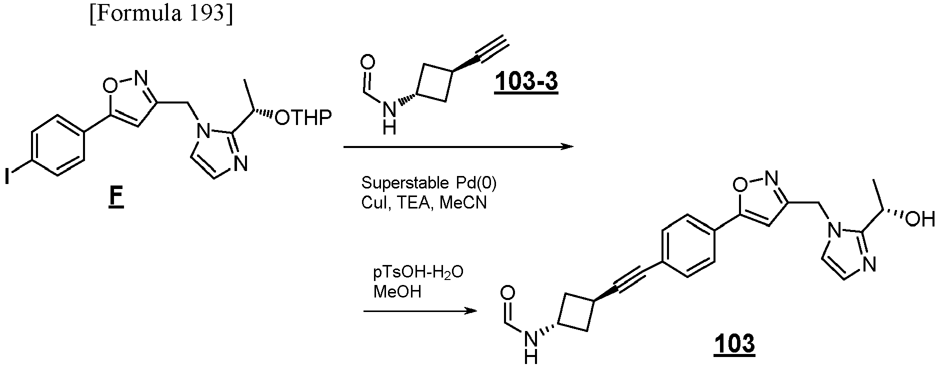

Compound 103 was produced by the same methods as in 4-1 and 4-3 of Example 2 with the use of the corresponding starting materials.

The ¹H-NMR and LCMS data are as shown in Table 2.

### Example 40

### 2-[(1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]-N-methyl-2-oxoacetamide (compound 105)

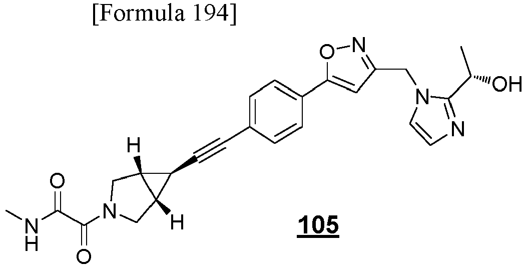

### 105-1) Synthesis of 2-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-N-methyl-2-oxoacetamide (compound 105)

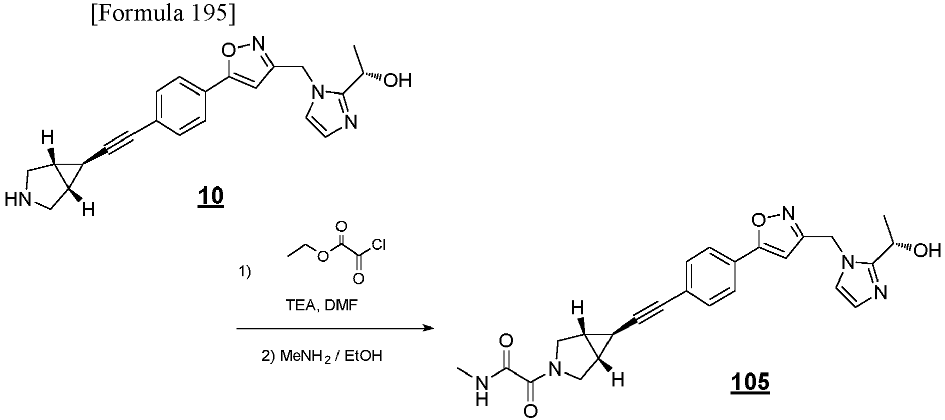

To a DMF (1.1 mL) solution of compound 10 (43 mg), TEA (48 µL) and chloro(oxo)ethyl acetate (27 µL) were added under ice cooling. After the mixture was stirred for 1.5 hours, an aqueous saturated sodium bicarbonate solution was added, and the mixture was extracted with ethyl acetate. The aqueous layer was extracted with ethyl acetate, the organic layer was dried over magnesium sulfate, and then, the solvent was distilled off under reduced pressure. To the residue, a 33 wt% methylamine-ethanol solution (1.0 mL) was added at room temperature, and the mixture was stirred for 20 hours and concentrated under reduced pressure. The residue was purified by OH type silica gel column chromatography (gradient elution with chloroform/methanol = 100/0 to 95/5) to obtain title compound 105 (pale yellow amorphous, 40 mg, yield 79%).

The ¹H-NMR and LCMS data are as shown in Table 2.

### Example 41

### (1S)-1-(1-{[5-(4-{[2,3-Bis(hydroxymethyl)cyclopropyl]ethynyl}phenyl)-1,2-oxazol-3-yl]methyl}-1H-imidazol-2-yl)ethan-1-ol (compound 109)

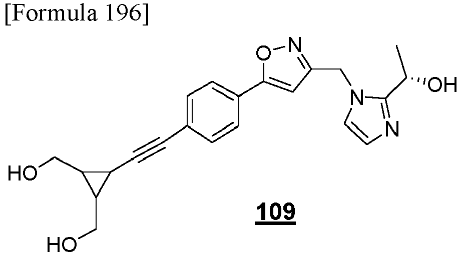

### 109-1) Synthesis of {3-[(trimethylsilyl)ethynyl]cyclopropane-1,2-diyl}dimethanol

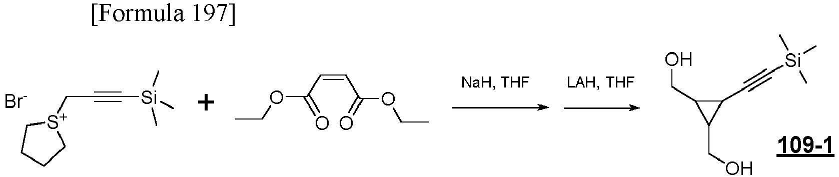

To a THF (12 mL) suspension of 1-[3-(trimethylsilyl)prop-2-in-1-yl]thiolan-1-ium bromide (0.67 g), 60% sodium hydride (0.14 g) was added under ice cooling, the mixture was stirred at room temperature for 30 min, and then, diethyl maleate (0.39 mL) was added, and the mixture was stirred at room temperature for 2 hours. The reaction mixture was poured into an ice-cooled aqueous saturated ammonium chloride solution and extracted with EtOAc, and the organic layer was washed with brine and concentrated under reduced pressure. The residue was purified by OH type silica gel column chromatography (gradient elution with n-hexane/ethyl acetate = 90/10 to 75/25) to obtain a crude product. A THF (3.0 mL) solution of the crude product obtained above was added dropwise via syringe to a solution obtained by placing LAH (0.11 g) into a reaction vessel under a nitrogen atmosphere, adding THF (10 mL), and stirring under ice cooling. After the mixture was stirred at 0°C for 2 hours, EtOAc (1.2 mL) was added dropwise to the reaction mixture, and anhydrous sodium sulfate (1.3 g) was added, followed by stirring for 30 min. Water was added to the reaction mixture, followed by extraction with EtOAc, and the organic layer was washed with brine and concentrated under reduced pressure. The residue was purified by OH type silica gel column chromatography (gradient elution with n-hexane/ethyl acetate = 50/50 to 0/100) to obtain title compound 109-1 (colorless oil, 0.11 g, yield 45%).
MS (ESI) m/z = 199 [M+1]⁺
LCMS retention time: 0.836 min (condition B)

### 109-2) Synthesis of [{3-[(trimethylsilyl)ethynyl]cyclopropane-1,2-diyl}bis(methyleneoxy)]bis[tert-butyl(dimethyl)silane]

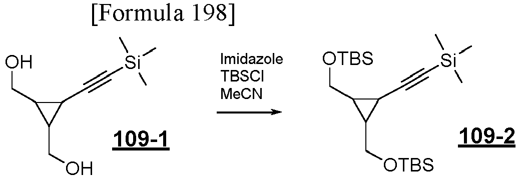

To an acetonitrile (5.4 mL) solution of compound 109-1 (0.11 g), imidazole (0.19 g) and TBSC1 (0.25 g) were added under ice cooling, and the mixture was stirred at room temperature for 1 hour. Water was added to the reaction mixture, the mixture was extracted with chloroform, and the organic layer was concentrated. The residue was purified by OH type silica gel column chromatography (gradient elution with n-hexane/ethyl acetate = 100/0 to 75/25) to obtain title compound 109-2 (colorless oil, 0.20 g, yield 87%).
¹H NMR (400 MHz, CHLOROFORM-d) δ ppm 0.03 (s, 6 H), 0.08 (s, 6 H), 0.13 (s, 9 H), 0.87 (s, 9 H), 0.91 (s, 9 H), 1.10 - 1.19 (m, 1 H), 1.22 - 1.35 (m, 1 H), 1.48 (dd, J=8.3, 5.1 Hz, 1 H), 3.58 - 3.65 (m, 2 H), 3.66 - 3.73 (m, 1 H), 3.76 - 3.84 (m, 1 H)

### 109-3) Synthesis of [(3-ethynylcyclopropane-1,2-diyl)bis(methyleneoxy)]bis[tert-butyl(dimethyl)silane]

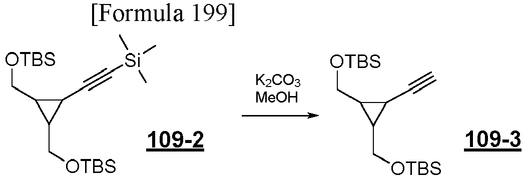

To a MeOH (4.7 mL) solution of compound 109-2 (0.20 g), potassium carbonate (98 mg) was added, and the mixture was stirred at room temperature for 3 hours. To the reaction mixture, an aqueous saturated ammonium chloride solution was added, the mixture was extracted with chloroform, and the organic layer was concentrated under reduced pressure. The residue was purified by OH type silica gel column chromatography (gradient elution with n-hexane/ethyl acetate = 100/0 to 90/10) to obtain title compound 109-3 (colorless oil, 0.16 g, yield 97%).
¹H NMR (400 MHz, CHLOROFORM-d) δ ppm 0.04 (s, 6 H), 0.08 (s, 6 H), 0.88 (s, 9 H), 0.91 (s, 9 H), 1.11 - 1.20 (m, 1 H), 1.23 - 1.34 (m, 1 H), 1.39 - 1.49 (m, 1 H), 1.83 (s, 1 H), 3.56 - 3.68 (m, 2 H), 3.68 - 3.83 (m, 2 H)

### 109-4) Synthesis of (1S)-1-(1-{[5-(4-{[2,3-bis(hydroxymethyl)cyclopropyl]ethynyl}phenyl)-1,2-oxazol-3-yl]methyl}-1H-imidazol-2-yl)ethan-1-ol (compound 109)

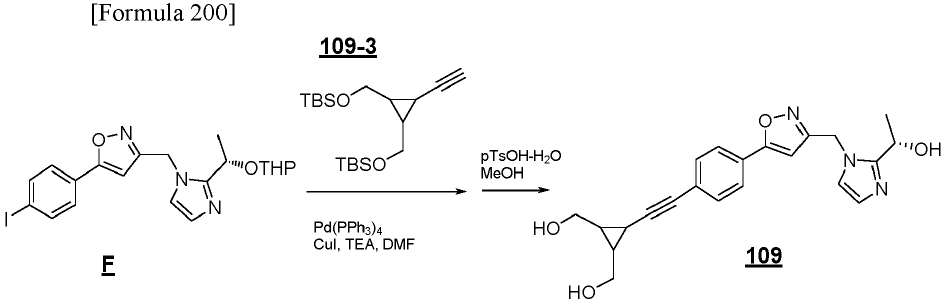

Compound 109 was produced by the same methods as in 4-1 and 4-3 with the use of the corresponding starting materials and reagents.

The ¹H-NMR and LCMS data are as shown in Table 2.

### Example 42

### (1S)-1-[1-({5-[4-({3-[(Oxetan-3-yl)amino]cyclobutyl}ethynyl)phenyl]-1,2-oxazol-3-yl}methyl)-1H-imidazol-2-yl]ethan-1-ol (compound 112)

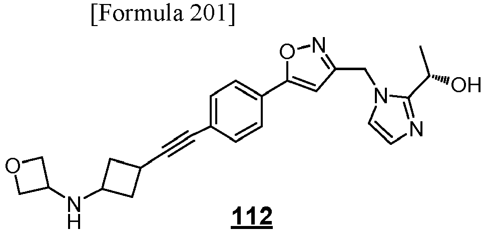

### 112-1) Synthesis of cis-3-[(4-{3-[(2-{(1S)-1-[(oxan-2-yl)oxy]ethyl}-1H-imidazol-1-yl)methyl]-1,2-oxazol-5-yl}phenyl)ethynyl]cyclobutan-1-ol

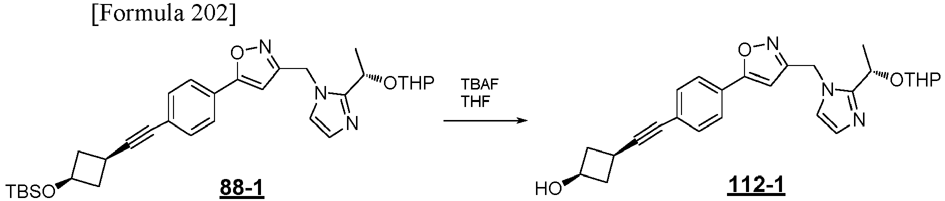

Compound 112-1 was produced by the same methods as in 19-2 of Example 11 with the use of the corresponding starting materials.
MS (ESI) m/z = 448 [M+1]⁺
LCMS retention time: 0.646 min (condition B)

### 112-2) Synthesis of cis-3-[(4-{3-[(2-{(1S)-1-[(oxan-2-yl)oxy]ethyl}-1H-imidazol-1-yl)methyl]-1,2-oxazol-5-yl}phenyl)ethynyl]cyclobutyl 4-methylbenzene-1-sulfonate

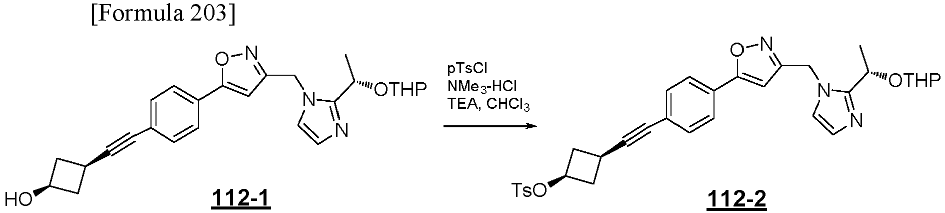

To a chloroform (2.9 mL) solution of compound 112-1 (0.13 g), TEA (61 µL), trimethylamine hydrochloride (7.0 mg), and TsCl (61 mg) were added under ice cooling, and the mixture was stirred at room temperature for 1 hour. TEA (61 µL) and TsCl (61 mg) were added, and the mixture was stirred at the same temperature for 30 min. Water was added to the reaction mixture, the mixture was extracted with chloroform, and the organic layer was dried over magnesium sulfate and concentrated under reduced pressure. The residue was purified by NH type silica gel column chromatography (gradient elution with n-hexane/ethyl acetate = 90/10 to 0/100 to gradient elution with ethyl acetate/methanol = 98/2 to 90/10) to obtain title compound 112-2 (pale yellow oil, 0.14 g, yield 82%).
MS (ESI) m/z = 602 [M+1]⁺
LCMS retention time: 0.895 min (condition B)

### 112-3) Synthesis of N-{3-[(4-{3-[(2-{(1S)-1-[(oxan-2-yl)oxy]ethyl}-1H-imidazol-1-yl)methyl]-1,2-oxazol-5-yl}phenyl)ethynyl]cyclobutyl}oxetan-3-amine

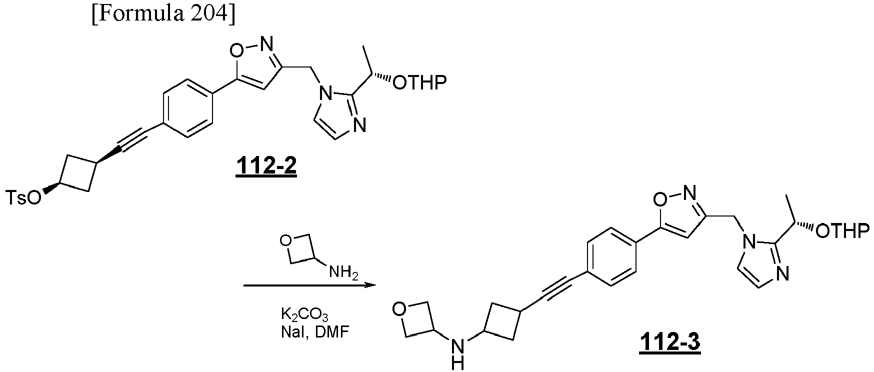

To a DMF (0.87 mL) solution of compound 112-2 (53 mg) and sodium iodide (13 mg), potassium carbonate (36 mg) and oxetan-3-amine (0.19 g) were added. The resulting mixture was irradiated in a microwave reactor at 100°C for 1 hour, and the mixture was stirred at room temperature for 24 hours. It was further irradiated in a microwave reactor at 100°C for 1.5 hours. To the reaction mixture, an aqueous saturated sodium bicarbonate solution was added, followed by extraction with ethyl acetate. After the organic layer was concentrated under reduced pressure, the resulting residue was purified by NH type silica gel column chromatography (gradient elution with n-hexane/ethyl acetate = 50/50 to 0/100 to gradient elution with ethyl acetate/methanol = 98/2 to 90/10) to obtain title compound 112-3 (colorless oil, 15 mg, yield 34%).
MS (ESI) m/z = 503 [M+1]⁺
LCMS retention time: 0.768 min (condition A)

### 112-4) Synthesis of (1S)-1-[1-({5-[4-({3-[(oxetan-3-yl)amino]cyclobutyl}ethynyl)phenyl]-1,2-oxazol-3-yl}methyl)-1H-imidazol-2-yl]ethan-1-ol (compound 112)

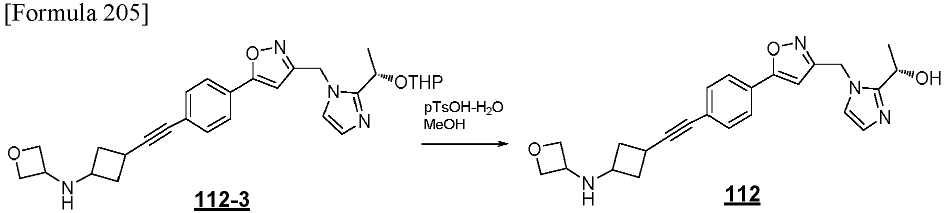

Compound 112 was produced by the same methods as in 4-3 with the use of the corresponding starting materials.

The ¹H-NMR and LCMS data are as shown in Table 2.

### Example 43

### 4-{(1R,5S,6s)-6-[(4-{3-[(1R)-2-Amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}butanoic acid (compound 113)

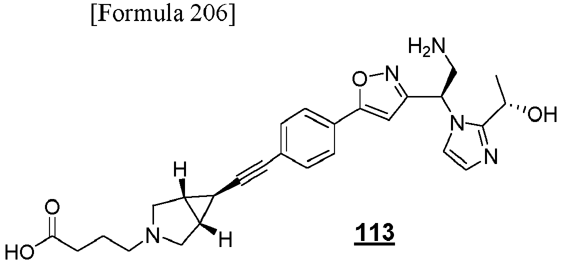

### 113-1) Synthesis of ethyl 4-[(1R,5S,6s)-6-ethynyl-3-azabicyclo[3.1.0]hexan-3-yl]butanoate

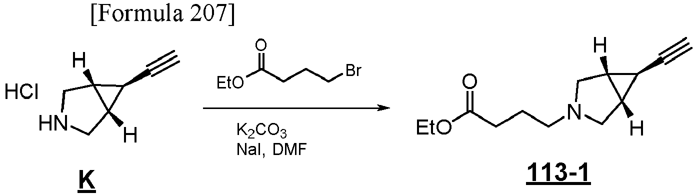

Compound 113-1 was produced by the same methods as in 101-1 of Example 38 with the use of the corresponding starting materials.
¹H NMR (400 MHz, CHLOROFORM-d) δ ppm 1.25 (t, J=7.1 Hz, 3 H), 1.61 - 1.76 (m, 5 H), 1.80 (s, 1 H), 2.20 - 2.32 (m, 4 H), 2.40 (br t, J=6.8 Hz, 2 H), 3.03 (d, J=8.9 Hz, 2 H), 4.12 (q, J=7.1 Hz, 2 H)

### 113-2) Synthesis of ethyl 4-{(1R,5S,6s)-6-[(4-{3-[(1R)-2-[(tert-butoxycarbonyl)amino]-1-{2-[(1S)-1-{[tert-butyl(dimethyl)silyl]oxy}ethyl]-1H-imidazol-1-yl}ethyl]-1,2-oxazol-5-yl}phenyl)ethynyl]-3-azabicyclo[3.1.0]hexan-3-yl}butanoate

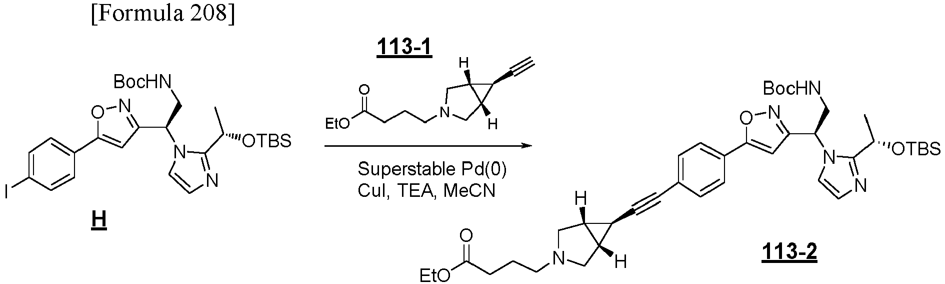

Compound 113-2 was produced by the same methods as in 10-1 of Example 3 with the use of the corresponding starting materials.
MS (ESI) m/z = 732 [M+1]⁺
LCMS retention time: 0.778 min (condition B)

### 113-3) Synthesis of ethyl 4-{(1R,5S,6s)-6-[(4-{3-[(1R)-2-[(tert-butoxycarbonyl)amino]-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}butanoate

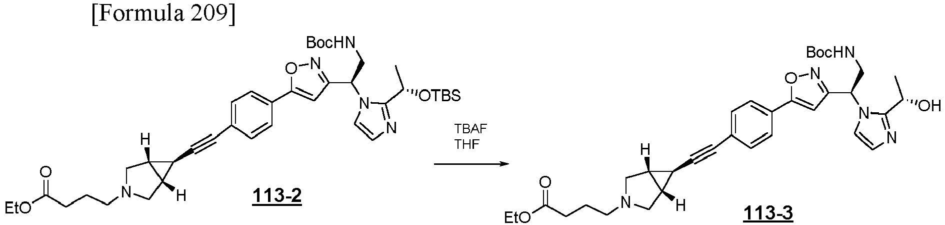

Compound 113-3 was produced by the same methods as in 19-2 of Example 11 with the use of the corresponding starting materials.
MS (ESI) m/z = 618 [M+1]⁺
LCMS retention time: 0.932 min (condition A)

### 113-4) Synthesis of ethyl 4-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3 . 1.0]hexan-3-yl }butanoate

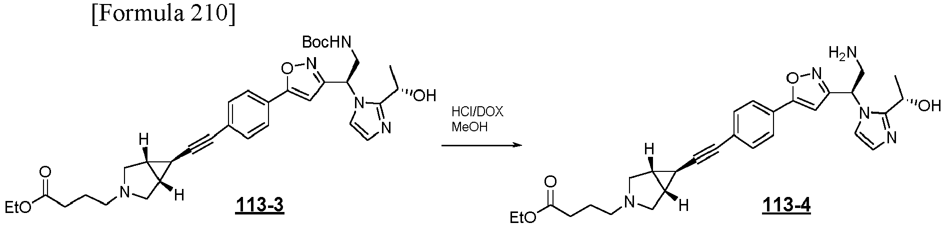

Compound 113-4 was produced by the same methods as in 10-2 of Example 3 with the use of the corresponding starting materials.
MS (ESI) m/z= 518 [M+1]⁺
LCMS retention time: 0.592 min (condition A)

### 113-5) Synthesis of 4-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}butanoic acid (compound 113)

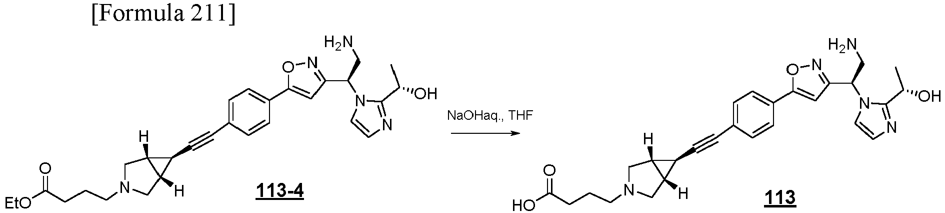

Compound 113 was produced by the same methods as in 67-2 of Example 25 with the use of the corresponding starting materials.

The ¹H-NMR and LCMS data are as shown in Table 2.

### Example 44

### 3-[(1R,5S,6s)-6-({4-[3-({2-[(1S)-1-Hydroxyethyl]-1H-imidazol-1-yl}methyl)-1,2-oxazol-5-yl]phenyl}ethynyl)-3-azabicyclo[3.1.0]hexan-3-yl]propanoic acid (compound 114)

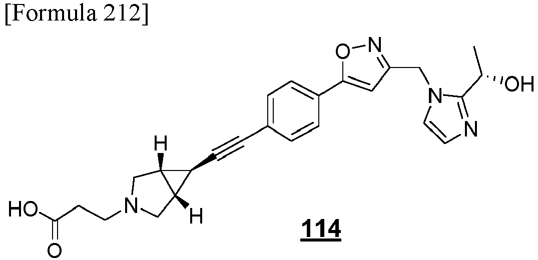

### 114-1) Synthesis of ethyl 3-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propanoate

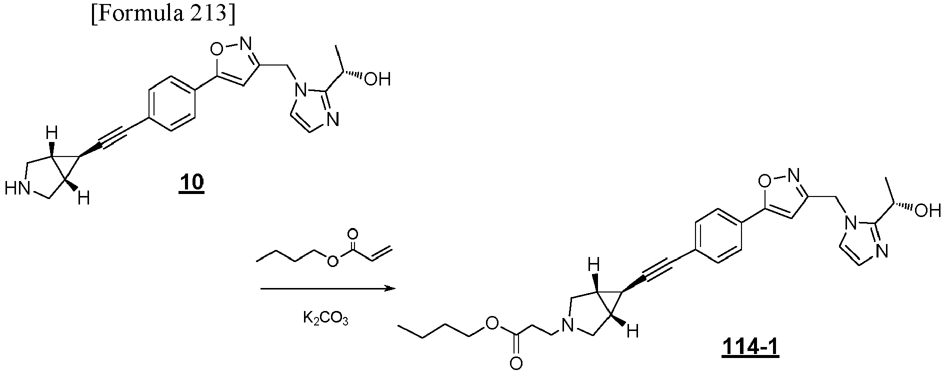

A mixture of compound 10 (55 mg), butyl prop-2-enoate (2.1 mL), and potassium carbonate (81 mg) was irradiated in a microwave reactor at 140°C for 2 hours. After the mixture was allowed to cool, the insolubles were filtered off, and the filtrate was concentrated under reduced pressure. The residue was purified by OH type silica gel column chromatography (gradient elution with chloroform/methanol = 100/0 to 96/4) to obtain title compound 114-1 (yellow oil, 21 mg, yield 29%).
MS (ESI) m/z = 503 [M+1]⁺
LCMS retention time: 0.666 min (condition A)

### 114-2) Synthesis of 3-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propanoic acid (compound 114)

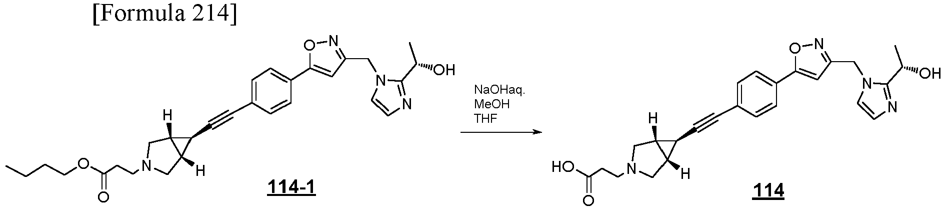

To a THF (1.0 mL) and MeOH (1.0 mL) solution of compound 114-1 (21 mg), a 1 moL/L-aqueous sodium hydroxide solution (2.0 mL) was added at room temperature, and the mixture was stirred for 4 hours. To the reaction mixture, 1 moL/L-hydrochloric acid was added under ice cooling to adjust the pH to 6, and then, the mixture was concentrated under reduced pressure. The residue was suspended in MeOH/chloroform (1:1), and the insolubles were filtered off. The filtrate was concentrated under reduced pressure, and the residue was purified by OH type silica gel column chromatography (gradient elution with chloroform/methanol = 90/10 to 50/50) to obtain a crude product. The crude product was further suspended in chloroform /methanol (9:1), the insolubles were filtered off, and the filtrate was concentrated under reduced pressure to obtain title compound 114 (yellow solid, 19 mg, yield 100%).

The ¹H-NMR and LCMS data are as shown in Table 2.

### Example 45

### (1R,5S,6s)-6-[(4-{3-[(1R)-2-Amino-1-{2-[(1S)-1-hydroxyethyl]-1H-imidazol-1-yl}ethyl]-l,2-oxazol-5-yl}phenyl)ethynyl]-3-azabicyclo[3.1.0]hexane-3-carbaldehyde (compound 117)

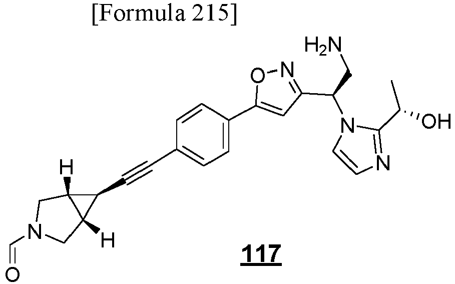

### 117-1) Synthesis of tert-butyl [(2R)-2-[5-(4-{[(1R,5S,6s)-3-azabicyclo[3.1.0]hexan-6-yl]ethynyl}phenyl)-1,2-oxazol-3-yl]-2-{2-[(1S)-1-{[tert-butyl(dimethyl)silyl]oxy}ethyl]-1H-imidazol-1-yl }ethyl]carbamate

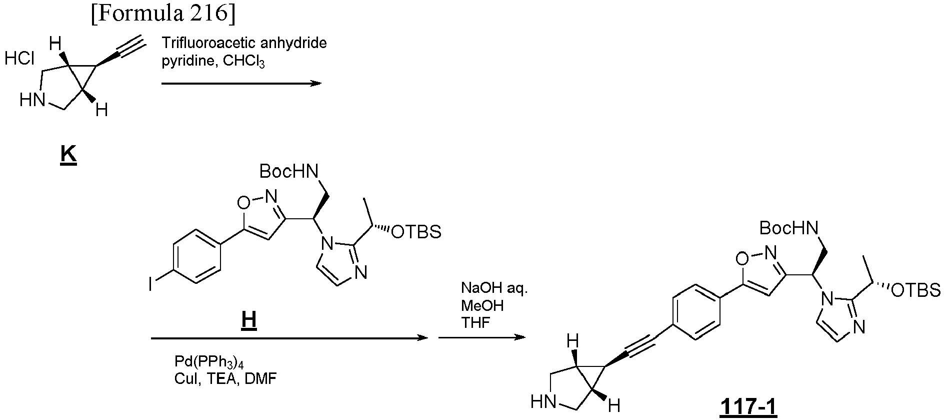

To a chloroform (18 mL) suspension of intermediate K (1.0 g), pyridine (2.3 mL) and trifluoroacetic acid anhydride (1.8 mL) were added under ice cooling, the mixture was stirred at room temperature for 1 hour, and the reaction mixture was concentrated under reduced pressure. The residue was purified by OH type silica gel column chromatography (gradient elution with n-hexane/ethyl acetate = 90/10 to 0/100) to obtain a crude product. To a DMF (4.0 mL) solution of compound H (1.0 g), tetrakis(triphenylphosphine)palladium(0) (0.18 g), copper iodide (30 mg), and TEA (2.2 mL) were added, and the mixture was stirred at 65°C. Thereinto, a DMF (3.8 mL) solution of the crude product (0.48 g) obtained above was added, and the mixture was stirred at the same temperature for 30 min. After allowing to cool, the reaction mixture was diluted with ethyl acetate and extracted with an ethyl acetate/toluene mixed solvent. The organic layer was washed with brine, dried over magnesium sulfate, and concentrated under reduced pressure. The residue was purified by NH type silica gel column chromatography (gradient elution with n-hexane/ethyl acetate = 50/50 to 0/100) to obtain a crude product. To a THF (3.9 mL)/MeOH (3.9 mL) solution of the obtained crude product, a 1 mol/L-aqueous sodium hydroxide solution (7.8 mL) was added, and the mixture was stirred at room temperature for 20 min. To the reaction mixture, a 20% aqueous potassium carbonate solution was added, the mixture was extracted with a chloroform/MeOH mixed solvent and then concentrated under reduced pressure. The residue was purified by NH type silica gel column chromatography (gradient elution with chloroform/methanol = 100/0 to 90/10) to obtain title compound 117-1 (pale yellow oil, 0.75 g, yield 77%).
MS (ESI) m/z = 618 [M+1]⁺
LCMS retention time: 0.723 min (condition B)

### 117-2) Synthesis of tert-butyl [(2R)-2-[5-(4-{[(1R,5S,6s)-3-azabicyclo[3.1.0]hexan-6-yl]ethynyl}phenyl)-1,2-oxazol-3-yl]-2-{2-[(1S)-1-hydroxy ethyl]-1H-imidazol-1-yl}ethyl]carbamate

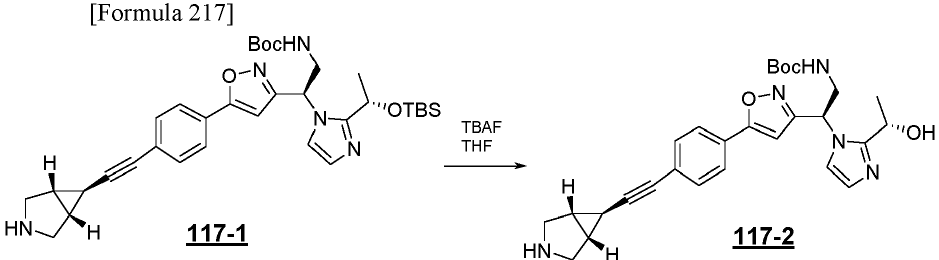

Compound 117-2 was produced by the same methods as in 19-2 of Example 11 with the use of the corresponding starting materials.
MS (ESI) m/z = 504 [M+1]⁺
LCMS retention time: 0.836 min (condition A)

### 117-3) Synthesis of tert-butyl [(2R)-2-[5-(4-{[(1R,5S,6s)-3-formyl-3-azabicyclo[3.1.0]hexan-6-yl]ethynyl}phenyl)-1,2-oxazol-3-yl]-2-{2-[(1S)-1-hydroxyethyl]-1H-imidazol-1-yl }ethyl]carbamate

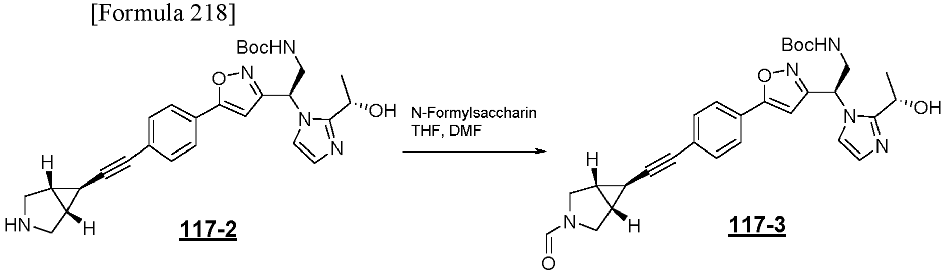

Compound 117-3 was produced by the same methods as in 73-1 of Example 29 with the use of the corresponding starting materials.
MS (ESI) m/z = 532 [M+1]⁺
LCMS retention time: 0.659 min (condition B)

### 117-4) Synthesis of (1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carbaldehyde (compound 117)

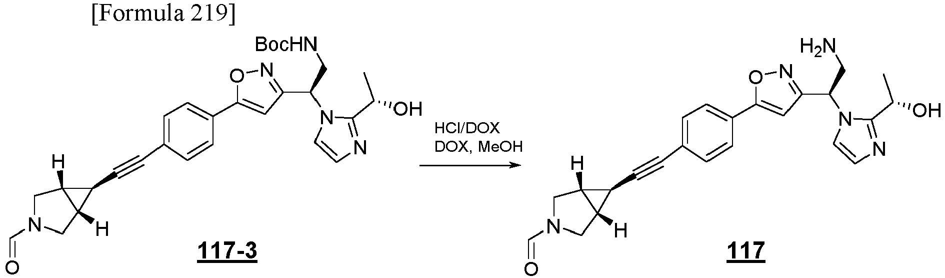

Compound 117 was produced by the same methods as in 10-2 with the use of the corresponding starting materials.

The ¹H-NMR and LCMS data are as shown in Table 2.

### Example 46

### Methyl (1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carboxylate (compound 118)

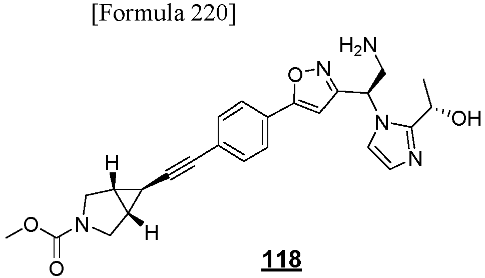

### 118-1) Synthesis of methyl (1R,5S,6s)-6-[(4-{3-[(1R)-2-[(tert-butoxycarbonyl)amino]-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carboxylate

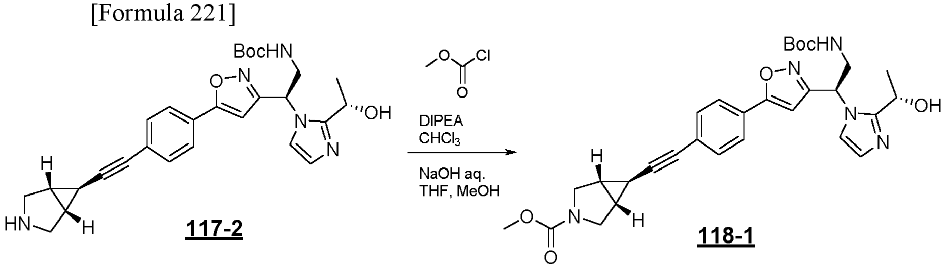

To a chloroform (2.0 mL) solution of compound 117-2 (30 mg), DIPEA (31 µL) and methyl chloroformate (10 µL) were added under ice cooling, and the mixture was stirred at room temperature for 2.5 hours. To the reaction mixture, a 20% aqueous potassium carbonate solution was added, the mixture was extracted with chloroform, and the organic layer was concentrated under reduced pressure. To the residue, THF (1.0 mL), MeOH (1.0 mL), and a 1 mol/L-aqueous sodium hydroxide solution (1.0 mL) were added, and the mixture was stirred at room temperature for 2 hours. To the reaction mixture, a 20% aqueous potassium carbonate solution was added, the mixture was extracted with chloroform, and the organic layer was concentrated under reduced pressure. The residue was purified by NH type silica gel column chromatography (gradient elution with ethyl acetate/methanol = 100/0 to 90/10) to obtain title compound 118-1 (colorless solid, 30 mg, yield 89%).
MS (ESI) m/z = 562 [M+1]⁺
LCMS retention time: 1.232 min (condition A)

### 118-2) Synthesis of methyl (1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carboxylate (compound 118)

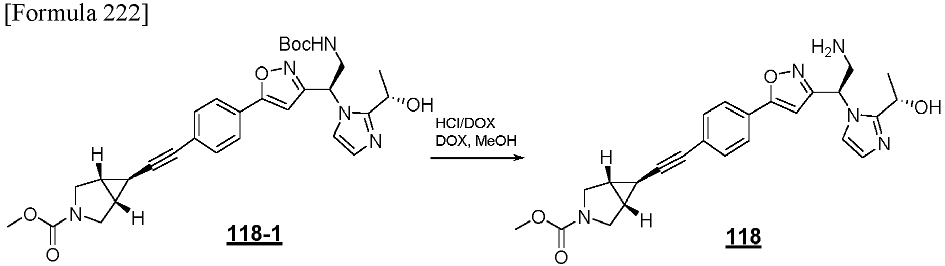

Compound 118 was produced by the same methods as in 10-2 with the use of the corresponding starting materials.

The ¹H-NMR and LCMS data are as shown in Table 2.

### Example 47

### 1-{(1R,5S,6s)-6-[(4-{3-[(1R)-2-Amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}propan-2-ol (compound 120)

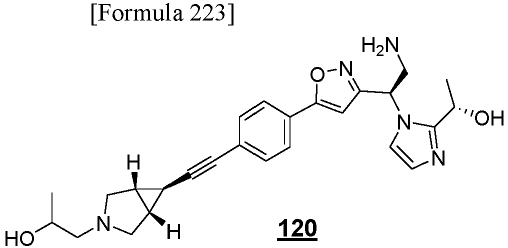

### 120-1) Synthesis of tert-butyl {(2R)-2-{2-[(1S)-1-hydroxyethyl]-1H-imidazol-1-yl}-2-[5-(4-{[(1R,5S,6s)-3-(2-hydroxypropyl)-3-azabicyclo[3.1.0]hexan-6-yl]ethynyl}phenyl)-1,2-oxazol-3-yl]ethyl}carbamate

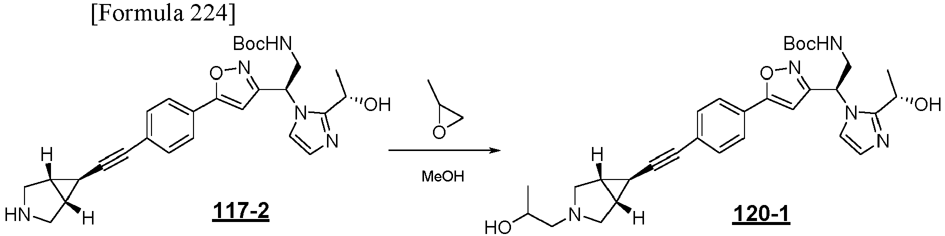

To a MeOH (1.0 mL) solution of compound 117-2 (30 mg), propylene oxide (21 µL) was added, the mixture was irradiated in a microwave reactor at 50°C for 30 min. Then, propylene oxide (42 µL) was further added, the mixture was irradiated in a microwave reactor at 50°C for 1 hour, and the reaction mixture was concentrated under reduced pressure. The residue was purified by NH type silica gel column chromatography (gradient elution with ethyl acetate/methanol = 100/0 to 90/10) to obtain title compound 120-1 (colorless solid, 28 mg, yield 85%).
MS (ESI) m/z = 562 [M+1]⁺
LCMS retention time: 0.363 min (condition B)

### 120-2) Synthesis of 1-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}propan-2-ol (compound 120)

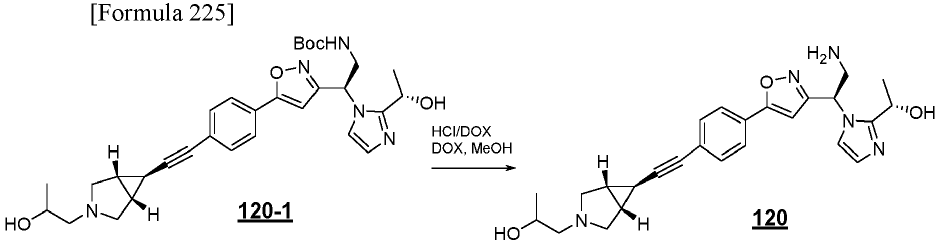

Compound 120 was produced by the same methods as in 10-2 of Example 3 with the use of the corresponding starting materials.

The ¹H-NMR and LCMS data are as shown in Table 2.

### Example 48

### (1S)-1-(1-{[5-(4-{[(1R,5S,6s)-3-(2-Aminoethyl)-3-azabicyclo[3.1.0]hexan-6-yl]ethynyl }phenyl)-1,2-oxazol-3-yl]methyl}-1H-imidazol-2-yl)ethan-1-ol (compound 122)

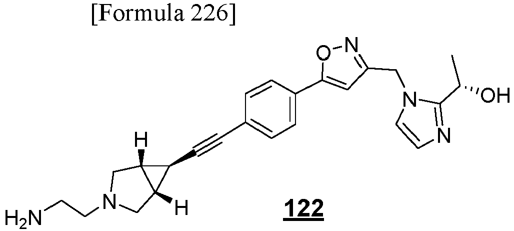

### 122-1) Synthesis of tert-butyl {2-[(1R,5S,6s)-6-ethynyl-3-azabicyclo[3.1.0]hexan-3-yl]ethyl}carbamate

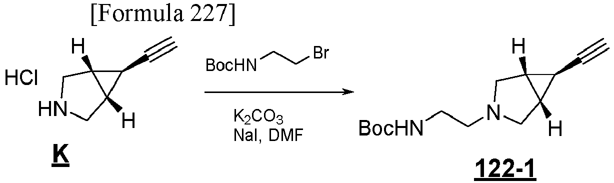

Compound 122-1 was produced by the same methods as in 101-1 of Example 38 with the use of the corresponding starting materials.
¹H NMR (400 MHz, CHLOROFORM-d) δ ppm 1.45 (s, 9 H), 1.64 - 1.69 (m, 1 H), 1.69 - 1.75 (m, 2 H), 1.82 (s, 1 H), 2.32 (br d, J=8.9Hz, 2 H), 2.51 (brt, J=5.9Hz, 2 H), 3.05 (d, J=8.9 Hz, 2 H), 3.10 - 3.20 (m, 2 H), 4.79 (br s, 1 H)

### 122-2) Synthesis of (1S)-1-(1-{[5-(4-{[(1R,5S,6s)-3-(2-aminoethyl)-3-azabicyclo[3.1.0]hexan-6-yl]ethynyl}phenyl)-1,2-oxazol-3-yl]methyl}-1H-imidazol-2-yl)ethan-1-ol (compound 122)

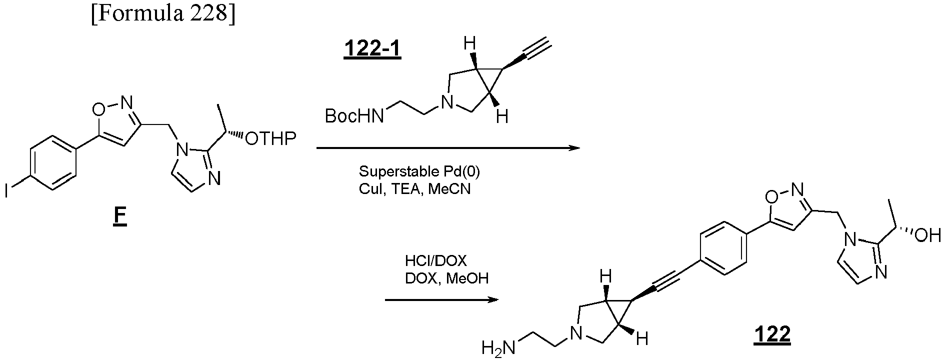

Compound 122 was produced by the same methods as in 10-1 and 10-2 of Example 3 with the use of the corresponding starting materials.

The ¹H-NMR and LCMS data are as shown in Table 2.

### Example 49

### N-{trans-3-[(4-{3-[(1R)-2-Amino-1-{2-[(1S)-1-hydroxyethyl]-1H-imidazol-1-yl}ethyl]-1,2-oxazol-5-yl}phenyl)ethynyl]cyclobutyl}-N-methylformamide (compound 123)

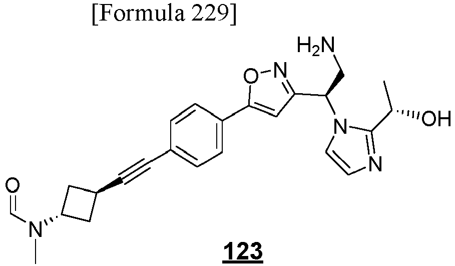

### 123-1) Synthesis of N-(trans-3-ethynylcyclobutyl)-N-methylformamide

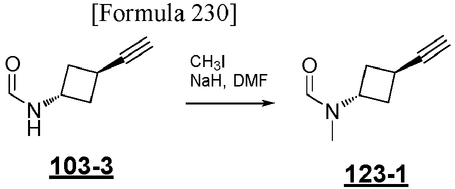

To a DMF (0.88 mL) solution of compound 103-3 (22 mg), iodomethane (13 µL) and 60% sodium hydride (7.8 mg) were added under ice cooling. After the reaction mixture was warmed to room temperature, the mixture was stirred for 1 hour. To the reaction mixture, acetic acid (11 µL) and water were added under ice cooling to terminate the reaction. After the resulting mixture was extracted with ethyl acetate, the organic layer was dried over magnesium sulfate, and the solvent was distilled off under reduced pressure. The residue was purified by OH type silica gel column chromatography (gradient elution with n-hexane/ethyl acetate = 50/50 to 0/100 to gradient elution with ethyl acetate/methanol = 99/1 to 90/10) to obtain title compound 123-1 (colorless solid, 19 mg, yield 78%).
MS (ESI) m/z= 138 [M+1]⁺
LCMS retention time: 0.527 min (condition B)

### 123-2) Synthesis of tert-butyl [(2R)-2-{2-[(1S)-1-{[tert-butyl(dimethyl)silyl]oxy}ethyl]-1H-imidazol-1-yl}-2-{5-[4-({trans-3-[formyl(methyl)amino]cyclobutyl}ethynyl)phenyl]-1,2-oxazol-3-yl}ethyl]carbamate

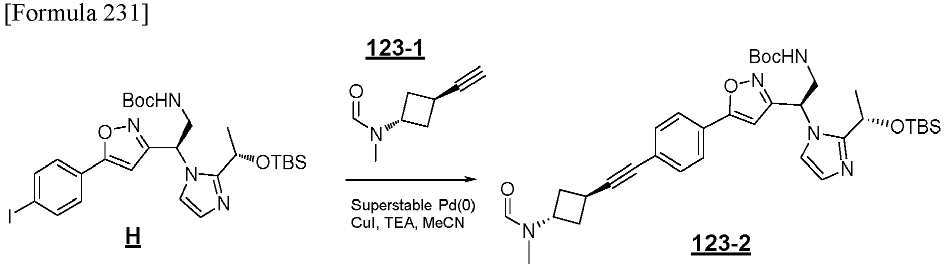

Compound 123-2 was produced by the same methods as in 10-1 of Example 3 with the use of the corresponding starting materials.
MS (ESI) m/z = 648 [M+1]⁺
LCMS retention time: 0.965 min (condition B)

### 123-3) Synthesis of N-{trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutyl}-N-methylformamide (compound 123)

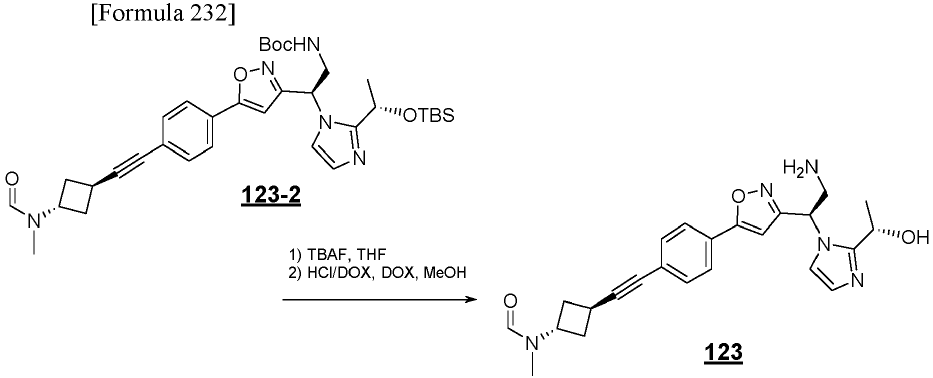

To a THF (0.77 mL) solution of compound 123-1 (50 mg), TBAF (85 µL, 1 mol/L-THF solution) was added, and the mixture was stirred for 1 hour. Water and brine were added to the reaction mixture, followed by extraction three times with ethyl acetate. The organic layer was dried over magnesium sulfate, and then, filtered, and concentrated under reduced pressure. The residue was purified by NH type silica gel column chromatography (gradient elution with ethyl acetate/methanol = 99/1 to 90/10). The resulting pale yellow oil was dissolved in methanol (0.50 mL), a 4 mol/L-hydrochloric acid-DOX solution (0.15 mL) was added at room temperature, and the mixture was stirred for 5 min. After the reaction mixture was concentrated under reduced pressure, an aqueous saturated sodium bicarbonate solution and a 20% aqueous potassium carbonate solution were added to the residue, and the mixture was extracted with chloroform. The organic layer was separated using a phase separator and concentrated under reduced pressure. The residue was purified by preparative TLC (OH type silica gel, developing solvent; chloroform/methanol/ammonia water = 100/10/1) to obtain title compound 123 (colorless solid, 23 mg, yield 70%).

The ¹H-NMR and LCMS data are as shown in Table 2.

### Example 50

### 2-{(1R,5S,6s)-6-[(4-{3-[(1R)-2-Amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}-2-oxoacetamide (compound 124)

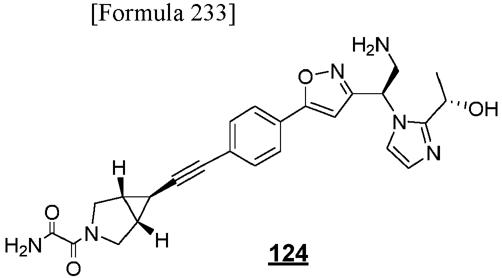

### 124-1) Synthesis of ethyl {(1R,5S,6s)-6-[(4-{3-[(1R)-2-[(tert-butoxycarbonyl)amino]-1-{2-[(1S)-1-{[tert-butyl(dimethyl)silyl]oxy}ethyl]-1H-imidazol-1-yl}ethyl]-1,2-oxazol-5-yl}phenyl)ethynyl]-3-azabicyclo[3.1.0]hexan-3-yl}(oxo)acetate

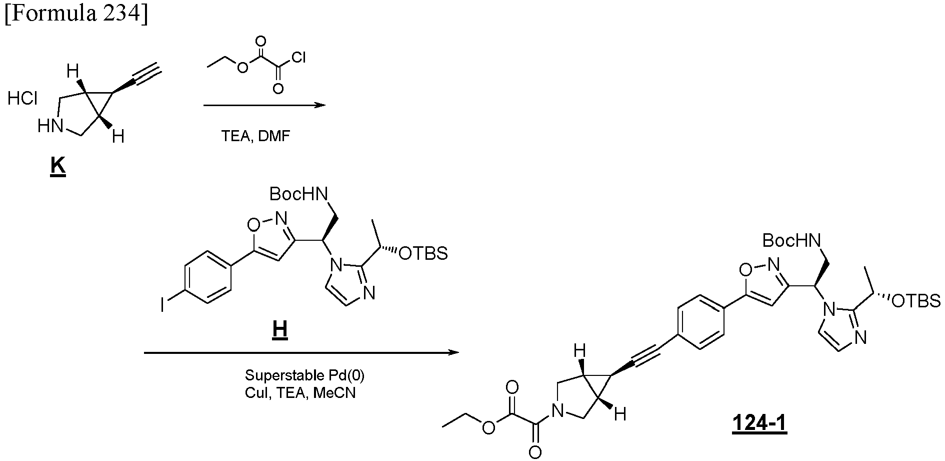

To a DMF (5.2 mL) solution of intermediate K (0.15 g), TEA (0.58 mL) and ethyl chloro(oxo)acetate (0.24 mL) were added under ice cooling. After the mixture was stirred for 2 hours, water was added, and the mixture was extracted with ethyl acetate. The aqueous layer was extracted with ethyl acetate, the organic layer was dried over magnesium sulfate, and the solvent was distilled off under reduced pressure. The residue was purified by OH type silica gel column chromatography (gradient elution with n-hexane/ethyl acetate = 90/10 to 20/80). Using the resulting crude product and intermediate H, compound 124-1 was produced by the same methods as in 10-1.
MS (ESI) m/z = 718 [M+1]⁺
LCMS retention time: 0.909 min (condition B)

### 124-2) Synthesis of tert-butyl ((R)-2-(5-(4-(((1R,5S,6s)-3-(2-amino-2-oxoacetyl)-3-azabicyclo[3 .1.0]hexan-6-yl)ethynyl)phenyl)isoxazol-3-yl)-2-(2-((S)-1-((tertbutyldimethylsilyl)oxy)ethyl)-1H-imidazol-1-yl)ethyl)carbamate

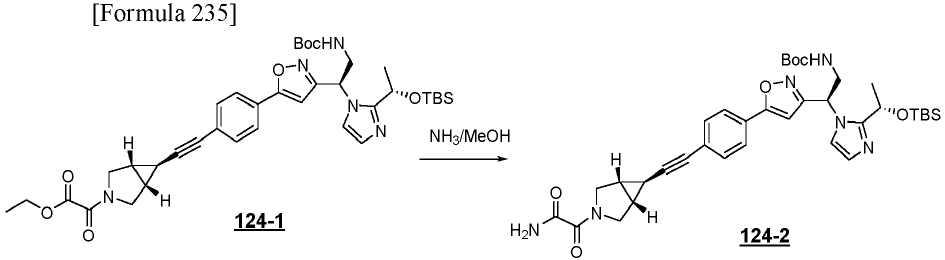

After a mixture of compound 124-2 (48 mg) and a 7 mol/L-ammonia-methanol solution (0.50 mL) was stirred at room temperature for 15 hours, the mixture was concentrated under reduced pressure. The residue was purified by OH type silica gel column chromatography (gradient elution with n-hexane/ethyl acetate = 90/10 to 0/100) to obtain title compound 124-2 (colorless oil, 44 mg, yield 96%).
MS (ESI) m/z = 689 [M+1]⁺
LCMS retention time: 0.786 min (condition B)

### 124-3) Synthesis of 2-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}-2-oxoacetamide (compound 124)

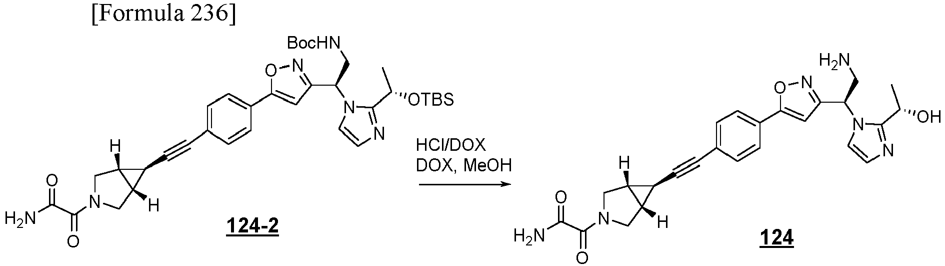

Compound 124 was produced by the same methods as in 22-3 of Example 12 with the use of the corresponding starting materials.

The ¹H-NMR and LCMS data are as shown in Table 2.

### Example 51

### (1S)-1-(1-{(1R)-2-Amino-1-[5-(4-{[3-(cyclopropylamino)cyclobutyl]ethynyl}phenyl)-1,2-oxazol-3-yl]ethyl}-1H-imidazol-2-yl)ethan-1-ol (compound 129)

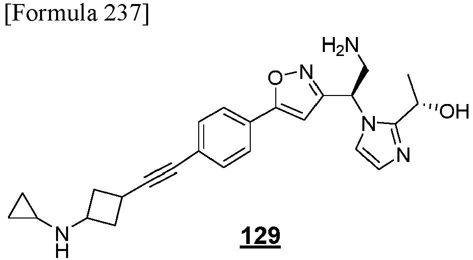

### 129-1) Synthesis of cis-3-ethynylcyclobutyl 4-methylbenzene-1-sulfonate

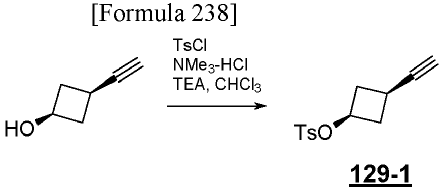

To a chloroform (7.0 mL) solution of 3-ethynylcyclobutanol (69 mg), TEA (0.20 mL), trimethylamine hydrochloride (17 mg), and TsCl (0.20 g) were added under ice cooling, and the mixture was stirred at room temperature for 1 hour, and then, TEA (0.20 mL) and TsCl (0.20 g) were added, and the mixture was stirred for 30 min. Water was added to the reaction mixture, the mixture was extracted with chloroform, and the organic layer was dried over magnesium sulfate and concentrated under reduced pressure. The residue was purified by OH type silica gel column chromatography (gradient elution with n-hexane/ethyl acetate = 90/10 to 50/50) to obtain title compound 129-1 (colorless oil, 0.16 g, yield 90%).
¹H NMR (400 MHz, CHLOROFORM-d) δ ppm 2.13 (s, 1 H), 2.21 - 2.34 (m, 2 H), 2.42 - 2.64 (m, 6 H), 4.59 - 4.74 (m, 1 H), 7.34 (d, J=8.0 Hz, 2 H), 7.78 (d, J=8.0 Hz, 2 H)

### 129-2) Synthesis of N-cyclopropyl-3-ethynylcyclobutan-1-amine

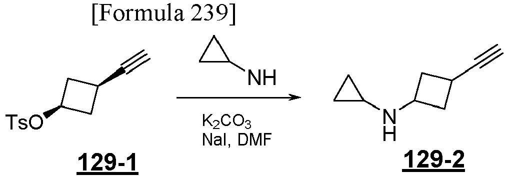

To a DMF (1.2 mL) solution of compound 129-1 (60 mg), sodium iodide (36 mg), potassium carbonate (99 mg), cyclopropylamine (0.17 mL) were added, and the mixture was irradiated in a microwave reactor at 60°C for 30 min. Cyclopropylamine (0.17 mL) was added thereto, and the mixture was irradiated at 80°C for 1 hour. Furthermore, an operation of adding cyclopropylamine (0.17 mL) and irradiating the mixture at 100°C for 1 hour was performed three times, and then, an aqueous saturated sodium bicarbonate solution was added to the reaction mixture, the mixture was extracted with chloroform, and the organic layer was concentrated under reduced pressure. The residue was purified by NH type silica gel column chromatography (gradient elution with n-hexane/ethyl acetate = 75/25 to 0/100) and OH type silica gel column chromatography (gradient elution with ethyl acetate/methanol = 100/0 to 95/5) to obtain title compound 129-2 (colorless oil, 17 mg, yield 53%).
MS (ESI) m/z = 136 [M+1]⁺
LCMS retention time: 0.229 min (condition A)

### 129-3) Synthesis of (1S)-1-(1-{(1R)-2-amino-1-[5-(4-{[3-(cyclopropylamino)cyclobutyl]ethynyl}phenyl)-1,2-oxazol-3-yl]ethyl}-1H-imidazol-2-yl)ethan-1-ol (compound 129)

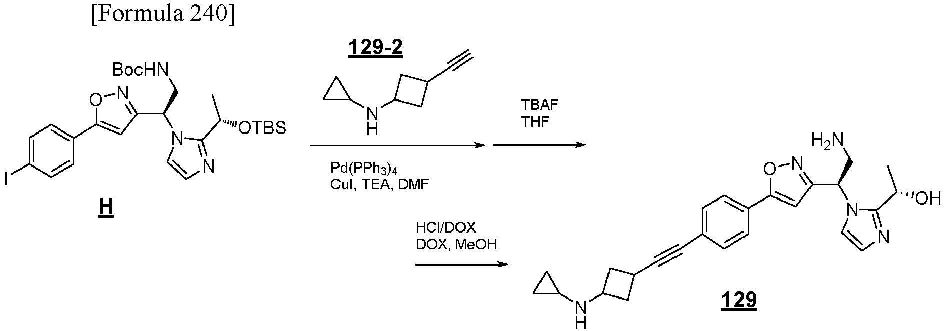

Compound 129 was produced by the same methods as in 113-2 to 113-4 of Example 43 with the use of the corresponding starting materials.

The ¹H-NMR and LCMS data are as shown in Table 2.

### Example 52

### (1S)-1-{1-[(1R)-2-Amino-1-{5-[4-({trans-3-[(2-hydroxyethyl)amino]cyclobutyl}ethynyl)phenyl]-1,2-oxazol-3-yl}ethyl]-1H-imidazol-2-yl}ethan-1-ol (compound 134)

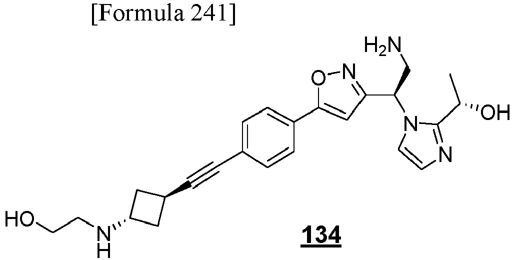

### 134-1) Synthesis of trans-N-(2-{[tert-butyl(dimethyl)silyl]oxy}ethyl)-3-ethynylcyclobutan-1-amine

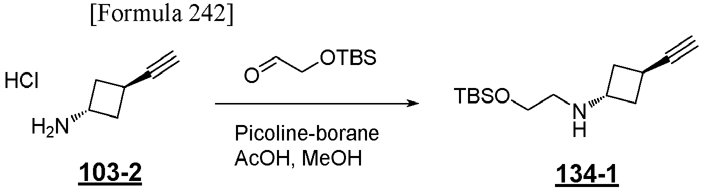

To a methanol (4.4 mL) solution of compound 103-2 (57 mg), acetic acid (75 µL) and {[tert-butyl(dimethyl)silyl]oxy}acetaldehyde (76 mg) were added, and the mixture was stirred at room temperature for 30 min. To this solution, 2-picoline borane (0.14 g) was added, and the mixture was stirred at room temperature for 16 hours. To the reaction mixture, an aqueous saturated sodium bicarbonate solution was added to terminate the reaction, followed by extraction with chloroform. The organic layer was separated using a phase separator, and the solvent was distilled off under reduced pressure. The residue was purified by OH type silica gel column chromatography (gradient elution with ethyl hexane/ethyl acetate = 70/30 to 0/100 to gradient elution with ethyl acetate/methanol = 99/1 to 90/10) to obtain title compound 134-1 (yellow oil, 49 mg, yield 44%).
¹H NMR (400 MHz, CHLOROFORM-d) δ ppm 0.06 (s, 6 H), 0.90 (s, 9 H), 1.98 - 2.43 (m, 5 H), 2.55 - 2.70 (m, 2 H), 2.84 - 3.04 (m, 1 H), 3.52 - 3.77 (m, 3 H)

### 134-2) Synthesis of (1S)-1-{1-[(1R)-2-amino-1-{5-[4-({trans-3-[(2-hydroxyethyl)amino]cyclobutyl}ethynyl)phenyl]-1,2-oxazol-3-yl}ethyl]-1H-imidazol-2-yl}ethan-l-ol (compound 134)

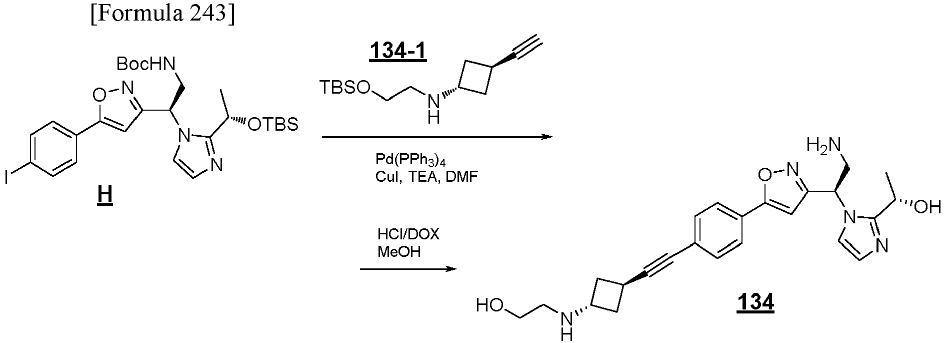

Compound 134 was produced by the same methods as in 10-1 of Example 3 and 22-3 of Example 12 with the use of the corresponding starting materials.

The ¹H-NMR and LCMS data are as shown in Table 2.

### Example 53

### (S)-1-(1-((5-(4-(((1r,3 S)-3-(Oxetan-3-ylamino)cyclobutyl)ethynyl)phenyl)isoxazol-3-yl)methyl)-1H-imidazol-2-yl)ethanol (compound 135)

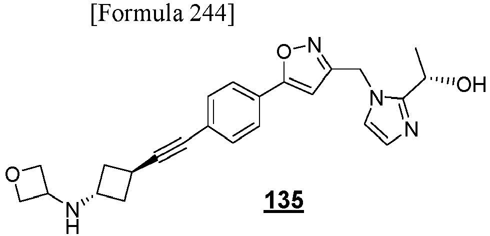

### 135-1) Synthesis of N-(trans-3-ethynylcyclobutyl)oxetan-3-amine

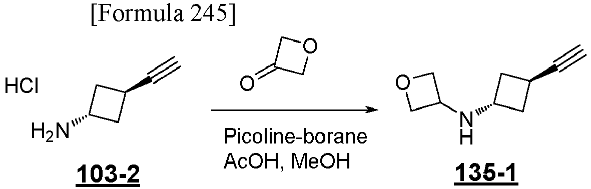

To a methanol (9.5 mL) solution of compound 103-2 (0.12 g), acetic acid (0.16 mL) and oxetan-3-one (68 mg) were added, and the mixture was stirred at room temperature for 30 min. To this solution, 2-picoline borane (0.30 g) was added, and the mixture was stirred at room temperature for 16 hours. To the reaction mixture, an aqueous saturated sodium bicarbonate solution was added to terminate the reaction, followed by extraction with chloroform. The organic layer was separated using a phase separator, and the solvent was distilled off under reduced pressure. The residue was purified by OH type silica gel column chromatography (gradient elution with n-hexane/ethyl acetate = 70/30 to 0/100) to obtain title compound 135-1 (yellow oil, 87 mg, yield 61%).
¹H NMR (400 MHz, CHLOROFORM-d) δ ppm 1.98 - 2.37 (m, 5 H), 2.97 (br s, 1 H), 3.52 - 3.65 (m, 1 H), 3.90 - 4.01 (m, 1 H), 4.38 - 4.47 (m, 2 H), 4.73 - 4.87 (m, 2 H)

### 135-2) Synthesis of (S)-1-(1-((5-(4-(((1r,3S)-3-(oxetan-3-ylamino)cyclobutyl)ethynyl)phenyl)isoxazol-3-yl)methyl)-1H-imidazol-2-yl)ethanol (compound 135)

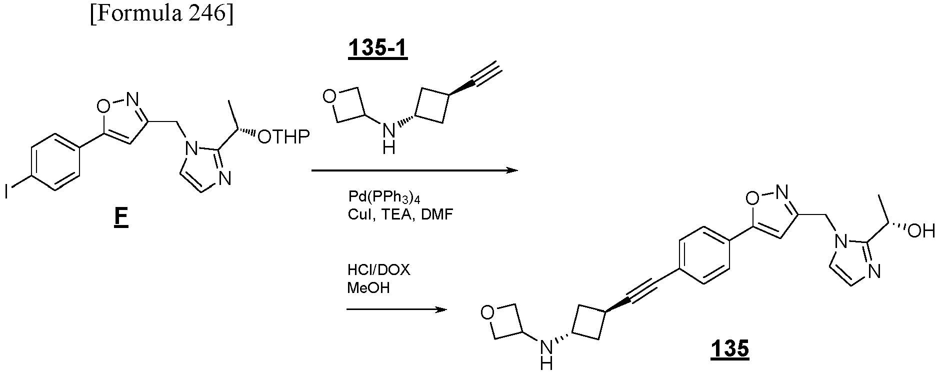

Compound 135 was produced by the same methods as in 4-1 and 4-3 of Example 2 with the use of the corresponding starting materials.

The ¹H-NMR and LCMS data are as shown in Table 2.

Compounds 2, 3, and 5 were produced by the same methods as compound 1 of Example 1 with the use of the corresponding materials.

Compounds 27 and 40 were produced by the same methods as compound 4 of Example 2 with the use of the corresponding materials.

Compounds 8, 9, 17, 23, 28, 29, 30, 56, and 83 were produced by the same methods as compound 10 of Example 3 with the use of the corresponding materials.

Compounds 55, 57, and 85 were produced by the same methods as compound 11 of Example 4 with the use of the corresponding materials.

Compounds 35 and 36 were produced by the same methods as compound 12 of Example 5 with the use of the corresponding materials.

Compound 96 was produced by the same methods as compound 16 of Example 9 with the use of the corresponding materials.

Compounds 21 and 60 were produced by the same methods as compound 18 of Example 10 with the use of the corresponding materials.

Compounds 34, 41, 84, and 99 were produced by the same methods as compound 19-1 of Example 11 with the use of the corresponding materials.

Compounds 20, 58, 74, 75, 80, 106, 115, 116, 131, 132, and 133 were produced by the same methods as compound 10-1 of Example 3 and 22-3 of Example 12 with the use of the corresponding materials.

Compounds 6, 7, 31, 32, 33, 43, 44, 46, 47, 48, 49, 52, 53, 65, 68, and 119 were produced by the same methods as compound 26 of Example 14 with the use of the corresponding materials.

Compounds 69, 86, and 87 were produced by the same methods as compound 37 of Example 15 with the use of the corresponding materials.

Compound 50 was produced by the same methods as compound 39-1 of Example 17 with the use of compound 45 as a material.

Compounds 55, 121, and 128 were produced by the same methods as compound 120-1 of Example 47 with the use of the corresponding materials.

Compounds 62 and 63 were produced by the same methods as compound 61 of Example 22 with the use of the corresponding materials.

Compounds 93, 94, 110, and 111 were produced by the same methods as compound 67 of Example 25 with the use of the corresponding materials.

Compound 125 was produced by the same methods as compound 70 of Example 26 with the use of the corresponding materials.

Compounds 102, 104, and 127 were produced by the same methods as compound 73 of Example 29 with the use of the corresponding materials.

Compounds 77, 79, and 95 were produced by the same methods as compound 76 of Example 30 with the use of the corresponding materials.

Compound 100 was produced by the same methods as compound 82 of Example 33 with the use of the corresponding materials.

Compound 90 was produced by the same methods as compound 89 of Example 35 with the use of the corresponding materials.

Compounds 107 and 108 were produced by the same methods as compound 105 of Example 40 with the use of the corresponding materials.

Compounds 51, 97, and 98 were produced by the same methods as compound 112 of Example 42 with the use of the corresponding materials.

Compounds 126 and 130 were produced by the same methods as compounds 113-2 to 113-5 of Example 43 with the use of the corresponding materials.

Compounds 136 and 137 were produced by the same methods as compound 120 of Example 47 with the use of the corresponding materials.

Table 2 shows the structural formulas, ¹H-NMR data, MS (m/z) data, LCMS retention times (min), and measurement methods (Method) of compounds 1 to 137.

**[Table 2-1]**

| Compound | Structural formula | ¹H-NMR | MS (m/z) | Retention time Method |
|---|---|---|---|---|
| 1 | 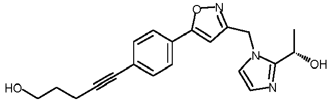 | ¹H NMR (600 MHz, CHLOROFORM-d) *δ* ppm 1. 70 (d, J=6.6 Hz, 3 H), 1. 88 (quin, J=66 Hz, 2 H), 2.37 (d, J=66 Hz, 1 H), 2.57 (t, J=66 Hz, 2 H), 3.80 - 3.85 (m, 2 H), 5.00 - 5.06 (m, 1 H), 5.28 - 5.41 (dd, J=157, 15.7, 2 H), 6.35 (s, 1 H), 6.97 (d, J=1.2 Hz, 1 H), 7.02 (d, J=1. 2 Hz, 1 H), 7.46 (d, J=8.3 Hz, 2 H), 7.65 (d, J=8.3 Hz. 2 H) | ESI 352[M+1]⁺ | 0.830 (A) |
| 2 | 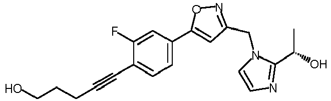 | ¹H NMR (400 MHz, DMSO-d6) *δ* ppm 1.47 (d, J=6.6 Hz, 3 H), 1.66 - 1.74 (m, 2 H), 3. 49 - 3. 56 (m, 2 H), 4.54 (t, J=52 Hz, 1 H), 4.83 - 4.91 (m, 1 H), 5. 36 - 5. 42 (m, 1 H), 5.42 - 5.50 (m, 2 H), 6.82 (s, 1 H), 7.04 (s, 1 H), 7.16 (s, 1 H), 7.57 - 7.63 (m, 1 H), 7.63 - 7.69 (m, 1 H), 7.78 (d, J=9.3 Hz. 1 H) | ESI 370[M+1]⁺ | 0.468 (B) |
| 3 | 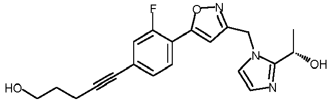 | ¹H NMR (400 MHz, DMSO-d6) *δ* ppm 1.47 (d, J=64 Hz, 3 H), 1. 61 - 1. 75 (m, 2 H), 3. 44 - 3.56 (m, 2 H), 4.54 (t, J=50 Hz, 1 H), 4.84 - 4.94 (m, 1 H), 5.37 - 5.53 (m, 3 H), 6.81 (s, 2 H), 7.19 (s, 1 H), 7.38 (d, J=8.3Hz, 1 H), 7.46 (d, J=11.6 Hz, 1 H), 7.87 (t, J=8.0 Hz, 1 H) | ESI 370[M+1]⁺ | 0.447 (B) |
| 4 | 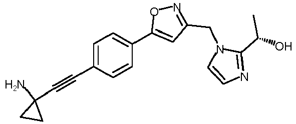 | ¹H NMR (600 MHz, CHLOROFORM-d) *δ* ppm 1.00 - 1. 08 (m, 4 H), 1. 69 (d, J=6.6 Hz, 3 H), 4.99 - 5.06 (m, 1 H), 5.28 - 5.33 (m, 1 H), 5.37 - 5.41 (m, 1 H), 6.36 (s, 1 H), 6.96 (d, J=1.2 Hz, 1 H), 7.01 (d, J=1.2 Hz, 1 H), 7.44 (d, J=8.3 Hz, 2 H), 7.65 (d, J=8.3 Hz, 2 H) | ESI 349[M+1]⁺ | 0.484 (A) |
| 5 | 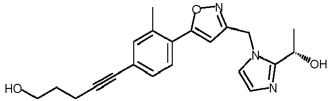 | ¹H NMR (400 MHz, METHANOL-d4) *δ* ppm 1.60 (d, J=6. 5 Hz, 3 H), 1. 74 - 1.86 (m, 2 H), 2.43 (s, 3 H), 2.52 (t, J=7.0 Hz, 2 H), 3.70 (t, J=6.2 Hz, 2 H), 5.00 - 5.11 (m, 1 H), 5.42 - 5.59 (m, 2 H), 6.59 (s, 1 H), 6.93 (s, 1 H), 7.16 (s, 1 H), 7.29 (d, J=8.2 Hz, 1 H), 7.33 (s, 1 H), 7.62 (d, J=8.2 Hz, 1 H) | ESI 366[M+1]⁺ | 0.500 (B) |
| 6 | 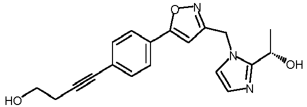 | ¹H NMR (600 MHz, CHLOROFORM-d) δ ppm 1.72 (d, J=6.6 Hz, 3 H), 2.72 (t, J=6.2 Hz, 2 H), 3.84 (t, J=6.2 Hz, 2 H), 5.10 - 5.22 (m, 1 H), 5.32 - 5. 39 (m, 1 H), 5.44 - 5.52 (m, 1 H), 6.47 (br s, 1 H), 7.01 (br s, 1 H), 7.09 (br s, 1 H), 7.49 (d, J=8. 3 Hz, 2 H), 7.68 (d. J=8.3 Hz. 2 H) | ESI 338[M+1]⁺ | 0.773 (A) |
| 7 | 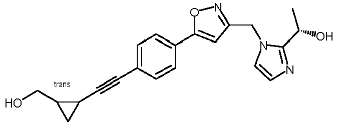 | ¹H NMR (400 MHz, METHANOL-d4) *δ* ppm 0.84 - 0.92 (m, 1 H), 0.92 - 1.00 (m, 1 H). 1. 38 - 1.51 (m, 2 H), 1.59 (d, J=66 Hz, 3 H), 3.37 -3.44 (m, 1 H), 3.51 - 3.59 (m, 1 H), 5.04 (q, J=64 Hz, 1 H), 5.42 - 5. 55 (m, 2 H), 6.70 (s, 1 H), 6.92 (s, 1 H), 7.15 (s, 1 H), 7.43 (d, J=8.2 Hz, 2 H), 7.72 (d, J=8.2 Hz. 2 H) | ESI 364[M+1]⁺ | 0.865 (A) |

**[Table 2-2]**

| Compound | Structural formula | ¹H-NMR | MS (m/z) | Retention time Method |
|---|---|---|---|---|
| 8 | 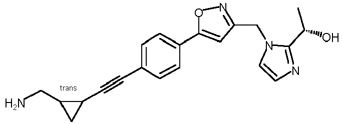 | ¹H NMR (400 MHz, METHANOL-d4) *δ* ppm 0.79 - 0.90 (m, 1 H), 0.93 - 1.03 (m, 1 H), 1.34 - 1.46 (m, 2 H), 1.59 (d, J=6.6 Hz, 3 H), 2.50 - 2.69 (m, 2 H), 5.04 (q, J=6. 6 Hz, 1 H), 5.41 - 5.57 (m, 2 H), 6.70 (s, 1 H), 6.92 (s, 1 H), 7.15 (s, 1 H), 7.42 (d, J=8.2 Hz, 2 H), 7.72 (d. J=8.2 Hz, 2 H) | ESI 363[M+1]⁺ | 0.556 (A) |
| 9 | 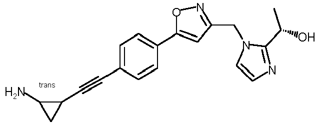 | ¹H NMR (400 MHz, METHANOL-d4) *δ* ppm 0.93 - 1.02 (m, 1 H), 1.21 - 1.48 (m, 2 H), 1.59 (d, J=6.5 Hz, 3 H), 2.54 - 2.64 (m, 1 H), 4.89 -5.16 (m, 1 H), 5.42 - 5.56 (m, 2 H), 6.70 (s, 1 H), 6.92 (s, 1 H), 7.15 (s, 1 H), 7.42 (d, J=8.6 Hz, 2 H), 7.72 (d, J=8.6 Hz, 2 H) | ESI 349[M+1]⁺ | 0.273 (A) |
| 10 | 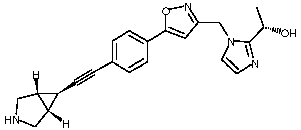 | ¹H NMR (400 MHz, METHANOL-d4) *δ* ppm 1.41 (br s, 1 H), 1.59 (d, J=6.6 Hz, 3 H), 1.87 (br s, 2 H), 2.85 (br d, J=11.5 Hz, 2 H), 3.06 (d, J=11. 5 Hz, 2 H), 5.04 (q, J=6.6 Hz, 1 H), 5.41 - 5.56 (m, 2 H), 6.70 (s, 1 H), 6.92 (s, 1 H), 7.15 (s, 1 H), 7.43 (d, J=8. 1 Hz, 2 H), 7.72 (d, J=8. 1 Hz, 2 H) | ESI 375[M+1]⁺ | 0.523 (A) |
| 11 | 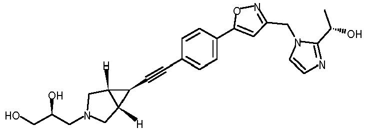 | ¹H NMR (600 MHz, METHANOL-d4) *δ* ppm 1.59 (d, J=6.6 Hz, 3 H), 1.80 - 1.84 (m, 2 H), 1.89 (t, J=3.1 Hz, 1 H), 2.44 (br d, J=7.0 Hz, 1 H), 2.50 (dd, J=12.4, 7.4 Hz, 2 H), 2.53 - 2.57 (m, 1 H), 3.18 (t, J=9.3 Hz, 2 H), 3.43 - 3.48 (m, 1 H), 3.50 - 3.54 (m, 1 H), 3.66 - 3. 71 (m, 1 H), 5.04 (q, J=6.6 Hz, 1 H), 5.43 - 5.54 (m, 2 H), 6.70 (s, 1 H), 6.92 (d, J=1.2 Hz, 1 H), 7.15 (d, J=1.2 Hz, 1 H), 740-744 (m, 2 H), 768-7.75 (m. 2 H) | ESI 449[M+1]⁺ | 0.516 (A) |
| 12 | 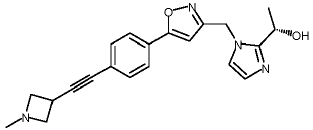 | ¹H NMR (600 MHz, METHANOL-d4) *δ* ppm 1.60 (d, J=6.6 Hz, 3 H), 2.34 (s, 3 H), 3.21 - 3.27 (m, 2 H), 3.42 - 3.49 (m, 1 H), 3.64 - 3.70 (m, 2 H), 5.01 - 5.07 (m, 1 H), 5.43 - 5.55 (m, 2 H), 6.73 (s, 1 H), 6.93 (d, J=1.2 Hz, 1 H), 7.15 (d, J=1.2 Hz, 1 H), 7.50 (d, J=8.3 Hz, 2 H), 7.76 (d, J=8.3 Hz. 2 H) | ESI 182[M+2]²⁺ | 0.245 (A) |
| 13 | 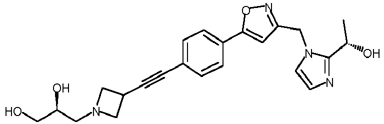 | ¹H NMR (600 MHz, METHANOL-d4) *δ* ppm 1.59 (d, J=6.6 Hz, 3 H), 2.52 - 2.59 (m, 1 H), 2.67 - 2. 73 (m, 1 H), 3.32 - 3.37 (m, 1 H), 3.47 (dd, J=5.6, 1.9 Hz, 2 H), 3.48 - 3. 55 (m, 1 H), 3.57 - 3.66 (m, 2 H), 3.74 - 3.82 (m, 2 H), 5.04 (q, J=6.6 Hz, 1 H), 5.44 - 5.48 (m, 1 H), 5.49 - 5.55 (m, 1 H), 6.73 (s, 1 H), 6.93 (d, J=1.7 Hz, 1 H), 7.15 (d, J=1.7 Hz, 1 H), 7.48 - 7.52 (m, 2 H), 7.74 - 7.78 (m. 2 H) | ESI 423[M+1]⁺ | 0.241 (A) |
| 14 | 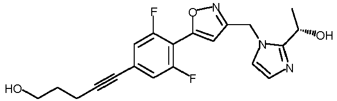 | ¹H NMR (400 MHz, DMSO-d6) *δ* ppm 1.47 (d, J=6.4 Hz, 3 H), 1.65 - 1.77 (m, 2 H), 3.46 - 3.60 (m, 2 H), 4.54 (t, J=5.1 Hz, 1 H), 4.84 - 4.93 (m, 1 H), 5.38 - 5.55 (m, 3 H), 6.82 (br s, 1 H), 6.86 (s, 1 H), 7.20 (s, 1 H), 7.37 (s, 1 H). 7.39 (s. 1 H) | ESI 388[M+1]⁺ | 0.489 (B) |

**[Table 2-3]**

| Compound | Structural formula | ¹H-NMR | MS (m/z) | Retention time Method |
|---|---|---|---|---|
| 15 | 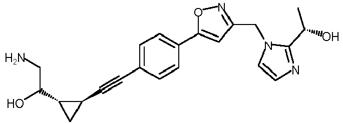 | ¹H NMR (400 MHz, METHANOL-d4) *δ* ppm 0.88 - 1.06 (m, 2 H), 1.26 - 1. 38 (m, 1 H), 1. 43 - 1.56 (m, 1 H), 1.59 (d, J=6.5 Hz, 3 H), 2.63 - 2.74 (m, 1 H), 2.75 - 2.86 (m, 1 H), 3.02 - 3.23 (m, 1 H), 5.04 (q, J=6.5 Hz, 1 H), 5.40 - 5.57 (m, 2 H), 6.69 (s, 1 H), 6.92 (s, 1 H), 7.15 (s, 1 H), 7.43 (d, J=8.2 Hz, 2 H), 7.71 (d, J=8.2 Hz. 2 H) | ESI 415[M+23]⁺ | 0.537 (A) |
| 16 | 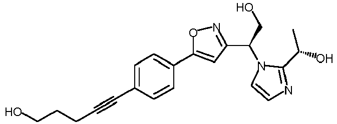 | ¹H NMR (600 MHz, METHANOL-d4) *δ* ppm 1.64 (d, J=6.6 Hz, 3 H), 1.81 (quin, J=6.6 Hz, 2 H), 2.53 (t, J=6.6 Hz, 2 H), 3.70 (t, J=6.6 Hz, 2 H), 4.15 - 4.29 (m, 2 H), 5.04 - 5.10 (m, 1 H), 6.05 - 6.09 (m, 1 H), 6.79 (s, 1 H), 6.94 (s, 1 H), 7.32 - 7.34 (m, 1 H), 7.47 (d, J=8.5 Hz, 2 H), 7.75 (d, J=8.5 Hz. 2 H) | ESI 382[M+1]⁺ | 0.816 (A) |
| 17 | 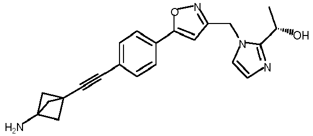 | ¹H NMR (400 MHz, METHANOL-d4) *δ* ppm 1.59 (d, J=6.6 Hz, 3 H), 2.12 (s, 6 H), 5.04 (q, J=6.6 Hz, 1 H), 5.41 - 5.58 (dd, J=159, 15.9, 2 H), 6.72 (s, 1 H), 6.92 (d, J=1.1 Hz, 1 H), 7.15 (d, J=1.1 Hz, 1 H), 7.46 (d, J=8.4 Hz, 2 H), 7.74 (d, J=8.4 Hz, 2 H) | ESI 375[M+1]⁺ | 0.442 (A) |
| 18 | 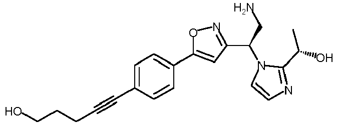 | ¹H NMR (400 MHz, METHANOL-d4) *δ* ppm 1.65 (d, J=6.7 Hz, 3 H), 1.81 (quin, J=6. 2 Hz, 2 H), 2.52 - 2.54 (m, 2 H), 3.37 - 3.41 (m, 1 H), 3.50 - 3.54 (m, 1 H), 3.70 (t, J=6. 2 Hz, 2 H), 5.08 - 5.1 1 (m, 1 H), 5.89 - 5.92 (m, 1 H), 6.78 (s, 1 H), 6.98 (s,, 1 H), 7.25 (s,, 1 H), 7.46 - 7.48 (m, 2 H). 7.75 - 7.76 (m, 2 H) | ESI 381[M+1]⁺ | 0.623 (A) |
| 19 | 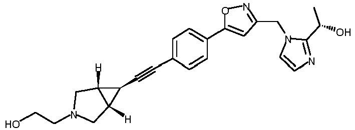 | ¹H NMR (400 MHz, CHLOROFORM-d) *δ* ppm 1.68 (d, J=6.6 Hz, 3 H), 1.82 - 1.92 (m, 3 H), 2.46 (br d, J=9.0 Hz, 2 H), 2.63 (t, J=5.3 Hz, 2 H), 3.16 (d, J=9.0 Hz, 2 H), 3.57 (t, J=5.3 Hz, 2 H), 5.02 (q, J=6.6 Hz, 1 H), 5.24 - 5.44 (m, 2 H), 6.35 (s, 1 H), 6.96 (br s, 1 H), 7.00 (br s, 1 H), 7.42 (d, J=8.3 Hz, 2 H), 7.63 (d, J=8.3 Hz. 2 H) | ESI 419[M+1]⁺ | 0.514 (A) |
| 20 | 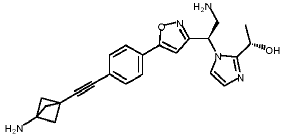 | ¹H NMR (600 MHz, METHANOL-d4) *δ* ppm 1.66 (d, J=6.3 Hz, 3 H), 2.12 (s, 6 H), 3.39 (dd, J=13.6, 8. 1 Hz, 1 H), 3.52 (dd, J=13.6, 6.2 Hz, 1 H), 5.09 (q, J=6.3 Hz, 1 H), 5.90 (dd, J=8. 1, 6.2 Hz, 1 H), 6.79 (s, 1 H), 6.97 (s, 1 H), 7.24 (s, 1 H), 7.46 (d, J=8.3 Hz, 2 H), 7.75 (d, J=8.3 Hz, 2 H) | ESI 404[M+1]⁺ | 0.221 (A) |
| 21 | 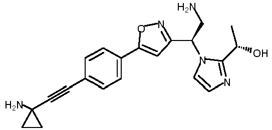 | ¹H NMR (600 MHz, METHANOL-d4) *δ* ppm 0.96 - 1.00 (m, 2 H), 1.02 - 1.06 (m, 2 H), 1.66 (d, J=6.6 Hz, 3 H), 3.39 (dd, J=13.6, 8.3 Hz, 1 H), 3.52 (dd, J=13.6, 6.2 Hz, 1 H), 5.09 (q, J=6.6 Hz, 1 H), 5.88 - 5.93 (m, 1 H), 6.78 (s, 1 H), 6.97 (d, J=1.2 Hz, 1 H), 7.24 (d, J=1.2 Hz, 1 H), 7.47 (d, J=8.3 Hz, 2 H), 7.76 (d, J=8.3 Hz, 2 H) | ESI378[M+1] + | 0.201 (A) |

**[Table 2-4]**

| Compound | Structural formula | ¹H-NMR | MS (m/z) | Retention time Method |
|---|---|---|---|---|
| 22 | 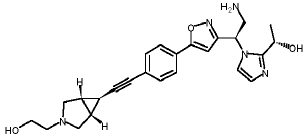 | ¹H NMR (400 MHz, CHLOROFORM-d) *δ* ppm 1.76 (d, J=6.4 Hz, 3 H), 1.82 - 1.92 (m, 3 H), 2.46 (br d, J=9.0 Hz, 2 H), 2.58 - 2.69 (m, 2 H), 3.16 (br d, J=9.0 Hz, 2 H), 3.40 - 3.50 (m, 1 H), 3.53 - 3.66 (m, 3 H), 5.05 (q, J=6.4 Hz, 1 H), 5.60 -5.68 (m, 1 H), 6.29 (s, 1 H), 6.95 (s, 1 H), 7.09 (s, 1 H), 7.43 (d, J=8.0 Hz, 2 H), 7.64 (d. J=8.0 Hz. 2 H) | ESI 448 [M+1]⁺ | 0.195 (A) |
| 23 | 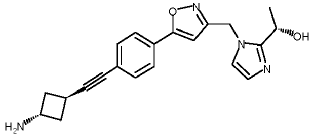 | ¹H NMR (400 MHz, METHANOL-d4) *δ* ppm 1.59 (d, J=6.6 Hz, 3 H), 2.14 - 2.28 (m, 2 H), 2.37 - 2.48 (m, 2 H), 3.18 - 3.26 (m, 1 H), 3.67 - 3.81 (m, 1 H), 4.99 - 5.08 (m, 1 H), 5.43 - 5.56 (m, 2 H), 6.72 (s, 1 H), 6.91 - 6.95 (m, 1 H), 7.15 (s, 1 H), 7.47 (d, J=8.4 Hz, 2 H), 7.75 (d, J=8.4 Hz, 2 H) | ESI 363[M+1]⁺ | 0.532 (A) |
| 24 | 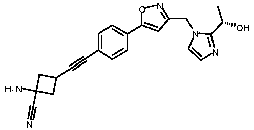 | ¹H NMR (400 MHz, METHANOL-d4) *δ* ppm 1.59 (d, J=6.6 Hz, 3 H), 2.32 - 2.48 (m, 2 H), 2.92 - 3.04 (m, 2 H), 3.35 - 3.45 (m, 1 H), 4.99 - 5.09 (m, 1 H), 5.40 - 5.59 (m, 2 H), 6.73 (s, 1 H), 6.93 (s, 1 H), 7.15 (s, 1 H), 7.48 (d, J=8.2 Hz, 2 H), 7.76 (d, J=8.2 Hz, 2 H) | ESI 388[M+1]⁺ | 0.689 (A) |
| 25 | 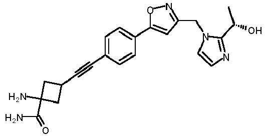 | ¹H NMR (400 MHz, METHANOL-d4) *δ* ppm 1.59 (d, J=6.7 Hz, 3 H), 2.15 - 2.28 (m, 2 H), 2.83 - 3.03 (m, 2 H), 3.43 - 3.55 (m, 1 H), 4.98 - 5.12 (m, 1 H), 5.37 - 5.60 (m, 2 H), 6.72 (s, 1 H), 6.93 (d, J=1.1 Hz, 1 H), 7.16 (s, 1 H), 7.47 (d, J=8.2 Hz, 2 H), 7.75 (d, J=8.2 Hz, 2 H) | ESI 406[M+1]⁺ | 0.256 (A) |
| 26 | 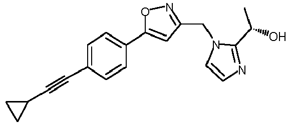 | ¹H NMR (600 MHz, CHLOROFORM-d) *δ* ppm 0.81 - 0.85 (m, 2 H), 0.88 - 0.93 (m, 2 H), 1.47 (tt, J=8.2, 5.0 Hz, 1 H), 1.70 (d, J=6.4 Hz, 3 H), 2.33 (br d, J=7.0 Hz, 1 H), 5.03 (br quin, J=6.4 Hz, 1 H), 5.28 - 5.32 (m, 1 H), 5.36 - 5.40 (m, 1 H), 6.34 (s, 1 H), 6.97 (s, 1 H), 7.02 (s, 1 H), 7.43 (d, J=8.7 Hz, 2 H), 7.63 (d, J=8.7 Hz. 2 H) | ESI 334[M+1]⁺ | 1.091 (A) |
| 27 | 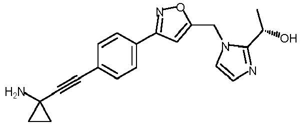 | ¹H NMR (400 MHz, METHANOL-d4) *δ* ppm 0.93 - 1.08 (m, 4 H), 1.59 (d, J=6.4 Hz, 3 H), 5.03 (q, J=6.4 Hz, 1 H), 5.61 (dd, J=14.9, 14.9 Hz, 2 H), 6.73 (s, 1 H), 6.94 (s, 1 H), 7.20 (s, 1 H), 7.45 (d, J=8.7 Hz, 2 H), 7.76 (d, J=8.7 Hz, 2 H) | ESI 349[M+1]⁺ | 0.393 (A) |
| 28 | 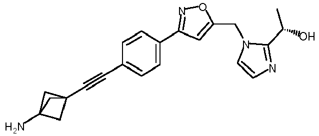 | ¹H NMR (400 MHz, METHANOL-d4) *δ* ppm 1.59 (d, J=6.5 Hz, 3 H), 2.15 (s, 6 H), 5.03 (q, J=6.5 Hz, 1 H), 5.61 (dd, J=15.7, 15.7 Hz, 2 H), 6.73 (s, 1 H), 6.94 (s, 1 H), 7.20 (s, 1 H), 7.45 (d, J=8.3 Hz, 2 H), 7.75 (d, J=8.3 Hz, 2 H) | ESI 375[M+1]⁺ | 0.426 (A) |

**[Table 2-5]**

| Compound | Structural formula | ¹H-NMR | MS (m/z) | Retention time Method |
|---|---|---|---|---|
| 29 | 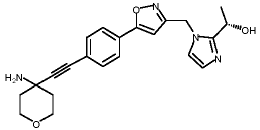 | ¹H NMR (600 MHz, CHLOROFORM-d) *δ* ppm 1.70 (d, J=6.6 Hz, 3 H), 1.73 - 1.79 (m, 2 H), 1.87 - 1.93 (m, 2 H), 3.70 - 3.76 (m, 2 H), 3.96 (dt, J=11. 9, 3.6 Hz, 2 H), 5.04 (q, J=6.6 Hz, 1 H), 5.29 - 5.34 (m, 1 H), 5.37 - 5.42 (m, 1 H), 6.38 (s, 1 H), 6.97 (d, J=1.2 Hz, 1 H), 7.03 (d, J=1.2 Hz, 1 H), 7.47 - 7.51 (m, 2 H), 7.66 - 7.70 (m. 2 H) | ESI 393[M+1]⁺ | 0.516 (A) |
| 30 | 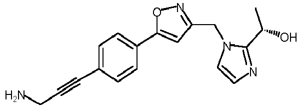 | ¹H NMR (600 MHz, CHLOROFORM-d) *δ* ppm 1.70 (d, J=6.6 Hz, 3 H), 3.68 (s, 2 H), 5.00 - 5.08 (m, 1 H), 5.28 - 5.34 (m, 1 H), 5.36 - 5.42 (m, 1 H), 6.36 (s, 1 H), 6.97 (s, 1 H), 7.03 (s, 1 H), 7.48 (d, J=8.3 Hz, 2 H), 7.67 (d, J=8.3 Hz. 2 H) | ESI 323[M+1]⁺ | 0.234 (A) |
| 31 | 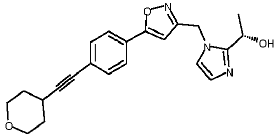 | ¹H NMR (400 MHz, METHANOL-d4) *δ* ppm 1.60 (d, J=6.5 Hz, 3 H), 1.66 - 1.80 (m, 2 H), 1.81 - 2.01 (m, 2 H), 2.86 - 2.96 (m, 1 H), 3.46 - 3.64 (m, 2 H), 3.83 - 3.98 (m, 2 H), 4.99 - 5.11 (m, 1 H), 5.43 - 5.57 (m, 2 H), 6.72 (s, 1 H), 6.93 (s, 1 H), 7.16 (s, 1 H), 7.48 (d, J=8.2 Hz, 2 H), 7.75 (d, J=8.2 Hz. 2 H) | ESI 378[M+1]⁺ | 0.583 (A) |
| 32 | 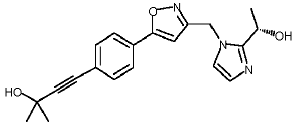 | ¹H NMR (600 MHz, CHLOROFORM-d) *δ* ppm 1.63 (s, 6 H), 1.70 (d, J=6.6 Hz, 3 H), 2.00 (s, 1 H), 2.32 (d, J=7.4 Hz, 1 H), 5.03 (quin, J=6.6 Hz, 1 H), 5.28 - 5.34 (m, 1 H), 5.36 - 5.42 (m, 1 H), 6.37 (s, 1 H), 6.97 (d, J=1.2 Hz, 1 H), 7.02 (d, J=1.2 Hz, 1 H), 7.47 - 7.51 (m. 2 H), 7.64 - 7.71 (m, 2 H) | ESI 352[M+1]⁺ | 0.460 (A) |
| 33 | 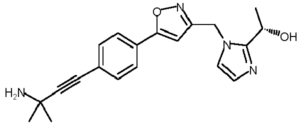 | ¹H NMR (600 MHz, CHLOROFORM-d) *δ* ppm 1.50 (s, 6 H), 1.70 (d, J=6.6 Hz, 3 H), 2.37 (br s, 1 H), 5.03 (q, J=6.6 Hz, 1 H), 5.25 - 5.34 (m, 1 H), 5.35 - 5.43 (m, 1 H), 6.36 (s, 1 H), 6.97 (d, J=1.2 Hz, 1 H), 7.02 (d, J=1.2 Hz, 1 H), 7.46 (d, J=8.7 Hz, 2 H), 7.63 - 7.70 (m. 2 H) | ESI 351[M+1]⁺ | 0.531 (A) |
| 34 | 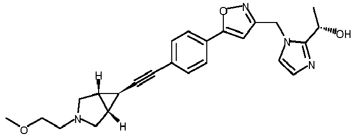 | ¹H NMR (400 MHz, METHANOL-d4) *δ* ppm 1.59 (d, J=6.6 Hz, 3 H), 1.79 - 1.90 (m, 3 H), 2.41 - 2.50 (m, 2 H), 2.64 (t, J=5.7 Hz, 2 H), 3.10 - 3.18 (m, 2 H), 3.33 (s, 3 H), 3.46 (t, J=5.7 Hz, 2 H), 4.99 - 5.07 (m, 1 H), 5.42 - 5.55 (m, 2 H), 6.70 (s, 1 H), 6.92 (s, 1 H), 7.15 (s, 1 H), 7.42 (d, J=8.3 Hz. 2 H), 7.72 (d. J=8.3 Hz. 2 H) | ESI 433[M+1]⁺ | 0.583 (A) |
| 35 | 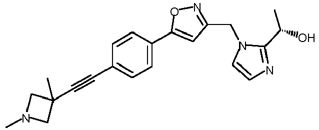 | ¹H NMR (400 MHz, METHANOL-d4) *δ* ppm 1.51 - 1.68 (m, 6 H), 2.37 (s, 3 H), 3.32 - 3.38 (m, 2 H), 3.47 (d, J=7.3 Hz, 2 H), 5.04 (q, J=6.6 Hz, 1 H), 5.49 (dd, J=15.9, 15.9 Hz, 2 H), 6.73 (s, 1 H), 6.93 (s, 1 H), 7.16 (s, 1 H), 7.50 (d, J=8.2 Hz, 2 H), 7.76 (d, J=8.2 Hz, 2 H) | ESI 377[M+1]⁺ | 0.544 (A) |

**[Table 2-6]**

| Compound | Structural formula | ¹H-NMR | MS (m/z) | Retention time Method |
|---|---|---|---|---|
| 36 | 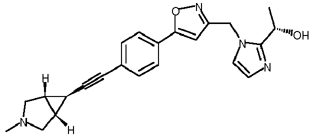 | ¹H NMR (400 MHz, METHANOL-d4) *δ* ppm 1.59 (d, J=6.6 Hz, 3 H), 1.77 - 1.92 (m, 3 H), 2.32 (s, 3 H), 2.37 - 2.49 (m, 2 H), 3.05 - 3.16 (m, 2 H), 5.00 - 5.06 (m, 1 H), 5.42 - 5.57 (m, 2 H), 6.70 (s, 1 H), 6.93 (s, 1 H), 7.15 (s, 1 H), 7.43 (d, J=8.4 Hz, 2 H), 7.72 (d, J=8.4 Hz, 2 H) | ESI 195[M+2]²⁺ | 0.511 (A) |
| 37 | 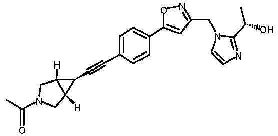 | ¹H NMR (400 MHz, METHANOL-d4) *δ* ppm 1.59 (d, J=6.6 Hz, 3 H), 1.97 - 2.13 (m, 6 H), 3.37 - 3.57 (m, 1 H), 3.59 - 3.83 (m, 3 H), 4.99 - 5.13 (m, 1 H), 5.42 - 5.55 (m, 2 H), 6.71 (s, 1 H), 6.93 (s, 1 H), 7.15 (s, 1 H), 7.45 (d, J=8.3 Hz, 2 H), 7.74 (d, J=8.3 Hz, 2 H) | ESI 417[M+1]⁺ | 0.869 (A) |
| 38 | 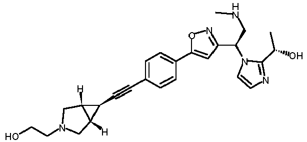 | ¹H NMR (400 MHz, METHANOL-d4) *δ* ppm 1.65 (d, J=6.5 Hz, 3 H), 1.76 - 1.96 (m, 3 H), 2.37 (s, 3 H), 2.41 - 2.51 (m, 2 H), 2.59 (t, J=5.9 Hz, 2 H), 3.10 - 3.21 (m, 2 H), 3.43 - 3.54 (m, 2 H), 3.56 - 3.63 (m, 2 H), 5.02 - 5.17 (m, 1 H), 6.05 (t, J=7.2 Hz, 1 H), 6.78 (s, 1 H), 6.96 (s, 1 H), 7.26 (s, 1 H), 7.43 (d, J=8.2 Hz, 2 H), 7.73 (d. J=8.2 Hz. 2 H) | ESI 462[M+1]⁺ | 0.211 (A) |
| 39 | 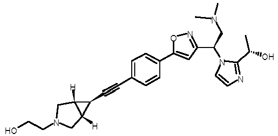 | ¹H NMR (400 MHz, METHANOL-d4) *δ* ppm 1.65 (d, J=6.5 Hz, 3 H), 1.79 - 1.86 (m, 2 H), 1.86 - 1.95 (m, 1 H), 2.30 (s, 6 H), 2.38 - 2.51 (m, 2 H), 2.52 - 2.65 (m, 2 H), 3.05 - 3.21 (m, 2 H), 3.41 - 3.50 (m, 2 H), 3.55 - 3.63 (m, 2 H), 5.05 - 5.14 (m, 1 H), 6.06 - 6.12 (m, 1 H), 6.83 (s, 1 H), 6.95 (s, 1 H), 7.27 (s, 1 H), 7.43 (d, J=8.3 Hz, 2 H), 7.73 (d, J=8.3 Hz. 2 H) | ESI 476[M+1]⁺ | 0.233 (A) |
| 40 | 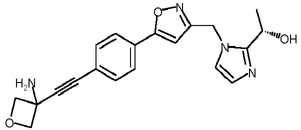 | ¹H NMR (400 MHz, METHANOL-d4) *δ* ppm 1.60 (d, J=6.5 Hz, 3 H), 4.64 (d, J=6.1 Hz, 2 H), 4.88 (d, J=6.1 Hz, 2 H), 5.04 (q, J=6.5 Hz, 1 H), 5.50 (dd, J=14.7, 14.7 Hz, 2 H), 6.75 (s, 1 H), 6.93 (s, 1 H), 7.16 (s, 1 H), 7.56 (d, J=8.4 Hz, 2 H), 7.80 (d, J=8.4 Hz, 2 H) | ESI 365[M+1]⁺ | 0.362 (A) |
| 41 | 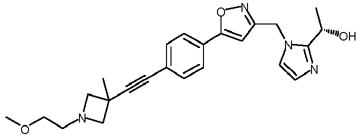 | ¹H NMR (400 MHz, METHANOL-d4) *δ* ppm 1.54 - 1.62 (m, 6 H), 2.71 (t, J=5.4 Hz, 2 H), 3.27 - 3.39 (m, 5 H), 3.42 (t, J=5.4 Hz, 2 H), 3.50 (d, J=7.3 Hz, 2 H), 5.04 (q, J=6.8 Hz, 1 H), 5.49 (dd, J=16.1, 16.1 Hz, 2 H), 6.73 (s, 1 H), 6.93 (s, 1 H), 7.16 (s, 1 H), 7.50 (d, J=8.1 Hz, 2 H), 7.76 (d, J=8.1 Hz. 2 H) | ESI 421[M+1]⁺ | 0.598 (A) |
| 42 | 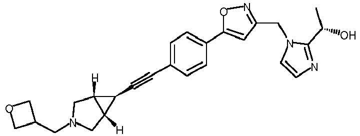 | ¹H NMR (400 MHz, METHANOL-d4) *δ* ppm 1.59 (d, J=6.5 Hz, 3 H), 1.81 (s, 3 H), 2.31 - 2.48 (m, 2 H), 2.76 (d, J=7.6 Hz, 2 H), 2.99 - 3.07 (m, 2 H), 4.38 (t, J=6.2 Hz, 2 H), 4.45 - 4.65 (m, 1 H), 4.67 - 4.79 (m, 2 H), 4.88 - 5. 15 (m, 1 H), 5.39 - 5.55 (m, 2 H), 6.70 (s, 1 H), 6.92 (s, 1 H), 7.15 (s, 1 H), 7.42 (d, J=8.3 Hz, 2 H), 7.72 (d. J=8.3 Hz. 2 H) | ESI 445[M+1]⁺ | 0.540 (A) |

**[Table 2-7]**

| Compound | Structural formula | ¹H-NMR | MS (m/z) | Retention time Method |
|---|---|---|---|---|
| 43 | 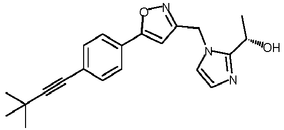 | ¹H NMR (600 MHz, CHLOROFORM-d) *δ* ppm 1.32 (s, 9 H), 1.69 (d, J=6.6 Hz, 3 H), 5.03 (q, J=6.6 Hz, 1 H), 5.28 - 5.32 (m, 1 H), 5.37 - 5.41 (m, 1 H), 6.35 (s, 1 H), 6.96 (s, 1 H), 7.01 (s, 1 H), 7.43 - 7.46 (m, 2 H), 7.63 (d, J=8.3 Hz, 2 H) | ESI 350[M+1]⁺ | 0.763 (A) |
| 44 | 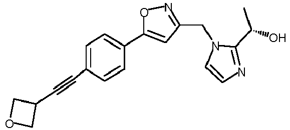 | ¹H NMR (400 MHz, METHANOL-d4) *δ* ppm 1.60 (d, J=6.6 Hz, 3 H), 4.15 (br t, J=7.8 Hz, 1 H), 4.69 - 4.80 (m, 2 H), 4.88 - 4.97 (m, 2 H), 4.98 - 5.20 (m, 1 H), 5.43 - 5.56 (m, 2 H), 6.74 (s, 1 H), 6.93 (s, 1 H), 7.16 (s, 1 H), 7.53 (d, J=8.2 Hz, 2 H), 7.78 (d, J=8.2 Hz, 2 H) | ESI 350[M+1]⁺ | 0.486 (B) |
| 45 | 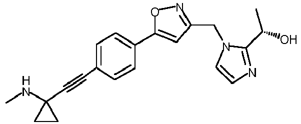 | ¹H NMR (600 MHz, CHLOROFORM-d) *δ* ppm 0.96 - 0.99 (m, 2 H), 1.02 - 1.06 (m, 2 H), 1. 70 (d, J=6.6 Hz, 3 H), 2.57 (s, 3 H), 5.03 (br q, J=6.6 Hz, 1 H), 5.28 - 5.33 (m, 1 H), 5.36 - 5.41 (m, 1 H), 6.35 (s, 1 H), 6.97 (d, J=1.2 Hz, 1 H), 7.02 (d, J=1.2 Hz, 1 H), 7.44 - 7.48 (m, 2 H), 7.63 - 7.67 (m, 2 H) | ESI 182[M+2]²⁺ | 0.541 (A) |
| 46 | 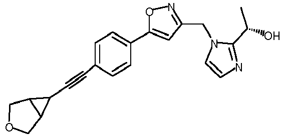 | ¹H NMR (400 MHz, CHLOROFORM-d) *δ* ppm 1.43 - 1.52 (m, 1 H), 1.69 (d, J=6.4 Hz, 3 H), 1.96 - 2.10 (m, 2 H), 3.73 (br d, J=8.7 Hz, 2 H), 3.95 (d, J=8.7 Hz, 2 H), 5.03 (q, J=6.4 Hz, 1 H), 5.24 - 5.45 (m, 2 H), 6.35 (s, 1 H), 6.96 (s, 1 H), 7.01 (s, 1 H), 7.43 (d, J=8.1 Hz, 2 H), 7.64 (d, J=8.1 Hz. 2 H) | ESI 376[M+1]⁺ | 0.587 (B) |
| 47 | 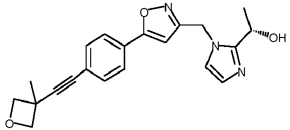 | ¹H NMR (400 MHz, METHANOL-d4) *δ* ppm 1.60 (d, J=6.6 Hz, 3 H), 1.70 (s, 3 H), 4.50 (d, J=5.4 Hz, 2 H), 4.87 - 4.95 (m, 2 H), 4.95 - 5.10 (m, 1 H), 5.43 - 5.56 (m, 2 H), 6.74 (s, 1 H), 6.93 (s, 1 H), 7.16 (s, 1 H), 7.52 (d, J=8.3 Hz, 2 H), 7.78 (d, J=8.3 Hz, 2 H) | ESI 364[M+1]⁺ | 0.553 (B) |
| 48 | 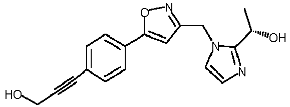 | ¹H NMR (400 MHz, METHANOL-d4) *δ* ppm 1.60 (d, J=6.6 Hz, 3 H), 4.41 (s, 2 H), 4.93 - 5.10 (m, 1 H), 5.43 - 5.57 (m, 2 H), 6.74 (s, 1 H), 6.93 (s, 1 H), 7.16 (s, 1 H), 7.53 (d, J=8.2 Hz, 2 H), 7.78 (d, J=8.2 Hz, 2 H) | ESI 324[M+1]⁺ | 0.702 (A) |
| 49 | 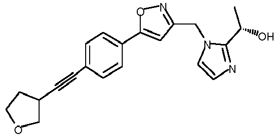 | ¹H NMR (400 MHz, METHANOL-d4) *δ* ppm 1.59 (d, J=6.6 Hz, 3 H), 2.00 - 2.09 (m, 1 H), 2.26 - 2.36 (m, 1 H), 3.23 - 3.31 (m, 1 H), 3.68 - 3.74 (m, 1 H), 3.82 - 3.89 (m, 1 H), 3.91 - 3.98 (m, 1 H), 4.03 (t, J=7.6 Hz, 1 H), 4.98 - 5.07 (m, 1 H), 5.43 - 5.55 (m, 2 H), 6.72 (s, 1 H), 6.93 (s, 1 H), 7.15 (s, 1 H), 7.48 (d, J=8.1 Hz, 2 H), 7.75 (d, J=8.1 Hz, 2 H) | ESI 364[M+1]⁺ | 0.547 (B) |

**[Table 2-8]**

| Compound | Structural formula | ¹H-NMR | MS (m/z) | Retention time Method |
|---|---|---|---|---|
| 50 | 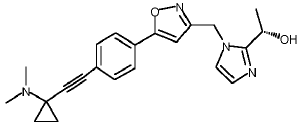 | ¹H NMR (600 MHz, CHLOROFORM-d) *δ* ppm 0.95 - 0.99 (m, 2H), 1.02 - 1.06 (m, 2H), 1.67 (d, J=6.6 Hz, 3H), 2.40 (s, 6H), 5.02 (q, J=6.5 Hz, 1H), 5.29 - 5.34 (m, 1 H), 5.38 - 5.43 (m, 1H), 6.37 (s, 1H), 6.95 (s, 1H), 6.98 (s, 1H), 7.45 - 7.50 (m, 2H), 7.62 - 7.67 (m, 2 H) | ESI 189 [M+2]²⁺ | 0.580 (A) |
| 51 | 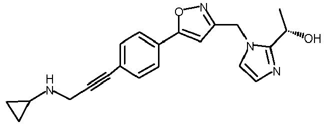 | ¹H NMR (400 MHz, METHANOL-d4) *δ* ppm 0.37 - 0.48 (m, 2H), 0.48 - 0.58 (m, 2H), 1.59 (d, J=6.6 Hz, 3H), 2.37 - 2.45 (m, 1H), 3.66 (s, 2H), 4.99 - 5.10 (m, 1 H), 5.43 - 5.58 (m, 2H), 6.73 (s, 1 H), 6.93 (s, 1H), 7.15 (s, 1H), 7.52 (d, J=8.2Hz, 2H), 7.77 (d, J=8.2 Hz, 2 H) | ESI 182[M+2]²⁺ | 0.252 (A) |
| 52 | 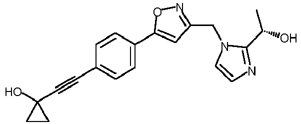 | ¹H NMR (400 MHz, METHANOL-d4) *δ* ppm 1.02 - 1.09 (m, 4 H), 1.59 (d, J=6.6 Hz, 3 H), 5.04 (q, J=6.6 Hz, 1H), 5.49 (dd, J=15.9, 15.9Hz, 2H), 6.73 (s, 1 H), 6.93 (s, 1H), 7.16 (s, 1H), 7.48 (d, J=8.3 Hz, 2H), 7.76 (d, J=8.3 Hz, 2 H) | ESI 350[M+1]⁺ | 0.860 (A) |
| 53 | 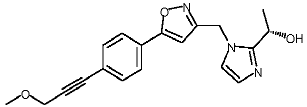 | ¹H NMR (600 MHz, CHLOROFORM-d) *δ* ppm 1.70 (d, J=6.2 Hz, 3 H), 3.46 (s, 3 H), 4.34 (s, 2 H), 5.03 (br s, 1 H), 5.28 - 5.34 (m, 1 H), 5.37 - 5.42 (m, 1 H), 6.38 (s, 1 H), 6.97 (br s, 1H), 7.03 (br s, 1 H), 7.51 - 7.54 (m, 2 H), 7.66 - 7.70 (m, 2 H) | ESI 338[M+1]⁺ | 0.524 (B) |
| 54 | 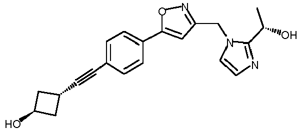 | ¹H NMR (400 MHz, CHLOROFORM-d) *δ* ppm 1.69 (d, J=6.4 Hz, 3H), 2.26 - 2.38 (m, 2H), 2.46 - 2.57 (m, 2 H), 3.18 - 3.28 (m, 1H), 4.66 (quin, J=6.7 Hz, 1H), 4.97 - 5.09 (m, 1H), 5.26 - 5.44 (m, 2 H), 6.36 (s, 1H), 6.97 (br s, 1 H), 7.02 (br s, 1H), 7.46 (d, J=8.1 Hz, 2H), 7.65 (d, J=8.1 Hz. 2 H) | ESI 364[M+1]⁺ | 0.460 (B) |
| 55 | 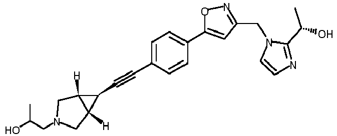 | ¹H NMR (400 MHz, METHANOL-d4) *δ* ppm 1.11 (d, J=6.2 Hz, 3 H), 1.59 (d, J=6.6 Hz, 3 H), 1. 77 - 1. 84 (m, 2 H), 1.90 - 1.96 (m, 1 H), 2.29 - 2.51 (m, 4 H), 3.08 - 3.21 (m, 2 H), 3.72 - 3.84 (m, 1H), 5.04 (q, J=6.6 Hz, 1H), 5.42 - 5.55 (m, 2H), 6.70 (s, 1H), 6.92 (s, 1H), 7.15 (s, 1H), 7.42 (d, J=8.2 Hz, 2H), 7.71 (d, J=8.2 Hz. 2 H) | ESI 433[M+1]⁺ | 0.545 (A) |
| 56 | 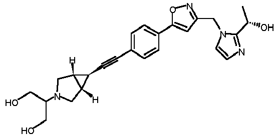 | ¹H NMR (400 MHz, METHANOL-d4) *δ* ppm 1.59 (d, J=6.4 Hz, 3 H), 1.80 - 1.97 (m, 3 H), 2.59 - 2.84 (m, 2 H), 3.04 - 3.24 (m, 2 H), 3.41 - 3.55 (m, 1 H), 3.56 - 3. 79 (m, 4 H), 5.01 - 5.11 (m, 1 H), 5.41 - 5.57 (m, 2 H), 6.70 (s, 1 H), 6.93 (s, 1 H), 7.15 (s, 1 H), 7.42 (d, J=8.2 Hz, 2 H), 7.72 (d, J=8.2 Hz. 2 H) | ESI 449[M+1]⁺ | 0.510 (A) |

**[Table 2-9]**

| Compound | Structural formula | ¹H-NMR | MS (m/z) | Retention time Method |
|---|---|---|---|---|
| 57 | 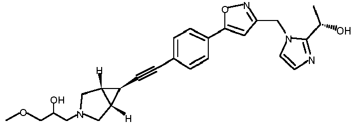 | ¹H NMR (400 MHz, METHANOL-d4) *δ* ppm 1.59 (d, J=6.6 Hz, 3 H), 1.76 - 1.86 (m, 2 H), 1.86 - 1.95 (m, 1 H), 2.38 - 2.57 (m, 4 H), 3 08 - 3.20 (m, 2 H), 3.24 - 3.47 (m, 5 H), 3.70 - 3.88 (m, 1H), 5.04 (q, J=6.6 Hz, 1H), 5.41 - 5.56 (m, 2 H), 6.70 (s, 1H), 6.92 (s, 1H), 7.15 (s, 1H), 7.42 (d, J=8.2 Hz, 2 H), 7.72 (d, J=8.2 Hz, 2 H) | ESI 463 [M+1]⁺ | 0.565 (A) |
| 58 | 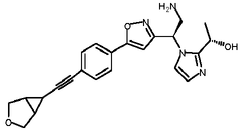 | ¹H NMR (400 MHz, CHLOROFORM-d) *δ* ppm 1.44 - 1.52 (m, 1H), 1.77 (d, J=6. 2 Hz, 3 H), 1.99 - 2.06 (m, 2 H), 3. 38 - 3. 51 (m, 1H), 3.58 - 3.68 (m, 1H), 3.73 (d, J=8.7 Hz, 2H), 3.95 (d, J=8.7 Hz, 2H), 5.05 (q, J=6.2 Hz, 1 H), 5.60 - 5.68 (m, 1H), 6.30 (s, 1 H), 6.95 (s, 1 H), 7.09 (s, 1 H), 7. 44 (d, J=7.9 Hz, 2 H), 7.65 (d. J=7.9 Hz. 2 H) | ESI 405 [M+1]⁺ | 0.768 (A) |
| 59 | 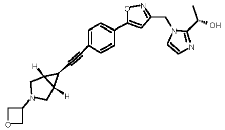 | ¹H NMR (400 MHz, METHANOL-d4) *δ* ppm 1.59 (d, J=6.6 Hz, 3 H), 1.84 - 2.00 (m, 3 H), 2.39 - 2.46 (m, 2H), 3.08 - 3.16 (m, 2H), 3.69 - 3.78 (m, 1H), 4.56 (t, J=5.9 Hz, 2H), 4.65 - 4.72 (m, 2H), 4.99 - 5.10 (m, 1H), 5.41 - 5.58 (m, 2H), 6.70 (s, 1H), 6.93 (s, 1H), 7.15 (s, 1H), 7.43 (d, J=8.4 Hz, 2 H), 7.72 (d, J=8.4 Hz, 2 H) | ESI 431 [M+1]⁺ | 0.576 (A) |
| 60 | 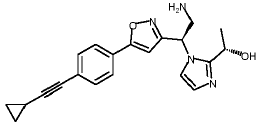 | ¹H NMR (400 MHz, CHLOROFORM-d) *δ* ppm 0.77 - 0.96 (m, 5 H), 1.77 (d, J=6.4 Hz, 3 H), 3.39 - 3.49 (m, 1 H), 3. 58 - 3. 67 (m, 1 H), 5.05 (q, J=6.0 Hz, 1 H), 5.64 (br dd, J=10.0, 4.0 Hz, 1 H), 6.29 (s, 1 H), 6.95 (s, 1 H), 7.09 (s, 1 H), 7.44 (d, J=7.9 Hz, 2 H), 7.64 (d, J=7.9 Hz, 2 H) | ESI 363 [M+1]⁺ | 0.856 (A) |
| 61 | 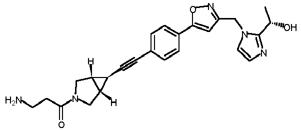 | ¹H NMR (600 MHz, METHANOL-d4) *δ* ppm 1.35 - 1.40 (m, 1 H), 1.59 (d, J=6.6 Hz, 3 H), 1.99 - 2.06 (m, 1 H), 2.06 - 2.12 (m, 1 H), 2.34 - 2.54 (m, 2H), 2.82 - 2. 92 (m, 2H), 3.40 - 3.48 (m, 1H), 3.61 - 3.69 (m, 1H), 3.74 - 3.89 (m, 2H), 5.04 (q, J=6.6 Hz, 1 H), 5.42 - 5.56 (m, 2H), 6.71 (s, 1H), 6.92 (d, J=1.2 Hz, 1 H), 7.15 (d, J=1.2 Hz, 1 H), 7.40 - 7.49 (m, 2 H), 7.69 - 7. 76 (m, 2 H) | ESI 446 [M+1]⁺ | 0.590 (A) |
| 62 | 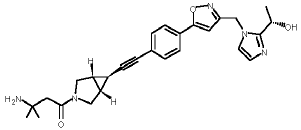 | ¹H NMR (600 MHz, METHANOL-d4) *δ* ppm 1.27 (s, 6 H), 1.34 - 1.37 (m, 1H), 1.59 (d, J=6.6 Hz, 3 H), 2.03 - 2.07 (m, 1H), 2.07 - 2.12 (m, 1 H), 2. 40 - 2. 52 (m, 2 H), 3. 45 (br dd, J=12.2, 4.3 Hz, 1 H), 3.66 (br dd, J=10. 7, 4. 1 Hz, 1H), 3.76 - 3.87 (m, 2H), 5.04 (q, J=6.6 Hz, 1H), 5.43 - 5.54 (m, 2 H), 6. 71 (s, 1H), 6.92 (d, J=1.2 Hz, 1 H), 7.15 (d, J=1.2 Hz, 1 H), 7.41 - 7.47 (m, 2 H). 7.69 - 7.75 (m. 2 H) | ESI 474 [M+1]⁺ | 0.654 (A) |
| 63 | 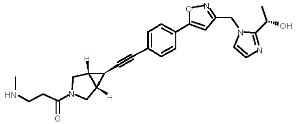 | ¹H NMR (600 MHz, METHANOL-d4) *δ* ppm 1.36 - 1.40 (m, 1 H), 1.59 (d, J=6.6 Hz, 3 H), 2.00 - 2.06 (m, 1 H), 2. 07 - 2. 12 (m, 1 H), 2.42 (s, 3 H), 2.44 - 2.51 (m, 1 H), 2.51 - 2.58 (m, 1H), 2.80 - 2.88 (m, 2 H), 3.42 - 3. 48 (m, 1 H), 3.67 (dd, J=10. 7, 4.1 Hz, 1 H), 3.78 (d, J=10.7 Hz, 1 H), 3.83 (d, J=12.4 Hz, 1 H), 5.04 (q, J=6.6 Hz, 1 H), 5.42 - 5.55 (m, 2 H), 6.71 (s, 1H), 6.92 (d, J=1.2 Hz, 1H), 7.15 (d, J=1.2 Hz, 1 H), 7.45 (d, J=8.3 Hz, 2 H), 7.73 (d, J=8. 3 Hz. 2 H) | ESI 460 [M+1]⁺ | 0.600 (A) |

**[Table 2-10]**

| Compound | Structural formula | ¹H-NMR | MS (m/z) | Retention time Method |
|---|---|---|---|---|
| 64 | 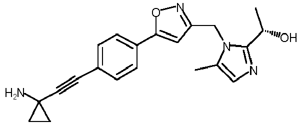 | ¹H NMR (400 MHz, METHANOL-d4) *δ* ppm 0.95 - 1.01 (m, 2 H), 1.01 - 1.08 (m, 2 H), 1.59 (d, J=6.6 Hz, 3 H), 2.20 (s, 3 H), 5.00 (q, J=6.6 Hz, 1 H), 5.36 - 5.52 (m, 2 H), 6.65 (s, 1 H), 6.67 (s, 1 H), 7.46 (d, J=8.2 Hz, 2 H), 7.74 (d, J=8.2 Hz, 2 H) | ESI 363[M+1]⁺ | 0.529 (A) |
| 65 | 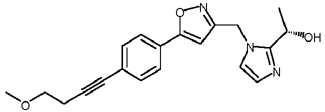 | ¹H NMR (400 MHz, METHANOL-d4) *δ* ppm 1.59 (d, J=6.6 Hz, 3 H), 2.65 - 2.73 (m, 2 H), 3.39 (s, 3 H), 3.56 - 3.62 (m, 2 H), 5.04 (q, J=6.6 Hz, 1 H), 5.41 - 5.57 (m, 2 H), 6.71 (s, 1 H), 6.93 (s, 1 H), 7.15 (s, 1 H), 7.48 (d, J=8.2 Hz, 2 H), 7.74 (d, J=8.2 Hz, 2 H) | ESI 352[M+1]⁺ | 0.950 (A) |
| 66 | 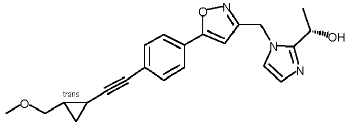 | ¹H NMR (400 MHz, METHANOL-d4) *δ* ppm 0.84 - 0.92 (m, 1 H), 0.94 - 1.02 (m, 1 H), 1.41 - 1.52 (m, 2 H), 1.59 (d, J=6.6 Hz, 3 H), 3.21 - 3.28 (m, 1 H), 3.36 (s, 3 H), 3.39 - 3.46 (m, 1 H), 5.04 (q, J=6.6 Hz, 1 H), 5.42 - 5.56 (m, 2 H), 6.70 (s, 1 H), 6.92 (s, 1 H), 7.15 (s, 1 H), 7.44 (d, J=8.3 Hz, 2 H). 7.72 (d. J=8.3 Hz. 2 H) | ESI 378[M+1]⁺ | 1.045 (A) |
| 67 | 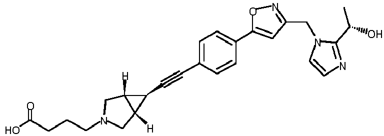 | ¹H NMR (400 MHz, METHANOL-d4) *δ* ppm 1.59 (d, J=6.5 Hz, 3 H), 1.71 - 1.95 (m, 3 H), 2.02 - 2. 13 (m, 2 H), 2.36 - 2.48 (m, 2 H), 2.85 - 3.00 (m, 4 H), 3.46 - 3.62 (m, 2 H), 4.95 - 5.12 (m, 1 H), 5.38 - 5.61 (m, 2 H), 6.71 (s, 1 H), 6.93 (s, 1 H), 7.15 (s, 1 H), 7.45 (d, J=8.1 Hz, 2 H), 7.73 (d, J=8.1 Hz, 2 H) | ESI 461 [M+1]⁺ | 0.641 (A) |
| 68 | 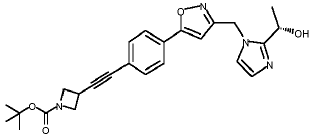 | ¹H NMR (400 MHz, CHLOROFORM-d) *δ* ppm 1.45 (s, 9 H), 1.70 (d, J=6.6 Hz, 3 H), 2.33 (d, J=7.3 Hz, 1 H), 3.51 - 3.59 (m, 1 H), 3.98 - 4.07 (m, 2 H), 4.17 - 4.26 (m, 2 H), 5.03 (s, 1 H), 5.28 - 5.42 (m, 2 H), 6.37 (s, 1 H), 6.97 (s, 1 H), 7.02 (s, 1 H), 7.48 (d, J=7.8 Hz, 2 H), 7.67 (d, J=7.8 Hz. 2 H) | ESI 449[M+1]⁺ | 1. 164 (A) |
| 69 | 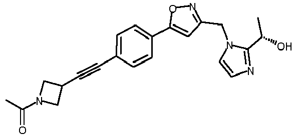 | ¹H NMR (400 MHz, CHLOROFORM-d) *δ* ppm 1.70 (d, J=6.5 Hz, 3 H), 1.89 (s, 3 H), 2.34 - 2.43 (m, 1 H), 3.58 - 3.67 (m, 1 H), 4.06 - 4. 13 (m, 1 H), 4.22 (t, J=6.9 Hz, 1 H), 4.32 (t, J=9.2 Hz, 1 H), 4.38 - 4.46 (m, 1 H), 4.99 - 5.08 (m, 1 H), 5.28 - 5.43 (m, 2 H), 6.38 (s, 1 H), 6.97 (s, 1 H), 7.02 (s, 1 H), 7.49 (d, J=8.1 Hz, 2 H), 7.68 (d. J=8. 1Hz. 2 H) | ESI 391 [M+1]⁺ | 0.796 (A) |
| 70 | 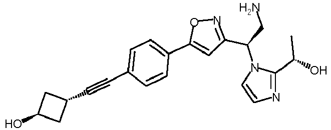 | ¹H NMR (400 MHz, CHLOROFORM-d) *δ* ppm 1.77 (d, J=6.4 Hz, 3 H), 2.24 - 2.40 (m, 2 H), 2.46 - 2.58 (m, 2 H), 3.18-3.31 (m, 1 H), 3.39 - 3.52 (m, 1 H), 3.63 (br dd, J=134, 3.8 Hz, 1 H), 4.60 - 4.72 (m, 1 H), 5.05 (q, J=6.4 Hz, 1 H), 5.65 (br dd, J=9.4, 3.8 Hz, 1 H), 6.30 (s, 1 H), 6.96 (s, 1 H), 7.09 (s, 1 H), 7.47 (d, J=8.1 Hz, 2 H). 7.66 (d. J=8.1 Hz. 2 H) | ESI 393 [M+1]⁺ | 0.710 (A) |

**[Table 2-11]**

| Compound | Structural formula | ¹H-NMR | MS (m/z) | Retention time Method |
|---|---|---|---|---|
| 71 | 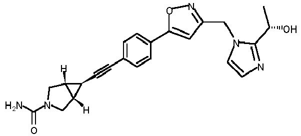 | ¹H NMR (400 MHz, METHANOL-d4) *δ* ppm 1.59 (d, J=6.8 Hz, 3 H), 1.89 - 1.91 (m, 1 H), 2.01 - 2.05 (m, 2 H), 3.39 - 3.53 (m, 2 H), 3.60 - 3.69 (m, 2 H), 4.98 - 5.07 (m, 1 H), 5.47 - 5.51 (m, 2 H), 6. 71 (s, 1 H), 6.92 (s, 1 H), 7.15 (s, 1 H), 7.45 (d, J=8.6 Hz, 2 H), 7.73 (d, J=8.6 Hz, 2 H) | ESI 418[M+1]⁺ | 0.775 (A) |
| 72 | 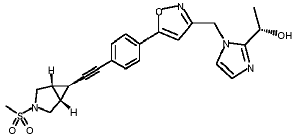 | ¹H NMR (400 MHz, METHANOL-d4) *δ* ppm 1.59 (d, J=6.5 Hz, 3 H), 1.62 - 1.66 (m, 1 H), 2.04 (br s, 2 H), 2.89 (s, 3 H), 3.38 - 3.45 (m, 2 H), 3.55 - 3.61 (m, 2 H), 5.04 (q, J=6.5 Hz, 1 H), 5.41 - 5.56 (m, 2 H), 6. 71 (s, 1 H), 6.93 (s, 1 H), 7.15 (s, 1 H), 7.45 (d, J=8.2 Hz, 2 H), 7.73 (d, J=8.2 Hz. 2 H) | ESI 453[M+1]⁺ | 0.944 (A) |
| 73 | 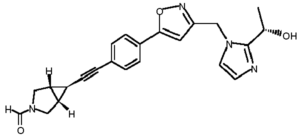 | ¹H NMR (400 MHz, METHANOL-d4) *δ* ppm 1.33 - 1.39 (m, 1 H), 1.59 (d, J=6.6 Hz, 3 H), 1.97 - 2.09 (m, 2 H), 3.23 - 3.39 (m, 1 H), 3.64 - 3.72 (m, 1 H), 3.79 - 3.95 (m, 2 H), 5.04 (br q, J=6.6 Hz, 1 H), 5.41 - 5.56 (m, 2 H), 6.71 (s, 1 H), 6.92 (s, 1 H), 7.15 (s, 1 H), 7.45 (d, J=8.2 Hz, 2 H), 7.73 (d, J=8.2 Hz, 2 H), 8.10 (s, 1 H) | ESI 403[M+1]⁺ | 0.838 (A) |
| 74 | 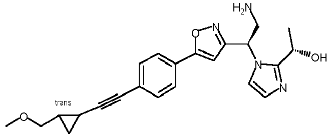 | ¹H NMR (400 MHz, METHANOL-d4) *δ* ppm 0.84 - 0.92 (m, 1 H), 0.94 - 1.03 (m, 1 H), 1.39 - 1.53 (m, 2 H), 1.66 (d, J=6.6 Hz, 3 H), 3.10 - 3.63 (m, 7 H), 5.09 (q, J=6.6 Hz, 1 H), 5.90 (t, J=7.0 Hz, 1 H), 6.76 (s, 1 H), 6.97 (s, 1 H), 7.24 (s, 1 H), 7.44 (d, J=8.3 Hz, 2 H). 7.73 (d. J=8.3 Hz. 2 H) | ESI 407[M+1]⁺ | 0.811 (A) |
| 75 | 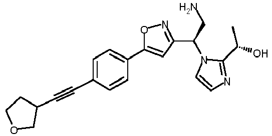 | ¹H NMR (400 MHz, METHANOL-d4) *δ* ppm 1.66 (d, J=6.5 Hz, 3 H), 1.98 - 2. 18 (m, 1 H), 2.19 - 2.44 (m, 1 H), 3.36 - 3.63 (m, 2 H), 3.68 - 3.74 (m, 1 H), 3.80 - 4.06 (m, 3 H), 4.57 (br s, 1 H), 5.03 - 5.16 (m, 1 H), 5.91 (dd, J=7.5, 6.4 Hz, 1 H), 6.79 (s, 1 H), 6.98 (s, 1 H), 7.24 (s, 1 H), 7.48 (d, J=8.1 Hz, 2 H), 7.76 (d, J=8.1 Hz. 2 H) | ESI 393[M+1]⁺ | 0.717 (A) |
| 76 | 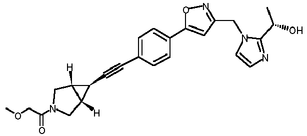 | ¹H NMR (400 MHz, METHANOL-d4) *δ* ppm 1.25 - 1.39 (m, 3 H), 1.59 (d, J=6.5 Hz, 3 H), 2.01 -2.12 (m, 2 H), 3.39 (s, 3 H), 3.43 - 3.67 (m, 2 H), 3.97 - 4.09 (m, 2 H), 5.04 (q, J=6.4 Hz, 1 H), 5.43 - 5.55 (m, 2 H), 6.71 (s, 1 H), 6.92 (s, 1 H), 7. 15 (s, 1 H), 7.45 (d, J=7.9 Hz, 2 H), 7.73 (d, J=7.9 Hz. 2 H) | ESI 447[M+1]⁺ | 0.865 (A) |
| 77 | 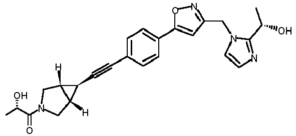 | ¹H NMR (400 MHz, METHANOL-d4) *δ* ppm 1.29 (s, 3 H), 1.59 (d, J=6.6 Hz, 3 H), 1.98 - 2.14 (m, 2 H), 3.37 - 4.02 (m, 5 H), 4.28 - 4.42 (m, 1 H), 5.04 (q, J=6.6 Hz, 1 H), 5.49 (dd, J=15.8 Hz, 2 H), 6.71 (s, 1 H), 6.92 (s, 1 H), 7.15 (s, 1 H), 7.45 (d, J=7.9 Hz, 2 H), 7.73 (d, J=7.9 Hz, 2 H) | ESI 447[M+1]⁺ | 0.831 (A) |

**[Table 2-12]**

| Compound | Structural formula | ¹H-NMR | MS (m/z) | Retention time Method |
|---|---|---|---|---|
| 78 | 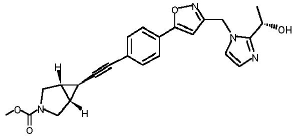 | ¹H NMR (400 MHz, METHANOL-d4) *δ* ppm 1.59 (d, J=6.6 Hz, 3 H), 2.00 (br s, 2 H), 3.37 - 3.51 (m, 3 H), 3.63 - 3. 71 (m, 5 H), 5.03 (q, J=6.6 Hz, 1 H), 5.49 (dd, J=16.4, 16.4 Hz, 2 H), 6.71 (s, 1 H), 6.93 (s, 1 H), 7.15 (s, 1 H), 7.44 (d, J=8.1 Hz, 2 H), 7.73 (d, J=8.1 Hz, 2 H) | ESI 433[M+1]⁺ | 1.024 (A) |
| 79 | 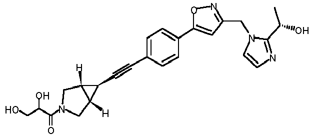 | ¹H NMR (400 MHz, METHANOL-d4) *δ* ppm 1.59 (d, J=6.7 Hz, 3 H), 1.99 -2.13 (m, 2 H), 3.41 - 3.53 (m, 1 H), 3.58 - 3.77 (m, 3 H), 3.83 - 4.08 (m, 3 H), 4.24 - 4.34 (m, 1 H), 5.03 (q, J=6.7 Hz, 1 H), 5.49 (dd, J=15.7, 15.7 Hz, 2 H), 6.71 (s, 1 H), 6.92 (s, 1 H), 7.15 (s, 1 H), 7.45 (d, J=8.1 Hz, 2 H). 7.73 (d. J=8.1 Hz. 2 H) | ESI 463[M+1]⁺ | 0.739 (A) |
| 80 | 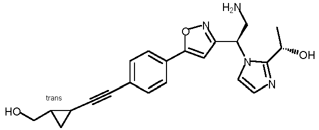 | ¹H NMR (400 MHz, METHANOL-d4) *δ* ppm 0.84 - 0.92 (m, 1 H), 0.92 - 1.00 (m, 1 H), 1.37 - 1.49 (m, 2 H), 1.66 (d, J=6.5 Hz, 3 H), 3.15 - 3.66 (m, 4 H), 5.09 (q, J=6.5 Hz, 1 H), 5.90 (t, J=6.9 Hz, 1 H), 6.76 (s, 1 H), 6.97 (s, 1 H), 7.24 (s, 1 H), 7.43 (d, J=8.1 Hz, 2 H), 7.73 (d, J=8.1 Hz, 2 H) | ESI 393[M+1]⁺ | 0.647 (A) |
| 81 | 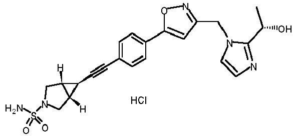 | ¹H NMR (400 MHz, METHANOL-d4) *δ* ppm 1.60 (d, J=6.6 Hz, 3 H), 1.95 - 2.07 (m, 3 H), 3.38 - 3.54 (m, 4 H), 5.14-5.26 (m, 1 H), 5.55 - 5.67 (m, 2 H), 6.82 (s, 1 H), 7.31 (br s, 1 H), 7.41 - 7.52 (m, 3 H), 7.75 (d, J=8.1 Hz, 2 H) | ESI 454[M+1]⁺ | 0.482 (A) |
| 82 | 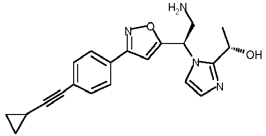 | ¹H NMR (400 MHz, CHLOROFORM-d) *δ* ppm 0.76 - 0.94 (m, 4 H), 1.42 - 1.51 (m, 1 H), 1. 76 (d, J=5.9 Hz, 3 H), 3.34 - 3.49 (m, 1 H), 3.49 - 3.61 (m, 1 H), 4.95 - 5. 10 (m, 1 H), 5.69 - 5.80 (m, 1 H), 6.46 (s, 1 H), 7.07 (br s, 1 H), 7.11 (br s, 1 H), 7.44 (d, J=7.9 Hz, 2 H), 7.67 (d, J=7.9 Hz, 2 H) | ESI 363[M+1]⁺ | 0.860 (A) |
| 83 | 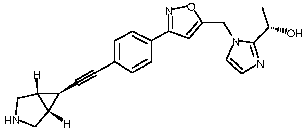 | ¹H NMR (400 MHz, METHANOL-d4) *δ* ppm 1.40 (br s, 1 H), 1.59 (d, J=6.5 Hz, 3 H), 1.86 (br s, 2 H), 2.84 (d, J=11.5 Hz, 2 H), 3.06 (d, J=11. 7Hz, 2 H), 4.98 - 5.09 (m, 1 H), 5.52 - 5.73 (m, 2 H), 6.72 (s, 1 H), 6.94 (s, 1 H), 7.20 (s, 1 H), 7.42 (d, J=8.0 Hz, 2 H), 7.74 (d, J=8.0 Hz, 2 H) | ESI 188 [M+2]²⁺ | 0.488 (A) |
| 84 | 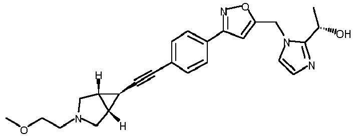 | ¹H NMR (400 MHz, METHANOL-d4) *δ* ppm 1.59 (d, J=6.7 Hz, 3 H), 1.78 - 1.84 (m, 2 H), 1.85 - 1.89 (m, 1 H), 2.45 (br d, J=9.5 Hz, 2 H), 2.63 (t, J=5.7 Hz, 2 H), 3.14 (d, J=9.5 Hz, 2 H), 3.33 (br s, 3 H), 3.46 (br t, J=5.7 Hz, 2 H), 4.98 - 5.07 (m, 1 H), 5.53 - 5.67 (m, 2 H), 6.72 (s, 1 H), 6.94 (s, 1 H), 7.19 (s, 1 H), 7.41 (d, J=8.0 Hz. 2 H). 7.73 (d. J=8.0 Hz. 2 H) | ESI 433[M+1]⁺ | 0.570 (A) |

**[Table 2-13]**

| Compound | Structural formula | ¹H-NMR | MS (m/z) | Retention time Method |
|---|---|---|---|---|
| 85 | 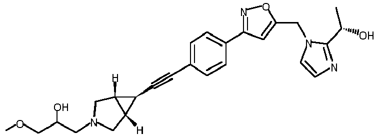 | ¹H NMR (400 MHz, METHANOL-d4) *δ* ppm 1.59 (d, J=6.6 Hz, 3 H), 1.78 - 1.83 (m, 2 H), 1.87 - 1.92 (m, 1 H), 2.38 - 2.54 (m, 4 H), 3.15 (br dd, J=9.0, 5.2 Hz, 2 H), 3.35 (s, 3 H), 3.36 - 3.42 (m, 2 H), 3.74 - 3.83 (m, 1 H), 5.03 (q, J=6.6 Hz, 1 H), 5.53 - 5.68 (m, 2 H), 6.72 (s, 1 H), 6.94 (s, 1 H), 7.19 (s, 1 H), 7.41 (d, J=8.1 Hz, 2 H). 7.73 (d. J=8.1 Hz. 2 H) | ESI 463[M+1]⁺ | 0.560 (A) |
| 86 | 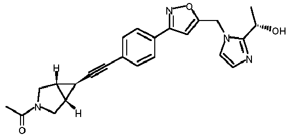 | ¹H NMR (400 MHz, METHANOL-d4) *δ* ppm 1.35 - 1.39 (m, 1 H), 1.59 (d, J=6.6 Hz, 3 H), 1.99 - 2.11 (m, 5 H), 3.38 - 3.50 (m, 1 H), 3.65 - 3.72 (m, 1 H), 3.74 - 3.85 (m, 2 H), 5.03 (q, J=6.6 Hz, 1 H), 5.53 - 5.67 (m, 2 H), 6.73 (s, 1 H), 6.94 (s, 1 H), 7.19 (s, 1 H), 7.43 (d, J=7.8 Hz, 2 H), 7.75 (d. J=7.8 Hz. 2 H) | ESI 417[M+1]⁺ | 0.474 (B) |
| 87 | 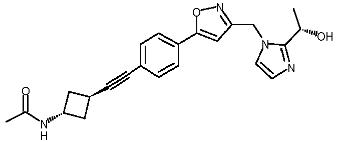 | ¹H NMR (400 MHz, METHANOL-d4) *δ* ppm 1.60 (d, J=6.6 Hz, 3 H), 1.92 (s, 3 H), 2.30 - 2.42 (m, 2 H), 2.42 - 2.55 (m, 2 H), 3.00 - 3.18 (m, 1 H), 4.54 - 4.61 (m, 1 H), 4.98 - 5. 11 (m, 1 H), 5.42 - 5.56 (m, 2 H), 6.72 (s, 1 H), 6.93 (s, 1 H), 7.16 (s, 1 H), 7.49 (d, J=7.6 Hz, 2 H), 7.76 (d, J=7.6 Hz. 2 H) | ESI 405[M+1]⁺ | 0.860 (A) |
| 88 | 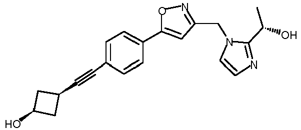 | ¹H NMR (400 MHz, METHANOL-d4) *δ* ppm 1.59 (d, J=6.6 Hz, 3 H), 1.98 - 2.12 (m, 2 H), 2.63 - 2. 78 (m, 2 H), 3.39 - 3.47 (m, 1 H), 4.06 - 4.14 (m, 1 H), 4.98 - 5.21 (m, 1 H), 5.43 - 5.56 (m, 2 H), 6. 71 (s, 1 H), 6.93 (s, 1 H), 7.15 (s, 1 H), 7.46 (d, J=7.7 Hz, 2 H), 7.74 (d, J=7.7 Hz, 2 H) | ESI 364[M+1]⁺ | 0.477 (B) |
| 89 | 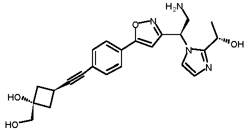 | ¹H NMR (400 MHz, CHLOROFORM-d) *δ* ppm 1.77 (d, J=6.1 Hz, 3 H), 2.30 (br dd, J=12.2, 4.8 Hz, 2 H), 2.44 - 2.55 (m, 2 H), 3.26 - 3.38 (m, 1 H), 3.41 - 3.52 (m, 1 H), 3.59 - 3.67 (m, 1 H), 3.82 (s, 2 H), 5.01 - 5.09 (m, 1 H), 5.60 - 5.68 (m, 1 H), 6.31 (s, 1 H), 6.96 (s, 1 H), 7.09 (s, 1 H), 7.46 (d, J=8.1 Hz, 2 H), 7.66 (d, J=8.1 Hz. 2 H) | ESI 423[M+1]⁺ | 0.599 (A) |
| 90 | 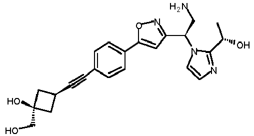 | ¹H NMR (400 MHz, CHLOROFORM-d) *δ* ppm 1.77 (d, J=6.1 Hz, 3 H), 2.30 (br dd, J=12.2, 4.8 Hz, 2 H), 2.44 - 2.55 (m, 2 H), 3.26 - 3.38 (m, 1H), 3.41 - 3.52 (m, 1 H), 3.59 - 3.67 (m, 1 H), 3.82 (s, 2 H), 5.01 - 5.09 (m, 1 H), 5.60 - 5.68 (m, 1 H), 6.31 (s, 1 H), 6.96 (s, 1 H), 7.09 (s, 1 H), 7.46 (d, J=8.1 Hz, 2 H), 7.66 (d, J=8.1 Hz. 2 H) | ESI 423[M+1]⁺ | 0.595 (A) |
| 91 | 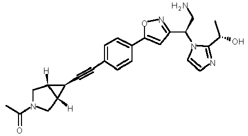 | ¹H NMR (400 MHz, METHANOL-d4) *δ* ppm 1.35 - 1.41 (m, 1 H), 1.66 (d, J=6.5 Hz, 3 H), 1.98-2.12 (m, 5 H), 3.08 - 3.89 (m, 6 H), 5.09 (q, J=6.5 Hz, 1 H), 5.90 (t, J=7.1 Hz, 1 H), 6.77 (s, 1 H), 6.97 (s, 1 H), 7.24 (s, 1 H), 7.45 (d, J=7.9 Hz, 2 H), 7.74 (d, J=7.9 Hz, 2 H) | ESI 446[M+1]⁺ | 0.696 (A) |

**[Table 2-14]**

| Compound | Structural formula | ¹H-NMR | MS (m/z) | Retention time Method |
|---|---|---|---|---|
| 92 | 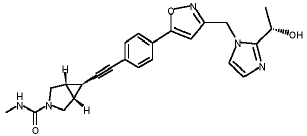 | ¹H NMR (400 MHz, METHANOL-d4) *δ* ppm 1.30 - 1.37 (m, 1 H), 1.59 (d, J=6.4 Hz, 3 H), 1.56 - 1.64 (m, 1 H), 1.98 - 2.06 (m, 2 H), 2.70 (s, 3 H), 3.35 - 3.43 (m, 2 H), 3.59 - 3.67 (m, 2 H), 5.04 (q, J=6.4 Hz, 1 H), 5.40 - 5.60 (m, 2 H), 6.71 (s, 1 H), 6.92 (s, 1 H), 7.15 (s, 1 H), 7.44 (d, J=7.7 Hz, 2 H), 7.73 (d, J=7.7 Hz. 2 H) | ESI 432[M+1]⁺ | 0.867 (A) |
| 93 | 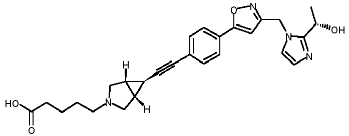 | ¹H NMR (400 MHz, METHANOL-d4) *δ* ppm 1.55 - 1.70 (m, 7 H), 1.87 - 1.92 (m, 1 H), 2.02 - 2.09 (m, 2 H), 2.23 - 2.30 (m, 2 H), 2.75 - 2.85 (m, 2 H), 2.93 (br d, J=10.6 Hz, 2 H), 3.43 (br d, J=10.6 Hz, 2 H), 5.04 (q, J=6.6 Hz, 1 H), 5.40 - 5.57 (m, 2 H), 6.71 (s, 1 H), 6.93 (s, 1 H), 7.16 (s, 1 H), 7.44 (d, J=8.1 Hz, 2 H), 7.73 (d, J=8.1 Hz. 2 H) | ESI 475[M+1]⁺ | 0.595 (A) |
| 94 | 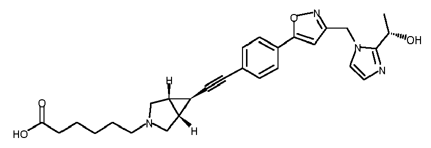 | ¹H NMR (400 MHz, METHANOL-d4) *δ* ppm 1.34 - 1.47 (m, 2 H), 1.54 - 1.72 (m, 7 H), 1.85 - 1.92 (m, 1 H), 2.10 - 2.18 (m, 2 H), 2.23 - 2.33 (m, 2 H), 2.87 - 2.99 (m, 2 H), 3.09 - 3.22 (m, 2 H), 3.44 - 3.60 (m, 2 H), 5.05 (q, J=6.5 Hz, 1 H), 5.43 - 5.57 (m, 2 H), 6. 72 (s, 1 H), 6.95 (s, 1 H), 7.17 (s, 1 H), 7.45 (d, J=8.2 Hz, 2 H), 7.74 (d. J=8.2 Hz. 2 H) | ESI 489[M+1]⁺ | 0.628 (A) |
| 95 | 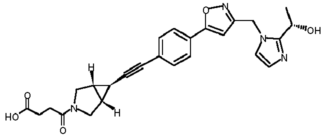 | ¹H NMR (400 MHz, METHANOL-d4) *δ* ppm 1.35 - 1.43 (m, 1 H), 1.53 - 1.66 (m, 3 H), 1.98 - 2.16 (m, 2 H), 2.44 - 2.64 (m, 4 H), 3.39 - 3.54 (m, 1 H), 3.64 - 3.73 (m, 1 H), 3.79 - 3.89 (m, 2 H), 5.01 - 5.10 (m, 1 H), 5.43 - 5.59 (m, 2 H), 6.72 (s, 1 H), 6.97 (s, 1 H), 7.19 (s, 1 H), 7.45 (d, J=8.0 Hz, 2 H). 7.74 (d. J=8.0 Hz. 2 H) | ESI 475[M+1]⁺ | 0.859 (A) |
| 96 | 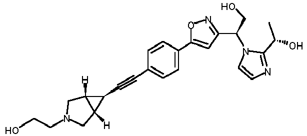 | ¹H NMR (400 MHz, METHANOL-d4) *δ* ppm 1.63 (d, J=6.5 Hz, 3 H), 1.82 - 1.97 (m, 3 H), 2.44 - 2.57 (m, 2 H), 2.57 - 2.67 (m, 2 H), 3.57 - 3.63 (m, 2 H), 4. 19 - 4.30 (m, 2 H), 4.96 - 5.15 (m, 3 H), 6.02 - 6.12 (m, 1 H), 6.78 (s, 1 H), 6.94 (s, 1 H), 7.33 (s, 1 H), 7.43 (d, J=8.6 Hz, 2 H), 7.73 (d, J=8.6 Hz. 2 H) | ESI 449[M+1]⁺ | 0.522 (A) |
| 97 | 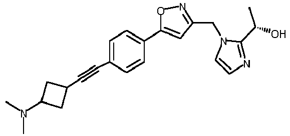 | ¹H NMR (400 MHz, METHANOL-d4) *δ* ppm 1.60 (d, J=6.6 Hz, 3 H), 1.95 - 2.22 (m, 8 H), 2.22 - 2.36 (m, 2 H), 2.48 - 2.72 (m, 1 H), 3.36 - 3.44 (m, 1 H), 4.99 - 5.09 (m, 1 H), 5.42 - 5.55 (m, 2 H), 6.72 (s, 1 H), 6.93 (s, 1 H), 7.15 (s, 1 H), 7.44 - 7.52 (m, 2 H), 7.71 - 7.80 (m, 2 H) | ESI 196[M+2]²⁺ | 0.561 (A) |
| 98 | 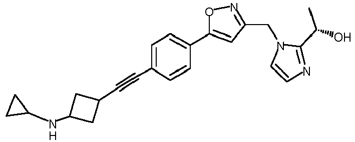 | ¹H NMR (400 MHz, METHANOL-d4) *δ* ppm 0.28 - 0.80 (m, 4 H), 1.60 (d, J=6.6 Hz, 3 H), 1.93 - 2.44 (m, 4 H), 2.58 - 2.98 (m, 2 H), 3.51 - 3.74 (m, 1 H), 5.00 - 5.09 (m, 1 H), 5.42 - 5.57 (m, 2 H), 6.71 (s, 1 H), 6.93 (s, 1 H), 7.15 (s, 1 H), 7.43 - 7.52 (m, 2 H), 7.69 - 7.80 (m, 2 H) | ESI 202 [M+2]²⁺ | 0.284 (A) |

**[Table 2-15]**

| Compound | Structural formula | ¹H-NMR | MS (m/z) | Retention time Method |
|---|---|---|---|---|
| 99 | 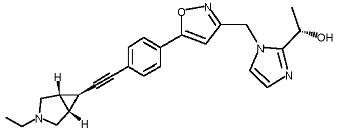 | ¹H NMR (400 MHz, METHANOL-d4) *δ* ppm 1.07 (t, J=7.2 Hz, 3 H), 1.59 (d, J=6.6 Hz, 3 H), 1.80 - 1.88 (m, 3 H), 2.41 (br d, J=9.8 Hz, 2 H), 2.45 - 2.53 (m, 2 H), 3.14 (br d, J=9.8 Hz, 2 H), 4.99 - 5. 08 (m, 1 H), 5. 41 - 5. 55 (m, 2 H), 6.70 (s, 1 H), 6.93 (s, 1 H), 7.15 (s, 1 H), 7.43 (d, J=7.5 Hz, 2 H), 7.72 (d. J=7.5 Hz. 2 H) | ESI 202[M+2]²⁺ | 0.569 (A) |
| 100 | 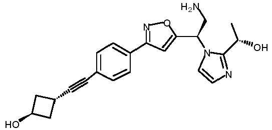 | ¹H NMR (400 MHz, CHLOROFORM-d) *δ* ppm 1.76 (d, J=6.4 Hz, 3 H), 2.29 - 2.37 (m, 2 H), 2.49 - 2.58 (m, 2 H), 3.19 - 3.27 (m, 1 H), 3.38 - 3.47 (m, 1 H), 3.52 - 3.60 (m, 1 H), 4.63 - 4.72 (m, 1 H), 5.03 (q, J=6.4 Hz, 1 H), 5. 70 - 5.80 (m, 1 H), 6.47 (s, 1 H), 7.07 (s, 1 H), 7.11 (s, 1 H), 7.47 (d, J=8.1 Hz, 2 H), 7.70 (d, J=8.1 Hz. 2 H) | ESI 393 [M+1]⁺ | 0.669 (A) |
| 101 | 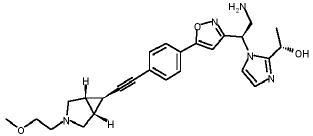 | ¹H NMR (400 MHz, METHANOL-d4) *δ* ppm 1.66 (d, J=6.5 Hz, 3 H), 1.78 - 1.85 (m, 2 H), 1.85 - 1.92 (m, 1 H), 2.40 - 2.51 (m, 2 H), 2.59 - 2.68 (m, 2 H), 3.09 - 3. 18 (m, 2 H), 3.24 - 3.57 (m, 7 H), 5.09 (q, J=6.5 Hz, 1 H), 5.90 (t, J=7.1 Hz, 1 H), 6.76 (s, 1 H), 6.97 (s, 1 H), 7.24 (s, 1 H), 7.43 (d, J=8.0 Hz, 2 H), 7.73 (d, J=8.0 Hz. 2 H) | ESI 232 [M+2]²⁺ | 0.339 (A) |
| 102 | 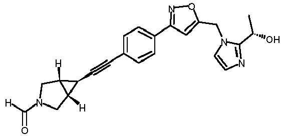 | ¹H NMR (400 MHz, METHANOL-d4) *δ* ppm 1.33 - 1.40 (m, 1 H), 1. 59 (d, J=6.6 Hz, 3 H), 1.97 - 2.08 (m, 2 H), 3.16 - 3.51 (m, 1 H), 3.62 - 3.74 (m, 1 H), 3.79 - 3.95 (m, 2 H), 5.03 (q, J=6.6 Hz, 1 H), 5.54 - 5.68 (m, 2 H), 6.73 (s, 1 H), 6.94 (s, 1 H), 7.19 (s, 1 H), 7.43 (d, J=7.9 Hz, 2 H), 7.75 (d, J=7.9 Hz, 2 H), 8.10 (s, 1 H) | ESI 403[M+1]⁺ | 0.849 (A) |
| 103 | 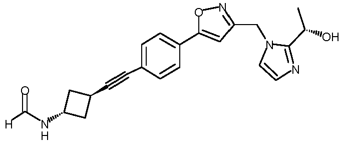 | ¹H NMR (400 MHz, METHANOL-d4) *δ* ppm 1.60 (d, J=6.5 Hz, 3 H), 2.26 - 2.57 (m, 5 H), 4.60 - 4. 71 (m, 1 H), 4.98 - 5.10 (m, 1 H), 5.41 - 5.58 (m, 2 H), 6.72 (s, 1 H), 6.93 (br s, 1 H), 7.16 (br s, 1 H), 7.50 (d, J=8.0 Hz, 2 H), 7.76 (d, J=8.0 Hz, 2 H), 7.93 - 7.98 (m, 1 H) | ESI 391 [M+1]⁺ | 0.478 (B) |
| 104 | 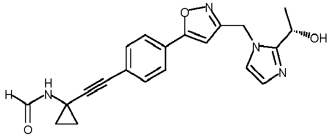 | ¹H NMR (400 MHz, METHANOL-d4) *δ* ppm 1.15 - 1. 41 (m, 4 H), 1.59 (d, J=6.6 Hz, 3 H), 5.04 (q, J=6.6 Hz, 1 H), 5.39 - 5.57 (m, 2 H), 6.72 (s, 1 H), 6.92 (s, 1 H), 7.15 (s, 1 H), 7.41 - 7.56 (m, 2 H), 7.69 - 7.81 (m, 2 H), 7.86 - 8.05 (m, 1 H) | ESI 377[M+1]⁺ | 0.815 (A) |
| 105 | 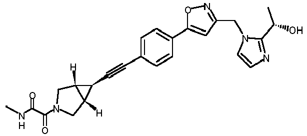 | ¹H NMR (400 MHz, CHLOROFORM-d) *δ* ppm 1.20 - 1.32 (m, 1 H), 1. 69 (d, J=6.5 Hz, 3 H), 2.01 (dt, J=7.2, 3.9 Hz, 1 H), 2.12 (br dd, J=7.3, 3.6 Hz, 1 H), 2.86 (d, J=5. 1 Hz, 3 H), 3.52 - 3.68 (m, 1 H), 3.87 - 4.07 (m, 2 H), 4.58 (d, J=13.0 Hz, 1 H), 5.04 (q, J=6.5 Hz, 1 H), 5.23 - 5.34 (m, 1 H), 5.35 - 5.46 (m, 1 H), 6.36 (s, 1 H), 6.96 (s, 1 H), 7.02 (s, 1 H), 7.43 (d, J=8.2 Hz, 2 H), 7.64 (d. J=8.2 Hz. 2 H) | ESI 460[M+1]⁺ | 0.882 (A) |

**[Table 2-16]**

| Compound | Structural formula | ¹H-NMR | MS (m/z) | Retention time Method |
|---|---|---|---|---|
| 106 | 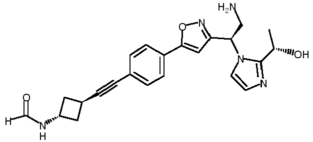 | ¹H NMR (400 MHz, METHANOL-d4) *δ* ppm 1.66 (d, J=6.3 Hz, 3 H), 2.32 - 2.58 (m, 4 H), 3.35 - 3.45 (m, 1 H), 3.49 - 3.56 (m, 1 H), 4.04 - 4. 13 (m, 1 H), 4.62 - 4. 70 (m, 1 H), 5.09 (q, J=6.3 Hz, 1 H), 5.91 (t, J=7.0 Hz, 1 H), 6.78 (s, 1 H), 6.98 (s, 1 H), 7.25 (s, 1 H), 7.50 (d, J=8.4 Hz, 2 H), 7.77 (d. J=8.4 Hz. 2 H). 7.96 (s. 1 H) | ESI 420[M+1]⁺ | 0.679 (A) |
| 107 | 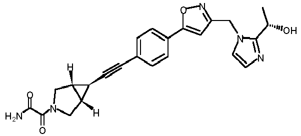 | ¹H NMR (400 MHz, METHANOL-d4) *δ* ppm 1.37 (br s, 1 H), 1.59 (d, J=6.6 Hz, 3 H), 2.01 - 2.08 (m, 1 H), 2.08 - 2. 17 (m, 1 H), 3.48 - 3.61 (m, 1 H), 3.85 (br d, J=8.3 Hz, 1 H), 3.93 (br d, J=12.6 Hz, 1 H), 4.15 (br d, J=12.0 Hz, 1 H), 5.04 (q, J=6.6 Hz, 1 H), 5.34 - 5.62 (m, 2 H), 6.71 (s, 1 H), 6.93 (s, 1 H), 7. 15 (s, 1 H), 7.45 (d, J=8. 1 Hz, 2 H), 7.73 (d, J=8. 1 Hz. 2 H) | ESI 446[M+1]⁺ | 0.818 (A) |
| 108 | 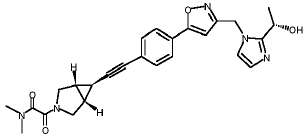 | ¹H NMR (400 MHz, METHANOL-d4) *δ* ppm 1.42 (br s, 1 H), 1.59 (d, J=6.5 Hz, 3 H), 2.10 (br s, 2 H), 2.98 (s, 3 H), 2.99 (s, 3 H), 3.44 - 3.57 (m, 1 H), 3.59 - 3.74 (m, 2 H), 3.95 (br d, J=12.2 Hz, 1 H), 5.04 (q, J=6.5 Hz, 1 H), 5.39 - 5.59 (m, 2 H), 6.71 (s, 1 H), 6.93 (s, 1 H), 7.15 (s, 1 H), 7.45 (d, J=8. 1 Hz, 2 H), 7.74 (d, J=8. 1 Hz. 2 H) | ESI 474[M+1]⁺ | 0.858 (A) |
| 109 | 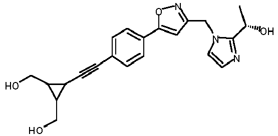 | ¹H NMR (400 MHz, METHANOL-d4) *δ* ppm 1.23 - 1.43 (m, 2 H), 1.59 (d, J=6.6 Hz, 3 H), 1.65 - 1.76 (m, 1 H), 3.42 - 3.51 (m, 1 H), 3.54 - 3.65 (m, 1 H), 3.74 (m, J=7.0 Hz, 2 H), 5.04 (q, J=6.6 Hz, 1 H), 5.38 - 5.58 (m, 2 H), 6.71 (s, 1 H), 6.93 (s, 1 H), 7.15 (s, 1 H), 7.46 (d, J=8.1 Hz, 2 H), 7.73 (d. J=8.1 Hz. 2 H) | ESI 394[M+1]⁺ | 0. 721 (A) |
| 110 | 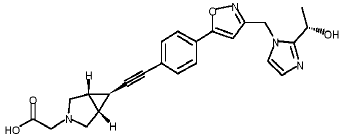 | ¹H NMR (400 MHz, METHANOL-d4) *δ* ppm 1.59 (d, J=6.6 Hz, 3 H), 1.90 - 2.02 (m, 1 H), 2.08 - 2.24 (m, 2 H), 3.19-3.42 (m, 2 H), 3.53 - 3.72 (m, 4 H), 5.04 (q, J=6.6 Hz, 1 H), 5.40 - 5.58 (m, 2 H), 6.72 (s, 1 H), 6.93 (s, 1 H), 7.15 (s, 1 H), 7.46 (d, J=8.2 Hz, 2 H), 7.74 (d, J=8.2 Hz, 2 H) | ESI 433[M+1]⁺ | 0.604 (A) |
| 111 | 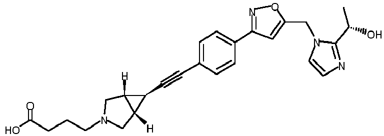 | ¹H NMR (400 MHz, METHANOL-d4) *δ* ppm 1.60 (d, J=6.6 Hz, 3 H), 1.80 - 1.96 (m, 3 H), 2. 18 - 2.28 (m, 2 H), 2.45 (t, J=6.5 Hz, 2 H), 3. 10 - 3.20 (m, 2 H), 3.22 - 3.44 (m, 2 H), 3.70 (br d, J=11.2 Hz, 2 H), 5.03 - 5.13 (m, 1 H), 5.56 - 5.71 (m, 2 H), 6.78 (s, 1 H), 7.04 (s, 1 H), 7.28 (s, 1 H), 7.45 (d, J=8. 1 Hz, 2 H), 7.77 (d, J=8. 1 Hz. 2 H) | ESI 461 [M+1]⁺ | 0.654 (A) |
| 112 | 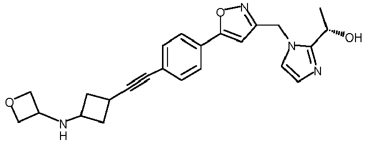 | ¹H NMR (400 MHz, METHANOL-d4) *δ* ppm 1.59 (d, J=6.6 Hz, 3 H), 1.86 - 2.68 (m, 4 H), 2.81 - 3.22 (m, 1 H), 3.52 - 3.62 (m, 1 H), 3.91 - 4.04 (m, 1 H), 4.43 - 4.54 (m, 2 H), 4.71 - 4.83 (m, 2 H), 5.01 - 5.09 (m, 1 H), 5.41 - 5.57 (m, 2 H), 6.71 (s, 1 H), 6.93 (s, 1 H), 7.15 (s, 1 H), 7.42 - 7.53 (m. 2 H), 7.74 (d. J=8.3 Hz. 2 H) | ESI 419[M+1]⁺ | 0.611 (A) |

**[Table 2-17]**

| Compound | Structural formula | ¹H-NMR | MS (m/z) | Retention time Method |
|---|---|---|---|---|
| 113 | 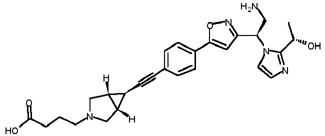 | ¹H NMR (400 MHz, METHANOL-d4) *δ* ppm 1.66 (d, J=6.5 Hz, 3 H), 1.75 - 1.88 (m, 3 H), 2.00 - 2.06 (m, 2 H), 2.34 (br t, J=6.5 Hz, 2 H), 2.80-2.91 (m, 4 H), 3.34 - 3.58 (m, 4 H), 5.09 (q, J=6.5 Hz, 1 H), 5.92 (br t, J=7.2 Hz, 1 H), 6.77 (s, 1 H), 6.98 (s, 1 H), 7.24 (s, 1 H), 7.44 (d, J=8.2 Hz, 2 H). 7.74 (d. J=8.2 Hz. 2 H) | ESI 490[M+1]⁺ | 0.514 (A) |
| 114 | 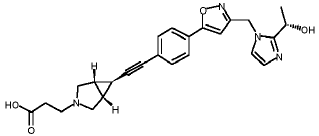 | ¹H NMR (400 MHz, METHANOL-d4) *δ* ppm 1.25 - 1.35 (m, 1 H), 1.59 (d, J=6.5 Hz, 3 H), 2. 10 (br s, 2 H), 2.45 (t, J=6.8 Hz, 2 H), 2.98 - 3.16 (m, 4 H), 3.49 (br d, J=10.8 Hz, 2 H), 5.04 (q, J=6.5 Hz, 1 H), 5.39 - 5.59 (m, 2 H), 6.71 (s, 1 H), 6.93 (s, 1 H), 7.15 (s, 1 H), 7.45 (d, J=8.1 Hz, 2 H). 7.74 (d. J=8.1 Hz. 2 H) | ESI 447[M+1]⁺ | 0.574 (A) |
| 115 | 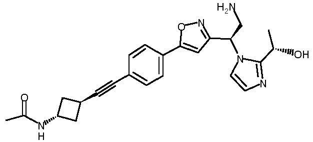 | ¹H NMR (400 MHz, METHANOL-d4) *δ* ppm 1.66 (d, J=6.6 Hz, 3 H), 1.92 (s, 3 H), 2.30 - 2.42 (m, 2 H), 2.42 - 2.52 (m, 2 H), 3.20 - 3.24 (m, 1 H), 3.35 - 3.43 (m, 1 H), 3.49 - 3.57 (m, 1 H), 4.53 - 4.64 (m, 1 H), 5.09 (q, J=6.6 Hz, 1 H), 5.86 - 5.94 (m, 1 H), 6.78 (s, 1 H), 6.98 (s, 1 H), 7.25 (s, 1 H), 7.50 (d, J=8.3 Hz, 2 H), 7.77 (d. J=8.3 Hz. 2 H) | ESI 434[M+1]⁺ | 0. 769 (A) |
| 116 | 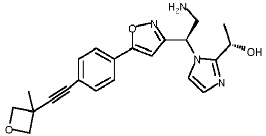 | ¹H NMR (400 MHz, METHANOL-d4) *δ* ppm 1.66 (d, J=6.6 Hz, 3 H), 1.70 (s, 3 H), 3.35 - 3.44 (m, 1 H), 3.49 - 3.58 (m, 1 H), 4.47 - 4.53 (m, 2 H), 4.85 - 4.91 (m, 2 H), 5.09 (q, J=6.6 Hz, 1 H), 5.87 - 5.94 (m, 1 H), 6.80 (s, 1 H), 6.98 (s, 1 H), 7.25 (s, 1 H), 7.52 (d, J=7.9 Hz, 2 H), 7.79 (d, J=7.9 Hz. 2 H) | ESI 393[M+1]⁺ | 0.258 (B) |
| 117 | 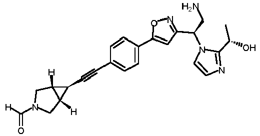 | ¹H NMR (400 MHz, METHANOL-d4) *δ* ppm 1.33 - 1.39 (m, 1 H), 1.66 (d, J=6.4 Hz, 3 H), 1.96 - 2.09 (m, 2 H), 3.16 - 3.45 (m, 2 H), 3.46 - 3.59 (m, 1 H), 3.61 - 3.75 (m, 1 H), 3.78 - 3.96 (m, 2 H), 5.03 - 5. 14 (m, 1 H), 5.85 - 5.94 (m, 1 H), 6.77 (s, 1 H), 6.97 (s, 1 H), 7.24 (s, 1 H), 7.45 (d, J=7.8 Hz, 2 H), 7.74 (d, J=7.8 Hz. 2 H), 8.10 (s. 1 H) | ESI 432[M+1]⁺ | 0.652 (A) |
| 118 | 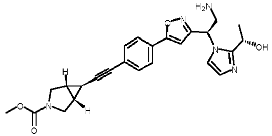 | ¹H NMR (400 MHz, METHANOL-d4) *δ* ppm 1.32 - 1. 37 (m, 1 H), 1. 66 (d, J=6.4 Hz, 3 H), 1.97 - 2.04 (m, 2 H), 3.18 - 3.73 (m, 9 H), 5.09 (q, J=6.4 Hz, 1 H), 5.90 (t, J=6.6 Hz, 1 H), 6.77 (s, 1 H), 6.97 (s, 1 H), 7.24 (s, 1 H), 7.45 (d, J=8.1 Hz, 2 H), 7.74 (d, J=8. 1 Hz, 2 H) | ESI 462[M+1]⁺ | 0.845 (A) |
| 119 | 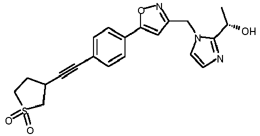 | ¹H NMR (400 MHz, METHANOL-d4) *δ* ppm 1.59 (d, J=6.5 Hz, 3 H), 2.25 - 2.44 (m, 1 H), 2.54 - 2.67 (m, 1 H), 3.06 - 3.53 (m, 4 H), 3.58 - 3.74 (m, 1 H), 5.04 (q, J=6.5 Hz, 1 H), 5.41 - 5.57 (m, 2 H), 6.74 (s, 1 H), 6.93 (s, 1 H), 7.15 (s, 1 H), 7.53 (d, J=7.8 Hz, 2 H), 7.78 (d, J=7.8 Hz, 2 H) | ESI 412[M+1]⁺ | 0.951 (A) |

**[Table 2-18]**

| Compound | Structural formula | ¹H-NMR | MS (m/z) | Retention time Method |
|---|---|---|---|---|
| 120 | 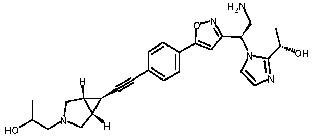 | ¹H NMR (400 MHz, METHANOL-d4) *δ* ppm 1.11 (d, J=6.2 Hz, 3 H), 1.66 (d, J=6.5 Hz, 3 H), 1. 77 - 1.86 (m, 2 H), 1.89 - 1.97 (m, 1 H), 2.28 - 2.52 (m, 4 H), 3.05 - 3.22 (m, 2 H), 3.23 - 3.44 (m, 1 H), 3.46 - 3.59 (m, 1 H), 3.71 - 3.87 (m, 1 H), 5.09 (q, J=6.5 Hz, 1 H), 5.90 (t, J=7.0 Hz, 1 H), 6.76 (s, 1 H), 6.97 (s, 1 H), 7.24 (s, 1 H), 7.42 (d, J=8.2 Hz, 2 H), 7.73 (d. J=8.2 Hz. 2 H) | ESI 462[M+1]⁺ | 0.242 (A) |
| 121 | 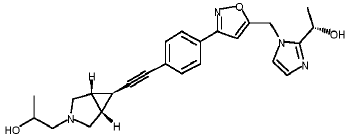 | ¹H NMR (400 MHz, METHANOL-d4) *δ* ppm 1.11 (d, J=6.2 Hz, 3 H), 1.59 (d, J=6.5 Hz, 3 H), 1. 76 - 1. 84 (m, 2 H), 1.89 - 1.97 (m, 1 H), 2.27 - 2.54 (m, 4 H), 3.08 - 3.21 (m, 2 H), 3.71 - 3.87 (m, 1 H), 5.03 (q, J=6.5 Hz, 1 H), 5.52 - 5.70 (m, 2 H), 6.72 (s, 1 H), 6.94 (s, 1 H), 7.19 (s, 1 H), 7.41 (d, J=8.2 Hz, 2 H), 7.73 (d, J=8.2 Hz. 2 H) | ESI 433[M+1]⁺ | 0.557 (A) |
| 122 | 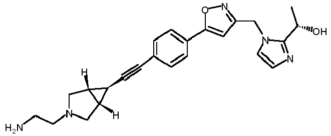 | ¹H NMR (400 MHz, CHLOROFORM-d) *δ* ppm 1.69 (d, J=6.4 Hz, 3 H), 1.79 - 1.85 (m, 2 H), 1.88 - 1.94 (m, 1 H), 2.37 (br d, J=8.9 Hz, 2 H), 2.50 (br t, J=5.8 Hz, 2 H), 2.68 - 2.76 (m, 2 H), 3. 11 (d, J=8.9 Hz, 2 H), 5.03 (q, J=6.4 Hz, 1 H), 5.23 - 5.45 (m, 2 H), 6.34 (s, 1 H), 6.96 (s, 1 H), 7.01 (s, 1 H), 7.42 (d, J=7.9 Hz, 2 H), 7.63 (d. J=7.9 Hz. 2 H) | ESI 418[M+1]⁺ | 0.364 (A) |
| 123 | 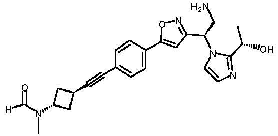 | ¹H NMR (400 MHz, METHANOL-d4) *δ* ppm 1.66 (d, J=6.5 Hz, 3 H), 2.33 - 2.48 (m, 2 H), 2.70 - 2.83 (m, 2 H), 2.88 (s, 3 H), 2.97 - 3.07 (m, 1 H), 3.35 - 3.44 (m, 1 H), 3.49 - 3.57 (m, 1 H), 4.52 - 4.73 (m, 1 H), 5.05 - 5.13 (m, 1 H), 5.91 (t, J=7.1 Hz, 1 H), 6.79 (s, 1 H), 6.98 (s, 1 H), 7.25 (s, 1 H), 7.51 (d, J=8.3 Hz, 2 H), 7.77 (d. J=8.3 Hz. 2 H), 8.13 (s. 1 H) | ESI 434[M+1]⁺ | 0.689 (A) |
| 124 | 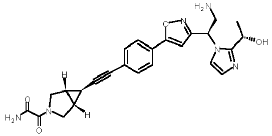 | ¹H NMR (400 MHz, CHLOROFORM-d) *δ* ppm 1.30 (br s, 1 H), 1. 77 (d, J=6.1 Hz, 3 H), 2.03 (br s, 1 H), 2. 12 (br s, 1 H), 3.38 - 3.52 (m, 1 H), 3.56 - 3.70 (m, 2 H), 3.91 - 4.08 (m, 2 H), 4.51 (br d, J=12.8 Hz, 1 H), 4.97 - 5. 12 (m, 1 H), 5.43 (br s, 1 H), 5.58 - δ.71 (m, 1 H), 6.30 (s, 1 H), 6.95 (s, 1 H), 7.09 (s, 1 H), 7.44 (d, J=8.3 Hz, 2 H), 7.65 (d, J=8.3 Hz. 2 H) | ESI 475[M+1]⁺ | 0.629 (A) |
| 125 | 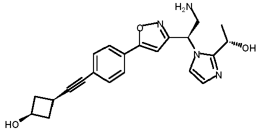 | ¹H NMR (400 MHz, METHANOL-d4) *δ* ppm 1.66 (d, J=6.5 Hz, 3 H), 1.98 - 2. 15 (m, 2 H), 2.60 - 2.80 (m, 3 H), 3.17-3.58 (m, 2 H), 4.03 - 4. 18 (m, 1 H), 5.09 (q, J=6.5 Hz, 1 H), 5.90 (t, J=6.9 Hz, 1 H), 6.77 (s, 1 H), 6.98 (s, 1 H), 7.25 (s, 1 H), 7.46 (d, J=7.3 Hz, 2 H), 7.75 (d, J=7.3 Hz, 2 H) | ESI 393 [M+1]⁺ | 0.630 (A) |
| 126 | 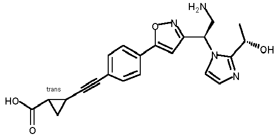 | ¹H NMR (400 MHz, METHANOL-d4) *δ* ppm 1.14 - 1. 23 (m, 1 H), 1. 33 - 1. 42 (m, 1 H), 1. 70 (d, J=6.2 Hz, 3 H), 1.86 - 1.99 (m, 2 H), 3.59 - 3.70 (m, 1 H), 3.75 - 3.85 (m, 1 H), 5.15 (q, J=6.2 Hz, 1 H), 6. 11 - 6.20 (m, 1 H), 6.78 (s, 1 H), 7.01 (s, 1 H), 7.24 (s, 1 H), 7.47 (d, J=8.2 Hz, 2 H), 7.75 (d, J=8.2 Hz, 2 H) | ESI 407[M+ 1]⁺ | 0.685 (A) |

**[Table 2-19]**

| Compound | Structural formula | ¹H-NMR | MS (m/z) | Retention time Method |
|---|---|---|---|---|
| 127 | 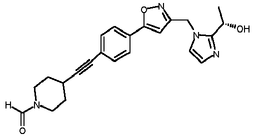 | ¹H NMR (400 MHz, METHANOL-d4) *δ* ppm 1.59 (d, J=6.6 Hz, 3 H), 1.63 - 1.78 (m, 2 H), 1.83 - 1.99 (m, 2 H), 3.00 - 3.06 (m, 1 H), 3.36 - 3.45 (m, 1 H), 3.62 - 3. 74 (m, 2 H), 3.80 - 3.92 (m, 1 H), 5.03 (q, J=6.6 Hz, 1 H), 5.49 (dd, J=16.3, 16.3 Hz, 2 H), 6.72 (s, 1 H), 6.93 (s, 1 H), 7.15 (s, 1 H), 7.50 (d, J=7.8 Hz, 2 H), 7.76 (d, J=7.8 Hz. 2 H). 8.01 (s. 1 H) | ESI 405[M+1]⁺ | 0.862 (A) |
| 128 | 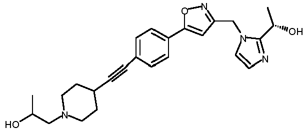 | ¹H NMR (400 MHz, METHANOL-d4) *δ* ppm 1.14 (d, J=6.1 Hz, 3 H), 1.60 (d, J=6.6 Hz, 3 H), 1. 66 - 1.84 (m, 2 H), 1.86 - 2.02 (m, 2 H), 2.22 - 2.48 (m, 4 H), 2.61 - 2.75 (m, 1 H), 2.75 - 2.98 (m, 2 H), 3.83 - 4.00 (m, 1 H), 5.04 (q, J=6.6 Hz, 1 H), 5.49 (dd, J=16.4, 16.4 Hz, 2 H), 6.71 (s, 1 H), 6.93 (s, 1 H), 7.15 (s, 1 H), 7.47 (d, J=7.7 Hz, 2 H), 7.74 (d, J=7.7 Hz. 2 H) | ESI 435[M+1]⁺ | 0.544 (A) |
| 129 | 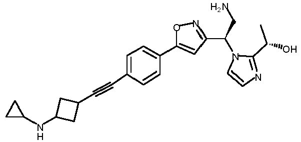 | ¹H NMR (400 MHz, METHANOL-d4) *δ* ppm 0.26 - 0.40 (m, 2 H), 0.40 - 0.54 (m, 2 H), 0.79 - 1. 19 (m, 2 H), 1.66 (d, J=6.0 Hz, 3 H), 1.87 - 2.48 (m, 4 H), 2.57 - 3.01 (m, 1 H), 3.09 - 3.88 (m, 2 H), 5.03 -5.17 (m, 1 H), 5.84 - 5.97 (m, 1 H), 6.78 (s, 1 H), 6.98 (s, 1 H), 7.25 (s, 1 H), 7.42 - 7.55 (m. 2 H). 7.70 - 7.83 (m, 2 H) | ESI 432[M+1]⁺ | 0.215 (A) |
| 130 | 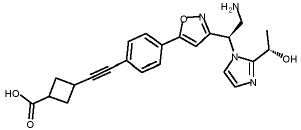 | ¹H NMR (400 MHz, DMSO-d6) *δ* ppm 1.51 (d, J=6.2 Hz, 3 H), 2.12 - 2.26 (m, 2 H), 2.29 - 2.40 (m, 2 H), 2.58 - 2. 72 (m, 1 H), 2.97 - 3.09 (m, 1 H), 3.13 - 3.48 (m, 2 H), 4.92 (br q, J=6.2 Hz, 1H), 5.75 (br t, J=6.5 Hz, 1 H), 6.84 (s, 1 H), 7.12 (s, 1H), 7.25 (s, 1 H), 7.51 (d, J=7.8 Hz, 2H), 7.78 (d. J=7.8 Hz. 2 H) | ESI 421 [M+1]⁺ | 0.710 (A) |
| 131 | 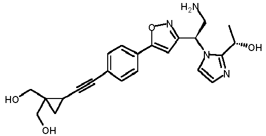 | ¹H NMR (400 MHz, METHANOL-d4) *δ* ppm 0.79 - 0.85 (m, 1 H), 1.06 - 1.15 (m, 1 H), 1.66 (d, J=6.4 Hz, 3 H), 1.68 - 1.74 (m, 1 H), 3.20 - 3.68 (m, 4 H), 3.73 - 3.92 (m, 2 H), 5.04 - 5.14 (m, 1 H), 5.90 (t, J=7.0 Hz, 1 H), 6.77 (s, 1 H), 6.97 (s, 1H), 7.24 (s, 1 H), 7.47 (d, J=7.9 Hz, 2 H), 7.74 (d, J=7.9 Hz. 2 H) | ESI 423[M+1]⁺ | 0.553 (A) |
| 132 | 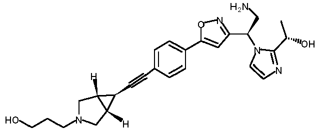 | ¹H NMR (400 MHz, CHLOROFORM-d) *δ* ppm 1.63 - 1.71 (m, 2 H), 1.73 (br s, 1H), 1.76 (d, J=6.2 Hz, 3 H), 1.84 (br s, 2H), 2.37 (br d, J=9.0 Hz, 2H), 2.70 (br t, J=5.2 Hz, 2H), 3.28 (br d, J=9.3 Hz, 2 H), 3.39 - 3.51 (m, 1 H), 3.63 (br dd, J=13.8, 3.2 Hz, 1 H), 3.77 (br t, J=5.1 Hz, 2 H), 4.98 - 5.10 (m, 1H), 5.64 (br dd, J=9.8, 3.8 Hz, 1H), 6.29 (s, 1 H), 6.95 (s, 1H), 7.08 (s, 1 H), 7.42 (d, J=7.9 Hz, 2 H), 7.63 (d, J=7.9 Hz. 2 H) | ESI 462[M+1]⁺ | 0.248 (A) |
| 133 | 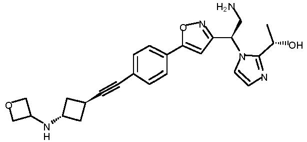 | ¹H NMR (400 MHz, METHANOL-d4) *δ* ppm 1.66 (d, J=6.6 Hz, 3 H), 2.13 - 2.44 (m, 4 H), 2.53 - 2.66 (m, 1 H), 3.41 - 3.42 (m, 1 H), 3.50 - 3.58 (m, 1H), 3.92 - 4.02 (m, 1H), 4.47 - 4.60 (m, 3 H), 4.76 (s, 2H), 5.09 (q, J=6.6 Hz, 1 H), 5.88 - 5.95 (m, 1 H), 6.77 (s, 1H), 6.98 (s, 1H), 7.24 (s, 1H), 7.47 (d, J=8.2 Hz, 2H), 7.76 (d. J=8.2 Hz. 2 H) | ESI 448[M+1]⁺ | 0.229 (A) |

**[Table 2-20]**

| Compound | Structural formula | ¹H-NMR | MS (m/z) | Retention time Method |
|---|---|---|---|---|
| 134 | 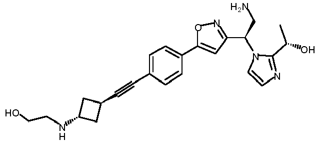 | ¹H NMR (400 MHz, METHANOL-d4) *δ* ppm 1.66 (d, J=6.6 Hz, 3 H), 2.17 - 2.30 (m, 2 H), 2.35 - 2.46 (m, 2 H), 2.62 - 2.69 (m, 2 H), 3.35 - 3.43 (m, 2 H), 3.49 - 3.56 (m, 1 H), 3.58 - 3.66 (m, 3 H), 5.09 (q, J=6.6 Hz, 1 H), 5.87 - 5.93 (m, 1H), 6.77 (s, 1 H), 6.98 (s, 1 H), 7.25 (s, 1 H), 7.48 (d, J=7.8 Hz, 2 H), 7.76 (d, J=7.8 Hz. 2 H) | ESI 436[M+1]⁺ | 0.221 (A) |
| 135 | 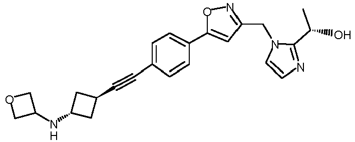 | ¹H NMR (400 MHz, METHANOL-d4) *δ* ppm 1.59 (d, J=6.3 Hz, 3 H), 2.12 - 2.38 (m, 4 H), 3.52 - 3.63 (m, 1 H), 3.92 - 4.15 (m, 2 H), 4.45 - 4.55 (m, 2 H), 4.74 - 4.78 (m, 2 H), 5.04 (q, J=6.3 Hz, 1 H), 5.49 (dd, J=16.3, 16.3 Hz, 2 H), 6.71 (s, 1 H), 6.93 (s, 1 H), 7.15 (s, 1 H), 7.47 (d, J=8.1 Hz, 2 H). 7.74 (d. J=8.1 Hz. 2 H) | ESI 419[M+1]⁺ | 0.532 (A) |
| 136 | 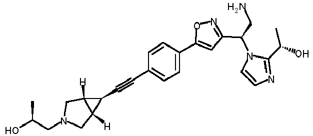 | ¹H NMR (400 MHz, METHANOL-d4) *δ* ppm 1.11 (d, J=5.6 Hz, 3 H), 1.66 (d, J=6.2 Hz, 3 H), 1. 77 - 1. 85 (m, 2 H), 1.90 - 1.97 (m, 1 H), 2.26 - 2.51 (m, 4H), 3.05 - 3.88 (m, 5 H), 5.03 - 5.14 (m, 1 H), 5.90 (t, J=6.9 Hz, 1 H), 6.75 (s, 1 H), 6.97 (s, 1 H), 7.24 (s, 1 H), 7.42 (d, J=8.0 Hz, 2 H), 7.73 (d. J=8.0 Hz. 2 H) | ESI 462[M+1]⁺ | 0.211 (A) |
| 137 | 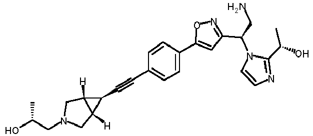 | ¹H NMR (400 MHz, METHANOL-d4) *δ* ppm 1.11 (d, J=6.2 Hz, 3 H), 1.66 (d, J=6.5 Hz, 3 H), 1. 78 - 1.84 (m, 2 H), 1.90 - 1.97 (m, 1 H), 2.30 - 2.52 (m, 4 H), 3.06 - 3.87 (m, 5 H), 5.05 - 5.13 (m, 1 H), 5.90 (t, J=7.3 Hz, 1 H), 6.75 (s, 1 H), 6.97 (s, 1 H), 7.24 (s, 1 H), 7.42 (d, J=7.9 Hz, 2 H), 7.73 (d, J=7.9 Hz, 2 H) | ESI 462[M+1]⁺ | 0.212 (A) |

The names of the compounds described in Table 2 are as follows:
Compound 1: 5-{4-[3-({2-[(1S)-1-hydroxyethyl]-1H-imidazol-1-yl}methyl)-1,2-oxazol-5-yl]phenyl}pent-4-yn-1-ol,
Compound 2: 5-{2-fluoro-4-[3-({2-[(1S)-1-hydroxyethyl]-1H-imidazol-1-yl}methyl)-1,2-oxazol-5-yl]phenyl}pent-4-yn-1-ol,
Compound 3: 5-{3-fluoro-4-[3-({2-[(1S)-1-hydroxyethyl]-1H-imidazol-1-yl}methyl)-1,2-oxazol-5-yl]phenyl}pent-4-yn-1-ol,
Compound 4: (1S)-1-{1-[(5-{4-[(1-aminocyclopropyl)ethynyl]phenyl}-1,2-oxazol-3-yl)methyl]-1H-imidazol-2-yl}ethan-1-ol,
Compound 5: 5-{4-[3-({2-[(1S)-1-hydroxyethyl]-1H-imidazol-1-yl}methyl)-1,2-oxazol-5-yl]-3-methylphenyl}pent-4-yn-1-ol,
Compound 6: 4-{4-[3-({2-[(1S)-1-hydroxyethyl]-1H-imidazol-1-yl}methyl)-1,2-oxazol-5-yl]phenyl}but-3-yn-1-ol,
Compound 7: (1S)-1-(1-{[5-(4-{[trans-2-(hydroxymethyl)cyclopropyl]ethynyl}phenyl)-1,2-oxazol-3-yljmethyl}-1H-imidazol-2-yl)ethan-1-ol,
Compound 8: (1S)-1-(1-{[5-(4-{[trans-2-(aminomethyl)cyclopropyl]ethynyl}phenyl)-1,2-oxazol-3-yl]methyl}-1H-imidazol-2-yl)ethan-1-ol,
Compound 9: (1S)-1-{1-[(5-{4-[(trans-2-aminocyclopropyl)ethynyl]phenyl}-1,2-oxazol-3-yl)methyl]-1H-imidazol-2-yl}ethan-1-ol,
Compound 10: (1S)-1-(1-{[5-(4-{[(1R,5S,6s)-3-azabicyclo[3.1.0]hexan-6-yl]ethynyl}phenyl)-1,2-oxazol-3-yl]methyl}-1H-imidazol-2-yl)ethan-1-ol,
Compound 11: (2S)-3-[(1R,5S, 6R)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1,2-diol,
Compound 12: (1S)-1-{1-[(5-{4-[(1-methylazetidin-3-yl)ethynyl]phenyl}-1,2-oxazol-3-yl)methyl]-1H-imidazol-2-yl}ethan-1-ol,
Compound 13: (2S)-3-[3-({4-[3-({2-[(1S)-1-hydroxyethyl]-1H-imidazol-1-yl}methyl)-1,2-oxazol-5-yl]phenyl}ethynyl)azetidin-1-yl]propan-1,2-diol,
Compound 14: 5-{3,5-difluoro-4-[3-({2-[(1S)-1-hydroxyethyl]-1H-imidazol-1-yl}methyl)-1,2-oxazol-5-yl]phenyl}pent-4-yn-1-ol,
Compound 15: (R)-2-amino-1-((1S,2S)-2-((4-(3-((2-((S)-1-hydroxyethyl)-1H-imidazol-1-yl)methyl)isoxazol-5-yl)phenyl)ethynyl)cyclopropyl)ethanol,
Compound 16: 5-(4-{3-[(1S)-2-hydroxy-1-{2-[(1S)-1-hydroxyethyl]-1H-imidazol-1-yl}ethyl]-1,2-oxazol-5-yl}phenyl)pent-4-yn-1-ol,
Compound 17: (1S)-1-{1-[(5-{4-[(3-aminobicyclo[1.1.1]pentan-1-yl)ethynyl]phenyl}-1,2-oxazol-3-yl)methyl]-1H-imidazol-2-yl}ethan-1-ol,
Compound 18: 5-(4-{3-[(1R)-2-amino-1-{2-[(1S)-1-hydroxyethyl]-1H-imidazol-1-yl}ethyl]-1,2-oxazol-5-yl}phenyl)pent-4-yn-1-ol,
Compound 19: (1S)-1-(1-{[5-(4-{[(1R,5S,6s)-3-(2-hydroxyethyl)-3-azabicyclo[3.1.0]hexan-6-yl]ethynyl}phenyl)-1,2-oxazol-3-yl]methyl}-1H-imidazol-2-yl)ethan-1-ol,
Compound 20: (1S)-1-{1-[(1R)-2-amino-1-(5-{4-[(3-aminobicyclo[1.1.1]pentan-1-yl)ethynyl]phenyl}-1,2-oxazol-3-yl)ethyl]-1H-imidazol-2-yl}ethan-1-ol,
Compound 21: (1S)-1-{1-[(1R)-2-amino-1-(5-{4-[(1-aminocyclopropyl)ethynyl]phenyl}-1,2-oxazol-3-yl)ethyl]-1H-imidazol-2-yl}ethan-1-ol,
Compound 22: (1S)-1-(1-{(1R)-2-amino-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol,
Compound 23: (1S)-1-{1-[(5-{4-[(trans-3-aminocyclobutyl)ethynyl]phenyl}-1,2-oxazol-3-yl)methyl]-1H-imidazol-2-yl}ethan-1-ol,
Compound 24: 1-amino-3-({4-[3-({2-[(1S)-1-hydroxyethyl]-1H-imidazol-1-yl}methyl)-1,2-oxazol-5-yl]phenyl}ethynyl)cyclobutane-1-carbonitrile,
Compound 25: 1-amino-3-({4-[3-({2-[(1S)-1-hydroxyethyl]-1H-imidazol-1-yl}methyl)-1,2-oxazol-5-yl]phenyl}ethynyl)cyclobutane-1-carboxamide,
Compound 26: (1S)-1-[1-({5-[4-(cyclopropylethynyl)phenyl]-1,2-oxazol-3-yl}methyl)-1H-imidazol-2-yl]ethan-1-ol,
Compound 27: (1S)-1-{1-[(3-{4-[(1-aminocyclopropyl)ethynyl]phenyl}-1,2-oxazol-5 yl)methyl]-1H-imidazol-2-yl}ethan-1-ol,
Compound 28: (1S)-1-{1-[(3-{4-[(3-aminobicyclo[1.1.1]pentane-1-yl)ethynyl]phenyl}-1,2-oxazol-5-yl)methyl]-1H-imidazol-2-yl}ethan-1-ol,
Compound 29: (1S)-1-{1-[(5-{4-[(4-aminooxan-4-yl)ethynyl]phenyl}-1,2-oxazol-3-yl)methyl]-1H-imidazol-2-yl}ethan-1-ol,
Compound 30: (1S)-1-[1-({5-[4-(3-aminoprop-1-yn-1-yl)phenyl]-1,2-oxazol-3-yl}methyl)-1H-imidazol-2-yl]ethan-1-ol,
Compound 31: (1S)-1-{1-[(5-{4-[(oxan-4-yl)ethynyl]phenyl}-l,2-oxazol-3-yl)methyl]-1H-imidazol-2-yl}ethan-1-ol,
Compound 32: 4-{4-[3-({2-[(1S)-1-hydroxyethyl]-1H-imidazol-1-yl}methyl)-1,2-oxazol-5-yl]phenyl}-2-methylbut-3-yn-2-ol,
Compound 33: (1S)-1-[1-({5-[4-(3-aminoprop-1-yn-1-yl)phenyl]-1,2-oxazol-3-yl}methyl)-1H-imidazol-2-yl]ethan-1-ol,
Compound 34: (1S)-1-(1-{[5-(4-{[(1R,5S,6s)-3-(2-methoxyethyl)-3-azabicyclo[3.1.0]hexan-6-yl]ethynyl}phenyl)-1,2-oxazol-3-yl]methyl}-1H-imidazol-2-yl)ethan-1-ol,
Compound 35: (1S)-1-{1-[(5-{4-[(1,3-dimethylazetidin-3-yl)ethynyl]phenyl}-1,2-oxazol-3-yl)methyl]-1H-imidazol-2-yl}ethan-1-ol,
Compound 36: (1S)-1-(1-{[5-(4-{[(1R,5S,6s)-3-methyl-3-azabicyclo[3.1.0]hexan-6-yl]ethynyl}phenyl)-1,2-oxazol-3-yl]methyl}-1H-imidazol-2-yl)ethan-1-ol,
Compound 37: 1-[(1R,5S,6s)-6-({4-[3-({2-[(1S)-1-hydroxyethyl]-1H-imidazol-1-yl}methyl)-1,2-oxazol-5-yl]phenyl]ethynyl)-3-azabicyclo[3.1.0]hexan-3-yl]ethan-1-one,
Compound 38: (1S)-1-{1-[(1R)-1-[5-(4-{[(1R,5S,6s)-3-(2-hydroxyethyl)-3-azabicyclo[3.1.0]hexan-6-yl]ethynyl}phenyl)-1,2-oxazol-3-yl]-2-(methylamino)ethyl]-1H-imidazol-2-yl}ethan-1-ol,
Compound 39: (1S)-1-(1-{(1R)-2-(dimethylamino)-1-[5-(4-{[(1R,5S,6s)-3-(2-hydroxyethyl)-3-azabicyclo[3.1.0]hexan-6-yl]ethynyl}phenyl)-1,2-oxazol-3-yl]ethyl}-1H-imidazol-2-yl)ethan-1-ol,
Compound 40: (1S)-1-{1-[(5-{ 4-[(3-aminooxetan-3-yl)ethynyl]phenyl}-1,2-oxazol-3-yl)methyl]-1H-imidazol-2-yl}ethan-1-ol,
Compound 41: (1S)-1-(1-{[5-(4-{[1-(2-methoxyethyl)-3-methylazetidin-3-yl]ethynyl}phenyl)-1,2-oxazol-3-yl]methyl}-1H-imidazol-2-yl)ethan-1-ol,
Compound 42: (1S)-1-[1-({5-[4-({(1R,5S,6s)-3-[(oxetan-3-yl)methyl]-3-azabicyclo[3.1.0]hexan-6-yl}ethynyl)phenyl]-1,2-oxazol-3-yl}methyl)-1H-imidazol-2-yl]ethan-1-ol,
Compound 43: (1S)-1-[1-({5-[4-(3,3-dimethylbut-1-yn-1-yl)phenyl]-1,2-oxazol-3-yl}methyl)-1H-imidazol-2-yl]ethan-1-ol,
Compound 44: (1S)-1-{1-[(5-{4-[(oxetan-3-yl)ethynyl]phenyl}-1,2-oxazol-3-yl)methyl]-1H-imidazol-2-yl}ethan-1-ol,
Compound 45: (1S)-1-(1-{[5-(4-{[1-(methylamino)cyclopropyl]ethynyl}phenyl)-1,2-oxazol-3-yl]methyl}-1H-imidazol-2-yl)ethan-1-ol,
Compound 46: (1S)-1-{1-[(5-{4-[(3-oxabicyclo[3.1.0]hexan-6-yl)ethynyl]phenyl}-1,2-oxazol-3-yl)methyl]-1H-imidazol-2-yl}ethan-1-ol,
Compound 47: (1S)-1-{1-[(5-{4-[(3-methyloxetan-3-yl)ethynyl]phenyl}-1,2-oxazol-3-yl)methyl]-1H-imidazol-2-yl}ethan-1-ol,
Compound 48: 3-{4-[3-({2-[(1S)-1-hydroxyethyl]-1H-imidazol-1-yl}methyl)-1,2-oxazol-5-yl]phenyl}prop-2-yn-1-ol,
Compound 49: (1S)-1-{1-[(5-{4-[(oxolan-3-yl)ethynyl]phenyl}-1,2-oxazol-3-yl)methyl]-1H-imidazol-2-yl}ethan-1-ol,
Compound 50: (1S)-1-(1-{[5-(4-{[1-(dimethylamino)cyclopropyl]ethynyl}phenyl)-1,2-oxazol-3-yl]methyl}-1H-imidazol-2-yl)ethan-1-ol,
Compound 51: (1S)-1-{1-[(5-{4-[3-(cyclopropylamino)prop-1-yn-1-yl]phenyl}-1,2-oxazol-3-yl)methyl]-1H-imidazol-2-yl}ethan-1-ol,
Compound 52: 1-({4-[3-({2-[(1S)-1-hydroxyethyl]-1H-imidazol-1-yl}methyl)-1,2-oxazol-5-yl]phenyl}ethynyl)cyclopropan-1-ol,
Compound 53: (1S)-1-[1-({5-[4-(3-methoxyprop-1-yn-1-yl)phenyl]-1,2-oxazol-3-yl}methyl)-1H-imidazol-2-yl]ethan-1-ol,
Compound 54: trans-3-({4-[3-({2-[(1S)-1-hydroxyethyl]-1H-imidazol-1-yl}methyl)-1,2-oxazol-5-yl]phenyl}ethynyl)cyclobutan-1-ol,
Compound 55: 1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-2-ol,
Compound 56: 2-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1,3-diol,
Compound 57: 1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-3-methoxypropan-2-ol,
Compound 58: (1S)-1-{1-[(1R)-2-amino-1-(5-{4-[(3-oxabicyclo[3.1.0]hexan-6-yl)ethynyl]phenyl}-1,2-oxazol-3-yl)ethyl]-1H-imidazol-2-yl}ethan-1-ol,
Compound 59: (1S)-1-(1-{[5-(4-{[(1R,5S,6s)-3-(oxetan-3-yl)-3-azabicyclo[3.1.0]hexan-6-yl]ethynyl}phenyl)-1,2-oxazol-3-yl]methyl}-1H-imidazol-2-yl)ethan-1-ol,
Compound 60: (1S)-1-{1-[(1R)-2-amino-1-{5-[4-(cyclopropylethynyl)phenyl]-1,2-oxazol-3-yl}ethyl]-1H-imidazol-2-yl}ethan-1-ol,
Compound 61: 3-amino-1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1-one,
Compound 62: 3-amino-1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-3-methylbutan-1-one,
Compound 63: 1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-3-(methylamino)propan-1-one,
Compound 64: (1S)-1-{1-[(5-{4-[(1-aminocyclopropyl)ethynyl]phenyl}-1,2-oxazol-3-yl)methyl]-5-methyl-1H-imidazol-2-yl}ethan-1-ol,
Compound 65: (1S)-1-[1-({5-[4-(4-methoxybut-1-yn-1-yl)phenyl]-1,2-oxazol-3-yl}methyl)-1H-imidazol-2-yl]ethan-1-ol,
Compound 66: (1S)-1-(1-{[5-(4-{[trans-2-(methoxymethyl)cyclopropyl]ethynyl}phenyl)-1,2-oxazol-3-yl]methyl}-1H-imidazol-2-yl)ethan-1-ol,
Compound 67: 4-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]butanoic acid
Compound 68: tert-butyl 3-({4-[3-({2-[(1S)-1-hydroxyethyl]-1H-imidazol-1-yl}methyl)-1,2-oxazol-5-yl]phenyl}ethynyl)azetidine-1-carboxylate
Compound 69: 1-[3-({4-[3-({2-[(1S)-1-hydroxyethyl]-1H-imidazol-1-yl}methyl)-1,2-oxazol-5-yl]phenyl}ethynyl)azetidin-1-yl]ethan-1-one
Compound 70: trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutan-1-ol,
Compound 71: (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carboxamide,
Compound 72: (1S)-1-(1-{[5-(4-{[(1R,5S,6s)-3-(methanesulfonyl)-3-azabicyclo[3.1.0]hexan-6-yl]ethynyl}phenyl)-1,2-oxazol-3-yl]methyl}-1H-imidazol-2-yl)ethan-1-ol,
Compound 73: (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carbaldehyde,
Compound 74: (1S)-1-(1-{(1R)-2-amino-1-[5-(4-{[trans-2-(methoxymethyl)cyclopropyl]ethynyl}phenyl)-1,2-oxazol-3-yl]ethyl}-1H-imidazol-2-yl)ethan-1-ol,
Compound 75: (1S)-1-{1-[(1R)-2-amino-1-(5-{4-[(oxolan-3-yl)ethynyl]phenyl}-1,2-oxazol-3-yl)ethyl]-1H-imidazol-2-yl}ethan-1-ol,
Compound 76: 1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-2-methoxyethan-1-one, Compound 77: (2S)-2-hydroxy-1-[(1R,5S,6R)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1-one,
Compound 78: methyl (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-carboxylate,
Compound 79: 2,3-dihydroxy-1-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propan-1-one,
Compound 80: (1S)-1-(1-{(1R)-2-amino-1-[5-(4-{[trans-2-(hydroxymethyl)cyclopropyl]ethynyl}phenyl)-1,2-oxazol-3-yl]ethyl}-1H-imidazol-2-yl)ethan-1-ol,
Compound 81: (1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexane-3-sulfonamide hydrochloride,
Compound 82: (1S)-1-{1-[(1R)-2-amino-1-{3-[4-(cyclopropylethynyl)phenyl]-1,2-oxazol-5-yl}ethyl]-1H-imidazol-2-yl}ethan-1-ol,
Compound 83: (1S)-1-(1-{[3-(4-{[(1R,5S,6s)-3-azabicyclo[3.1.0]hexan-6-yl]ethynyl}phenyl)-1,2-oxazol-5-yl]methyl}-1H-imidazol-2-yl)ethan-1-ol,
Compound 84: (1S)-1-(1-{[3-(4-{[(1R,5S,6s)-3-(2-methoxyethyl)-3-azabicyclo[3.1.0]hexan-6-yl]ethynyl}phenyl)-1,2-oxazol-5-yl]methyl}-1H-imidazol-2-yl)ethan-1-ol,
Compound 85: 1-[(1R,5S,6s)-6-({4-[5-({2-[(1S)-1-hydroxyethyl]-1H-imidazol-1-yl}methyl)-1,2-oxazol-3-yl]phenyl}ethynyl)-3-azabicyclo[3.1.0]hexan-3-yl]-3-methoxypropan-2-ol, Compound 86: 1-[(1R,5S,6s)-6-({4-[5-({2-[(1S)-1-hydroxyethyl]-1H-imidazol-1-yl}methyl)-1,2-oxazol-3-yl]phenyl}ethynyl)-3-azabicyclo[3.1.0]hexan-3-yl]ethan-1-one,
Compound 87: N-[trans-3-({4-[3-({2-[(1S)-1-hydroxyethyl]-1H-imidazol-1-yl}methyl)-1,2-oxazol-5-yl]phenyl}ethynyl)cyclobutyl]acetamide,
Compound 88: cis-3-({4-[3-({2-[(1S)-1-hydroxyethyl]-1H-imidazol-1-yl}methyl)-1,2-oxazol-5-yl]phenyl}ethynyl)cyclobutan-1-ol,
Compound 89: trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]-1-(hydroxymethyl)cyclobutan-1-ol,
Compound 90: cis-3-[(4-{3-[(R)-2-amino-1-{2-[(1S)-1-hydroxyethyl]-1H-imidazol-1-yl}ethyl]-1,2-oxazol-5-yl}phenyl)ethynyl]-1-(hydroxymethyl)cyclobutan-1-ol,
Compound 91: 1-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}ethan-1-one,
Compound 92: (1R,5S,6s)-6-({4-[3-({2-[(1S)-1-hydroxyethyl]-1H-imidazol-1-yl}methyl)-1,2-oxazol-5-yl]phenyl}ethynyl)-N-methyl-3-azabicyclo[3.1.0]hexane-3-carboxamide,
Compound 93: 5-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]pentanoic acid,
Compound 94: 6-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]hexanoic acid,
Compound 95: 4-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-4-oxobutanoic acid,
Compound 96: (2S)-2-[5-(4-{[(1R,5S,6s)-3-(2-hydroxyethyl)-3-azabicyclo[3.1.0]hexan-6-yl]ethynyl}phenyl)-1,2-oxazol-3-yl]-2-{2-[(1S)-1-hydroxyethyl]-1H-imidazol-1-yl}ethan-1-ol,
Compound 97: (1S)-1-(1-{[5-(4-{[3-(dimethylamino)cyclobutyl]ethynyl}phenyl)-1,2-oxazol-3-yl]methyl}-1H-imidazol-2-yl)ethan-1-ol,
Compound 98: (1S)-1-(1-{[5-(4-{[3-(cyclopropylamino)cyclobutyl]ethynyl}phenyl)-1,2-oxazol-3-yl]methyl}-1H-imidazol-2-yl)ethan-1-ol,
Compound 99: (1S)-1-(1-{[5-(4-{[(1R,5S,6s)-3-ethyl-3-azabicyclo[3.1.0]hexan-6-yl]ethynyl}phenyl)-1,2-oxazol-3-yl]methyl}-1H-imidazol-2-yl)ethan-1-ol,
Compound 100: trans-3-[(4-{5-[(1R)-2-amino-1-{2-[(1S)-1-hydroxyethyl]-1H-imidazol-1-yl}ethyl]-1,2-oxazol-3-yl}phenyl)ethynyl]cyclobutan-1-ol,
Compound 101: (1S)-1-(1-{(1R)-2-amino-1-[5-(4-{[(1R,5S,6s)-3-(2-methoxyethyl)-3-azabicyclo[3.1.0]hexan-6-yl]ethynyl}phenyl)-1,2-oxazol-3-yl]ethyl}-1H-imidazol-2-yl)ethan-1-ol,
Compound 102: (1R,5S,6s)-6-({4-[5-({2-[(1S)-1-hydroxyethyl]-1H-imidazol-1-yl}methyl)-1,2-oxazol-3-yl]phenyl}ethynyl)-3-azabicyclo[3.1.0]hexane-3-carbaldehyde,
Compound 103 N-[trans-3-({4-[3-({2-[(1S)-1-hydroxyethyl]-1H-imidazol-1-yl}methyl)-1,2-oxazol-5-yl]phenyl}ethynyl)cyclobutyl]formamide,
Compound 104: N-[1-({4-[3-({2-[(1S)-1-hydroxyethyl]-1H-imidazol-1-yl}methyl)-1,2-oxazol-5-yl]phenyl}ethynyl)cyclopropyl]formamide,
Compound 105: 2-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-N-methyl-2-oxoacetamide,
Compound 106: N-{trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutyl}formamide,
Compound 107: 2-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-2-oxoacetamide, Compound 108: 2-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]-N,N-dimethyl-2-oxoacetamide,
Compound 109: (1S)-1-(1-{[5-(4-{[2,3-bis(hydroxymethyl)cyclopropyl]ethynyl}phenyl)-1,2-oxazol-3-yl]methyl}-1H-imidazol-2-yl)ethan-1-ol,
compound 110: [(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]acetic acid
Compound 111: 4-[(1R,5S,6s)-6-({4-[5-({2-[(1S)-1-hydroxyethyl]-1H-imidazol-1-yl}methyl)-1,2-oxazol-3-yl]phenyl}ethynyl)-3-azabicyclo[3.1.0]hexan-3-yl]butanoic acid,
Compound 112: (1S)-1-[1-({5-[4-({3-[(oxetan-3-yl)amino]cyclobutyl}ethynyl)phenyl]-1,2-oxazol-3-yl}methyl)-1H-imidazol-2-yl]ethan-1-ol,
Compound 113: 4-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}butanoic acid,
Compound 114: 3-[(1R,5S,6s)-6-({4-[3-({2-[(1S)-1-hydroxyethyl]-1H-imidazol-1-yl}methyl)-1,2-oxazol-5-yl]phenyl}ethynyl)-3-azabicyclo[3.1.0]hexan-3-yl]propanoic acid,
Compound 115: N-{trans-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutyl}acetamide,
Compound 116: (1S)-1-{1-[(1R)-2-amino-1-(5-{4-[(3-methyloxetan-3-yl)ethynyl]phenyl}-1,2-oxazol-3-yl)ethyl]-1H-imidazol-2-yl}ethan-1-ol,
Compound 117: (1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carbaldehyde,
Compound 118: methyl (1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexane-3-carboxylate,
Compound 119: 3-((4-(3-((2-((S)-1-hydroxyethyl)-1H-imidazol-1-yl)methyl)isoxazol-5-yl)phenyl)ethynyl)tetrahydrothiophene 1,1-dioxide,
Compound 120: 1-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}propan-2-ol,
Compound 121: 1-[(1R,5S,6s)-6-({4-[5-({2-[(1S)-1-hydroxyethyl]-1H-imidazol-1-yl}methyl)-1,2-oxazol-3-yl]phenyl}ethynyl)-3-azabicyclo[3.1.0]hexan-3-yl]propan-2-ol,
Compound 122: (1S)-1-(1-{[5-(4-{[(1R,5S,6s)-3-(2-aminoethyl)-3-azabicyclo[3.1.0]hexan-6-yl]ethynyl}phenyl)-1,2-oxazol-3-yl]methyl}-1H-imidazol-2-yl)ethan-1-ol,
Compound 123: N-{trans-3-[(4-{3-[(1R)-2-amino-1-{2-[(1S)-1-hydroxyethyl]-1H-imidazol-1-yl} ethyl]-1,2-oxazol-5-yl}phenyl)ethynyl]cyclobutyl}-N-methylformamide,
Compound 124: 2-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1.0]hexan-3-yl}-2-oxoacetamide,
Compound 125: cis-3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutan-1-ol,
Compound 126: trans-2-[(4-{3-[(1R)-2-amino-1-{2-[(1S)-1-hydroxyethyl]-1H-imidazol-1-yl}ethyl]-1,2-oxazol-5-yl}phenyl)ethynyl]cyclopropane-1-carboxylate,
Compound 127: 4-({4-[3-({2-[(1S)-1-hydroxyethyl]-1H-imidazol-1-yl}methyl)-1,2-oxazol-5-yl]phenyl}ethynyl)piperidine-1-carbaldehyde,
Compound 128: 1-[4-({4-[3-({2-[(1S)-1-hydroxyethyl]-1H-imidazol-1-yl}methyl)-1,2-oxazol-5-yl]phenyl}ethynyl)piperidin-1-yl]propan-2-ol,
Compound 129: (1S)-1-(1-{(1R)-2-amino-1-[5-(4-{[3-(cyclopropylamino)cyclobutyl]ethynyl}phenyl)-1,2-oxazol-3-yl]ethyl}-1H-imidazol-2-yl)ethan-1-ol,
Compound 130: 3-[(4-{3-[(1R)-2-amino-1-{2-[(1S)-1-hydroxyethyl]-1H-imidazol-1-yl}ethyl]-1,2-oxazol-5-yl}phenyl)ethynyl]cyclobutane-1-carboxylate,
Compound 131: (1S)-1-(1-{(1R)-2-amino-1-[5-(4-{[2,2-bis(hydroxymethyl)cyclopropyl]ethynyl}phenyl)-1,2-oxazol-3-yl]ethyl}-1H-imidazol-2-yl)ethan-1-ol,
Compound 132: 3-{(1R,5S,6s)-6-[(4-{3-[(1R)-2-amino-1-{2-[(1S)-1-hydroxyethyl]-1H-imidazol-1-yl}ethyl]-1,2-oxazol-5-yl}phenyl)ethynyl]-3-azabicyclo[3.1,0]hexan-3-yl}propan-1-ol,
Compound 133: (1S)-1-{1-[(1R)-2-amino-1-{5-[4-({trans-3-[(oxetan-3-yl)amino]cyclobutyl}ethynyl)phenyl]-1,2-oxazol-3-yl}ethyl]-1H-imidazol-2-yl}ethan-1-ol,
Compound 134: (1S)-1-{1-[(1R)-2-amino-1-{5-[4-({trans-3-[(2-hydroxyethyl)amino]cyclobutyl}ethynyl)phenyl]-1,2-oxazol-3-yl}ethyl]-1H-imidazol-2-yl}ethan-1-ol,
Compound 135: (S)-1-(1-((5-(4-(((1r,3S)-3-(oxetan-3-ylamino)cyclobutyl)ethynyl)phenyl)isoxazol-3-yl)methyl)-1H-imidazol-2-yl)ethanol,
Compound 136: (S)-1-((1R,5S,6R)-6-((4-(3-((R)-2-amino-1-(2-((S)-1-hydroxyethyl)-1H-imidazol-1-yl)ethyl)isoxazol-5-yl)phenyl)ethynyl)-3-azabicyclo[3.1.0]hexan-3-yl)propan-2-ol,
Compound 137: (R)-1-((1R,5S,6R)-6-((4-(3-((R)-2-amino-1-(2-((S)-1-hydroxyethyl)-1H-imidazol-1-yl)ethyl)isoxazol-5-yl)phenyl)ethynyl)-3-azabicyclo[3.1.0]hexan-3-yl)propan-2-ol.

### Test Examples

The action of the compounds of the present invention was confirmed by the following pharmacological tests.

### Test Example 1 Evaluation of the inhibitory activity on Pseudomonas aeruginosa LpxC enzyme (Fluoresamine method)

To assay the activity of *Pseudomonas aeruginosa* LpxC enzyme, LpxC was reacted with its substrate, UDP-3-O-(R-3-hydroxydecanoyl)-N-acetylglucosamine, and the amount of the reaction product was quantified by the amino groups present in the product.

Specifically, to 3.6 nmol/L *Pseudomonas aeruginosa* LpxC enzyme (as acquired by preparing chromosomal DNA from *Pseudomonas aeruginosa,* obtaining the *Pseudomonas aeruginosa* LpxC genes by PCR (polymerase chain reaction) using LpxC specific primer, and incorporating the genes into a vector, followed by expression using *Escherichia coli*), 20 µmol/L UDP-3-O-(R-3-hydroxydecanoyl)-N-acetylglucosamine (Carbosynth) was added, and the mixture was incubated at room temperature for 60 minutes. This reaction was performed in a 40 mmol/L-HEPES buffer solution (pH 8.0) containing 0.02% Brij 35 and 25 nmol/L-zinc chloride. The reaction was terminated by the addition of 1.0 mg/mL-fluorescamine dissolved in a solvent obtained by mixing an equal amount of acetonitrile and dimethylformamide with the reaction mixture, and then, a 0.2 mol/L-sodium phosphate buffer solution (pH 8.0) was added thereto, and the amount of the reaction product was detected at an excitation wavelength/fluorescence wavelength = 390 nm/495 nm. An inhibition curve was obtained by performing the aforementioned reaction in the presence of various concentrations of test compounds. From this inhibition curve, the concentration of test compound at which the amount of the reaction product was suppressed by 50% (IC₅₀ value) was determined, which was used as an index for the inhibitory activity on *Pseudomonas aeruginosa* LpxC enzyme. Table 3 shows the test results for the compounds (inhibitory activity on *Pseudomonas aeruginosa* LpxC enzyme IC₅₀ (µmol/L)).

**[Table 3]**

| Compound | IC₅₀ (*µ*mol/L) | | Compound | IC₅₀ (*µ*mol/L) |
|---|---|---|---|---|
| 1 | 0.0132 | | 11 | 0.00378 |
| 2 | 0.00692 | | 12 | 0.046 |
| 3 | 0.00436 | | 13 | 0.0219 |
| 4 | 0.0167 | | 14 | 0.0154 |
| 5 | 0.132 | | 15 | 0.0084 |
| 6 | 0.0226 | | 16 | 0.0026 |
| 7 | 0.00444 | | 17 | 0.0138 |
| 8 | 0.0305 | | 18 | 0.00604 |
| 9 | 0.0223 | | 19 | 0.00203 |
| 10 | 0.0143 | | | |

Test Example 2 Evaluation of the inhibitory activity on *Escherichia coli* LpxC enzyme (Fluoresamine method) To assay the activity of *Escherichia coli* LpxC enzyme, LpxC was reacted with its substrate, UDP-3-O-(R-3-hydroxymyristoyl)-N-acetylglucosamine, and the amount of the reaction product was quantified by the amino groups present in the product.

Specifically, to 3.1 nmol/L of *Escherichia coli* LpxC enzyme (as acquired by preparing chromosomal DNA from *Escherichia coli,* obtaining the *Escherichia coli* LpxC genes by PCR (polymerase chain reaction) using LpxC specific primer, and incorporating the genes into a vector, followed by expression using *Escherichia coli*), 20 µmol/L UDP-3-O-(R-3-hydroxymyristoyl)-N-acetylglucosamine (Alberta) was added, and the mixture was incubated at room temperature for 120 minutes. This reaction was performed in a 40 mmol/L-2-morpholinoethanesulfonic acid buffer solution (pH 6.5) containing 0.02% Brij 35, 25 nmol/L-zinc chloride, and 80 µmol/L-dithiothreitol. The reaction was terminated by the addition of 1.0 mg/mL-fluorescamine dissolved in a solvent obtained by mixing an equal amount of acetonitrile and dimethylformamide with the reaction mixture, and then, a 0.2 mol/L-sodium phosphate buffer solution (pH 8.0) was added thereto, and the amount of the reaction product was detected at an excitation wavelength/fluorescence wavelength = 390 nm/495 nm. An inhibition curve was obtained by performing the aforementioned reaction in the presence of various concentrations of test compounds. From this inhibition curve, the concentration of test compound at which the amount of the reaction product was suppressed by 50% (IC₅₀ value) was determined, which was used as an index for the inhibitory activity on *Escherichia coli* LpxC enzyme. Table 4 shows the test results for the representative compounds (inhibitory activity on *Escherichia coli* LpxC enzyme IC₅₀ (µmol/L) (Fluoresamine method)).

In the table, ND means "Not Determined", and represents the case where the detected value of a reaction product of amino groups possessed by a test compound and fluorescamine shows a value 150% or more of the detected value of only the reaction mixture and fluorescamine (background), and the detected value of the reaction product of LpxC and its substrate, UDP-3-O-(R-3-hydroxymyristoyl)-N-acetylglucosamine is affected, which is the case where IC₅₀ value was not determined from the inhibition curve from which the test compound concentration was eliminated.

**[Table 4-1]**

| Compound | IC₅₀ (*µ*mol/L) | | Compound | IC₅₀ (*µ*mol/L) |
|---|---|---|---|---|
| 1 | 0.051 | | 80 | 0.0807 |
| 2 | 0.0169 | | 81 | 0.0696 |
| 3 | 0.0161 | | 82 | 0.082 |
| 4 | ND | | 83 | 0.136 |
| 7 | 0.0154 | | 84 | 0.102 |
| 9 | 0.183 | | 85 | 0.0968 |
| 10 | 0.123 | | 86 | 0.0464 |
| 11 | 0.102 | | 87 | 0.0252 |
| 13 | 0.651 | | 89 | ND |
| 17 | ND | | 90 | ND |
| 19 | 0.123 | | 91 | ND |
| 21 | ND | | 92 | 0.0549 |
| 22 | ND | | 93 | 0.371 |
| 23 | ND | | 94 | 0.412 |
| 26 | <0.0100 | | 95 | 0.134 |
| 31 | 0.0328 | | 96 | 0.259 |
| 32 | 0.335 | | 97 | 0.0997 |
| 34 | 0.108 | | 98 | 0.0514 |
| 35 | 0.815 | | 99 | 0.0918 |
| 36 | 0.0987 | | 100 | ND |
| 37 | 0.0582 | | 102 | 0.103 |
| 39 | 1.82 | | 103 | 0.088 |
| 41 | 0.231 | | 104 | 0.685 |
| 42 | 0.0468 | | 105 | 0.0638 |
| 43 | 0.0471 | | 106 | ND |
| 44 | 0.0227 | | 107 | 0.0786 |

**[Table 4-2]**

| Compound | IC₅₀ (*µ*mol/L) | | Compound | IC₅₀ (*µ*mol/L) |
|---|---|---|---|---|
| 45 | 0.268 | | 108 | 0.122 |
| 46 | 0.0134 | | 109 | 0.131 |
| 47 | 0.0193 | | 110 | 0.219 |
| 48 | 0.362 | | 111 | 0.197 |
| 49 | 0.0296 | | 112 | 0.0797 |
| 50 | 0.205 | | 113 | ND |
| 51 | 0.182 | | 114 | 0.291 |
| 52 | 0.0835 | | 115 | ND |
| 53 | 0.0817 | | 116 | 0.0791 |
| 55 | 0.0977 | | 117 | ND |
| 56 | 0.0837 | | 119 | 0.265 |
| 57 | 0.071 | | 120 | ND |
| 59 | 0.0355 | | 121 | 0.0985 |
| 63 | 0.105 | | 122 | 0.0846 |
| 65 | 0.0391 | | 123 | ND |
| 66 | <0.0100 | | 124 | ND |
| 67 | 0.0848 | | 125 | ND |
| 68 | 0.0136 | | 126 | ND |
| 69 | 0.0542 | | 127 | 0.0601 |
| 71 | 0.029 | | 128 | 0.331 |
| 72 | 0.112 | | 129 | ND |
| 73 | 0.0385 | | 130 | ND |
| 74 | 0.0396 | | 131 | ND |
| 76 | 0.038 | | 132 | ND |
| 77 | 0.0674 | | 133 | ND |
| 78 | 0.0138 | | 134 | ND |
| 79 | 0.0927 | | | |

### Test Example 3 Evaluation of the inhibitory activity on Escherichia coli LpxC enzyme (LCMS method)

To assay the activity of *Escherichia coli* LpxC enzyme, LpxC was reacted with its substrate, UDP-3-O-(R-3-hydroxymyristoyl)-N-acetylglucosamine, and the amount of the reaction product was quantified by liquid chromatography tandem mass spectrometry.

Specifically, to 3.1 nmol/L *Escherichia coli* LpxC enzyme (as acquired by preparing chromosomal DNA from *Escherichia coli,* obtaining the *Escherichia coli* LpxC genes by PCR (polymerase chain reaction) using LpxC specific primer, and incorporating the genes into a vector, followed by expression using *Escherichia coli*), 20 µmol/L UDP-3-O-(R-3-hydroxymyristoyl)-N-acetylglucosamine (Alberta) was added, and the mixture was incubated at room temperature for 120 minutes. This reaction was performed in a 40 mmol/L-2-morpholinoethanesulfonic acid buffer solution (pH 6.5) containing 0.02% Brij 35, 25 nmol/L-zinc chloride, and 80 µmol/L-dithiothreitol. The reaction was terminated by the addition of acetonitrile containing 25 µmol/L-UDP-N-acetylglucosamine as an internal standard to the reaction mixture. Centrifugation was performed and the amount of the substrate and the reaction product in the supernatant was detected by liquid chromatography tandem mass spectrometry. An inhibition curve was obtained by performing the aforementioned reaction in the presence of various concentrations of test compounds. From this inhibition curve, the concentration of test compound at which the amount of the reaction product was suppressed by 50% (IC₅₀ value) was determined, which was used as an index for the inhibitory activity on *Escherichia coli* LpxC enzyme. Table 5 shows the test results for the representative compounds (inhibitory activity on *Escherichia coli* LpxC enzyme IC₅₀ (µmol/L) (LCMS method)).

**[Table 5]**

| Compound | IC₅₀ (*µ*mol/L) | | Compound | IC₅₀ (*µ*mol/L) |
|---|---|---|---|---|
| 8 | 0.0678 | | 28 | 0.0646 |
| 17 | 0.0491 | | 29 | > 3.00 |
| 18 | 0.0697 | | 33 | > 3.00 |
| 20 | 0.279 | | 40 | 0.14 |
| 21 | 0.282 | | 61 | <0.0370 |
| 22 | 0.655 | | 62 | <0.0370 |
| 24 | <0.0370 | | 64 | 0.176 |
| 25 | 0.0522 | | 70 | <0.0370 |
| 27 | 0.193 | | | |

### Test Example 4 Evaluation of the antimicrobial activity

For minimum inhibitory concentration (MIC) measurement, the following broth microdilution method was applied as adapted from the standard procedure recommended by the CLSI (Clinical and Laboratory Standards Institute).

The bacteria used were *Pseudomonas aeruginosa* strain ATCC27853, *Escherichia coli* strain ATCC25922, and *Klebsiella pneumoniae* strain ATCC13883. After culture overnight on a heart infusion agar medium, the cells of a test bacterium were scraped off, and a bacterial solution suspended to have a turbidity of level 0.5 on the McFarland scale was diluted and used as an inoculum solution. The inoculum solution was inoculated in a cation-adjusted Mueller-Hinton broth medium containing a test compound so that the final inoculation concentration was about 5 x 10⁵ CFU/mL, and cultured at 35 °C for about 18 hours. A minimum drug concentration at which no cell growth was visible to the naked eye was designated as MIC. Table 6 shows the test results of the representative compounds (antimicrobial activity MIC (µg/mL) on *Pseudomonas aeruginosa, Escherichia coli,* and *Klebsiella pneumoniae*). In the table, ND represents the case where suppression of the cell growth in a drug concentration-dependent manner was not observed (Not Determined), and NT represents that no test was conducted (Not Tested).

**[Table 6-1]**

| Compound | *Pseudomonas aeruginosa* ATCC27853 | *Escherichia coli* ATCC25922 | *Klebsiella pneumoniae* ATCC 13883 |
|---|---|---|---|
| 1 | 64 | 1 | 2 |
| 2 | 64 | 1 | 2 |
| 3 | 32 | 0.5 | 1 |
| 4 | 64 | 0.5 | 1 |
| 5 | > 64 | NT | 16 |
| 6 | 64 | NT | 4 |
| 7 | 16 | 0.25 | 0.5 |
| 8 | 16 | NT | 64 |
| 9 | 16 | NT | 4 |
| 10 | 8 | NT | ND |
| 11 | 8 | 1 | 2 |
| 12 | 32 | NT | 16 |
| 13 | 32 | NT | 16 |
| 14 | 64 | NT | 8 |
| 15 | 16 | NT | 64 |
| 16 | 16 | NT | 4 |
| 17 | 16 | 0.5 | 0.5 |
| 18 | 16 | NT | 8 |
| 19 | 8 | 0.5 | 1 |
| 20 | 4 | 0.5 | 2 |
| 21 | 32 | 2 | 8 |
| 22 | 2 | 2 | 4 |
| 23 | 16 | 4 | 8 |
| 24 | 128 | 2 | 4 |
| 25 | 32 | 2 | 8 |
| 26 | > 8 | 0.25 | 0.25 |
| 27 | > 128 | 1 | 4 |
| 28 | 64 | 0.5 | 2 |
| 29 | > 128 | 64 | 128 |
| 30 | 64 | 64 | > 128 |
| 31 | > 128 | 1 | 2 |
| 32 | > 128 | 8 | 16 |
| 33 | > 128 | 32 | 64 |
| 34 | 8 | 0.25 | 0.5 |
| 35 | > 128 | 4 | 16 |
| 36 | 16 | 0.5 | 1 |
| 37 | 32 | 1 | 2 |
| 38 | 4 | 2 | ND |
| 39 | 8 | 2 | 4 |
| 40 | > 128 | 16 | 32 |

**[Table 6-2]**

| Compound | *Pseudomonas aeruginosa* ATCC27853 | *Escherichia coli* ATCC25922 | *Klebsiella pneumoniae* ATCC13883 |
|---|---|---|---|
| 41 | > 128 | 2 | 4 |
| 42 | 16 | 0.25 | 0.5 |
| 43 | > 32 | 1 | 2 |
| 44 | 128 | 2 | 2 |
| 45 | 128 | 0.25 | 0.5 |
| 46 | > 32 | 0.12 | 0.12 |
| 47 | > 128 | 0.5 | 1 |
| 48 | > 32 | 16 | 32 |
| 49 | > 32 | 0.5 | 1 |
| 50 | > 128 | 2 | 4 |
| 51 | > 32 | 1 | 2 |
| 52 | 128 | 2 | 4 |
| 53 | > 128 | 4 | 2 |
| 54 | 16 | 0.12 | 0.25 |
| 55 | 16 | 0.5 | 1 |
| 56 | 8 | 1 | 2 |
| 57 | 8 | 0.5 | 0.5 |
| 58 | 8 | ND | 0.25 |
| 59 | > 16 | 0.5 | 0.5 |
| 60 | 8 | 0.25 | 0.25 |
| 61 | 4 | 8 | 16 |
| 62 | 8 | 8 | 32 |
| 63 | 8 | 8 | 16 |
| 64 | > 128 | 2 | 4 |
| 65 | > 128 | 0.5 | 1 |
| 66 | 32 | 0.12 | 0.12 |
| 67 | 8 | 2 | 4 |
| 68 | 32 | 2 | 4 |
| 69 | 64 | 4 | 8 |
| 70 | 4 | 0.25 | 1 |
| 71 | > 16 | 1 | 2 |
| 72 | > 16 | 0.5 | 1 |
| 73 | 64 | 1 | 2 |
| 74 | 16 | 0.12 | 0.25 |
| 75 | 64 | 0.25 | 2 |
| 76 | 32 | 1 | 2 |
| 77 | 32 | 2 | 2 |
| 78 | 32 | 0.5 | 0.5 |
| 79 | 32 | 2 | 4 |
| 80 | 4 | 0.25 | 1 |

**[Table 6-3]**

| Compound | *Pseudomonas aeruginosa* ATCC27853 | *Escherichia coli* ATCC25922 | *Klebsiella pneumoniae* ATCC13883 |
|---|---|---|---|
| 81 | > 4 | 0.5 | 1 |
| 82 | 16 | 0.12 | 0.25 |
| 83 | 8 | 8 | 8 |
| 84 | 16 | 0.5 | 0.5 |
| 85 | 8 | 0.5 | 0.5 |
| 86 | 32 | 1 | 1 |
| 87 | > 128 | 0.5 | 1 |
| 88 | 16 | 0.12 | 0.25 |
| 89 | 4 | 2 | 8 |
| 90 | 4 | 4 | 8 |
| 91 | 32 | ND | 8 |
| 92 | 16 | 0.5 | 1 |
| 93 | 8 | 4 | 4 |
| 94 | 16 | 2 | 4 |
| 95 | 16 | 2 | 4 |
| 96 | 4 | 1 | 2 |
| 97 | 32 | 4 | 8 |
| 98 | 16 | 0.25 | 0.5 |
| 99 | 16 | 2 | 2 |
| 100 | 8 | 1 | 4 |
| 101 | 2 | 0.5 | 1 |
| 102 | 64 | 0.5 | 1 |
| 103 | > 128 | 1 | 2 |
| 104 | > 128 | 8 | 16 |
| 105 | 16 | 1 | 1 |
| 106 | 8 | 2 | 4 |
| 107 | 8 | 2 | 1 |
| 108 | 16 | 4 | 4 |
| 109 | 64 | 16 | 32 |
| 110 | 2 | 4 | 4 |
| 111 | 8 | 1 | 4 |
| 112 | 32 | 0.5 | 1 |
| 113 | 4 | 4 | 16 |
| 114 | 4 | ND | 4 |
| 115 | 16 | 4 | 8 |
| 116 | 64 | 0.5 | 2 |
| 117 | 8 | 1 | 2 |
| 118 | 4 | 0.25 | 0.5 |
| 119 | > 128 | 8 | 16 |
| 120 | 2 | 1 | 2 |

**[Table 6-4]**

| Compound | *Pseudomonas aeruginosa* ATCC27853 | *Escherichia coli* ATCC25922 | *Klebsiella pneumoniae* ATCC13883 |
|---|---|---|---|
| 121 | 16 | 0.5 | 1 |
| 122 | 4 | 4 | 4 |
| 123 | 32 | 4 | 2 |
| 124 | 2 | 2 | 4 |
| 125 | 8 | 2 | 2 |
| 126 | 16 | 16 | 64 |
| 127 | > 64 | 2 | 2 |
| 128 | > 128 | 8 | 16 |
| 129 | 8 | 1 | 2 |
| 130 | 8 | ND | 32 |
| 131 | 16 | 8 | 32 |
| 132 | 2 | 8 | 8 |
| 133 | 8 | 1 | 4 |
| 134 | 4 | ND | 32 |
| 135 | 32 | 0.5 | 2 |
| 136 | 2 | 1 | 4 |
| 137 | 4 | ND | 4 |

### INDUSTRIAL APPLICABILITY

The present invention can provide a potent antimicrobial active compound against gram-negative bacteria such as *Pseudomonas aeruginosa, Escherichia coli,* and *Klebsiella pneumoniae,* and their drug-resistant strains based on a LpxC-inhibiting action, and therefore, can provide a useful pharmaceutical product.

## Claims

1. A compound represented by general formula [1] or [2] or a pharmaceutically acceptable salt thereof: 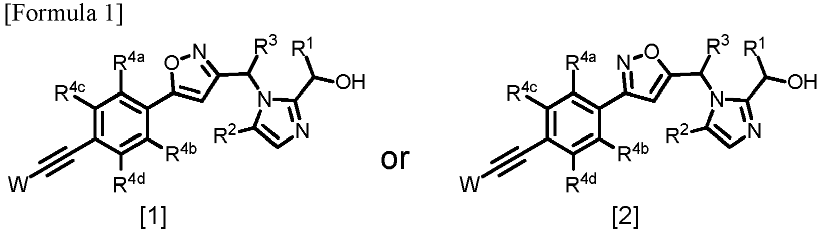 wherein
R¹ represents a C₁₋₄ alkyl group,
R² represents a hydrogen atom or a C₁₋₄ alkyl group,
R³ represents a hydrogen atom, an amino C₁₋₄ alkyl group (the amino group in the amino C₁₋₄ alkyl group may be substituted with 1 to 2 C₁₋₄ alkyl groups which are the same or different), or a hydroxy C₁₋₄ alkyl group,
R^{4a}, R^{4b}, R^{4c}, and R^{4d} are the same or different and each represent a hydrogen atom, a halogen atom, or a C₁₋₄ alkyl group,
W represents any one of the structures in the following formula group [3], and is any one of groups represented by 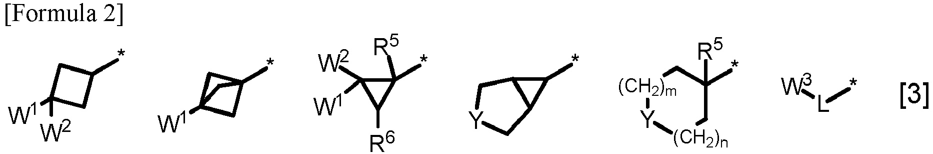 wherein
W¹ represents A^{1a}- or A^{1b}-B¹-,
A^{1a} represents a hydrogen atom, a hydroxy group, an amino group, a carboxy group, a hydroxy C₁₋₄ alkyl group, an amino C₁₋₄ alkyl group, or a C₂₋₆ alkoxyalkyl group (the hydroxy C₁₋₄ alkyl group, the amino C₁₋₄ alkyl group, and the C₂₋₆ alkoxyalkyl group may be substituted with 1 to 3 hydroxy groups),
A^{1b} represents a hydrogen atom, a C₁₋₄ alkyl group, a hydroxy C₁₋₄ alkyl group, a C₃₋₆ cycloalkyl group, or a saturated heterocyclyl group containing an oxygen atom in the ring,
B¹ represents -NR¹¹- or -CONR¹¹-,
W² represents a hydrogen atom, a hydroxy group, a cyano group, a carbamoyl group, or a hydroxy C₁₋₄ alkyl group,
W³ represents a hydrogen atom, a hydroxy group, -NR¹¹R¹³, or a C₁₋₄ alkoxy group,
L represents a C₁₋₆ alkylene group,
Y represents W⁴-N, an oxygen atom, or SO₂,
W⁴ represents A^{2a}-, A^{2b}-X¹-, A^{2a}-B²-, A^{2b}-X²-B²-, or Q-X²-,
A^{2a} represents a hydrogen atom or a C₁₋₄ alkyl group,
A^{2b} represents a hydrogen atom, a hydroxy group, an amino group, a carboxy group, a C₁₋₄ alkylamino group, or a C₁₋₄ alkoxy group,
B² represents -CO-, -OCO-, -NR¹¹CO-, -NR¹¹COCO-, -SO₂- or -NR¹¹SO₂-,
X¹ represents a C₁₋₆ alkylene group (the C₁₋₆ alkylene group may be substituted with 1 to 3 hydroxy groups),
X² represents a C₁₋₆ alkylene group (the C₁₋₆ alkylene group may be substituted with 1 to 3 hydroxy groups) or a bond,
Q represents a saturated heterocyclyl group containing an oxygen atom in the ring,
R⁵ represents a hydrogen atom, a hydroxy group, a C₁₋₄ alkyl group, -NR¹¹COR¹², or -NR¹¹R¹²,
R⁶ represents a hydrogen atom or a hydroxy C₁₋₄ alkyl group,
R¹¹ and R¹² are the same or different and each represent a hydrogen atom or a C₁₋₄ alkyl group,
R¹³ represents a hydrogen atom or a C₃₋₆ cycloalkyl group, and
m and n are the same or different and each represent 0 or 1.

2. A compound represented by general formula [1] or [2] or a pharmaceutically acceptable salt thereof: 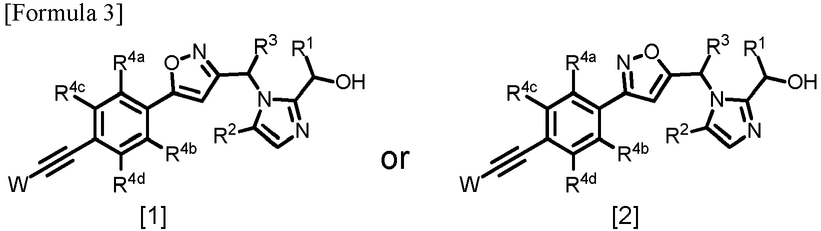 wherein
R¹ represents a C₁₋₄ alkyl group,
R² represents a hydrogen atom or a C₁₋₄ alkyl group,
R³ represents a hydrogen atom, an amino C₁₋₄ alkyl group (the amino group in the amino C₁₋₄ alkyl group may be substituted with 1 to 2 C₁₋₄ alkyl groups which are the same or different), or a hydroxy C₁₋₄ alkyl group,
R^{4a}, R^{4b}, R^{4c}, and R^{4d} are the same or different and each represent a hydrogen atom, a halogen atom, or a C₁₋₄ alkyl group,
W represents any one of the structures in the following formula group [3], and is any one of groups represented by 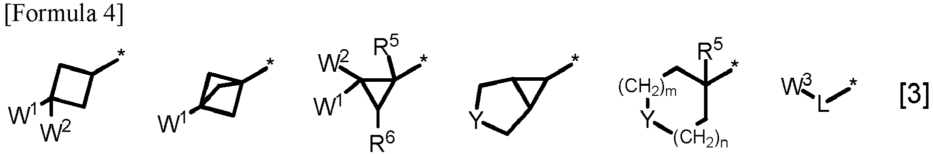 wherein
W¹ represents A^{1a}- or A^{1b}-B¹-,
A^{1a} represents a hydrogen atom, a hydroxy group, an amino group, a carboxy group, a hydroxy C₁₋₄ alkyl group, an amino C₁₋₄ alkyl group, or a C₂₋₆ alkoxyalkyl group (the hydroxy C₁₋₄ alkyl group, the amino C₁₋₄ alkyl group, and the C₂₋₆ alkoxyalkyl group may be substituted with 1 to 3 hydroxy groups),
A^{1b} represents a hydrogen atom, a C₁₋₄ alkyl group, a hydroxy C₁₋₄ alkyl group, a C₃₋₆ cycloalkyl group, or a saturated heterocyclyl group containing an oxygen atom in the ring,
B¹ represents -NR¹¹- or -CONR¹¹-,
W² represents a hydrogen atom, a hydroxy group, a cyano group, a carbamoyl group, or a hydroxy C₁₋₄ alkyl group,
W³ represents a hydrogen atom, a hydroxy group, -NR¹¹R¹³, or a C₁₋₄ alkoxy group,
L represents a C₁₋₆ alkylene group,
Y represents W⁴-N, an oxygen atom, or SO₂,
W⁴ represents A^{2a}-, A^{2b}-X¹-, A^{2a}-B²-, A^{2b}-X²-B²-, or Q-X²-,
A^{2a} represents a hydrogen atom or a C₁₋₄ alkyl group,
A^{2b} represents a hydrogen atom, a hydroxy group, an amino group, a carboxy group, a C₁₋₄ alkylamino group, or a C₁₋₄ alkoxy group,
B² represents -CO-, -OCO-, -NR¹¹CO- , -NR¹¹COCO-, -SO₂- or -NR¹¹SO₂-,
X¹ represents a C₁₋₆ alkylene group (the C₁₋₆ alkylene group may be substituted with 1 to 3 hydroxy groups),
X² represents a C₁₋₆ alkylene group (the C₁₋₆ alkylene group may be substituted with 1 to 3 hydroxy groups) or a bond,
Q represents a saturated heterocyclyl group containing an oxygen atom in the ring,
R⁵ represents a hydrogen atom, a hydroxy group, a C₁₋₄ alkyl group, or -NR¹¹R¹²,
R⁶ represents a hydrogen atom or a hydroxy C₁₋₄ alkyl group,
R¹¹ and R¹² are the same or different and each represent a hydrogen atom or a C₁₋₄ alkyl group,
R¹³ represents a hydrogen atom or a C₃₋₆ cycloalkyl group, and
m and n are the same or different and each represent 0 or 1.

3. The compound or the pharmaceutically acceptable salt thereof according to claim 1 or 2, wherein R¹ is a methyl group.

4. The compound or the pharmaceutically acceptable salt thereof according to any one of claims 1 to 3, wherein R² is a hydrogen atom.

5. The compound or the pharmaceutically acceptable salt thereof according to any one of claims 1 to 4, wherein all of R^{4a}, R^{4b}, R^{4c}, and R^{4d} are hydrogen atoms.

6. The compound or the pharmaceutically acceptable salt thereof according to any one of claims 1 to 5, wherein R³ is a hydrogen atom, an aminomethyl group, or a hydroxymethyl group.

7. The compound or the pharmaceutically acceptable salt thereof according to any one of claims 1 to 6, wherein W is formula [4] 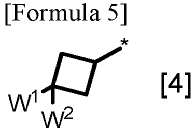 wherein
W¹ represents a hydroxy group or A^{1b}-B¹-,
A^{1b} represents a hydrogen atom or a C₁₋₄ alkyl group,
B¹ represents -CONR¹¹-,
R¹¹ represents a hydrogen atom or a C₁₋₄ alkyl group, and
W² represents a hydrogen atom.

8. The compound or the pharmaceutically acceptable salt thereof according to any one of claims 1 to 6, wherein W is formula [5] 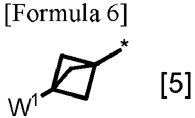 wherein
W¹ represents an amino group.

9. The compound or the pharmaceutically acceptable salt thereof according to any one of claims 1 to 6, wherein W is formula [6] 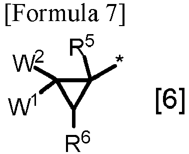 wherein
W¹ represents a hydrogen atom, a hydroxy C₁₋₄ alkyl group, or a C₂₋₆ alkoxyalkyl group,
W² represents a hydrogen atom,
R⁵ represents a hydrogen atom or an amino group, and
R⁶ represents a hydrogen atom.

10. The compound or the pharmaceutically acceptable salt thereof according to any one of claims 1 to 6, wherein W is formula [7] 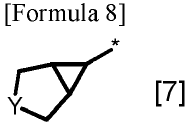 wherein
Y represents W⁴-N,
W⁴ represents A^{2b}-X¹- or A^{2a}-B²-,
A^{2a} represents a hydrogen atom or a C₁₋₄ alkyl group,
A^{2b} represents a hydrogen atom, a hydroxy group, or a carboxy group,
B² represents -CO-, and
X¹ represents a C₁₋₆ alkylene group (the C₁₋₆ alkylene group may be substituted with 1 to 3 hydroxy groups).

11. The compound or the pharmaceutically acceptable salt thereof according to any one of claims 1 to 6, wherein W is formula [8] 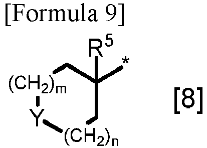 wherein
Y represents an oxygen atom,
m represents 0,
n represents 0 or 1, and
R⁵ represents a hydrogen atom or a C₁₋₄ alkyl group.

12. A pharmaceutical composition comprising the compound or the pharmaceutically acceptable salt thereof according to any one of claims 1 to 11 as an active ingredient.

13. An LpxC inhibitor comprising the compound or the pharmaceutically acceptable salt thereof according to any one of claims 1 to 11 as an active ingredient.

14. An antimicrobial agent comprising the compound or the pharmaceutically acceptable salt thereof according to any one of claims 1 to 11 as an active ingredient.
